(19) Europäisches Patentamt
European Patent Office
Office européen des brevets

(11) EP 4 155 309 A1

(12) **EUROPEAN PATENT APPLICATION**
published in accordance with Art. 153(4) EPC

(43) Date of publication:
**29.03.2023 Bulletin 2023/13**

(21) Application number: **21808197.4**

(22) Date of filing: **21.05.2021**

(51) International Patent Classification (IPC):
***C07D 491/048*** (2006.01) ***C09K 11/06*** (2006.01)
***C07D 487/04*** (2006.01) ***C07D 519/00*** (2006.01)
***H01L 51/50*** (2006.01)

(52) Cooperative Patent Classification (CPC):
**C07D 487/04; C07D 491/048; C07D 519/00; C09K 11/06;** H10K 50/00

(86) International application number:
**PCT/JP2021/019431**

(87) International publication number:
**WO 2021/235549 (25.11.2021 Gazette 2021/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.05.2020 JP 2020090095**
**01.09.2020 JP 2020147167**
**12.11.2020 JP 2020188657**
**26.04.2021 JP 2021074492**

(71) Applicant: **Kyulux, Inc.**
**Fukuoka-shi, Fukuoka 819-0388 (JP)**

(72) Inventors:
- **YAMASHITA Masataka**
  **Fukuoka-shi, Fukuoka 819-0388 (JP)**
- **CHENG Shuo-Hsien**
  **Fukuoka-shi, Fukuoka 819-0388 (JP)**
- **BALIJAPALLI Umamahesh**
  **Fukuoka-shi, Fukuoka 819-0388 (JP)**
- **YANG YuSeok**
  **Fukuoka-shi, Fukuoka 819-0388 (JP)**

(74) Representative: **Zimmermann & Partner Patentanwälte mbB**
**Postfach 330 920**
**80069 München (DE)**

(54) **COMPOUND, LIGHT-EMITTING MATERIAL, AND LIGHT-EMITTING ELEMENT**

(57) To provide an excellent light emitting material. A compound represented by the following general formula is used as the light emitting material. R is a hydrogen atom, a deuterium atom, an aryl group, or a heteroaryl group bonding via a carbon atom, Ar is an aryl group, or a heteroaryl group bonding via a carbon atom, $D^1$ and $D^2$ each are a donor group, and at least one is a hetero ring-condensed carbazol-9-yl group.

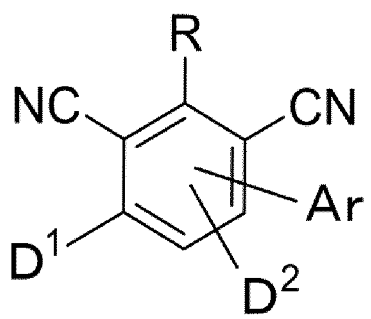

EP 4 155 309 A1

**Description**

Technical Field

The present invention relates to a compound useful as a light emitting material, and a light emitting device using the compound.

Background Art

Studies for enhancing the light emission efficiency of light emitting devices such as organic electroluminescent devices (organic EL devices) are being made actively. In particular, various kinds of efforts have been made for increasing light emission efficiency by newly developing and combining an electron transporting material and a hole transporting material to constitute an organic electroluminescent device. Among them, there are seen some studies relating to an organic electroluminescent device that utilizes a delayed fluorescent material.

A delayed fluorescent material is a material which, in an excited state, after having undergone reverse intersystem crossing from an excited triplet state to an excited singlet state, emits fluorescence when returning back from the excited singlet state to a ground state thereof. Fluorescence through the route is observed later than fluorescence from the excited singlet state directly occurring from the ground state (ordinary fluorescence), and is therefore referred to as delayed fluorescence. Here, for example, in the case where a light emitting compound is excited through carrier injection thereinto, the occurring probability of the excited singlet state and the excited triplet state is statistically 25%/75%, and therefore improvement of light emission efficiency by the fluorescence alone from the directly occurring excited singlet state is limited. On the other hand, in a delayed fluorescent material, not only the excited singlet state thereof but also the excited triplet state can be utilized for fluorescent emission through the route via the above-mentioned reverse intersystem crossing, and therefore as compared with an ordinary fluorescent material, a delayed fluorescent material can realize a higher emission efficiency.

Since such principles have been clarified, various delayed fluorescent materials have become discovered by various studies. However, every material capable of emitting delayed fluorescence is not always immediately useful as a light emitting material. Some delayed fluorescent materials are relatively less likely to undergo reverse intersystem crossing, and some delayed fluorescent materials have a long lifetime. In a high current density region, excitons may accumulate to lower emission efficiency, or in continuous long-time driving, some materials may rapidly worsen. Consequently, in fact, there are very many delayed fluorescent materials with room for improvement in point of practicability. Therefore, it is pointed out that benzonitrile compounds that are known as delayed fluorescent materials also have various problems. For example, a compound having the following structure is a material that emits delayed fluorescence (see PTL 1), but has a problem in that the lifetime of delayed fluorescence thereof is long and the device durability is insufficient.

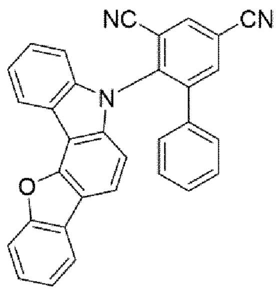

Citation List

Patent Literature

PTL 1: WO2014/208698A1

Summary of Invention

Technical Problem

Although it has been pointed out that such a problem has been encountered, it cannot be said that the relationship

between the chemical structure and the characteristics of the delayed fluorescent material has been sufficiently elucidated. Consequently, at present, it is difficult to generalize the chemical structure of a compound useful as a light emitting material, and there are many unclear points.

Given the situation, the present inventors have made repeated studies for the purpose of providing a compound more useful as a light emitting material for light emitting devices. With that, the inventors have further made assiduous studies in order to derive and generalize a general formula of a compound more useful as a light emitting material.

Solution to Problem

As a result of further promoting assiduous studies for attaining the above-mentioned object, the present inventors have found that, among isophthalonitrile derivatives, compounds having a structure that satisfies a specific requirement are useful as a light emitting material. The present invention has been proposed on the basis of these findings, and specifically has the following constitution.

[1] A compound represented by the following general formula (1):

General Formula (1)

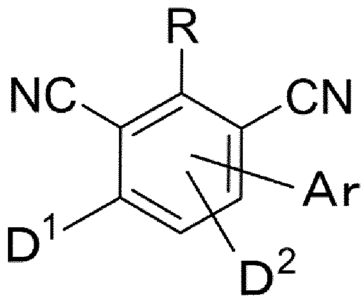

In the general formula (1);

R represents a hydrogen atom, a deuterium atom, a substituted or unsubstituted aryl group, or a substituted or unsubstituted heteroaryl group bonding via a carbon atom,
Ar represents a substituted or unsubstituted aryl group, or a substituted or unsubstituted heteroaryl group bonding via a carbon atom,
$D^1$ and $D^2$ each independently represent a donor group, and at least one of them is a hetero ring-condensed carbazol-9-yl group in which the hetero ring and the carbazol can be substituted.

[2] The compound according to [1], in which the compound is represented by the following general formula (2):

General Formula (2)

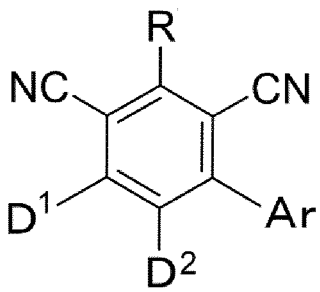

[3] The compound according to [1], in which the compound is represented by the following general formula (3):

General Formula (3)

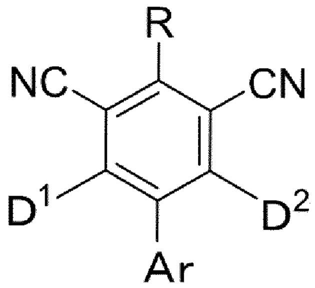

[4] The compound according to any one of [1] to [3], in which $D^1$ and $D^2$ are the same.

[5] The compound according to any one of [1] to [3], in which $D^1$ and $D^2$ are different.

[6] The compound according to any one of [1] to [5], in which the hetero ring condensed with the carbazol-9-yl group of the hetero ring-condensed carbazol-9-yl group is a substituted or unsubstituted furan ring, a substituted or unsubstituted thiophene ring, or a substituted or unsubstituted pyrrole ring, and any other ring can be further condensed with the furan ring, the thiophene ring and the pyrrole ring.

[7] The compound according to any one of [1] to [6], in which the hetero ring-condensed carbazol-9-yl group has a structure of any of the following:

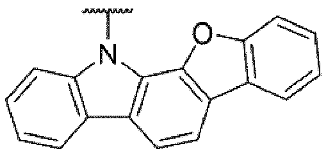
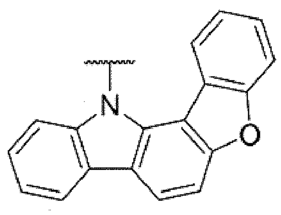
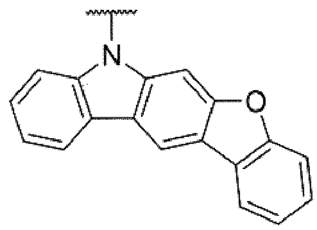

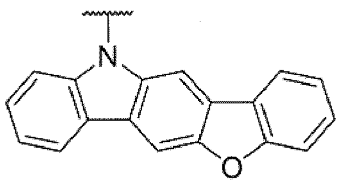
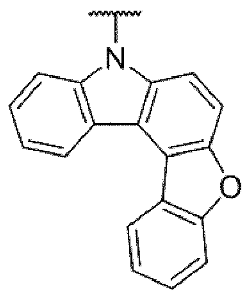
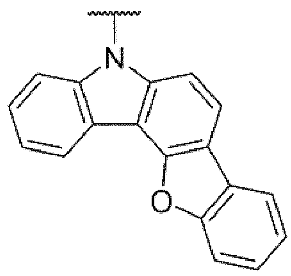

In the above structures, hydrogen atoms can be substituted, with which, however, any further hetero atom is not condensed.

[8] The compound according to any one of [1] to [6], in which the hetero ring-condensed carbazol-9-yl group has a structure of any of the following:

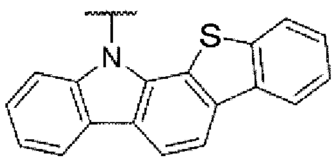
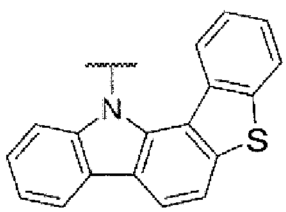
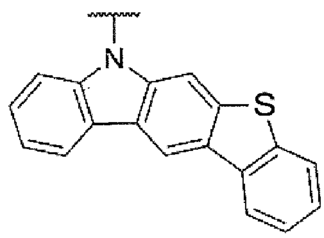

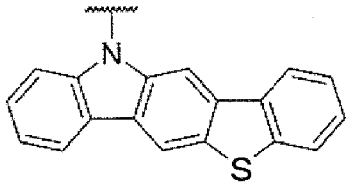
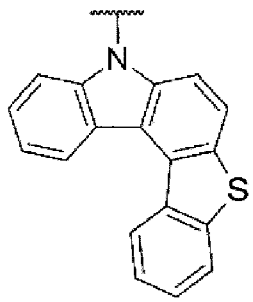
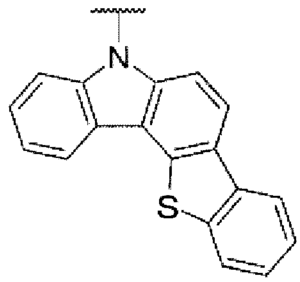

In the above structures, hydrogen atoms can be substituted, with which, however, any further hetero atom is not condensed.

[9] The compound according to any one of [1] to [6], in which the hetero ring-condensed carbazol-9-yl group has a structure of any of the following:

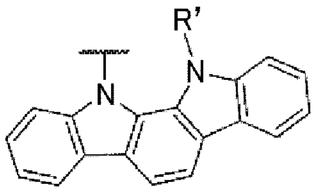
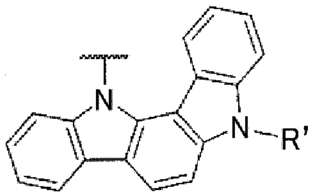
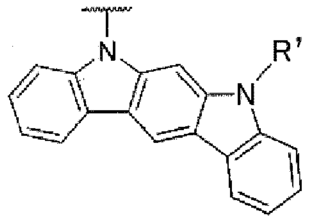

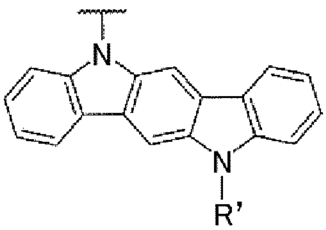
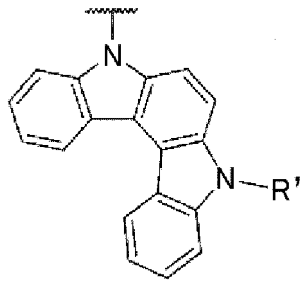
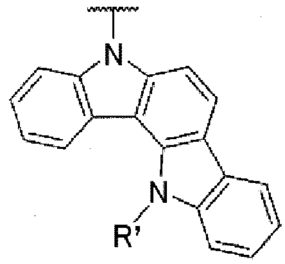

In the above structures, hydrogen atoms can be substituted, with which, however, any further hetero atom is not condensed. R' represents a hydrogen atom, a deuterium atom or a substituent.

[10] The compound according to any one of [1] to [9], in which two hetero rings selected from the group consisting of a substituted or unsubstituted furan ring, a substituted or unsubstituted thiophene ring and a substituted or unsubstituted pyrrole ring, in which any other ring can be condensed with the furan ring, the thiophene ring and the pyrrole ring, are condensed with the carbazol-9-yl group of the hetero ring-condensed carbazol-9-yl group.

[11] The compound according to any one of [1] to [10], in which the hetero ring-condensed carbazol-9-yl group has a structure with a hetero ring condensed at 1,2-positions of the carbazole ring.

[12] The compound according to any one of [1] to [10], in which the hetero ring-condensed carbazol-9-yl group has a structure with a hetero ring condensed at 2,3-positions of the carbazole ring.

[13] The compound according to any one of [1] to [10], in which the hetero ring-condensed carbazol-9-yl group has a structure with a hetero ring condensed at 3,4-positions of the carbazole ring.

[14] The compound according to any one of [1] to [13], in which R and Ar differ.

[15] The compound according to any one of [1] to [13], in which R is a hydrogen atom or a deuterium atom.

[16] The compound according to any one of [1] to [15], in which Ar is a substituted or unsubstituted phenyl group, or a substituted or unsubstituted pyridyl group.

[17] The compound according to any one of [1] to [16], which is composed of atoms selected from the group consisting of a carbon atom, a hydrogen atom, a deuterium atom, a nitrogen atom, an oxygen atom and a sulfur atom.

[18] A light emitting material of a compound of any one of [1] to [17].

[19] A light emitting device characterized by containing a compound of any one of [1] to [17].

[20] The light emitting device according to [19], in which the light emitting device has a light emitting layer and the

light emitting layer contains the above compound and a host material.

[21] The light emitting device according to [20], in which the light emitting device has a light emitting layer, the light emitting layer contains the above compound and a light emitting material, and the light emitting material mainly emits light.

Advantageous Effects of Invention

The compound of the present invention is useful as a light emitting material. Also, the compound of the present invention includes a compound having a short delayed fluorescence lifetime. Further, an organic light emitting device using the compound of the present invention has high device durability and is useful.

Brief Description of Drawing

[Fig. 1] This is a schematic cross-sectional view showing an example of a layer configuration of an organic electroluminescent device.

Description of Embodiments

The contents of the invention will be described in detail below. The constitutional elements may be described below with reference to representative embodiments and specific examples of the invention, but the invention is not limited to the embodiments and the examples. In the description herein, a numerical range expressed as "to" means a range that includes the numerical values described before and after "to" as the lower limit and the upper limit. A part of all of the hydrogen atoms that are present in the molecule of the compound used in the invention can be substituted with a deuterium atom ($^2$H, deuterium D). In the chemical structural formulae in the present description, a hydrogen atom is expressed as H, or the expression is omitted. For example, when the expression of the atoms bonding to the ring skeleton-constituting carbon atoms of a benzene ring is omitted, H bonds to the ring skeleton-constituting carbon atom in the site where the expression is omitted. In the chemical structural formulae in the present description, a deuterium atom is expressed as D.

(Compound Represented by General Formula (1))

General Formula (1)

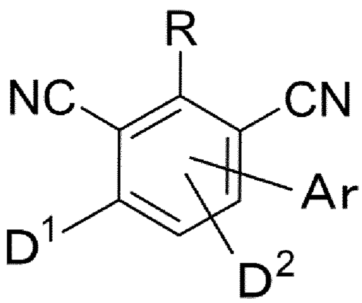

At least one of $D^1$ and $D^2$ in the general formula (1) represents a hetero ring-condensed carbazol-9-yl group. The hetero ring and the carbazole ring constituting the hetero ring-condensed carbazol-9-yl group each may be substituted or may not be substituted.

The number of the hetero ring condensed with the carbazol-9-yl group is 1 or more, preferably 1 or 2, more preferably 1. When 2 or more hetero rings are condensed, these hetero rings can be the same or different. In one embodiment of the present invention, the hetero ring is condensed at the 1,2-positions of the carbazol-9-yl group. In another embodiment of the present invention, the hetero ring is condensed at the 2,3-positions of the carbazol-9-yl group. In still another embodiment of the present invention, the hetero ring is condensed at the 3,4-positions of the carbazol-9-yl group.

The hetero ring condensed with the carbazol-9-yl group is a ring containing a hetero atom. The hetero atom is preferably selected from an oxygen atom, a sulfur atom, a nitrogen atom and a silicon atom, more preferably selected from an oxygen atom, a sulfur atom and a nitrogen atom. In one preferred embodiment, the hetero atom is an oxygen atom. In another preferred embodiment, the hetero atom is a sulfur atom. In still another preferred embodiment, the

hetero atom is an nitrogen atom. The number of the hetero atom contained as a ring skeleton constituting atom of the hetero ring is 1 or more, preferably 1 to 3, more preferably 1 or 2. In one preferred embodiment, the number of the hetero atom is 1. When the number of the hetero atom is 2 or more, they are preferably hetero atoms of the same species, but may be composed of hetero atoms of different species. For example, two or more hetero atoms can be all nitrogen atoms. The other ring skeleton constituting atoms than the hetero atom are carbon atoms.

The number of the ring skeleton constituting atoms that constitute the hetero ring condensed with the carbazol-9-yl group is preferably 4 to 8, more preferably 5 to 7, even more preferably 5 or 6. In one preferred embodiment, the number of the ring skeleton constituting atoms that constitute the hetero ring is 5. Preferably, the hetero ring has 2 or more conjugated double bonds, and preferably, through condensation with the hetero ring, the conjugated system of the carbazole ring is extended (preferably having aromaticity). Preferred examples of the hetero ring include a furan ring, a thiophene ring, and a pyrrole ring.

The hetero ring condensed with the carbazol-9-yl group can be further condensed with any other ring. The ring to be condensed can be a single ring or a condensed ring. The ring to be condensed includes an aromatic hydrocarbon ring, an aromatic hetero ring, an aliphatic hydrocarbon ring and an aliphatic hetero ring. The aromatic hydrocarbon ring includes a benzene ring. The aromatic hetero ring includes a pyridine ring, a pyridazine ring, a pyrimidine ring, a pyrazine ring, a triazine ring, a pyrrole ring, a pyrazole ring and an imidazole ring. The aliphatic hydrocarbon ring includes a cyclopentane ring, a cyclohexane ring, and a cycloheptane ring. The aliphatic hetero ring includes a piperidine ring, a pyrrolidine ring, and an imidazolidine ring. Specific examples of the condensed ring include a naphthalene ring, an anthracene ring, a phenanthrene ring, a pyran ring, a tetracene ring, an indole ring, an isoindole ring, a benzimidazole ring, a benzotriazole ring, a quinoline ring, an isoquinoline ring, a quinazoline ring, a quinoxaline ring and a cinnoline ring.

In one preferred embodiment of the present invention, the hetero ring-condensed carbazol-9-yl group is a benzofuran-condensed carbazol-9-yl group, a benzothiophene-condensed carbazol-9-yl group, an indole-condensed carbazol-9-yl group or a silaindenecondensed carbazol-9-yl group. In a more preferred embodiment of the present invention, the hetero ring-condensed carbazol-9-yl group is a benzofuran-condensed carbazol-9-yl group, a benzothiophene-condensed carbazol-9-yl group, or an indole-condensed carbazol-9-yl group.

In the present invention, as the benzofuran-condensed carbazol-9-yl group, a substituted or unsubstituted benzofuro[2,3-a]carbazol-9-yl group can be employed. Also a substituted or unsubstituted benzofuro[3,2-a]carbazol-9-yl group can be employed. Also a substituted or unsubstituted benzofuro[2,3-b]carbazol-9-yl group can be employed. Also a substituted or unsubstituted benzofuro[3,2-b]carbazol-9-yl group can be employed. Also a substituted or unsubstituted benzofuro[2,3-c]carbazol-9-yl group can be employed. Also a substituted or unsubstituted benzofuro[3,2-c]carbazol-9-yl group can be employed.

A preferred benzofuran-condensed carbazol-9-yl group is a carbazol-9-yl group in which only one benzofuran ring is condensed at 2,3-positions and any other hetero ring is not condensed (in which, however, a benzene ring can be condensed). Specifically, preferred are groups having any of the following structures, and in the following structures, the hydrogen atom can be substituted. For example, preferably exemplified are those in which a part of the hydrogen atoms in the following structure are substituted deuterium atoms, or those in which all hydrogen atoms in the following structure are substituted with deuterium atoms. Unsubstituted structures are also preferably employable.

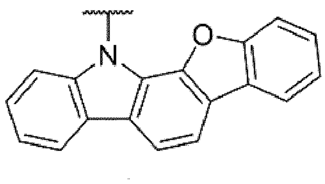
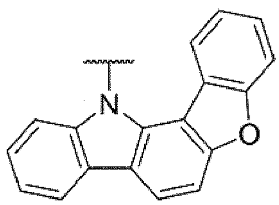
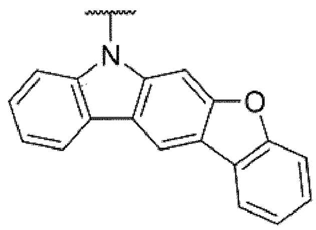

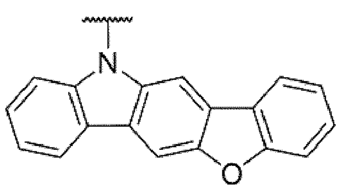
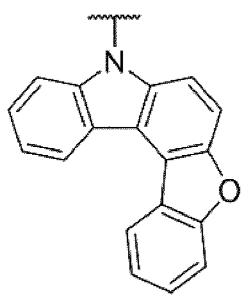
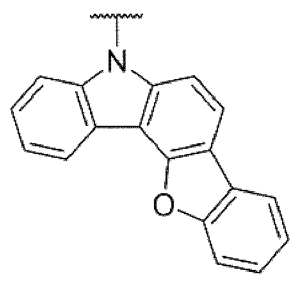

Also preferred is a carbazol-9-yl group in which two benzofuran rings are condensed at 2,3-positions and any other hetero ring is condensed therein (in which, however, a benzene ring can be condensed). Specifically, preferred are groups having any of the following structures, and in the following structures, the hydrogen atom can be substituted. For example, preferably exemplified are those in which a part of the hydrogen atoms in the following structure are substituted with deuterium atoms, or those in which all hydrogen atoms in the following structure are substituted with deuterium atoms. Unsubstituted structures are also preferably employable.

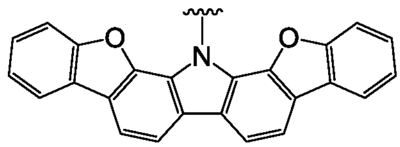
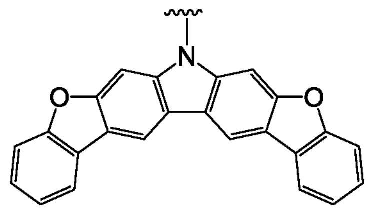
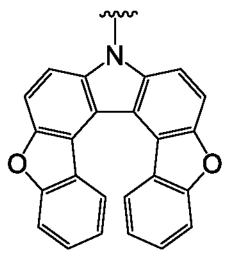
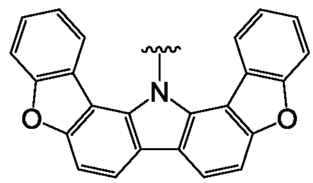
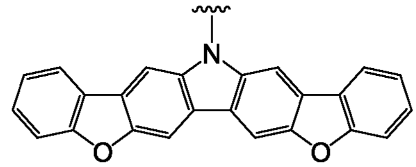
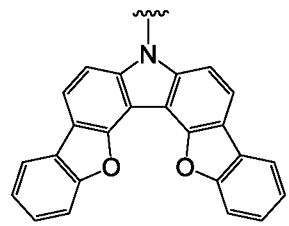

In the present invention, as the benzothiophene-condensed carbazol-9-yl group, a substituted or unsubstituted benzothieno[2,3-a]carbazol-9-yl group is employable. Also a substituted or unsubstituted benzothieno[3,2-a]carbazol-9-yl group is employable. Also, a substituted or unsubstituted benzothieno[2,3-b]carbazol-9-yl group is employable. Also a substituted or unsubstituted benzothieno[3,2-b]carbazol-9-yl group is employable. Also a substituted or unsubstituted benzothieno[2,3-c]carbazol-9-yl group is employable. Also a substituted or unsubstituted benzothieno[3,2-c]carbazol-9-yl group is employable.

A preferred benzothiophene-condensed carbazol-9-yl group is a carbazol-9-yl group in which only one benzothiophene ring is condensed at 2,3-positions and any other hetero ring is not condensed (in which, however, a benzene ring can be condensed). Specifically, preferred are groups having any of the following structures, and in the following structures, the hydrogen atom can be substituted. For example, preferably exemplified are those in which a part of the hydrogen atoms in the following structure are substituted with deuterium atoms, or those in which all hydrogen atoms in the following structure are substituted with deuterium atoms. Unsubstituted structures are also preferably employable.

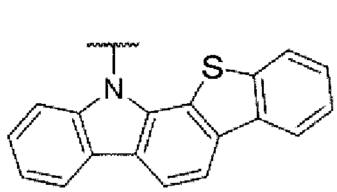
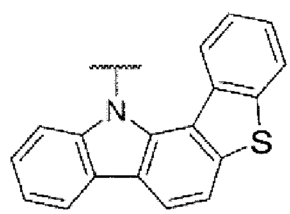
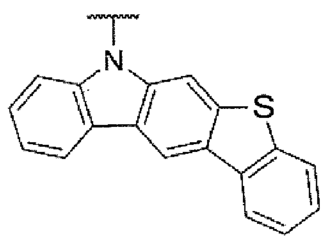

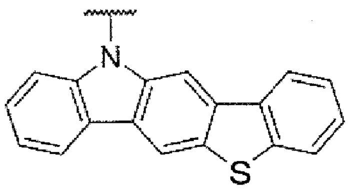
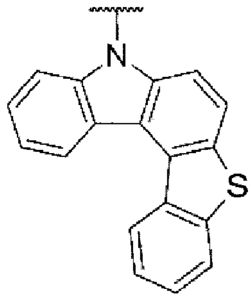
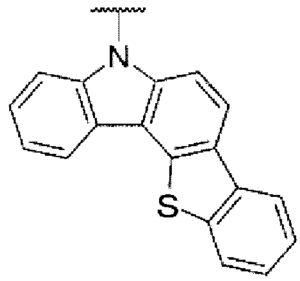

Also preferred is a carbazol-9-yl group in which two benzothiophene rings are condensed at 2,3-positions and any other hetero ring is condensed therein (in which, however, a benzene ring can be condensed). Specifically, preferred are groups having any of the following structures, and in the following structures, the hydrogen atom can be substituted. For example, preferably exemplified are those in which a part of the hydrogen atoms in the following structure are substituted with deuterium atoms, or those in which all hydrogen atoms in the following structure are substituted with deuterium atoms. Unsubstituted structures are also preferably employable.

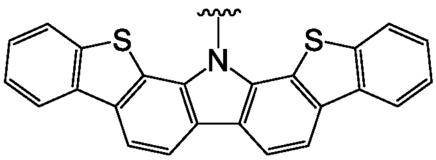
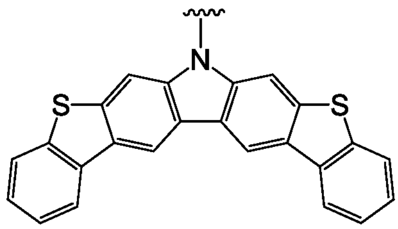
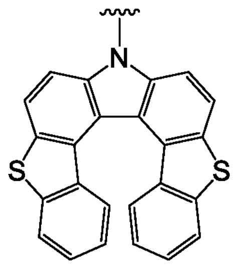

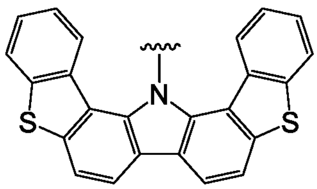
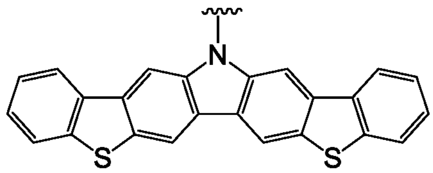
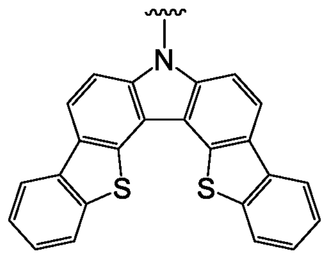

In the present invention, as the indole-condensed carbazol-9-yl group, a substituted or unsubstituted indolo[2,3-a]carbazol-9-yl group is employable. Also a substituted or unsubstituted indolo[3,2-a]carbazol-9-yl group is employable. Also, a substituted or unsubstituted indolo[2,3-b]carbazol-9-yl group is employable. Also a substituted or unsubstituted indolo[3,2-b]carbazol-9-yl group is employable. Also a substituted or unsubstituted indolo[2,3-c]carbazol-9-yl group is employable. Also a substituted or unsubstituted indolo[3,2-c]carbazol-9-yl group is employable.

A preferred indolo-condensed carbazol-9-yl group is a carbazol-9-yl group in which only one indole ring is condensed at 2,3-positions and any other hetero ring is not condensed (in which, however, a benzene ring can be condensed). Specifically, preferred are groups having any of the following structures, and in the following structures, R' represents a hydrogen atom, a deuterium atom or a substituent, and preferably, R' is a substituent. R' is preferably a substituted or unsubstituted aryl group. In the following structures, the hydrogen atom can be substituted. For example, preferably exemplified are those in which a part of the hydrogen atoms in the following structure are substituted with deuterium atoms, or those in which all hydrogen atoms in the following structure are substituted with deuterium atoms. Unsubstituted structures are also preferably employable.

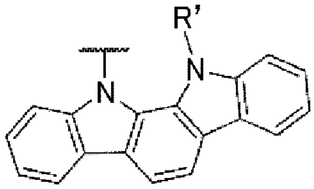
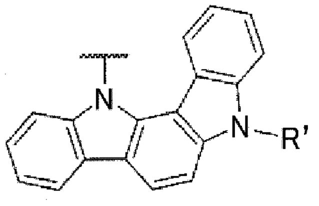
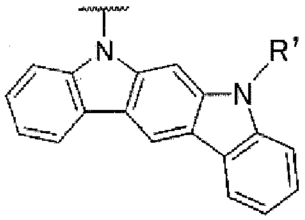

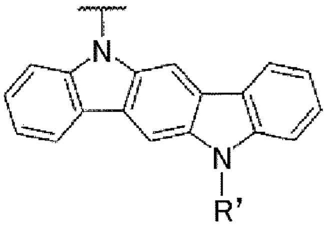
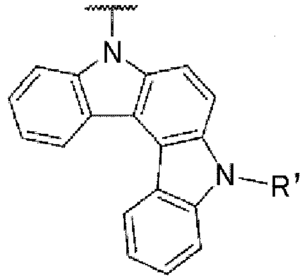
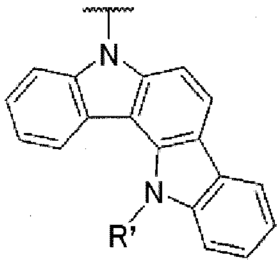

The hetero ring and the carbazole ring constituting the hetero ring-condensed carbazol-9-yl group each can be substituted. In the case where the rings are substituted, they may be substituted with a deuterium atom or can be substituted with any other substituent. The substituent as referred to herein includes an alkyl group, an alkenyl group, an aryl group, a heteroaryl group, an alkoxy group, an alkylthio group, an aryloxy group, an arylthio group, a heteroaryloxy group, a heteroarylthio group, and a cyano group. These substituents can be substituted with any other substituents. For example, there are mentioned embodiments substituted with a deuterium atom, an alkyl group, an aryl group, an alkoxy group or an alkylthio group.

The "alkyl group" as referred to herein may be any of a linear, branched or cyclic one. The group may have two or more kinds of a linear moiety, a cyclic moiety and a branched moiety as combined. The carbon number of the alkyl group may be, for example, 1 or more, 2 or more, or 4 or more. The carbon number may be 30 or less, 20 or less, 10 or less, 6 or less, or 4 or less. Specific examples of the alkyl group include a methyl group, an ethyl group, an n-propyl group, an isopropyl group, an n-butyl group, an isobutyl group, a tert-butyl group, an n-pentyl group, an isopentyl group, an n-hexyl group, an isohexyl group, a 2-ethylhexyl group, an n-heptyl group, an isoheptyl group, an n-octyl group, an isooctyl group, an n-nonyl group, an isononyl group, an n-decanyl group, an isodecanyl group, a cyclopentyl group, a cyclohexyl group, and a cycloheptyl group. The alkyl group to be a substituent may be further substituted with a deuterium atom, an aryl group, an alkoxy group, an aryloxy group or a halogen atom.

The "alkenyl group" as referred to herein may be any of a linear, branched or cyclic one. The group may have two or more kinds of a linear moiety, a cyclic moiety and a branched moiety as combined. The carbon number of the alkenyl group may be, for example, 2 or more, or 4 or more. The carbon number may be 30 or less, 20 or less, 10 or less, 6 or less, or 4 or less. Specific examples of the alkenyl group include an ethenyl group, an n-propenyl group, an isopropenyl group, an n-butenyl group, an isobutenyl group, an n-pentenyl group, an isopentenyl group, an n-hexenyl group, an isohexenyl group, and a 2-ethylhexenyl group. The alkenyl group to be a substituent may be further substituted.

The "aryl group" and the "heteroaryl group" each may be a single ring or may be a condensed ring of two or more kinds of rings. In the case of a condensed ring, the number of the rings that are condensed is preferably 2 to 6, and, for example, can be selected from 2 to 4. Specific examples of the ring include a benzene ring, a pyridine ring, a pyrimidine ring, a triazine ring, a naphthalene ring, an anthracene ring, a phenanthrene ring, a triphenylene ring, a quinoline ring, a pyrazine ring, a quinoxaline ring, and a naphthyridine ring. Specific examples of the arylene group or the heteroarylene group include a phenyl group, a 1-naphthyl group, a 2-naphthyl group, a 1-anthracenyl group, a 2-anthracenyl group, a 9-anthracenyl group, a 2-pyridyl group, a 3-pyridyl group, and a 4-pyridyl group.

Regarding the alkyl moiety in the "alkoxy group" and the "alkylthio group", reference can be made to the description and the specific examples of the alkyl group mentioned above. Regarding the aryl moiety in the "aryloxy group" and the "arylthio group", reference can be made to the description and the specific examples of the aryl group mentioned above. Regarding the heteroaryl moiety in the "heteroaryloxy group" and the "heteroarylthio group", reference can be made to the description and the specific examples of the heteroaryl group mentioned above.

The number of the atoms except hydrogen atoms and deuterium atoms constituting the hetero ring-condensed carbazol-9-yl group is preferably 16 or more, more preferably 20 or more, and can be, for example 16 or more. Also preferably the number is 80 or less, more preferably 50 or less, even more preferably 30 or less.

In the general formula (1), the hetero ring-condensed carbazol-9-yl group can be $D^1$ alone, or can be $D^2$ alone. In one preferred embodiment of the present invention, $D^1$ and $D^2$ are both hetero ring-condensed carbazol-9-yl groups. In that case, $D^1$ and $D^2$ can have the same structure, or can be different hetero ring-condensed carbazol-9-yl groups.

In the case where any one of $D^1$ and $D^2$ is a hetero ring-condensed carbazol-9-yl group, the other is a donor group except the hetero ring-condensed carbazol-9-yl (hereinafter this is referred to as "the other donor group"). The other donor group as referred to herein is a group having a negative Hammett's σp value. Here, "Hammett's $\sigma_p$ value" is one propounded by L. P. Hammett, and is one to quantify the influence of a substituent on the reaction rate or the equilibrium of a para-substituted benzene derivative. Specifically, the value is a constant ($\sigma_p$) peculiar to the substituent in the following equation that is established between a substituent and a reaction rate constant or an equilibrium constant in a para-substituted benzene derivative:

$$\log(k/k_0) = \rho\sigma_p$$

or

$$\log(K/K_0) = \rho\sigma_p$$

In the above equations, k represents a rate constant of a benzene derivative not having a substituent; $k_0$ represents a rate constant of a benzene derivative substituted with a substituent; K represents an equilibrium constant of a benzene derivative not having a substituent; $K_0$ represents an equilibrium constant of a benzene derivative substituted with a substituent; ρ represents a reaction constant to be determined by the kind and the condition of reaction. Regarding the description relating to the "Hammett's $\sigma_p$ value" and the numerical value of each substituent in the present invention, reference may be made to the description relating to $\sigma_p$ value in Hansch, C. et. al., Chem. Rev., 91, 165-195 (1991). A group having a negative Hammett's $\sigma_p$ value tends to exhibit an electron donor property, and a group having a positive Hammett's $\sigma_p$ value tends to exhibit an electron acceptor property.

The other donor group in the present invention is preferably a group containing a substituted amino group. The substituent bonding to the nitrogen atom of the amino group is preferably a substituted or unsubstituted alkyl group, a substituted or unsubstituted alkenyl group, a substituted or unsubstituted aryl group or a substituted or unsubstituted heteroaryl group, more preferably a substituted or unsubstituted aryl group or a substituted or unsubstituted heteroaryl group. Especially, the substituted amino group is preferably a substituted or unsubstituted diarylamino group or a substituted or unsubstituted diheteroarylamino group. In the present invention, the donor group can be a group bonding at the nitrogen atom of a substituted amino group, or can be a group bonding to the substituted amino group-bonding group. The substituted amino group-bonding group is preferably a π-conjugated group. More preferred is a group bonding at the nitrogen atom of the substituted amino group. Regarding the alkyl group, the alkenyl group, the aryl group and the heteroaryl group as referred to herein as substituents, reference can be made to the corresponding description relating to the substituents for the aromatic hydrocarbon ring group and the aromatic hetero ring group mentioned hereinabove.

The other donor group especially preferred in the present invention is a substituted or unsubstituted carbazol-9-yl group. The carbazol-9-yl group can be condensed with a benzene ring or a hetero ring (excepting a benzofuran ring, a benzothiophene ring, an indole ring, an indene ring and a silaindene ring). The substituent for the carbazol-9-yl group includes an alkyl group, an alkenyl group, an aryl group, a heteroaryl group, an alkoxy group, an alkylthio group, an aryloxy group, an arylthio group, a heteroaryloxy group, a heteroarylthio group, and a substituted amino group. Preferred substituents are an alkyl group, an aryl group and a substituted amino group. Regarding the description of the substituted amin group, reference can be made to the description in the preceding paragraph. The substituted amino group as referred to herein includes a substituted or unsubstituted carbazolyl group, and for example, includes a substituted or unsubstituted carbazol-3-yl group and a substituted or unsubstituted carbazol-9-yl group.

The number of the atoms except hydrogen atoms and deuterium atoms constituting the other donor group in the present invention is preferably 8 or more, more preferably 12 or more, and for example, can be 16 or more. Also preferably the number is 80 or less, more preferably 60 or less, even more preferably 40 or less.

In the following, specific examples of the donor group that $D^1$ and $D^2$ in the general formula (1) can represent are shown. D13 to D78, D84 to D119, D150 to D161, D168 to D209, D215 to D268, and D270 to D324 are specific examples of a hetero ring-condensed carbazol-9-l group, and D1 to D12, D79 to 83, D120 to 149, D162 to D167, D210 to D214, and D269 are specific examples of other donor groups. In the following structural formulae, Ph represents a phenyl group, and * indicates a bonding position.

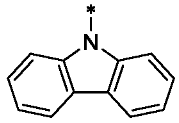
D1
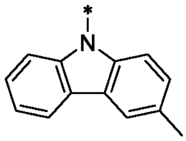
D2
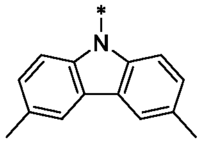
D3
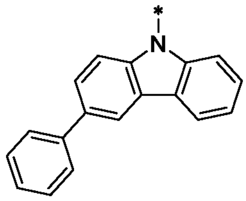
D4
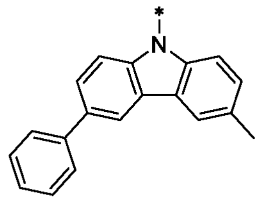
D5

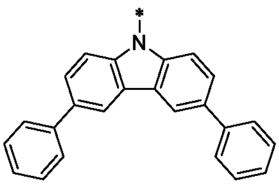
D6
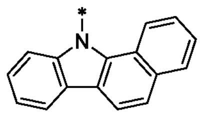
D7
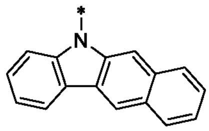
D8
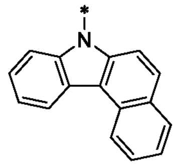
D9
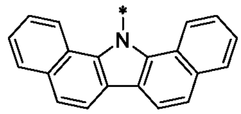
D10

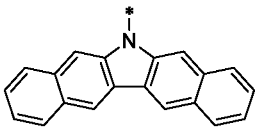
D11
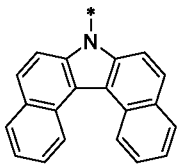
D12
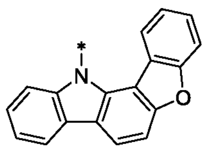
D13
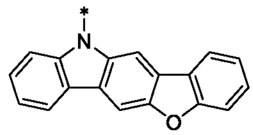
D14
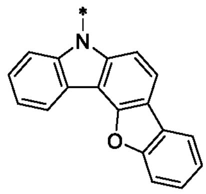
D15

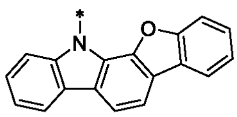
D16
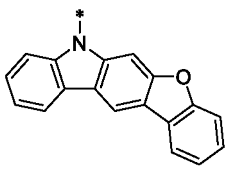
D17
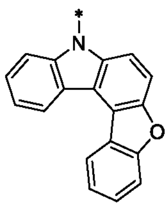
D18
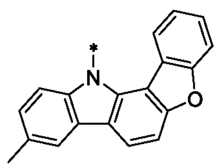
D19
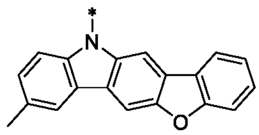
D20

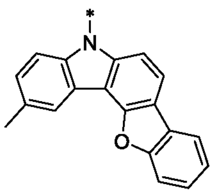
D21
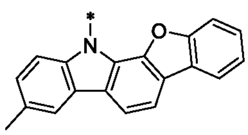
D22
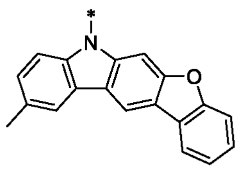
D23
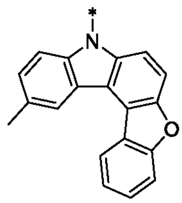
D24
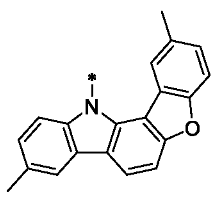
D25

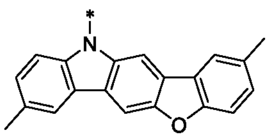
D26
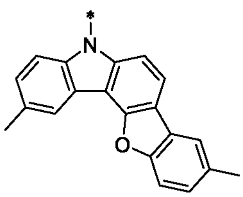
D27
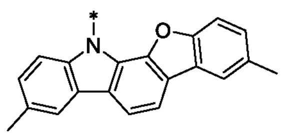
D28
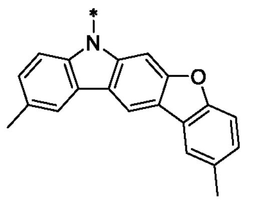
D29

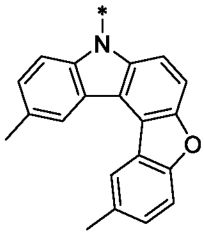

**D30**

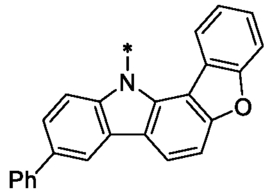

**D31**

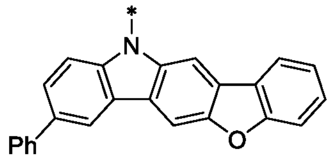

**D32**

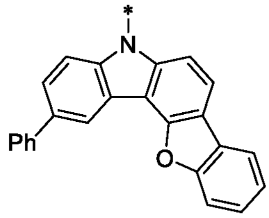

**D33**

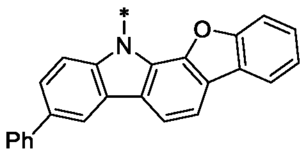

**D34**

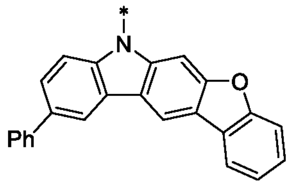

**D35**

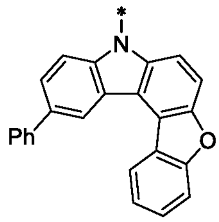

**D36**

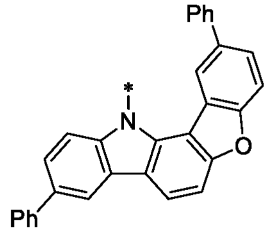

**D37**

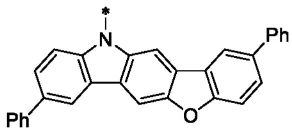

**D38**

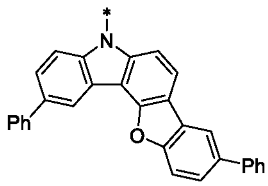

**D39**

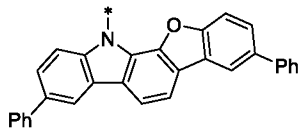

**D40**

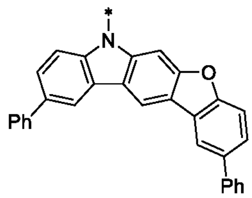

**D41**

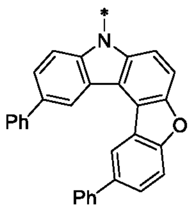

**D42**

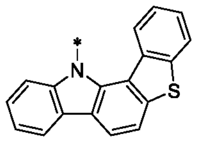

**D43**

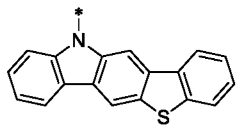

**D44**

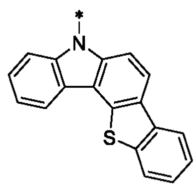

**D45**

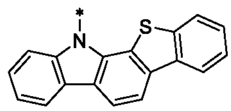

**D46**

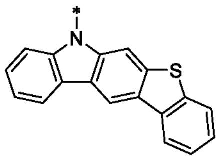

**D47**

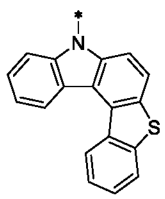

**D48**

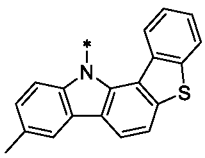

**D49**

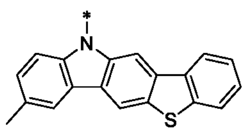

**D50**

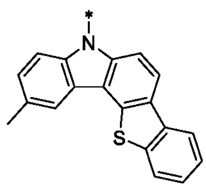

**D51**

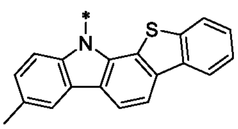

**D52**

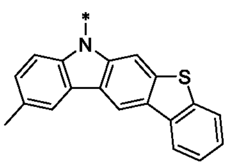

**D53**

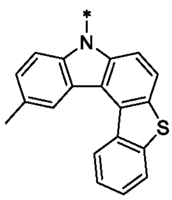

**D54**

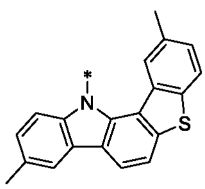

**D55**

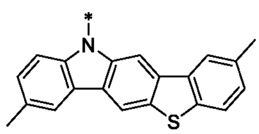

**D56**

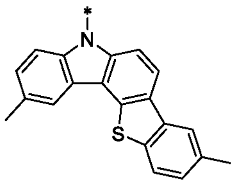
D57

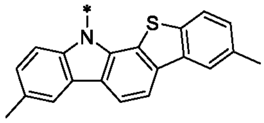
D58

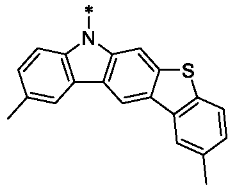
D59

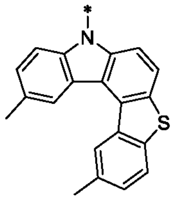
D60

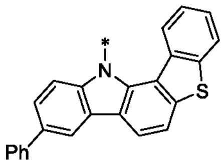
D61

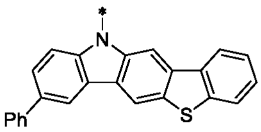
D62

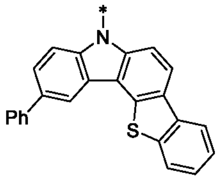
D63

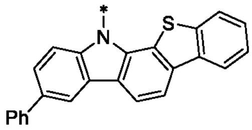
D64

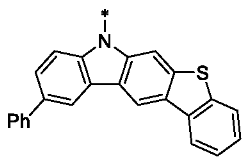
D65

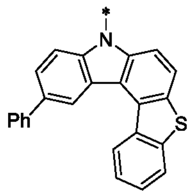
D66

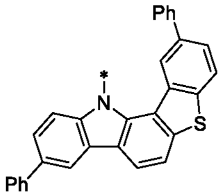
D67

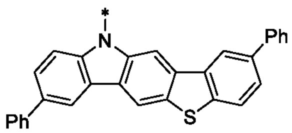
D68

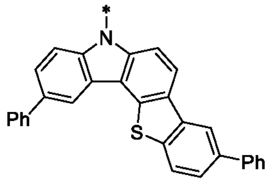
D69

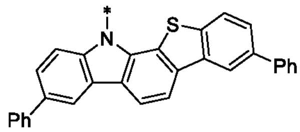
D70

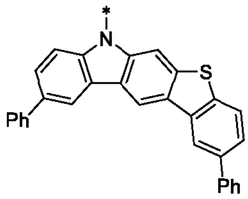
D71

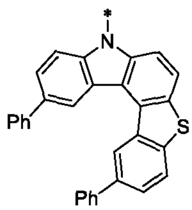
D72

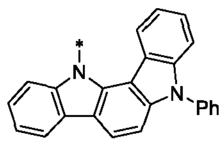
D73

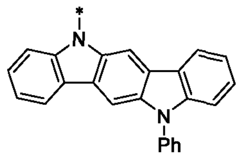
D74

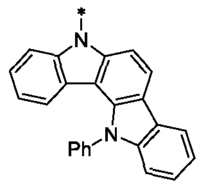
D75

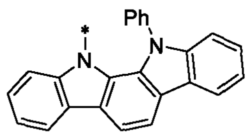
D76

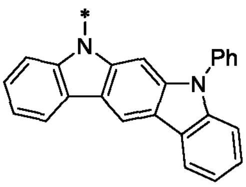
D77

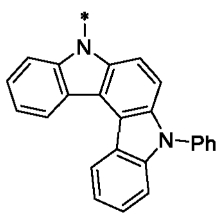
D78

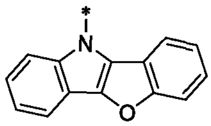
D79

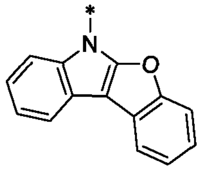
D80

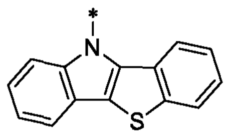
D81

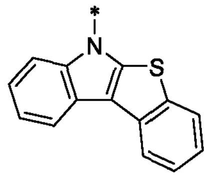
D82

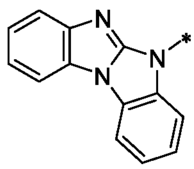
D83

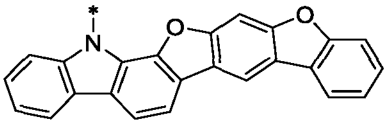
D84

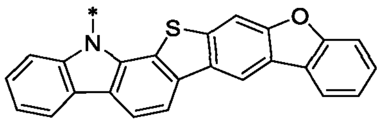
D85

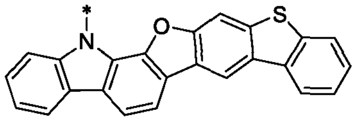
D86

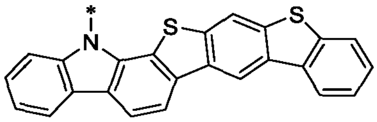
D87

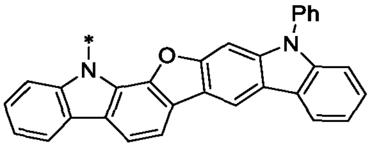
D88

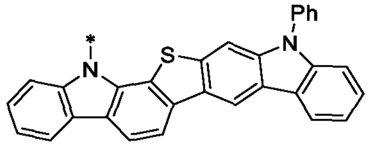
D89

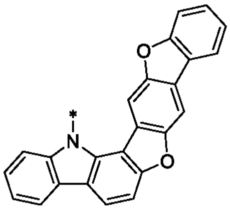
D90

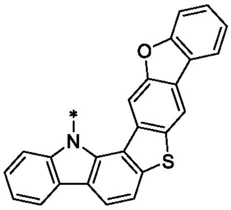
D91

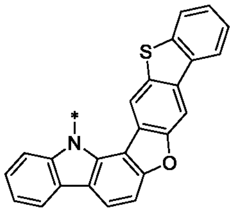
D92

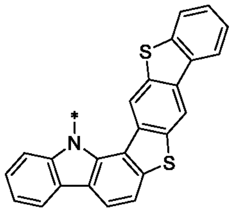
D93

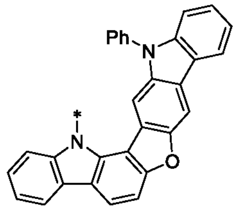
D94

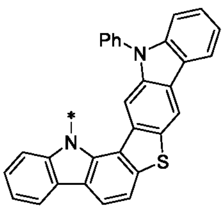
D95

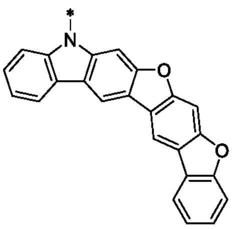
D96

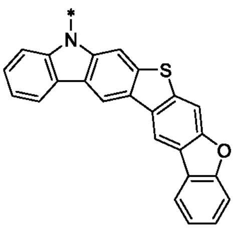
D97

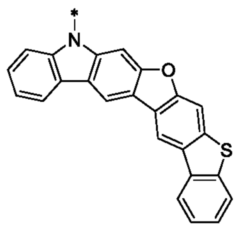
D98

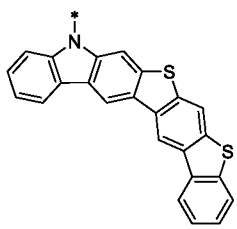
D99

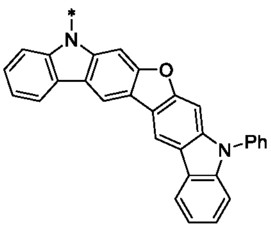
D100

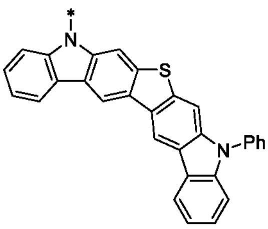
D101

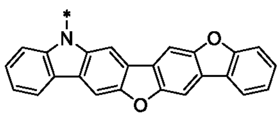
D102

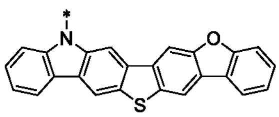
D103

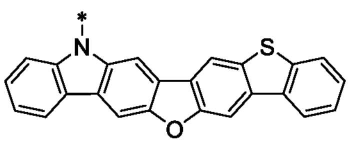
D104

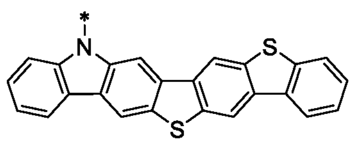
D105

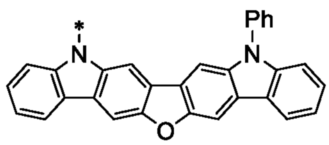
D106

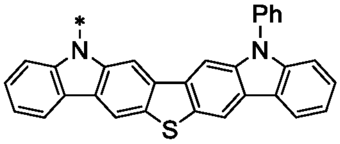

D107

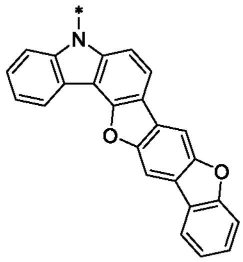

D108

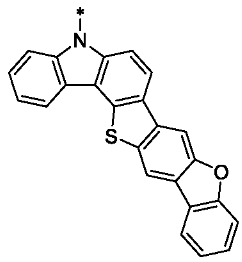

D109

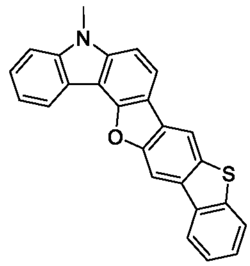

D110

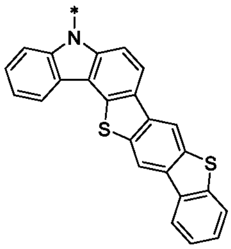

D111

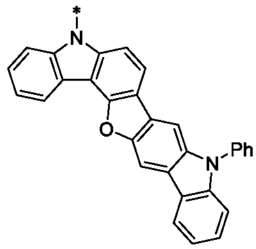

D112

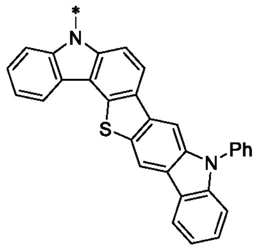

D113

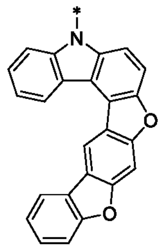

D114

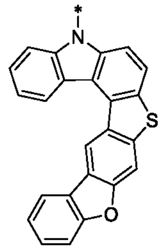

D115

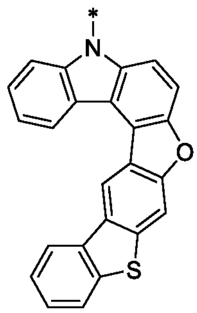

D116

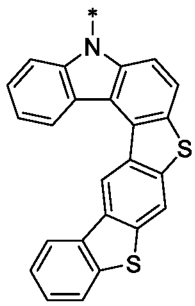

D117

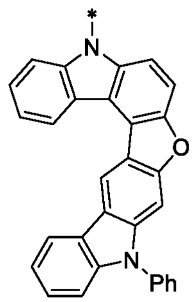

D118

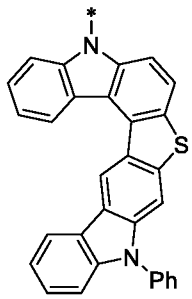

D119

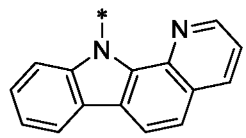

D120

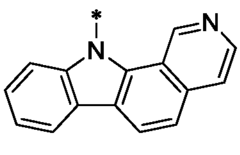

D121

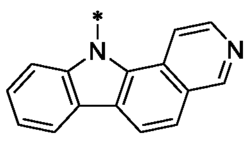

D122

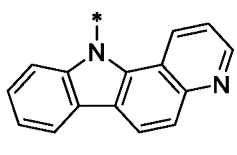

D123

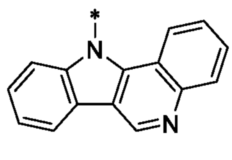

D124

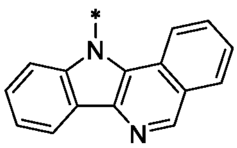

D125

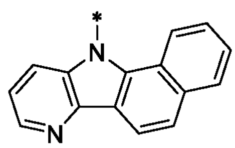

D126

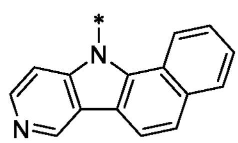

D127

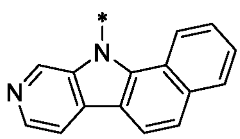

D128

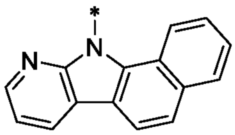
**D129**

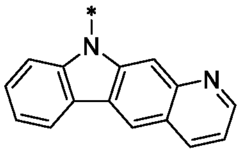
**D130**

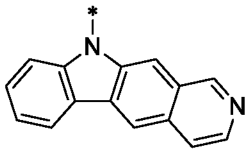
**D131**

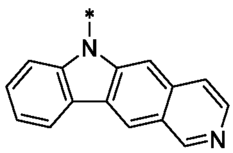
**D132**

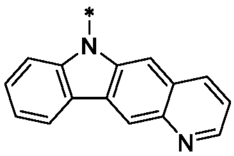
**D133**

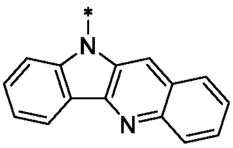
**D134**

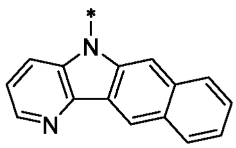
**D135**

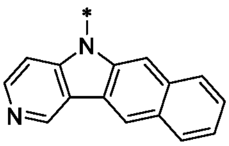
**D136**

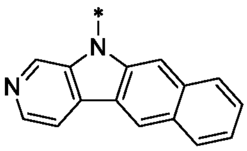
**D137**

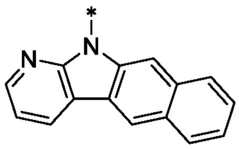
**D138**

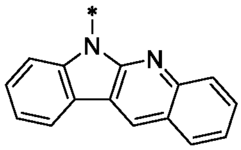
**D139**

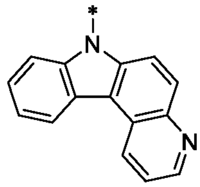
**D140**

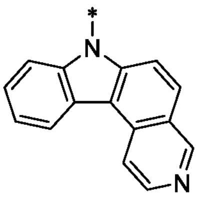
**D141**

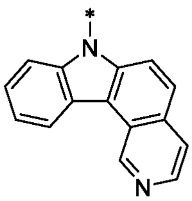
**D142**

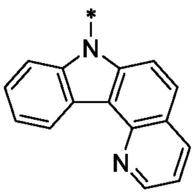
**D143**

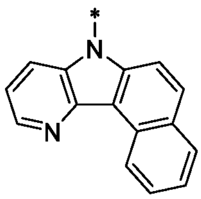
**D144**

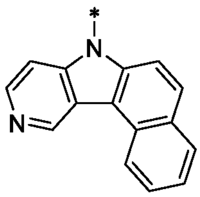
**D145**

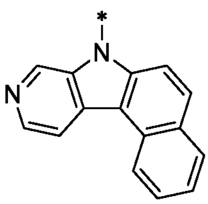
**D146**

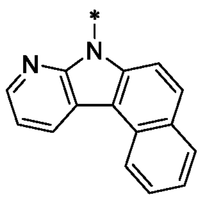
**D147**

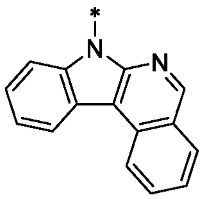
**D148**

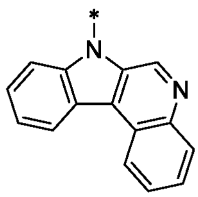
**D149**

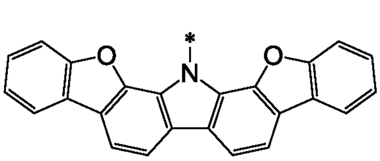
**D150**

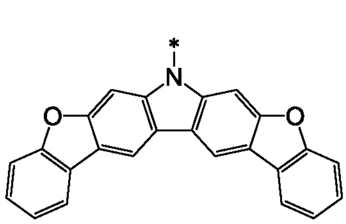
**D151**

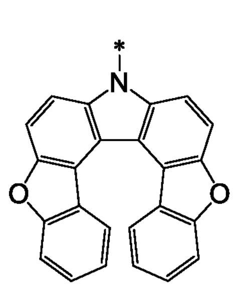
**D152**

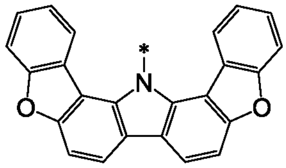

**D153**

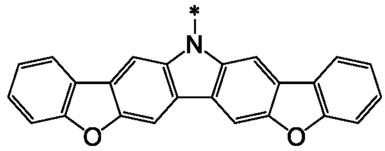

**D154**

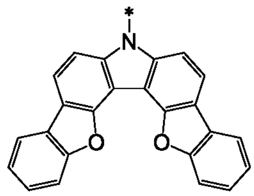

**D155**

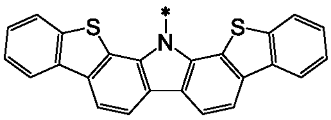

**D156**

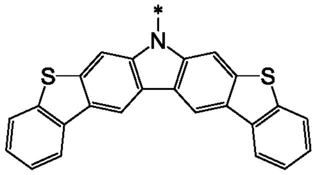

**D157**

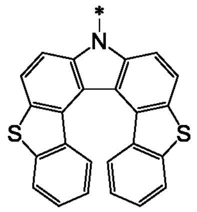

**D158**

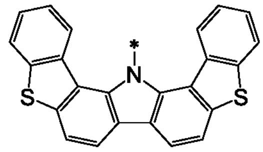

**D159**

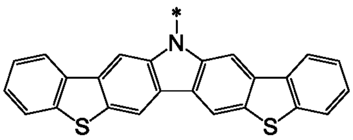

**D160**

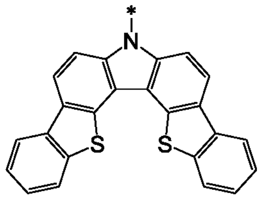

**D161**

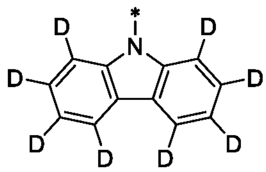

**D162**

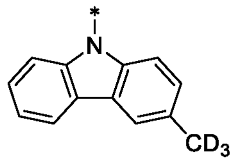

**D163**

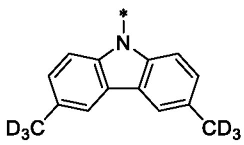

**D164**

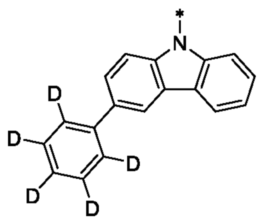

**D165**

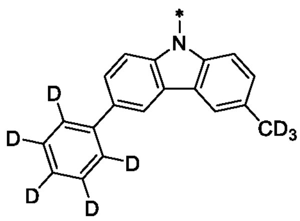

**D166**

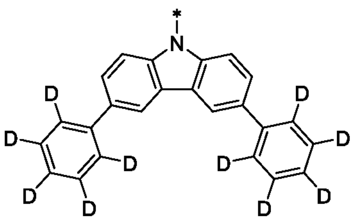

**D167**

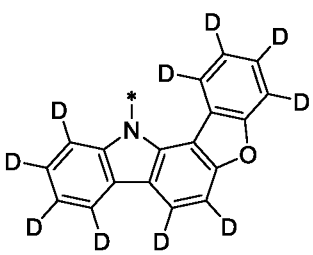

**D168**

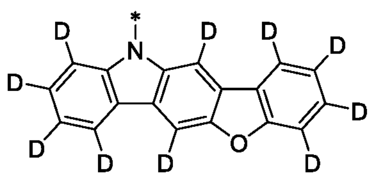

**D169**

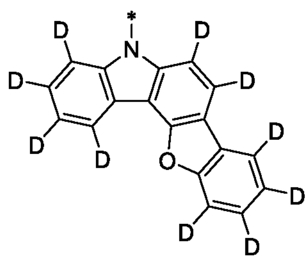

**D170**

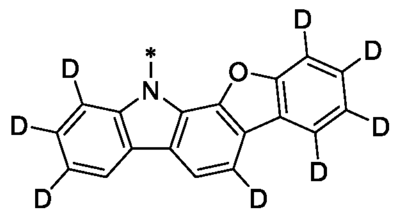

**D171**

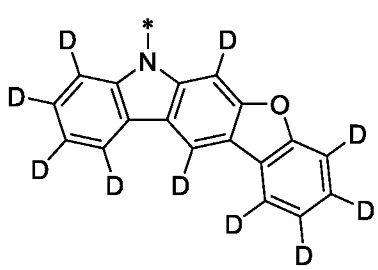

**D172**

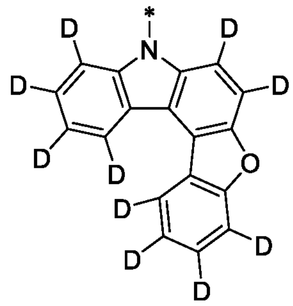

**D173**

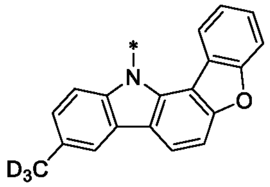

**D174**

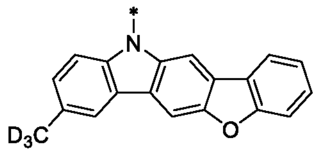

**D175**

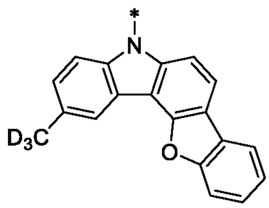

**D176**

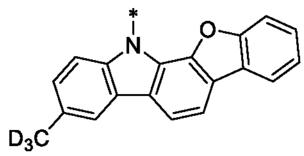

**D177**

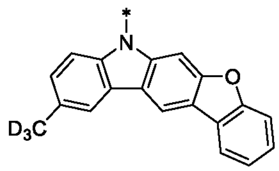

**D178**

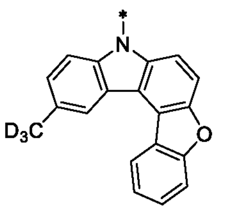

**D179**

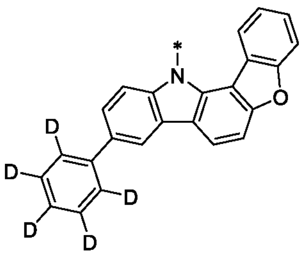

**D180**

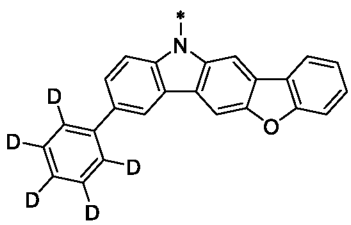

**D181**

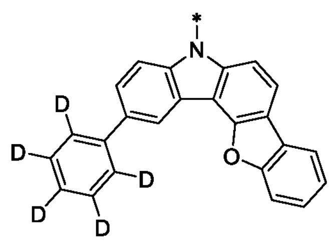

**D182**

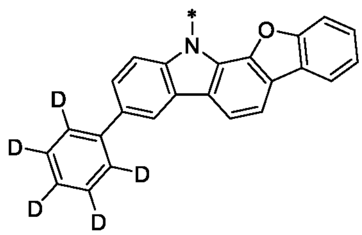

**D183**

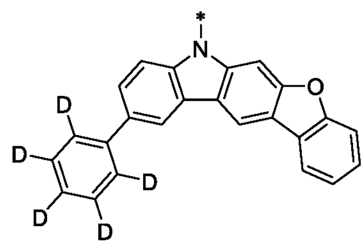

**D184**

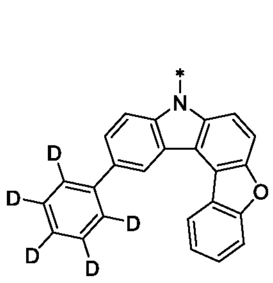

**D185**

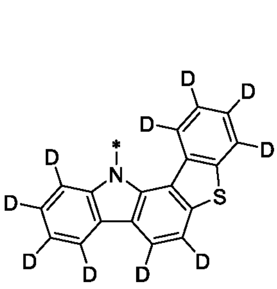

**D186**

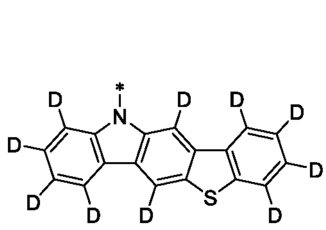

**D187**

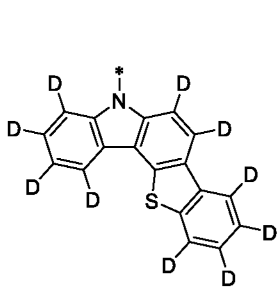

**D188**

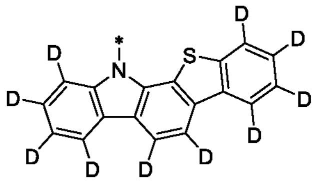

D189

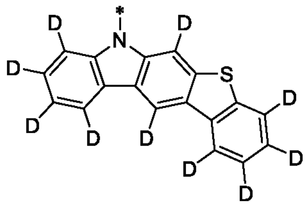

D190

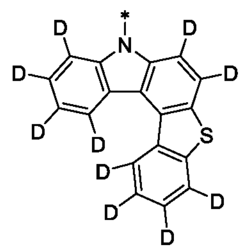

D191

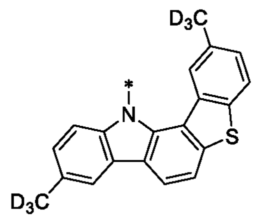

D192

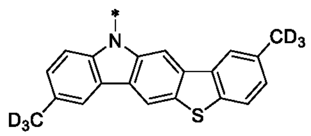

D193

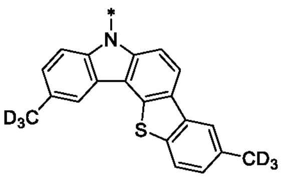

D194

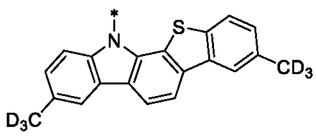

D195

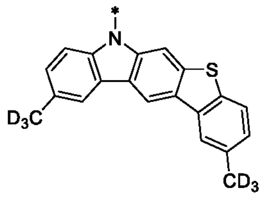

D196

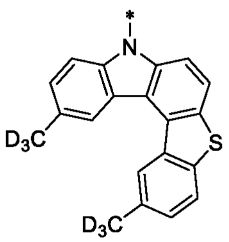

D197

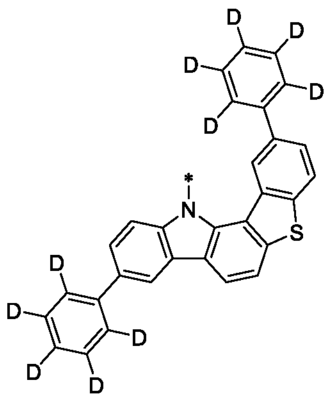

D198

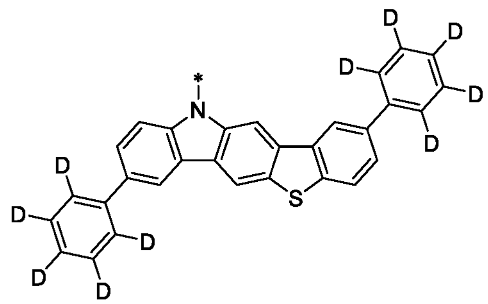

D199

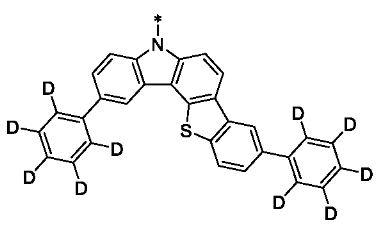

D200

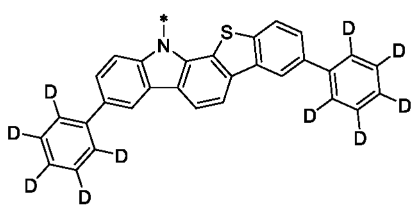

D201

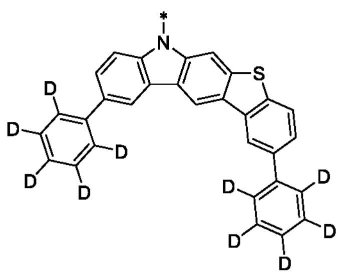

D202

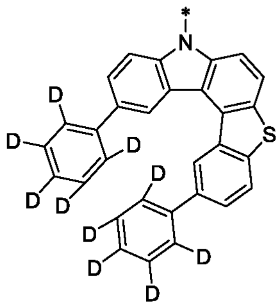
D203

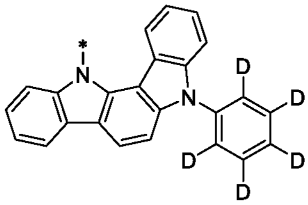
D204

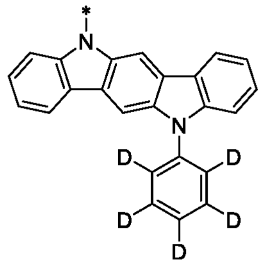
D205

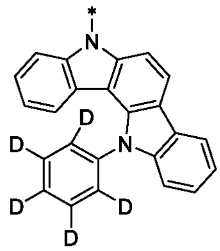
D206

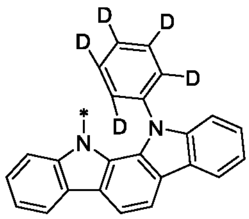
D207

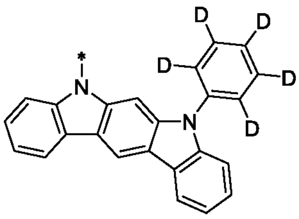
D208

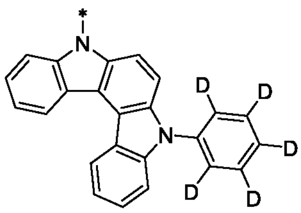
D209

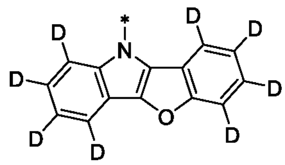
D210

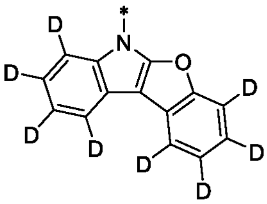
D211

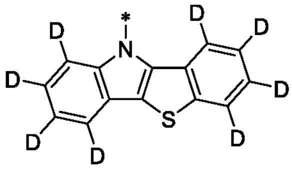
D212

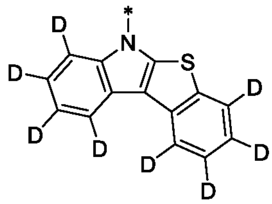
D213

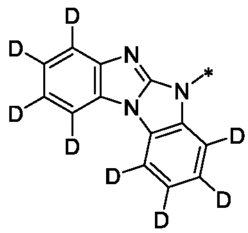
D214

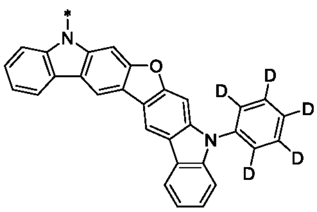
D215

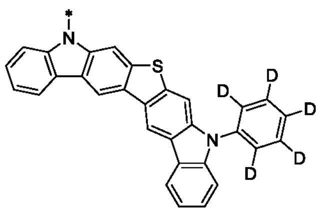
D216

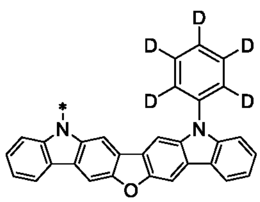
D217

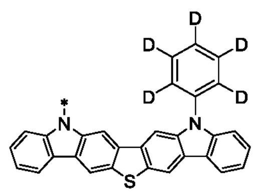
D218

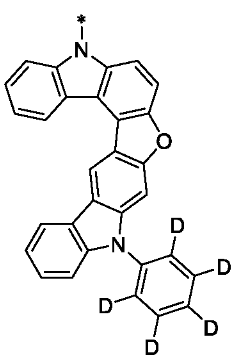
D219

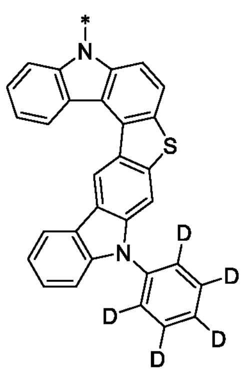
D220

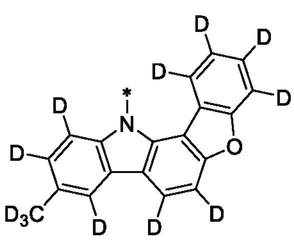
D221

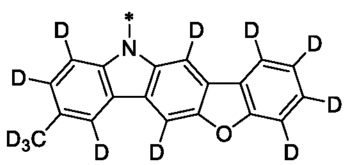
D222

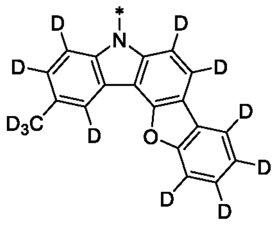 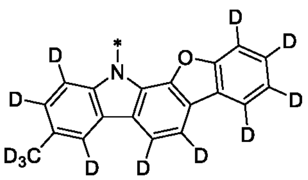 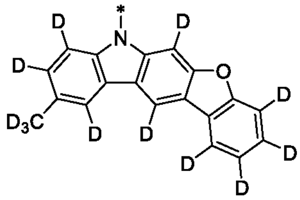 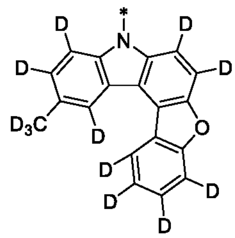

**D223** **D224** **D225** **D226**

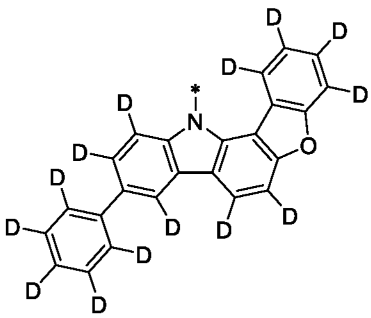 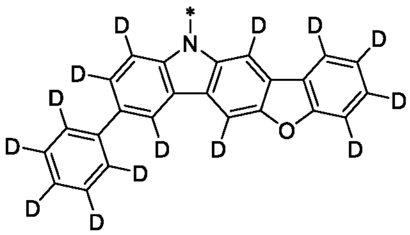 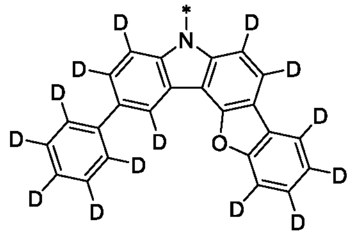

**D227** **D228** **D229**

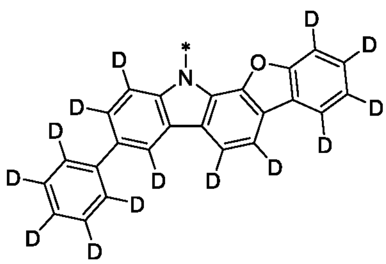 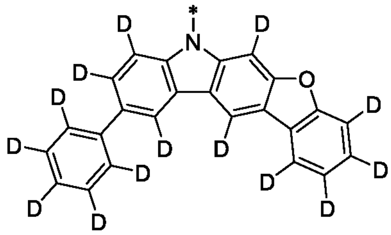 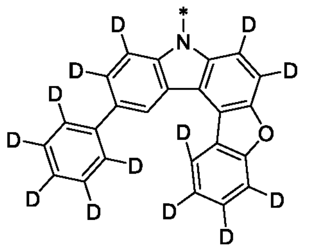

**D230** **D231** **D232**

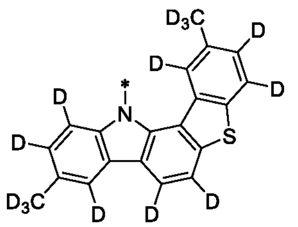 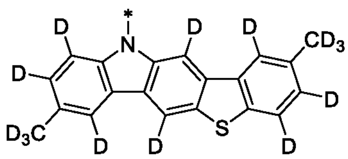 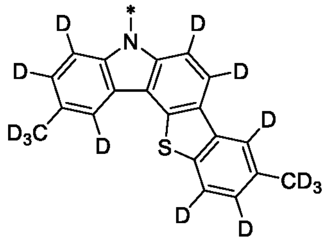

**D233** **D234** **D235**

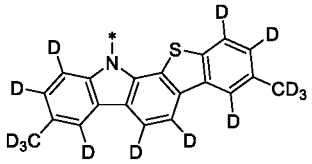
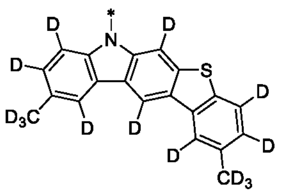
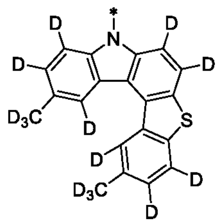
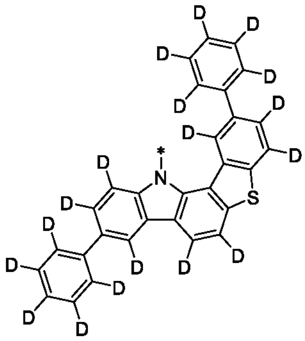

**D236** **D237** **D238** **D239**

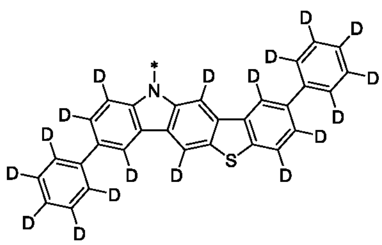
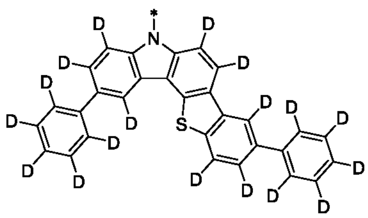
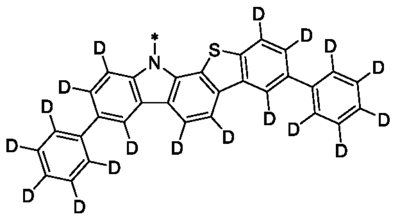

**D240** **D241** **D242**

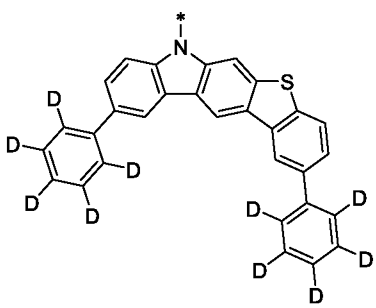
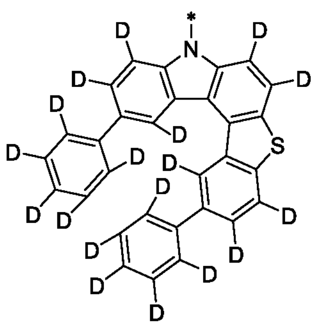
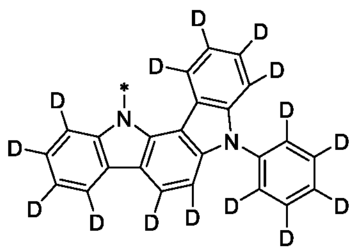

**D243** **D244** **D245**

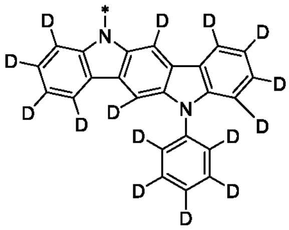
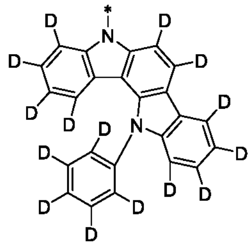
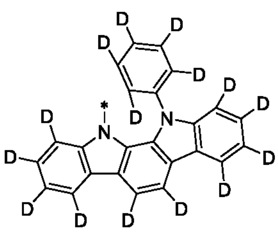
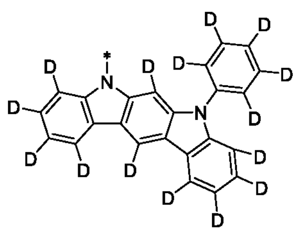

**D246** **D247** **D248** **D249**

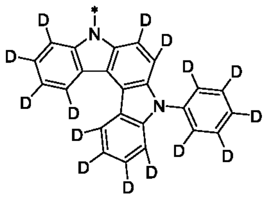

**D250**

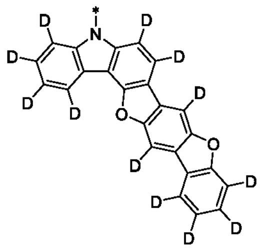

**D251**

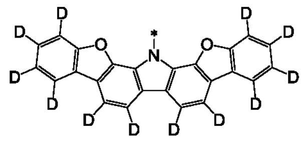

**D252**

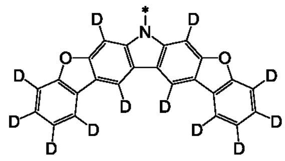

**D253**

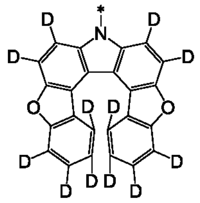

**D254**

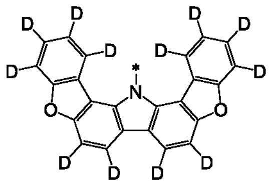

**D255**

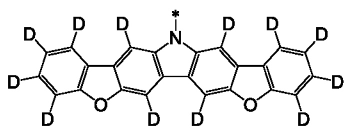

**D256**

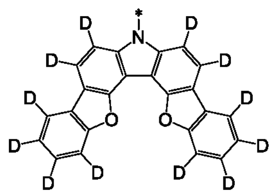

**D257**

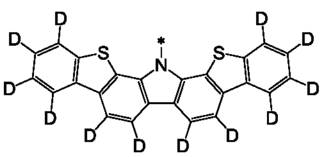

**D258**

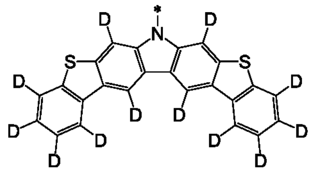

**D259**

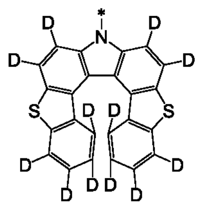

**D260**

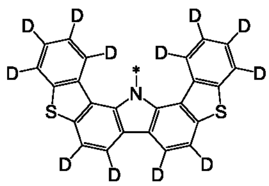

**D261**

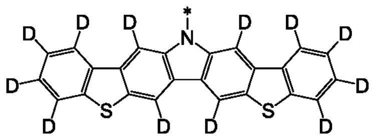

**D262**

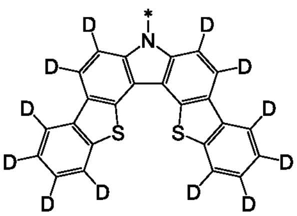

**D263**

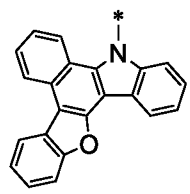

**D264**

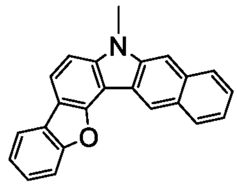

**D265**

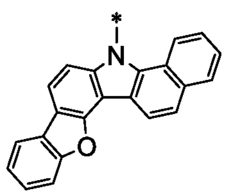

**D266**

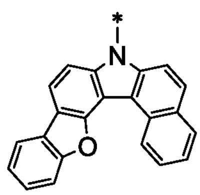

**D267**

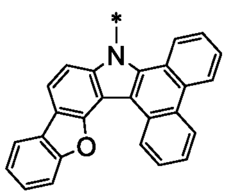

**D268**

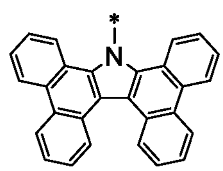

**D269**

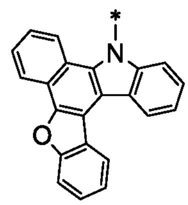

**D270**

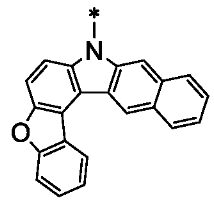
D271
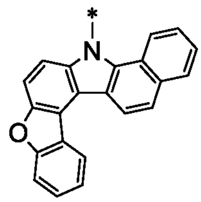
D272
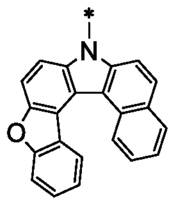
D273
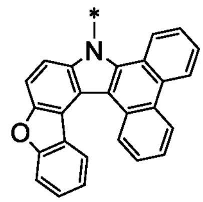
D274
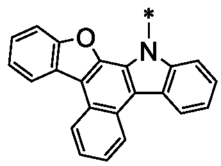
D275

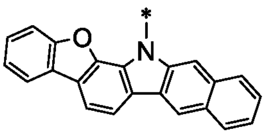
D276
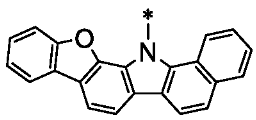
D277
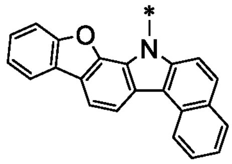
D278
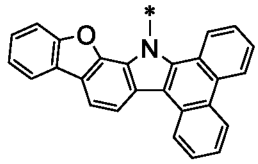
D279

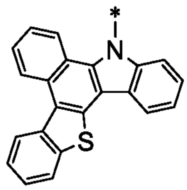
D280
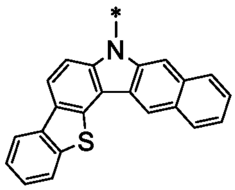
D281
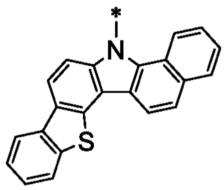
D282
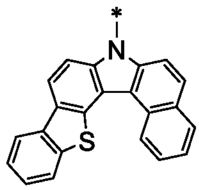
D283
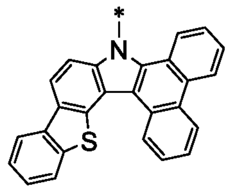
D284

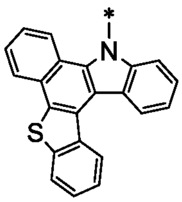
D285
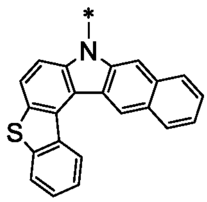
D286
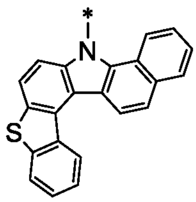
D287
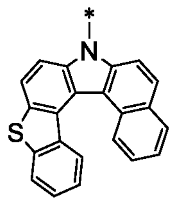
D288
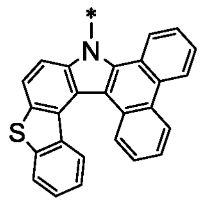
D289

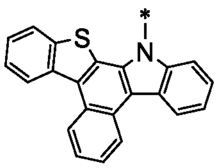
D290
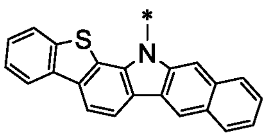
D291
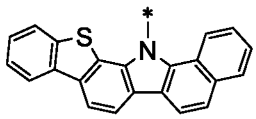
D292
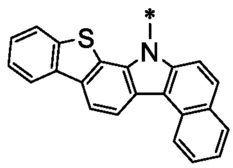
D293

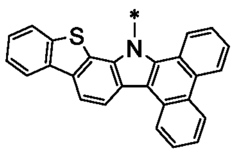
D294
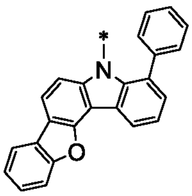
D295
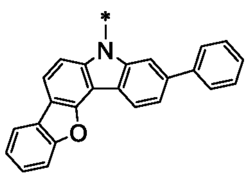
D296
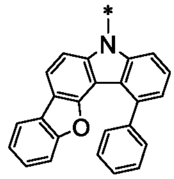
D297
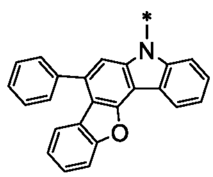
D298

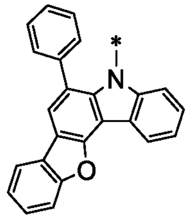
D299

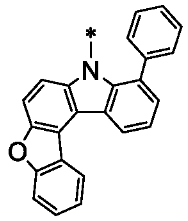
D300

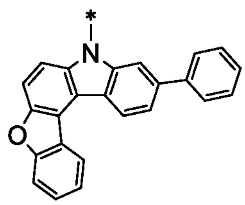
D301

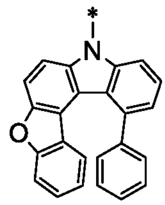
D302

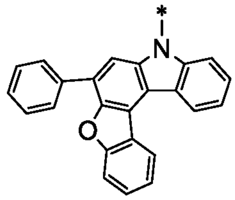
D303

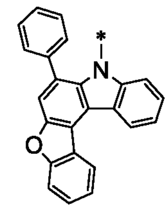
D304

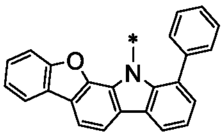
D305

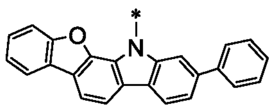
D306

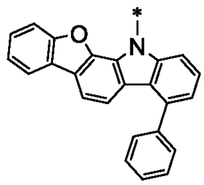
D307

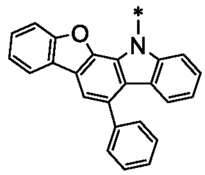
D308

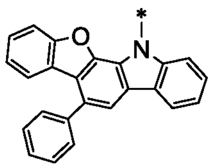
D309

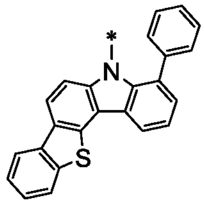
D310

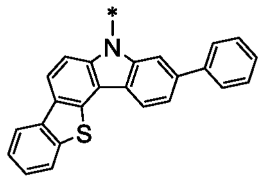
D311

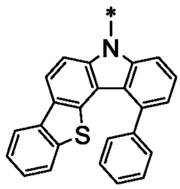
D312

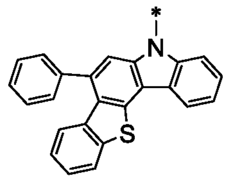
D313

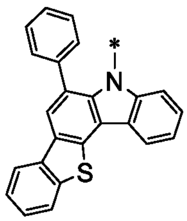
D314

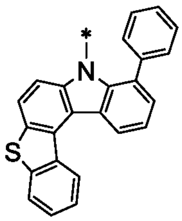
D315

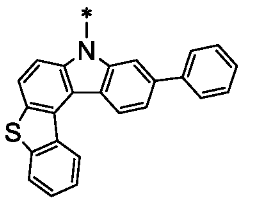
D316

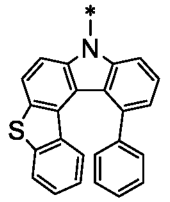
D317

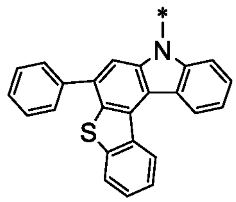
D318

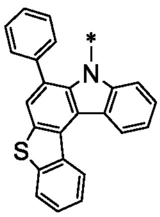
D319

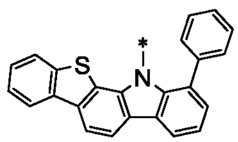
D320

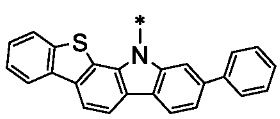
D321

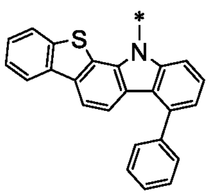
D322

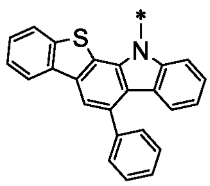
D323

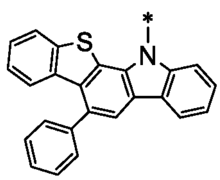
D324

R in the general formula (1) represents a hydrogen atom, a deuterium atom, a substituted or unsubstituted aryl group, or a substituted or unsubstituted heteroaryl group bonding via a carbon atom. In one preferred embodiment of the present invention, R is a hydrogen atom or a deuterium atom. However, herein also employable are an embodiment where R is a substituted or unsubstituted aryl group, and an embodiment where R is a substituted or unsubstituted heteroaryl group bonding via a carbon atom. In the case where R is an aryl group, it is preferably a substituted aryl group. In the case where R is a heteroaryl group, it is preferably a substituted heteroaryl group.

Ar in the general formula (1) represents a substituted or unsubstituted aryl group, or a substituted or unsubstituted heteroaryl group bonding via a carbon atom. In one preferred embodiment of the present invention, Ar is a substituted or unsubstituted aryl group. However, herein also employable is an embodiment where Ar is a substituted or unsubstituted heteroaryl group

Regarding the description and the preferred range of the aryl group and the heteroaryl group that R and Ar can represent, reference can be made to the description of the aryl group and the heteroaryl group in the substituent for the hetero ring-condensed carbazol-9-yl group. However, the heteroaryl group is a heteroaryl group bonding via a carbon atoms. The substituent for the aryl group and the substituent for the heteroaryl group include an alkyl group, an alkenyl group, an aryl group, a heteroaryl group, an alkoxy group, an alkylthio group, an aryloxy group, an arylthio group, a heteroaryloxy group, a heteroarylthio group and a cyano group. These substituents can be further substituted with any other substituent. The group of preferred substituents includes an alkyl group, an aryl group, an alkoxy group, an alkylthio group and a cyano group.

In one preferred embodiment of the present invention, R is a hydrogen atom or a deuterium atom, and Ar is a substituted or unsubstituted phenyl group, in which the phenyl group can be condensed with one or more rings selected from a benzene ring, a pyridine ring, a furan ring, a thiophene ring and a pyrrole ring. In another preferred embodiment of the present invention, R is a hydrogen atom or a deuterium atom, and Ar is a substituted or unsubstituted pyridyl group, in which the pyridyl group can be condensed with one or more rings selected from a benzene ring, a pyridine ring, a furan ring, a thiophene ring and a pyrrole ring. In another preferred embodiment of the present invention, R is a hydrogen atom or a deuterium atom, and Ar is a substituted phenyl group, in which the phenyl group is substituted with one or more groups selected from a substituted or unsubstituted phenyl group, and a substituted or unsubstituted pyridyl group. In another preferred embodiment of the present invention, R is a hydrogen atom or a deuterium atom, and Ar is a substituted pyridyl group, in which the pyridyl group is substituted with one or more groups selected from a substituted or unsubstituted phenyl group, and a substituted or unsubstituted pyridyl group.

In the following, shown are specific examples of the substituted or unsubstituted aryl group which can be employed by R and Ar in the general formula (1), and the substituted or unsubstituted heteroaryl group bonding via a carbon atom. In the following structural formulae, * indicates a bonding position.

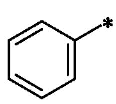
**Ar1**

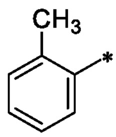
**Ar2**

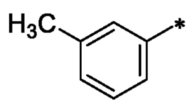
**Ar3**

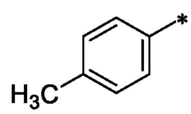
**Ar4**

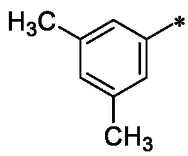
**Ar5**

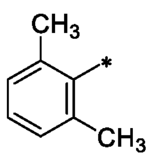
**Ar6**

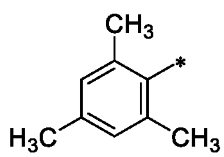
**Ar7**

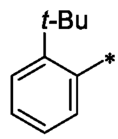
**Ar8**

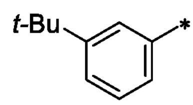
**Ar9**

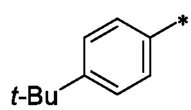
**Ar10**

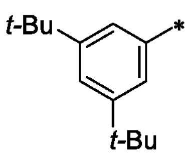
**Ar11**

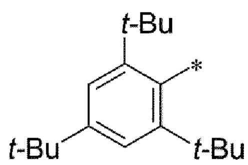
**Ar12**

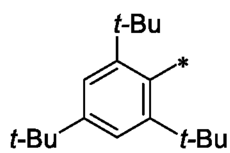
**Ar13**

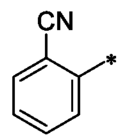
**Ar14**

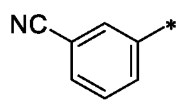
**Ar15**

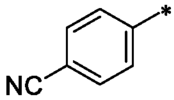
Ar16
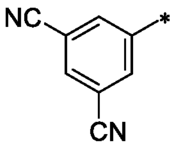
Ar17
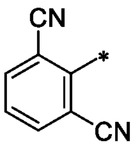
Ar18
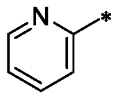
Ar19
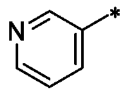
Ar20
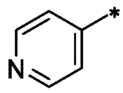
Ar21

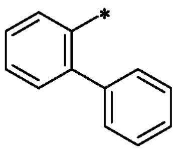
Ar22
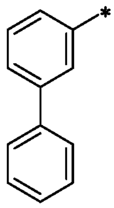
Ar23
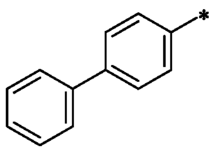
Ar24
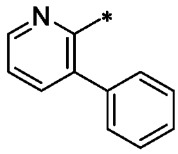
Ar25
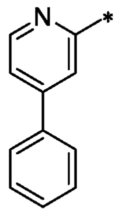
Ar26

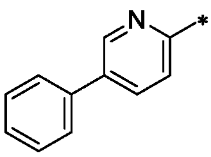
Ar27
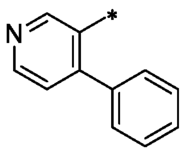
Ar28
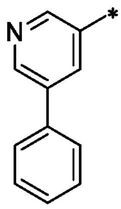
Ar29
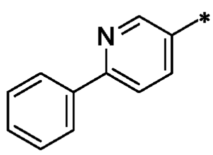
Ar30
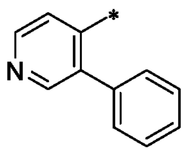
Ar31

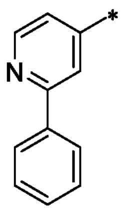
Ar32
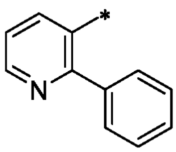
Ar33
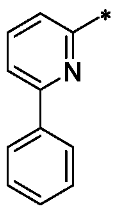
Ar34
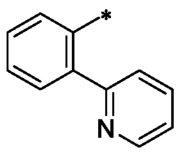
Ar35
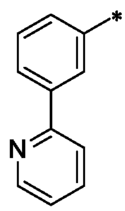
Ar36

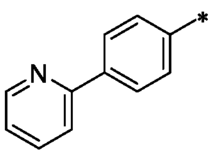
Ar37
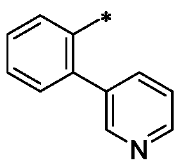
Ar38
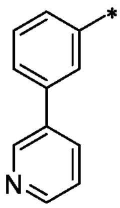
Ar39
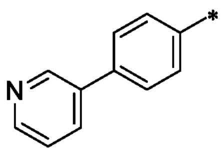
Ar40
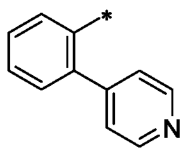
Ar41

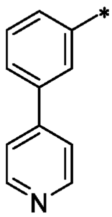
Ar42
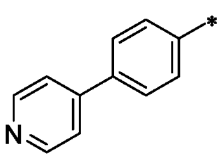
Ar43
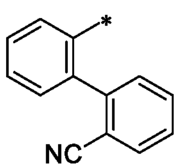
Ar44
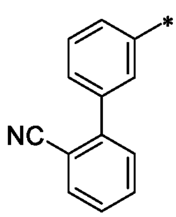
Ar45
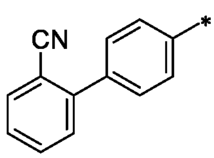
Ar46

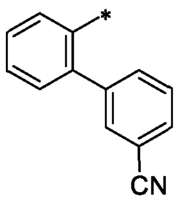
Ar47

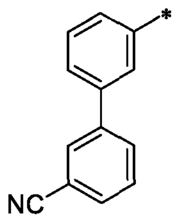
Ar48

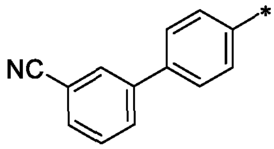
Ar49

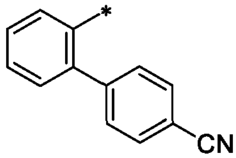
Ar50

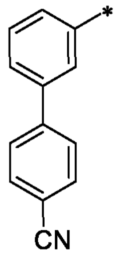
Ar51

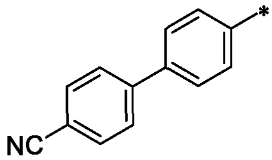
Ar52

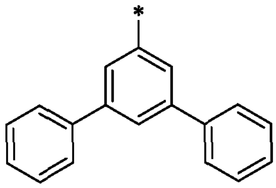
Ar53

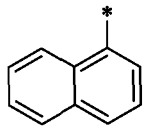
Ar54

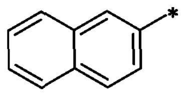
Ar55

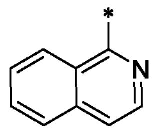
Ar56

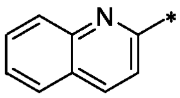
Ar57

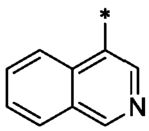
Ar58

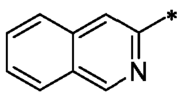
Ar59

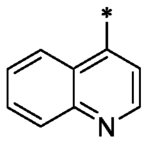
Ar60

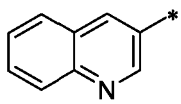
Ar61

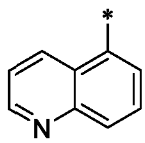
Ar62

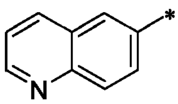
Ar63

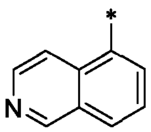
Ar64

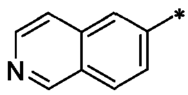
Ar65

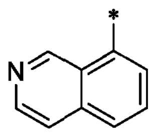
Ar66

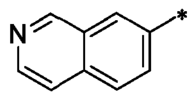
Ar67

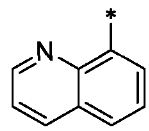
Ar68

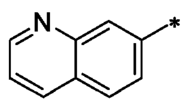
Ar69

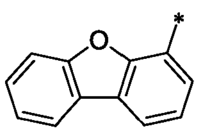
Ar70

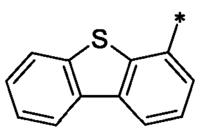
Ar71

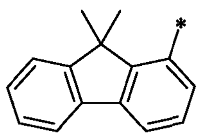
Ar72

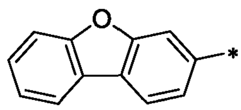
Ar73

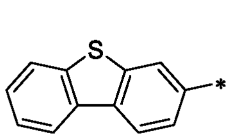
Ar74

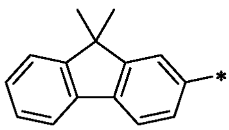
Ar75

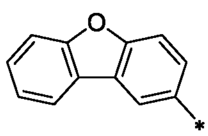
Ar76

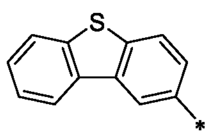
Ar77

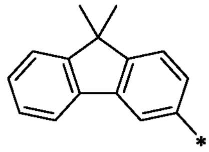
Ar78

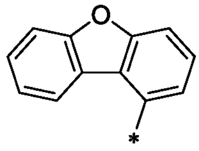
**Ar79**

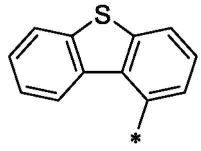
**Ar80**

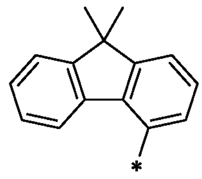
**Ar81**

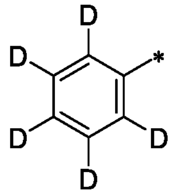
**Ar82**

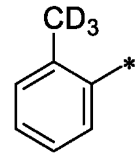
**Ar83**

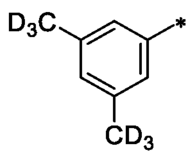
**Ar84**

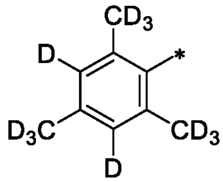
**Ar85**

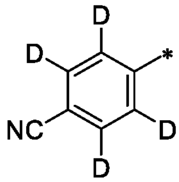
**Ar86**

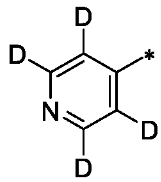
**Ar87**

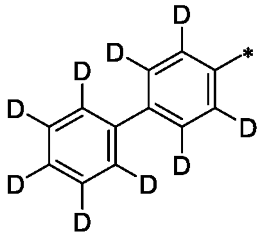
**Ar88**

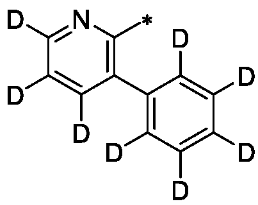
**Ar89**

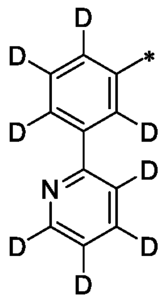
**Ar90**

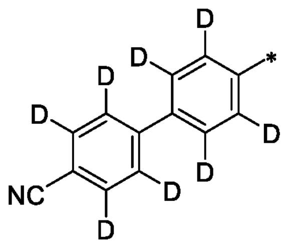
**Ar91**

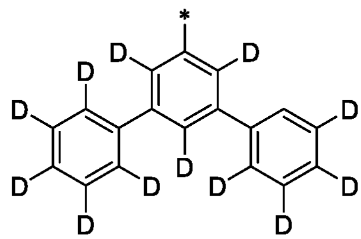
**Ar92**

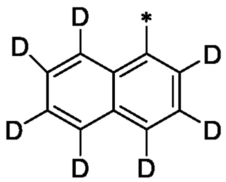
**Ar93**

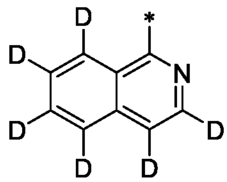
**Ar94**

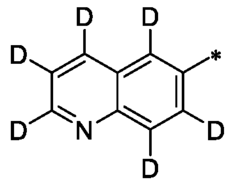
**Ar95**

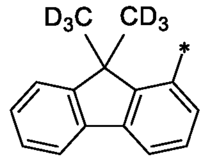
**Ar96**

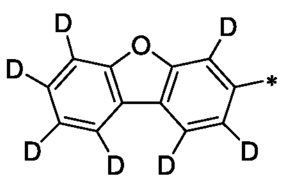
**Ar97**

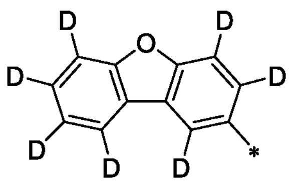
**Ar98**

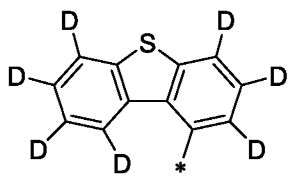
**Ar99**

The compound represented by the general formula (1) can be a compound composed of atoms alone selected from the group consisting of a carbon atom, a hydrogen atom, a deuterium atom, a nitrogen atom, an oxygen atom and a sulfur atom. In one preferred embodiment of the present invention, the compound represented by the general formula (1) is composed of atoms alone selected from the group consisting of a carbon atom, a hydrogen atom, a deuterium atom, a nitrogen atom, and an oxygen atom. The compound represented by the general formula (1) can also be a compound composed of atoms alone selected from the group consisting of a carbon atom, a hydrogen atom, a deuterium atom, a nitrogen atom and a sulfur atom. The compound represented by the general formula (1) can also be a compound

composed of atoms alone selected from the group consisting of a carbon atom, a hydrogen atom and a nitrogen atom. Further, the compound represented by the general formula (1) can be a compound not containing a hydrogen atom but containing a deuterium atom. For example, the compound represented by the general formula (1) can be a compound composed of atoms alone selected from the group consisting of a carbon atom, a deuterium atom, a nitrogen atom, an oxygen atom and a sulfur atom.

In one embodiment of the present invention, the compound represented by the general formula (1) has a symmetric structure.

In one preferred embodiment of the present invention, the compound represented by the general formula (1) has a structure represented by the following general formula (2).

General Formula (2)

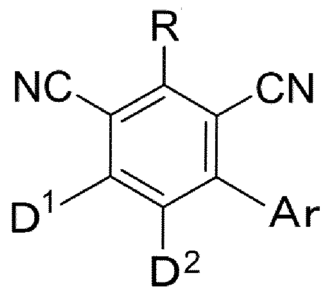

In one preferred embodiment of the present invention, the compound represented by the general formula (1) has a structure represented by the following general formula (3).

General Formula (3)

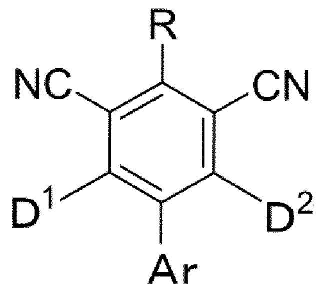

Regarding the definition and the description of R, Ar, $D^1$ and $D^2$ in the general formula (2) and the general formula (3), reference can be made to the corresponding description of the general formula (1).

In the following, shown are specific examples of the compound represented by the general formula (1). Specific examples show individual compounds by defining $R^1$ to $R^4$ in the following general formula. R in the general formula (1) corresponds to $R^1$ in the following general formula, and $D^1$ in the general formula corresponds to $R^2$ in the following general formula. In the case where the following compounds have rotational isomers, a mixture of the rotational isomers and each isolated rotational isomer are considered to be disclosed in the present description.

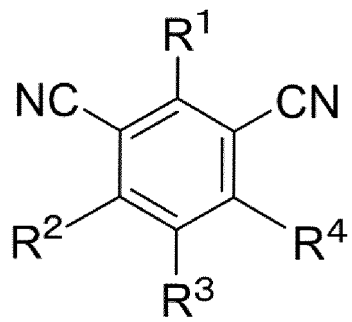

[Table 1]

| No. | $R^1$ = H | | |
|---|---|---|---|
| | $R^2$ | $R^3$ | $R^4$ |
| 1 | D15 | D15 | Ar1 |
| 2 | D16 | | |
| 3 | D18 | | |
| 4 | D21 | | |
| 5 | D33 | | |
| 6 | D34 | | |
| 7 | D36 | | |
| 8 | D45 | | |
| 9 | D46 | | |
| 10 | D48 | | |
| 11 | D63 | | |
| 12 | D64 | | |
| 13 | D66 | | |
| 14 | D73 | | |
| 15 | D74 | | |
| 16 | D75 | | |
| 17 | D76 | | |
| 18 | D78 | | |
| 19 | D155 | | |
| 20 | D182 | | |
| 21 | D183 | | |
| 22 | D185 | | |
| 23 | D246 | | |
| 24 | D265 | | |
| 25 | D266 | | |
| 26 | D267 | | |
| 27 | D268 | | |
| 28 | D269 | | |
| 29 | D296 | | |
| 30 | D298 | | |
| 31 | D15 | D16 | |
| 32 | D16 | | |
| 33 | D18 | | |
| 34 | D21 | | |
| 35 | D33 | | |
| 36 | D34 | | |
| 37 | D36 | | |
| 38 | D45 | | |
| 39 | D46 | | |
| 40 | D48 | | |
| 41 | D63 | | |
| 42 | D64 | | |
| 43 | D66 | | |
| 44 | D73 | | |
| 45 | D74 | | |
| 46 | D75 | | |
| 47 | D76 | | |
| 48 | D78 | | |
| 49 | D155 | | |
| 50 | D182 | | |
| 51 | D183 | | |
| 52 | D185 | | |
| 53 | D246 | | |
| 54 | D265 | | |
| 55 | D266 | | |
| 56 | D267 | | |
| 57 | D268 | | |
| 58 | D269 | | |
| 59 | D296 | | |
| 60 | D298 | | |

| No. | $R^1$ = H | | |
|---|---|---|---|
| | $R^2$ | $R^3$ | $R^4$ |
| 61 | D15 | D18 | Ar1 |
| 62 | D16 | | |
| 63 | D18 | | |
| 64 | D21 | | |
| 65 | D33 | | |
| 66 | D34 | | |
| 67 | D36 | | |
| 68 | D45 | | |
| 69 | D46 | | |
| 70 | D48 | | |
| 71 | D63 | | |
| 72 | D64 | | |
| 73 | D66 | | |
| 74 | D73 | | |
| 75 | D74 | | |
| 76 | D75 | | |
| 77 | D76 | | |
| 78 | D78 | | |
| 79 | D155 | | |
| 80 | D182 | | |
| 81 | D183 | | |
| 82 | D185 | | |
| 83 | D246 | | |
| 84 | D265 | | |
| 85 | D266 | | |
| 86 | D267 | | |
| 87 | D268 | | |
| 88 | D269 | | |
| 89 | D296 | | |
| 90 | D298 | | |
| 91 | D15 | D21 | |
| 92 | D16 | | |
| 93 | D18 | | |
| 94 | D21 | | |
| 95 | D33 | | |
| 96 | D34 | | |
| 97 | D36 | | |
| 98 | D45 | | |
| 99 | D46 | | |
| 100 | D48 | | |
| 101 | D63 | | |
| 102 | D64 | | |
| 103 | D66 | | |
| 104 | D73 | | |
| 105 | D74 | | |
| 106 | D75 | | |
| 107 | D76 | | |
| 108 | D78 | | |
| 109 | D155 | | |
| 110 | D182 | | |
| 111 | D183 | | |
| 112 | D185 | | |
| 113 | D246 | | |
| 114 | D265 | | |
| 115 | D266 | | |
| 116 | D267 | | |
| 117 | D268 | | |
| 118 | D269 | | |
| 119 | D296 | | |
| 120 | D298 | | |

| No. | $R^1$ = H | | |
|---|---|---|---|
| | $R^2$ | $R^3$ | $R^4$ |
| 121 | D15 | D33 | Ar1 |
| 122 | D16 | | |
| 123 | D18 | | |
| 124 | D21 | | |
| 125 | D33 | | |
| 126 | D34 | | |
| 127 | D36 | | |
| 128 | D45 | | |
| 129 | D46 | | |
| 130 | D48 | | |
| 131 | D63 | | |
| 132 | D64 | | |
| 133 | D66 | | |
| 134 | D73 | | |
| 135 | D74 | | |
| 136 | D75 | | |
| 137 | D76 | | |
| 138 | D78 | | |
| 139 | D155 | | |
| 140 | D182 | | |
| 141 | D183 | | |
| 142 | D185 | | |
| 143 | D246 | | |
| 144 | D265 | | |
| 145 | D266 | | |
| 146 | D267 | | |
| 147 | D268 | | |
| 148 | D269 | | |
| 149 | D296 | | |
| 150 | D298 | | |
| 151 | D15 | D34 | |
| 152 | D16 | | |
| 153 | D18 | | |
| 154 | D21 | | |
| 155 | D33 | | |
| 156 | D34 | | |
| 157 | D36 | | |
| 158 | D45 | | |
| 159 | D46 | | |
| 160 | D48 | | |
| 161 | D63 | | |
| 162 | D64 | | |
| 163 | D66 | | |
| 164 | D73 | | |
| 165 | D74 | | |
| 166 | D75 | | |
| 167 | D76 | | |
| 168 | D78 | | |
| 169 | D155 | | |
| 170 | D182 | | |
| 171 | D183 | | |
| 172 | D185 | | |
| 173 | D246 | | |
| 174 | D265 | | |
| 175 | D266 | | |
| 176 | D267 | | |
| 177 | D268 | | |
| 178 | D269 | | |
| 179 | D296 | | |
| 180 | D298 | | |

| No. | $R^1$ = H | | |
|---|---|---|---|
| | $R^2$ | $R^3$ | $R^4$ |
| 181 | D15 | D36 | Ar1 |
| 182 | D16 | | |
| 183 | D18 | | |
| 184 | D21 | | |
| 185 | D33 | | |
| 186 | D34 | | |
| 187 | D36 | | |
| 188 | D45 | | |
| 189 | D46 | | |
| 190 | D48 | | |
| 191 | D63 | | |
| 192 | D64 | | |
| 193 | D66 | | |
| 194 | D73 | | |
| 195 | D74 | | |
| 196 | D75 | | |
| 197 | D76 | | |
| 198 | D78 | | |
| 199 | D155 | | |
| 200 | D182 | | |
| 201 | D183 | | |
| 202 | D185 | | |
| 203 | D246 | | |
| 204 | D265 | | |
| 205 | D266 | | |
| 206 | D267 | | |
| 207 | D268 | | |
| 208 | D269 | | |
| 209 | D296 | | |
| 210 | D298 | | |
| 211 | D15 | D45 | |
| 212 | D16 | | |
| 213 | D18 | | |
| 214 | D21 | | |
| 215 | D33 | | |
| 216 | D34 | | |
| 217 | D36 | | |
| 218 | D45 | | |
| 219 | D46 | | |
| 220 | D48 | | |
| 221 | D63 | | |
| 222 | D64 | | |
| 223 | D66 | | |
| 224 | D73 | | |
| 225 | D74 | | |
| 226 | D75 | | |
| 227 | D76 | | |
| 228 | D78 | | |
| 229 | D155 | | |
| 230 | D182 | | |
| 231 | D183 | | |
| 232 | D185 | | |
| 233 | D246 | | |
| 234 | D265 | | |
| 235 | D266 | | |
| 236 | D267 | | |
| 237 | D268 | | |
| 238 | D269 | | |
| 239 | D296 | | |
| 240 | D298 | | |

| No. | $R^1$ = H | | |
|---|---|---|---|
| | $R^2$ | $R^3$ | $R^4$ |
| 241 | D15 | D46 | Ar1 |
| 242 | D16 | | |
| 243 | D18 | | |
| 244 | D21 | | |
| 245 | D33 | | |
| 246 | D34 | | |
| 247 | D36 | | |
| 248 | D45 | | |
| 249 | D46 | | |
| 250 | D48 | | |
| 251 | D63 | | |
| 252 | D64 | | |
| 253 | D66 | | |
| 254 | D73 | | |
| 255 | D74 | | |
| 256 | D75 | | |
| 257 | D76 | | |
| 258 | D78 | | |
| 259 | D155 | | |
| 260 | D182 | | |
| 261 | D183 | | |
| 262 | D185 | | |
| 263 | D246 | | |
| 264 | D265 | | |
| 265 | D266 | | |
| 266 | D267 | | |
| 267 | D268 | | |
| 268 | D269 | | |
| 269 | D296 | | |
| 270 | D298 | | |
| 271 | D15 | D48 | |
| 272 | D16 | | |
| 273 | D18 | | |
| 274 | D21 | | |
| 275 | D33 | | |
| 276 | D34 | | |
| 277 | D36 | | |
| 278 | D45 | | |
| 279 | D46 | | |
| 280 | D48 | | |
| 281 | D63 | | |
| 282 | D64 | | |
| 283 | D66 | | |
| 284 | D73 | | |
| 285 | D74 | | |
| 286 | D75 | | |
| 287 | D76 | | |
| 288 | D78 | | |
| 289 | D155 | | |
| 290 | D182 | | |
| 291 | D183 | | |
| 292 | D185 | | |
| 293 | D246 | | |
| 294 | D265 | | |
| 295 | D266 | | |
| 296 | D267 | | |
| 297 | D268 | | |
| 298 | D269 | | |
| 299 | D296 | | |
| 300 | D298 | | |

| No. | $R^1$ = H | | |
|---|---|---|---|
| | $R^2$ | $R^3$ | $R^4$ |
| 301 | D15 | D63 | Ar1 |
| 302 | D16 | | |
| 303 | D18 | | |
| 304 | D21 | | |
| 305 | D33 | | |
| 306 | D34 | | |
| 307 | D36 | | |
| 308 | D45 | | |
| 309 | D46 | | |
| 310 | D48 | | |
| 311 | D63 | | |
| 312 | D64 | | |
| 313 | D66 | | |
| 314 | D73 | | |
| 315 | D74 | | |
| 316 | D75 | | |
| 317 | D76 | | |
| 318 | D78 | | |
| 319 | D155 | | |
| 320 | D182 | | |
| 321 | D183 | | |
| 322 | D185 | | |
| 323 | D246 | | |
| 324 | D265 | | |
| 325 | D266 | | |
| 326 | D267 | | |
| 327 | D268 | | |
| 328 | D269 | | |
| 329 | D296 | | |
| 330 | D298 | | |
| 331 | D15 | D64 | |
| 332 | D16 | | |
| 333 | D18 | | |
| 334 | D21 | | |
| 335 | D33 | | |
| 336 | D34 | | |
| 337 | D36 | | |
| 338 | D45 | | |
| 339 | D46 | | |
| 340 | D48 | | |
| 341 | D63 | | |
| 342 | D64 | | |
| 343 | D66 | | |
| 344 | D73 | | |
| 345 | D74 | | |
| 346 | D75 | | |
| 347 | D76 | | |
| 348 | D78 | | |
| 349 | D155 | | |
| 350 | D182 | | |
| 351 | D183 | | |
| 352 | D185 | | |
| 353 | D246 | | |
| 354 | D265 | | |
| 355 | D266 | | |
| 356 | D267 | | |
| 357 | D268 | | |
| 358 | D269 | | |
| 359 | D296 | | |
| 360 | D298 | | |

| No. | $R^1$ = H | | |
|---|---|---|---|
| | $R^2$ | $R^3$ | $R^4$ |
| 361 | D15 | D66 | Ar1 |
| 362 | D16 | | |
| 363 | D18 | | |
| 364 | D21 | | |
| 365 | D33 | | |
| 366 | D34 | | |
| 367 | D36 | | |
| 368 | D45 | | |
| 369 | D46 | | |
| 370 | D48 | | |
| 371 | D63 | | |
| 372 | D64 | | |
| 373 | D66 | | |
| 374 | D73 | | |
| 375 | D74 | | |
| 376 | D75 | | |
| 377 | D76 | | |
| 378 | D78 | | |
| 379 | D155 | | |
| 380 | D182 | | |
| 381 | D183 | | |
| 382 | D185 | | |
| 383 | D246 | | |
| 384 | D265 | | |
| 385 | D266 | | |
| 386 | D267 | | |
| 387 | D268 | | |
| 388 | D269 | | |
| 389 | D296 | | |
| 390 | D298 | | |
| 391 | D15 | D73 | |
| 392 | D16 | | |
| 393 | D18 | | |
| 394 | D21 | | |
| 395 | D33 | | |
| 396 | D34 | | |
| 397 | D36 | | |
| 398 | D45 | | |
| 399 | D46 | | |
| 400 | D48 | | |
| 401 | D63 | | |
| 402 | D64 | | |
| 403 | D66 | | |
| 404 | D73 | | |
| 405 | D74 | | |
| 406 | D75 | | |
| 407 | D76 | | |
| 408 | D78 | | |
| 409 | D155 | | |
| 410 | D182 | | |
| 411 | D183 | | |
| 412 | D185 | | |
| 413 | D246 | | |
| 414 | D265 | | |
| 415 | D266 | | |
| 416 | D267 | | |
| 417 | D268 | | |
| 418 | D269 | | |
| 419 | D296 | | |
| 420 | D298 | | |

| No. | $R^1$ = H | | |
|---|---|---|---|
| | $R^2$ | $R^3$ | $R^4$ |
| 421 | D15 | D74 | Ar1 |
| 422 | D16 | | |
| 423 | D18 | | |
| 424 | D21 | | |
| 425 | D33 | | |
| 426 | D34 | | |
| 427 | D36 | | |
| 428 | D45 | | |
| 429 | D46 | | |
| 430 | D48 | | |
| 431 | D63 | | |
| 432 | D64 | | |
| 433 | D66 | | |
| 434 | D73 | | |
| 435 | D74 | | |
| 436 | D75 | | |
| 437 | D76 | | |
| 438 | D78 | | |
| 439 | D155 | | |
| 440 | D182 | | |
| 441 | D183 | | |
| 442 | D185 | | |
| 443 | D246 | | |
| 444 | D265 | | |
| 445 | D266 | | |
| 446 | D267 | | |
| 447 | D268 | | |
| 448 | D269 | | |
| 449 | D296 | | |
| 450 | D298 | | |
| 451 | D15 | D75 | |
| 452 | D16 | | |
| 453 | D18 | | |
| 454 | D21 | | |
| 455 | D33 | | |
| 456 | D34 | | |
| 457 | D36 | | |
| 458 | D45 | | |
| 459 | D46 | | |
| 460 | D48 | | |
| 461 | D63 | | |
| 462 | D64 | | |
| 463 | D66 | | |
| 464 | D73 | | |
| 465 | D74 | | |
| 466 | D75 | | |
| 467 | D76 | | |
| 468 | D78 | | |
| 469 | D155 | | |
| 470 | D182 | | |
| 471 | D183 | | |
| 472 | D185 | | |
| 473 | D246 | | |
| 474 | D265 | | |
| 475 | D266 | | |
| 476 | D267 | | |
| 477 | D268 | | |
| 478 | D269 | | |
| 479 | D296 | | |
| 480 | D298 | | |

| No. | $R^1$ = H | | |
|---|---|---|---|
| | $R^2$ | $R^3$ | $R^4$ |
| 481 | D15 | D76 | Ar1 |
| 482 | D16 | | |
| 483 | D18 | | |
| 484 | D21 | | |
| 485 | D33 | | |
| 486 | D34 | | |
| 487 | D36 | | |
| 488 | D45 | | |
| 489 | D46 | | |
| 490 | D48 | | |
| 491 | D63 | | |
| 492 | D64 | | |
| 493 | D66 | | |
| 494 | D73 | | |
| 495 | D74 | | |
| 496 | D75 | | |
| 497 | D76 | | |
| 498 | D78 | | |
| 499 | D155 | | |
| 500 | D182 | | |
| 501 | D183 | | |
| 502 | D185 | | |
| 503 | D246 | | |
| 504 | D265 | | |
| 505 | D266 | | |
| 506 | D267 | | |
| 507 | D268 | | |
| 508 | D269 | | |
| 509 | D296 | | |
| 510 | D298 | | |
| 511 | D15 | D78 | |
| 512 | D16 | | |
| 513 | D18 | | |
| 514 | D21 | | |
| 515 | D33 | | |
| 516 | D34 | | |
| 517 | D36 | | |
| 518 | D45 | | |
| 519 | D46 | | |
| 520 | D48 | | |
| 521 | D63 | | |
| 522 | D64 | | |
| 523 | D66 | | |
| 524 | D73 | | |
| 525 | D74 | | |
| 526 | D75 | | |
| 527 | D76 | | |
| 528 | D78 | | |
| 529 | D155 | | |
| 530 | D182 | | |
| 531 | D183 | | |
| 532 | D185 | | |
| 533 | D246 | | |
| 534 | D265 | | |
| 535 | D266 | | |
| 536 | D267 | | |
| 537 | D268 | | |
| 538 | D269 | | |
| 539 | D296 | | |
| 540 | D298 | | |

| No. | $R^1$ = H | | |
|---|---|---|---|
| | $R^2$ | $R^3$ | $R^4$ |
| 541 | D15 | D155 | Ar1 |
| 542 | D16 | | |
| 543 | D18 | | |
| 544 | D21 | | |
| 545 | D33 | | |
| 546 | D34 | | |
| 547 | D36 | | |
| 548 | D45 | | |
| 549 | D46 | | |
| 550 | D48 | | |
| 551 | D63 | | |
| 552 | D64 | | |
| 553 | D66 | | |
| 554 | D73 | | |
| 555 | D74 | | |
| 556 | D75 | | |
| 557 | D76 | | |
| 558 | D78 | | |
| 559 | D155 | | |
| 560 | D182 | | |
| 561 | D183 | | |
| 562 | D185 | | |
| 563 | D246 | | |
| 564 | D265 | | |
| 565 | D266 | | |
| 566 | D267 | | |
| 567 | D268 | | |
| 568 | D269 | | |
| 569 | D296 | | |
| 570 | D298 | | |
| 571 | D15 | D182 | |
| 572 | D16 | | |
| 573 | D18 | | |
| 574 | D21 | | |
| 575 | D33 | | |
| 576 | D34 | | |
| 577 | D36 | | |
| 578 | D45 | | |
| 579 | D46 | | |
| 580 | D48 | | |
| 581 | D63 | | |
| 582 | D64 | | |
| 583 | D66 | | |
| 584 | D73 | | |
| 585 | D74 | | |
| 586 | D75 | | |
| 587 | D76 | | |
| 588 | D78 | | |
| 589 | D155 | | |
| 590 | D182 | | |
| 591 | D183 | | |
| 592 | D185 | | |
| 593 | D246 | | |
| 594 | D265 | | |
| 595 | D266 | | |
| 596 | D267 | | |
| 597 | D268 | | |
| 598 | D269 | | |
| 599 | D296 | | |
| 600 | D298 | | |

| No. | $R^1$ = H | | |
|---|---|---|---|
| | $R^2$ | $R^3$ | $R^4$ |
| 601 | D15 | D183 | Ar1 |
| 602 | D16 | | |
| 603 | D18 | | |
| 604 | D21 | | |
| 605 | D33 | | |
| 606 | D34 | | |
| 607 | D36 | | |
| 608 | D45 | | |
| 609 | D46 | | |
| 610 | D48 | | |
| 611 | D63 | | |
| 612 | D64 | | |
| 613 | D66 | | |
| 614 | D73 | | |
| 615 | D74 | | |
| 616 | D75 | | |
| 617 | D76 | | |
| 618 | D78 | | |
| 619 | D155 | | |
| 620 | D182 | | |
| 621 | D183 | | |
| 622 | D185 | | |
| 623 | D246 | | |
| 624 | D265 | | |
| 625 | D266 | | |
| 626 | D267 | | |
| 627 | D268 | | |
| 628 | D269 | | |
| 629 | D296 | | |
| 630 | D298 | | |
| 631 | D15 | D185 | |
| 632 | D16 | | |
| 633 | D18 | | |
| 634 | D21 | | |
| 635 | D33 | | |
| 636 | D34 | | |
| 637 | D36 | | |
| 638 | D45 | | |
| 639 | D46 | | |
| 640 | D48 | | |
| 641 | D63 | | |
| 642 | D64 | | |
| 643 | D66 | | |
| 644 | D73 | | |
| 645 | D74 | | |
| 646 | D75 | | |
| 647 | D76 | | |
| 648 | D78 | | |
| 649 | D155 | | |
| 650 | D182 | | |
| 651 | D183 | | |
| 652 | D185 | | |
| 653 | D246 | | |
| 654 | D265 | | |
| 655 | D266 | | |
| 656 | D267 | | |
| 657 | D268 | | |
| 658 | D269 | | |
| 659 | D296 | | |
| 660 | D298 | | |

| No. | $R^1$ = H | | |
|---|---|---|---|
| | $R^2$ | $R^3$ | $R^4$ |
| 661 | D15 | D246 | Ar1 |
| 662 | D16 | | |
| 663 | D18 | | |
| 664 | D21 | | |
| 665 | D33 | | |
| 666 | D34 | | |
| 667 | D36 | | |
| 668 | D45 | | |
| 669 | D46 | | |
| 670 | D48 | | |
| 671 | D63 | | |
| 672 | D64 | | |
| 673 | D66 | | |
| 674 | D73 | | |
| 675 | D74 | | |
| 676 | D75 | | |
| 677 | D76 | | |
| 678 | D78 | | |
| 679 | D155 | | |
| 680 | D182 | | |
| 681 | D183 | | |
| 682 | D185 | | |
| 683 | D246 | | |
| 684 | D265 | | |
| 685 | D266 | | |
| 686 | D267 | | |
| 687 | D268 | | |
| 688 | D269 | | |
| 689 | D296 | | |
| 690 | D298 | | |
| 691 | D15 | D265 | |
| 692 | D16 | | |
| 693 | D18 | | |
| 694 | D21 | | |
| 695 | D33 | | |
| 696 | D34 | | |
| 697 | D36 | | |
| 698 | D45 | | |
| 699 | D46 | | |
| 700 | D48 | | |
| 701 | D63 | | |
| 702 | D64 | | |
| 703 | D66 | | |
| 704 | D73 | | |
| 705 | D74 | | |
| 706 | D75 | | |
| 707 | D76 | | |
| 708 | D78 | | |
| 709 | D155 | | |
| 710 | D182 | | |
| 711 | D183 | | |
| 712 | D185 | | |
| 713 | D246 | | |
| 714 | D265 | | |
| 715 | D266 | | |
| 716 | D267 | | |
| 717 | D268 | | |
| 718 | D269 | | |
| 719 | D296 | | |
| 720 | D298 | | |

| No. | $R^1$ = H | | |
|---|---|---|---|
| | $R^2$ | $R^3$ | $R^4$ |
| 721 | D15 | D266 | Ar1 |
| 722 | D16 | | |
| 723 | D18 | | |
| 724 | D21 | | |
| 725 | D33 | | |
| 726 | D34 | | |
| 727 | D36 | | |
| 728 | D45 | | |
| 729 | D46 | | |
| 730 | D48 | | |
| 731 | D63 | | |
| 732 | D64 | | |
| 733 | D66 | | |
| 734 | D73 | | |
| 735 | D74 | | |
| 736 | D75 | | |
| 737 | D76 | | |
| 738 | D78 | | |
| 739 | D155 | | |
| 740 | D182 | | |
| 741 | D183 | | |
| 742 | D185 | | |
| 743 | D246 | | |
| 744 | D265 | | |
| 745 | D266 | | |
| 746 | D267 | | |
| 747 | D268 | | |
| 748 | D269 | | |
| 749 | D296 | | |
| 750 | D298 | | |
| 751 | D15 | D267 | |
| 752 | D16 | | |
| 753 | D18 | | |
| 754 | D21 | | |
| 755 | D33 | | |
| 756 | D34 | | |
| 757 | D36 | | |
| 758 | D45 | | |
| 759 | D46 | | |
| 760 | D48 | | |
| 761 | D63 | | |
| 762 | D64 | | |
| 763 | D66 | | |
| 764 | D73 | | |
| 765 | D74 | | |
| 766 | D75 | | |
| 767 | D76 | | |
| 768 | D78 | | |
| 769 | D155 | | |
| 770 | D182 | | |
| 771 | D183 | | |
| 772 | D185 | | |
| 773 | D246 | | |
| 774 | D265 | | |
| 775 | D266 | | |
| 776 | D267 | | |
| 777 | D268 | | |
| 778 | D269 | | |
| 779 | D296 | | |
| 780 | D298 | | |

| No. | $R^1$ = H | | |
|---|---|---|---|
| | $R^2$ | $R^3$ | $R^4$ |
| 781 | D15 | D268 | Ar1 |
| 782 | D16 | | |
| 783 | D18 | | |
| 784 | D21 | | |
| 785 | D33 | | |
| 786 | D34 | | |
| 787 | D36 | | |
| 788 | D45 | | |
| 789 | D46 | | |
| 790 | D48 | | |
| 791 | D63 | | |
| 792 | D64 | | |
| 793 | D66 | | |
| 794 | D73 | | |
| 795 | D74 | | |
| 796 | D75 | | |
| 797 | D76 | | |
| 798 | D78 | | |
| 799 | D155 | | |
| 800 | D182 | | |
| 801 | D183 | | |
| 802 | D185 | | |
| 803 | D246 | | |
| 804 | D265 | | |
| 805 | D266 | | |
| 806 | D267 | | |
| 807 | D268 | | |
| 808 | D269 | | |
| 809 | D296 | | |
| 810 | D298 | | |
| 811 | D15 | D269 | |
| 812 | D16 | | |
| 813 | D18 | | |
| 814 | D21 | | |
| 815 | D33 | | |
| 816 | D34 | | |
| 817 | D36 | | |
| 818 | D45 | | |
| 819 | D46 | | |
| 820 | D48 | | |
| 821 | D63 | | |
| 822 | D64 | | |
| 823 | D66 | | |
| 824 | D73 | | |
| 825 | D74 | | |
| 826 | D75 | | |
| 827 | D76 | | |
| 828 | D78 | | |
| 829 | D155 | | |
| 830 | D182 | | |
| 831 | D183 | | |
| 832 | D185 | | |
| 833 | D246 | | |
| 834 | D265 | | |
| 835 | D266 | | |
| 836 | D267 | | |
| 837 | D268 | | |
| 838 | D269 | | |
| 839 | D296 | | |
| 840 | D298 | | |

| No. | $R^1$ = H | | |
|---|---|---|---|
| | $R^2$ | $R^3$ | $R^4$ |
| 841 | D15 | D296 | Ar1 |
| 842 | D16 | | |
| 843 | D18 | | |
| 844 | D21 | | |
| 845 | D33 | | |
| 846 | D34 | | |
| 847 | D36 | | |
| 848 | D45 | | |
| 849 | D46 | | |
| 850 | D48 | | |
| 851 | D63 | | |
| 852 | D64 | | |
| 853 | D66 | | |
| 854 | D73 | | |
| 855 | D74 | | |
| 856 | D75 | | |
| 857 | D76 | | |
| 858 | D78 | | |
| 859 | D155 | | |
| 860 | D182 | | |
| 861 | D183 | | |
| 862 | D185 | | |
| 863 | D246 | | |
| 864 | D265 | | |
| 865 | D266 | | |
| 866 | D267 | | |
| 867 | D268 | | |
| 868 | D269 | | |
| 869 | D296 | | |
| 870 | D298 | | |
| 871 | D15 | D298 | |
| 872 | D16 | | |
| 873 | D18 | | |
| 874 | D21 | | |
| 875 | D33 | | |
| 876 | D34 | | |
| 877 | D36 | | |
| 878 | D45 | | |
| 879 | D46 | | |
| 880 | D48 | | |
| 881 | D63 | | |
| 882 | D64 | | |
| 883 | D66 | | |
| 884 | D73 | | |
| 885 | D74 | | |
| 886 | D75 | | |
| 887 | D76 | | |
| 888 | D78 | | |
| 889 | D155 | | |
| 890 | D182 | | |
| 891 | D183 | | |
| 892 | D185 | | |
| 893 | D246 | | |
| 894 | D265 | | |
| 895 | D266 | | |
| 896 | D267 | | |
| 897 | D268 | | |
| 898 | D269 | | |
| 899 | D296 | | |
| 900 | D298 | | |

| No. | $R^1$ = H | | |
|---|---|---|---|
| | $R^2$ | $R^3$ | $R^4$ |
| 901 | D15 | D15 | Ar16 |
| 902 | D16 | | |
| 903 | D18 | | |
| 904 | D21 | | |
| 905 | D33 | | |
| 906 | D34 | | |
| 907 | D36 | | |
| 908 | D45 | | |
| 909 | D46 | | |
| 910 | D48 | | |
| 911 | D63 | | |
| 912 | D64 | | |
| 913 | D66 | | |
| 914 | D73 | | |
| 915 | D74 | | |
| 916 | D75 | | |
| 917 | D76 | | |
| 918 | D78 | | |
| 919 | D155 | | |
| 920 | D182 | | |
| 921 | D183 | | |
| 922 | D185 | | |
| 923 | D246 | | |
| 924 | D265 | | |
| 925 | D266 | | |
| 926 | D267 | | |
| 927 | D268 | | |
| 928 | D269 | | |
| 929 | D296 | | |
| 930 | D298 | | |
| 931 | D15 | D16 | |
| 932 | D16 | | |
| 933 | D18 | | |
| 934 | D21 | | |
| 935 | D33 | | |
| 936 | D34 | | |
| 937 | D36 | | |
| 938 | D45 | | |
| 939 | D46 | | |
| 940 | D48 | | |
| 941 | D63 | | |
| 942 | D64 | | |
| 943 | D66 | | |
| 944 | D73 | | |
| 945 | D74 | | |
| 946 | D75 | | |
| 947 | D76 | | |
| 948 | D78 | | |
| 949 | D155 | | |
| 950 | D182 | | |
| 951 | D183 | | |
| 952 | D185 | | |
| 953 | D246 | | |
| 954 | D265 | | |
| 955 | D266 | | |
| 956 | D267 | | |
| 957 | D268 | | |
| 958 | D269 | | |
| 959 | D296 | | |
| 960 | D298 | | |

| No. | $R^1$ = H | | |
|---|---|---|---|
| | $R^2$ | $R^3$ | $R^4$ |
| 961 | D15 | D18 | Ar16 |
| 962 | D16 | | |
| 963 | D18 | | |
| 964 | D21 | | |
| 965 | D33 | | |
| 966 | D34 | | |
| 967 | D36 | | |
| 968 | D45 | | |
| 969 | D46 | | |
| 970 | D48 | | |
| 971 | D63 | | |
| 972 | D64 | | |
| 973 | D66 | | |
| 974 | D73 | | |
| 975 | D74 | | |
| 976 | D75 | | |
| 977 | D76 | | |
| 978 | D78 | | |
| 979 | D155 | | |
| 980 | D182 | | |
| 981 | D183 | | |
| 982 | D185 | | |
| 983 | D246 | | |
| 984 | D265 | | |
| 985 | D266 | | |
| 986 | D267 | | |
| 987 | D268 | | |
| 988 | D269 | | |
| 989 | D296 | | |
| 990 | D298 | | |
| 991 | D15 | D21 | |
| 992 | D16 | | |
| 993 | D18 | | |
| 994 | D21 | | |
| 995 | D33 | | |
| 996 | D34 | | |
| 997 | D36 | | |
| 998 | D45 | | |
| 999 | D46 | | |
| 1000 | D48 | | |
| 1001 | D63 | | |
| 1002 | D64 | | |
| 1003 | D66 | | |
| 1004 | D73 | | |
| 1005 | D74 | | |
| 1006 | D75 | | |
| 1007 | D76 | | |
| 1008 | D78 | | |
| 1009 | D155 | | |
| 1010 | D182 | | |
| 1011 | D183 | | |
| 1012 | D185 | | |
| 1013 | D246 | | |
| 1014 | D265 | | |
| 1015 | D266 | | |
| 1016 | D267 | | |
| 1017 | D268 | | |
| 1018 | D269 | | |
| 1019 | D296 | | |
| 1020 | D298 | | |

| No. | $R^1$ = H | | |
|---|---|---|---|
| | $R^2$ | $R^3$ | $R^4$ |
| 1021 | D15 | D33 | Ar16 |
| 1022 | D16 | | |
| 1023 | D18 | | |
| 1024 | D21 | | |
| 1025 | D33 | | |
| 1026 | D34 | | |
| 1027 | D36 | | |
| 1028 | D45 | | |
| 1029 | D46 | | |
| 1030 | D48 | | |
| 1031 | D63 | | |
| 1032 | D64 | | |
| 1033 | D66 | | |
| 1034 | D73 | | |
| 1035 | D74 | | |
| 1036 | D75 | | |
| 1037 | D76 | | |
| 1038 | D78 | | |
| 1039 | D155 | | |
| 1040 | D182 | | |
| 1041 | D183 | | |
| 1042 | D185 | | |
| 1043 | D246 | | |
| 1044 | D265 | | |
| 1045 | D266 | | |
| 1046 | D267 | | |
| 1047 | D268 | | |
| 1048 | D269 | | |
| 1049 | D296 | | |
| 1050 | D298 | | |
| 1051 | D15 | D34 | |
| 1052 | D16 | | |
| 1053 | D18 | | |
| 1054 | D21 | | |
| 1055 | D33 | | |
| 1056 | D34 | | |
| 1057 | D36 | | |
| 1058 | D45 | | |
| 1059 | D46 | | |
| 1060 | D48 | | |
| 1061 | D63 | | |
| 1062 | D64 | | |
| 1063 | D66 | | |
| 1064 | D73 | | |
| 1065 | D74 | | |
| 1066 | D75 | | |
| 1067 | D76 | | |
| 1068 | D78 | | |
| 1069 | D155 | | |
| 1070 | D182 | | |
| 1071 | D183 | | |
| 1072 | D185 | | |
| 1073 | D246 | | |
| 1074 | D265 | | |
| 1075 | D266 | | |
| 1076 | D267 | | |
| 1077 | D268 | | |
| 1078 | D269 | | |
| 1079 | D296 | | |
| 1080 | D298 | | |

| No. | $R^1$ = H | | |
|---|---|---|---|
| | $R^2$ | $R^3$ | $R^4$ |
| 1081 | D15 | D36 | Ar16 |
| 1082 | D16 | | |
| 1083 | D18 | | |
| 1084 | D21 | | |
| 1085 | D33 | | |
| 1086 | D34 | | |
| 1087 | D36 | | |
| 1088 | D45 | | |
| 1089 | D46 | | |
| 1090 | D48 | | |
| 1091 | D63 | | |
| 1092 | D64 | | |
| 1093 | D66 | | |
| 1094 | D73 | | |
| 1095 | D74 | | |
| 1096 | D75 | | |
| 1097 | D76 | | |
| 1098 | D78 | | |
| 1099 | D155 | | |
| 1100 | D182 | | |
| 1101 | D183 | | |
| 1102 | D185 | | |
| 1103 | D246 | | |
| 1104 | D265 | | |
| 1105 | D266 | | |
| 1106 | D267 | | |
| 1107 | D268 | | |
| 1108 | D269 | | |
| 1109 | D296 | | |
| 1110 | D298 | | |
| 1111 | D15 | D45 | |
| 1112 | D16 | | |
| 1113 | D18 | | |
| 1114 | D21 | | |
| 1115 | D33 | | |
| 1116 | D34 | | |
| 1117 | D36 | | |
| 1118 | D45 | | |
| 1119 | D46 | | |
| 1120 | D48 | | |
| 1121 | D63 | | |
| 1122 | D64 | | |
| 1123 | D66 | | |
| 1124 | D73 | | |
| 1125 | D74 | | |
| 1126 | D75 | | |
| 1127 | D76 | | |
| 1128 | D78 | | |
| 1129 | D155 | | |
| 1130 | D182 | | |
| 1131 | D183 | | |
| 1132 | D185 | | |
| 1133 | D246 | | |
| 1134 | D265 | | |
| 1135 | D266 | | |
| 1136 | D267 | | |
| 1137 | D268 | | |
| 1138 | D269 | | |
| 1139 | D296 | | |
| 1140 | D298 | | |

| No. | $R^1$ = H | | |
|---|---|---|---|
| | $R^2$ | $R^3$ | $R^4$ |
| 1141 | D15 | D46 | Ar16 |
| 1142 | D16 | | |
| 1143 | D18 | | |
| 1144 | D21 | | |
| 1145 | D33 | | |
| 1146 | D34 | | |
| 1147 | D36 | | |
| 1148 | D45 | | |
| 1149 | D46 | | |
| 1150 | D48 | | |
| 1151 | D63 | | |
| 1152 | D64 | | |
| 1153 | D66 | | |
| 1154 | D73 | | |
| 1155 | D74 | | |
| 1156 | D75 | | |
| 1157 | D76 | | |
| 1158 | D78 | | |
| 1159 | D155 | | |
| 1160 | D182 | | |
| 1161 | D183 | | |
| 1162 | D185 | | |
| 1163 | D246 | | |
| 1164 | D265 | | |
| 1165 | D266 | | |
| 1166 | D267 | | |
| 1167 | D268 | | |
| 1168 | D269 | | |
| 1169 | D296 | | |
| 1170 | D298 | | |
| 1171 | D15 | D48 | |
| 1172 | D16 | | |
| 1173 | D18 | | |
| 1174 | D21 | | |
| 1175 | D33 | | |
| 1176 | D34 | | |
| 1177 | D36 | | |
| 1178 | D45 | | |
| 1179 | D46 | | |
| 1180 | D48 | | |
| 1181 | D63 | | |
| 1182 | D64 | | |
| 1183 | D66 | | |
| 1184 | D73 | | |
| 1185 | D74 | | |
| 1186 | D75 | | |
| 1187 | D76 | | |
| 1188 | D78 | | |
| 1189 | D155 | | |
| 1190 | D182 | | |
| 1191 | D183 | | |
| 1192 | D185 | | |
| 1193 | D246 | | |
| 1194 | D265 | | |
| 1195 | D266 | | |
| 1196 | D267 | | |
| 1197 | D268 | | |
| 1198 | D269 | | |
| 1199 | D296 | | |
| 1200 | D298 | | |

| No. | $R^1$ = H | | |
|---|---|---|---|
| | $R^2$ | $R^3$ | $R^4$ |
| 1201 | D15 | D63 | Ar16 |
| 1202 | D16 | D63 | Ar16 |
| 1203 | D18 | D63 | Ar16 |
| 1204 | D21 | D63 | Ar16 |
| 1205 | D33 | D63 | Ar16 |
| 1206 | D34 | D63 | Ar16 |
| 1207 | D36 | D63 | Ar16 |
| 1208 | D45 | D63 | Ar16 |
| 1209 | D46 | D63 | Ar16 |
| 1210 | D48 | D63 | Ar16 |
| 1211 | D63 | D63 | Ar16 |
| 1212 | D64 | D63 | Ar16 |
| 1213 | D66 | D63 | Ar16 |
| 1214 | D73 | D63 | Ar16 |
| 1215 | D74 | D63 | Ar16 |
| 1216 | D75 | D63 | Ar16 |
| 1217 | D76 | D63 | Ar16 |
| 1218 | D78 | D63 | Ar16 |
| 1219 | D155 | D63 | Ar16 |
| 1220 | D182 | D63 | Ar16 |
| 1221 | D183 | D63 | Ar16 |
| 1222 | D185 | D63 | Ar16 |
| 1223 | D246 | D63 | Ar16 |
| 1224 | D265 | D63 | Ar16 |
| 1225 | D266 | D63 | Ar16 |
| 1226 | D267 | D63 | Ar16 |
| 1227 | D268 | D63 | Ar16 |
| 1228 | D269 | D63 | Ar16 |
| 1229 | D296 | D63 | Ar16 |
| 1230 | D298 | D63 | Ar16 |
| 1231 | D15 | D64 | Ar16 |
| 1232 | D16 | D64 | Ar16 |
| 1233 | D18 | D64 | Ar16 |
| 1234 | D21 | D64 | Ar16 |
| 1235 | D33 | D64 | Ar16 |
| 1236 | D34 | D64 | Ar16 |
| 1237 | D36 | D64 | Ar16 |
| 1238 | D45 | D64 | Ar16 |
| 1239 | D46 | D64 | Ar16 |
| 1240 | D48 | D64 | Ar16 |
| 1241 | D63 | D64 | Ar16 |
| 1242 | D64 | D64 | Ar16 |
| 1243 | D66 | D64 | Ar16 |
| 1244 | D73 | D64 | Ar16 |
| 1245 | D74 | D64 | Ar16 |
| 1246 | D75 | D64 | Ar16 |
| 1247 | D76 | D64 | Ar16 |
| 1248 | D78 | D64 | Ar16 |
| 1249 | D155 | D64 | Ar16 |
| 1250 | D182 | D64 | Ar16 |
| 1251 | D183 | D64 | Ar16 |
| 1252 | D185 | D64 | Ar16 |
| 1253 | D246 | D64 | Ar16 |
| 1254 | D265 | D64 | Ar16 |
| 1255 | D266 | D64 | Ar16 |
| 1256 | D267 | D64 | Ar16 |
| 1257 | D268 | D64 | Ar16 |
| 1258 | D269 | D64 | Ar16 |
| 1259 | D296 | D64 | Ar16 |
| 1260 | D298 | D64 | Ar16 |

| No. | $R^1$ = H | | |
|---|---|---|---|
| | $R^2$ | $R^3$ | $R^4$ |
| 1261 | D15 | D66 | Ar16 |
| 1262 | D16 | D66 | Ar16 |
| 1263 | D18 | D66 | Ar16 |
| 1264 | D21 | D66 | Ar16 |
| 1265 | D33 | D66 | Ar16 |
| 1266 | D34 | D66 | Ar16 |
| 1267 | D36 | D66 | Ar16 |
| 1268 | D45 | D66 | Ar16 |
| 1269 | D46 | D66 | Ar16 |
| 1270 | D48 | D66 | Ar16 |
| 1271 | D63 | D66 | Ar16 |
| 1272 | D64 | D66 | Ar16 |
| 1273 | D66 | D66 | Ar16 |
| 1274 | D73 | D66 | Ar16 |
| 1275 | D74 | D66 | Ar16 |
| 1276 | D75 | D66 | Ar16 |
| 1277 | D76 | D66 | Ar16 |
| 1278 | D78 | D66 | Ar16 |
| 1279 | D155 | D66 | Ar16 |
| 1280 | D182 | D66 | Ar16 |
| 1281 | D183 | D66 | Ar16 |
| 1282 | D185 | D66 | Ar16 |
| 1283 | D246 | D66 | Ar16 |
| 1284 | D265 | D66 | Ar16 |
| 1285 | D266 | D66 | Ar16 |
| 1286 | D267 | D66 | Ar16 |
| 1287 | D268 | D66 | Ar16 |
| 1288 | D269 | D66 | Ar16 |
| 1289 | D296 | D66 | Ar16 |
| 1290 | D298 | D66 | Ar16 |
| 1291 | D15 | D73 | Ar16 |
| 1292 | D16 | D73 | Ar16 |
| 1293 | D18 | D73 | Ar16 |
| 1294 | D21 | D73 | Ar16 |
| 1295 | D33 | D73 | Ar16 |
| 1296 | D34 | D73 | Ar16 |
| 1297 | D36 | D73 | Ar16 |
| 1298 | D45 | D73 | Ar16 |
| 1299 | D46 | D73 | Ar16 |
| 1300 | D48 | D73 | Ar16 |
| 1301 | D63 | D73 | Ar16 |
| 1302 | D64 | D73 | Ar16 |
| 1303 | D66 | D73 | Ar16 |
| 1304 | D73 | D73 | Ar16 |
| 1305 | D74 | D73 | Ar16 |
| 1306 | D75 | D73 | Ar16 |
| 1307 | D76 | D73 | Ar16 |
| 1308 | D78 | D73 | Ar16 |
| 1309 | D155 | D73 | Ar16 |
| 1310 | D182 | D73 | Ar16 |
| 1311 | D183 | D73 | Ar16 |
| 1312 | D185 | D73 | Ar16 |
| 1313 | D246 | D73 | Ar16 |
| 1314 | D265 | D73 | Ar16 |
| 1315 | D266 | D73 | Ar16 |
| 1316 | D267 | D73 | Ar16 |
| 1317 | D268 | D73 | Ar16 |
| 1318 | D269 | D73 | Ar16 |
| 1319 | D296 | D73 | Ar16 |
| 1320 | D298 | D73 | Ar16 |

| No. | $R^1$ = H | | |
|---|---|---|---|
| | $R^2$ | $R^3$ | $R^4$ |
| 1321 | D15 | D74 | Ar16 |
| 1322 | D16 | D74 | Ar16 |
| 1323 | D18 | D74 | Ar16 |
| 1324 | D21 | D74 | Ar16 |
| 1325 | D33 | D74 | Ar16 |
| 1326 | D34 | D74 | Ar16 |
| 1327 | D36 | D74 | Ar16 |
| 1328 | D45 | D74 | Ar16 |
| 1329 | D46 | D74 | Ar16 |
| 1330 | D48 | D74 | Ar16 |
| 1331 | D63 | D74 | Ar16 |
| 1332 | D64 | D74 | Ar16 |
| 1333 | D66 | D74 | Ar16 |
| 1334 | D73 | D74 | Ar16 |
| 1335 | D74 | D74 | Ar16 |
| 1336 | D75 | D74 | Ar16 |
| 1337 | D76 | D74 | Ar16 |
| 1338 | D78 | D74 | Ar16 |
| 1339 | D155 | D74 | Ar16 |
| 1340 | D182 | D74 | Ar16 |
| 1341 | D183 | D74 | Ar16 |
| 1342 | D185 | D74 | Ar16 |
| 1343 | D246 | D74 | Ar16 |
| 1344 | D265 | D74 | Ar16 |
| 1345 | D266 | D74 | Ar16 |
| 1346 | D267 | D74 | Ar16 |
| 1347 | D268 | D74 | Ar16 |
| 1348 | D269 | D74 | Ar16 |
| 1349 | D296 | D74 | Ar16 |
| 1350 | D298 | D74 | Ar16 |
| 1351 | D15 | D75 | Ar16 |
| 1352 | D16 | D75 | Ar16 |
| 1353 | D18 | D75 | Ar16 |
| 1354 | D21 | D75 | Ar16 |
| 1355 | D33 | D75 | Ar16 |
| 1356 | D34 | D75 | Ar16 |
| 1357 | D36 | D75 | Ar16 |
| 1358 | D45 | D75 | Ar16 |
| 1359 | D46 | D75 | Ar16 |
| 1360 | D48 | D75 | Ar16 |
| 1361 | D63 | D75 | Ar16 |
| 1362 | D64 | D75 | Ar16 |
| 1363 | D66 | D75 | Ar16 |
| 1364 | D73 | D75 | Ar16 |
| 1365 | D74 | D75 | Ar16 |
| 1366 | D75 | D75 | Ar16 |
| 1367 | D76 | D75 | Ar16 |
| 1368 | D78 | D75 | Ar16 |
| 1369 | D155 | D75 | Ar16 |
| 1370 | D182 | D75 | Ar16 |
| 1371 | D183 | D75 | Ar16 |
| 1372 | D185 | D75 | Ar16 |
| 1373 | D246 | D75 | Ar16 |
| 1374 | D265 | D75 | Ar16 |
| 1375 | D266 | D75 | Ar16 |
| 1376 | D267 | D75 | Ar16 |
| 1377 | D268 | D75 | Ar16 |
| 1378 | D269 | D75 | Ar16 |
| 1379 | D296 | D75 | Ar16 |
| 1380 | D298 | D75 | Ar16 |

| No. | $R^1$ = H | | |
|---|---|---|---|
| | $R^2$ | $R^3$ | $R^4$ |
| 1381 | D15 | D76 | Ar16 |
| 1382 | D16 | D76 | Ar16 |
| 1383 | D18 | D76 | Ar16 |
| 1384 | D21 | D76 | Ar16 |
| 1385 | D33 | D76 | Ar16 |
| 1386 | D34 | D76 | Ar16 |
| 1387 | D36 | D76 | Ar16 |
| 1388 | D45 | D76 | Ar16 |
| 1389 | D46 | D76 | Ar16 |
| 1390 | D48 | D76 | Ar16 |
| 1391 | D63 | D76 | Ar16 |
| 1392 | D64 | D76 | Ar16 |
| 1393 | D66 | D76 | Ar16 |
| 1394 | D73 | D76 | Ar16 |
| 1395 | D74 | D76 | Ar16 |
| 1396 | D75 | D76 | Ar16 |
| 1397 | D76 | D76 | Ar16 |
| 1398 | D78 | D76 | Ar16 |
| 1399 | D155 | D76 | Ar16 |
| 1400 | D182 | D76 | Ar16 |
| 1401 | D183 | D76 | Ar16 |
| 1402 | D185 | D76 | Ar16 |
| 1403 | D246 | D76 | Ar16 |
| 1404 | D265 | D76 | Ar16 |
| 1405 | D266 | D76 | Ar16 |
| 1406 | D267 | D76 | Ar16 |
| 1407 | D268 | D76 | Ar16 |
| 1408 | D269 | D76 | Ar16 |
| 1409 | D296 | D76 | Ar16 |
| 1410 | D298 | D76 | Ar16 |
| 1411 | D15 | D78 | Ar16 |
| 1412 | D16 | D78 | Ar16 |
| 1413 | D18 | D78 | Ar16 |
| 1414 | D21 | D78 | Ar16 |
| 1415 | D33 | D78 | Ar16 |
| 1416 | D34 | D78 | Ar16 |
| 1417 | D36 | D78 | Ar16 |
| 1418 | D45 | D78 | Ar16 |
| 1419 | D46 | D78 | Ar16 |
| 1420 | D48 | D78 | Ar16 |
| 1421 | D63 | D78 | Ar16 |
| 1422 | D64 | D78 | Ar16 |
| 1423 | D66 | D78 | Ar16 |
| 1424 | D73 | D78 | Ar16 |
| 1425 | D74 | D78 | Ar16 |
| 1426 | D75 | D78 | Ar16 |
| 1427 | D76 | D78 | Ar16 |
| 1428 | D78 | D78 | Ar16 |
| 1429 | D155 | D78 | Ar16 |
| 1430 | D182 | D78 | Ar16 |
| 1431 | D183 | D78 | Ar16 |
| 1432 | D185 | D78 | Ar16 |
| 1433 | D246 | D78 | Ar16 |
| 1434 | D265 | D78 | Ar16 |
| 1435 | D266 | D78 | Ar16 |
| 1436 | D267 | D78 | Ar16 |
| 1437 | D268 | D78 | Ar16 |
| 1438 | D269 | D78 | Ar16 |
| 1439 | D296 | D78 | Ar16 |
| 1440 | D298 | D78 | Ar16 |

| No. | $R^1$ = H | | |
|---|---|---|---|
| | $R^2$ | $R^3$ | $R^4$ |
| 1441 | D15 | D155 | Ar16 |
| 1442 | D16 | | |
| 1443 | D18 | | |
| 1444 | D21 | | |
| 1445 | D33 | | |
| 1446 | D34 | | |
| 1447 | D36 | | |
| 1448 | D45 | | |
| 1449 | D46 | | |
| 1450 | D48 | | |
| 1451 | D63 | | |
| 1452 | D64 | | |
| 1453 | D66 | | |
| 1454 | D73 | | |
| 1455 | D74 | | |
| 1456 | D75 | | |
| 1457 | D76 | | |
| 1458 | D78 | | |
| 1459 | D155 | | |
| 1460 | D182 | | |
| 1461 | D183 | | |
| 1462 | D185 | | |
| 1463 | D246 | | |
| 1464 | D265 | | |
| 1465 | D266 | | |
| 1466 | D267 | | |
| 1467 | D268 | | |
| 1468 | D269 | | |
| 1469 | D296 | | |
| 1470 | D298 | | |
| 1471 | D15 | D182 | |
| 1472 | D16 | | |
| 1473 | D18 | | |
| 1474 | D21 | | |
| 1475 | D33 | | |
| 1476 | D34 | | |
| 1477 | D36 | | |
| 1478 | D45 | | |
| 1479 | D46 | | |
| 1480 | D48 | | |
| 1481 | D63 | | |
| 1482 | D64 | | |
| 1483 | D66 | | |
| 1484 | D73 | | |
| 1485 | D74 | | |
| 1486 | D75 | | |
| 1487 | D76 | | |
| 1488 | D78 | | |
| 1489 | D155 | | |
| 1490 | D182 | | |
| 1491 | D183 | | |
| 1492 | D185 | | |
| 1493 | D246 | | |
| 1494 | D265 | | |
| 1495 | D266 | | |
| 1496 | D267 | | |
| 1497 | D268 | | |
| 1498 | D269 | | |
| 1499 | D296 | | |
| 1500 | D298 | | |

| No. | $R^1$ = H | | |
|---|---|---|---|
| | $R^2$ | $R^3$ | $R^4$ |
| 1501 | D15 | D183 | Ar16 |
| 1502 | D16 | | |
| 1503 | D18 | | |
| 1504 | D21 | | |
| 1505 | D33 | | |
| 1506 | D34 | | |
| 1507 | D36 | | |
| 1508 | D45 | | |
| 1509 | D46 | | |
| 1510 | D48 | | |
| 1511 | D63 | | |
| 1512 | D64 | | |
| 1513 | D66 | | |
| 1514 | D73 | | |
| 1515 | D74 | | |
| 1516 | D75 | | |
| 1517 | D76 | | |
| 1518 | D78 | | |
| 1519 | D155 | | |
| 1520 | D182 | | |
| 1521 | D183 | | |
| 1522 | D185 | | |
| 1523 | D246 | | |
| 1524 | D265 | | |
| 1525 | D266 | | |
| 1526 | D267 | | |
| 1527 | D268 | | |
| 1528 | D269 | | |
| 1529 | D296 | | |
| 1530 | D298 | | |
| 1531 | D15 | D185 | |
| 1532 | D16 | | |
| 1533 | D18 | | |
| 1534 | D21 | | |
| 1535 | D33 | | |
| 1536 | D34 | | |
| 1537 | D36 | | |
| 1538 | D45 | | |
| 1539 | D46 | | |
| 1540 | D48 | | |
| 1541 | D63 | | |
| 1542 | D64 | | |
| 1543 | D66 | | |
| 1544 | D73 | | |
| 1545 | D74 | | |
| 1546 | D75 | | |
| 1547 | D76 | | |
| 1548 | D78 | | |
| 1549 | D155 | | |
| 1550 | D182 | | |
| 1551 | D183 | | |
| 1552 | D185 | | |
| 1553 | D246 | | |
| 1554 | D265 | | |
| 1555 | D266 | | |
| 1556 | D267 | | |
| 1557 | D268 | | |
| 1558 | D269 | | |
| 1559 | D296 | | |
| 1560 | D298 | | |

| No. | $R^1$ = H | | |
|---|---|---|---|
| | $R^2$ | $R^3$ | $R^4$ |
| 1561 | D15 | D246 | Ar16 |
| 1562 | D16 | | |
| 1563 | D18 | | |
| 1564 | D21 | | |
| 1565 | D33 | | |
| 1566 | D34 | | |
| 1567 | D36 | | |
| 1568 | D45 | | |
| 1569 | D46 | | |
| 1570 | D48 | | |
| 1571 | D63 | | |
| 1572 | D64 | | |
| 1573 | D66 | | |
| 1574 | D73 | | |
| 1575 | D74 | | |
| 1576 | D75 | | |
| 1577 | D76 | | |
| 1578 | D78 | | |
| 1579 | D155 | | |
| 1580 | D182 | | |
| 1581 | D183 | | |
| 1582 | D185 | | |
| 1583 | D246 | | |
| 1584 | D265 | | |
| 1585 | D266 | | |
| 1586 | D267 | | |
| 1587 | D268 | | |
| 1588 | D269 | | |
| 1589 | D296 | | |
| 1590 | D298 | | |
| 1591 | D15 | D265 | |
| 1592 | D16 | | |
| 1593 | D18 | | |
| 1594 | D21 | | |
| 1595 | D33 | | |
| 1596 | D34 | | |
| 1597 | D36 | | |
| 1598 | D45 | | |
| 1599 | D46 | | |
| 1600 | D48 | | |
| 1601 | D63 | | |
| 1602 | D64 | | |
| 1603 | D66 | | |
| 1604 | D73 | | |
| 1605 | D74 | | |
| 1606 | D75 | | |
| 1607 | D76 | | |
| 1608 | D78 | | |
| 1609 | D155 | | |
| 1610 | D182 | | |
| 1611 | D183 | | |
| 1612 | D185 | | |
| 1613 | D246 | | |
| 1614 | D265 | | |
| 1615 | D266 | | |
| 1616 | D267 | | |
| 1617 | D268 | | |
| 1618 | D269 | | |
| 1619 | D296 | | |
| 1620 | D298 | | |

| No. | $R^1$ = H | | |
|---|---|---|---|
| | $R^2$ | $R^3$ | $R^4$ |
| 1621 | D15 | D266 | Ar16 |
| 1622 | D16 | | |
| 1623 | D18 | | |
| 1624 | D21 | | |
| 1625 | D33 | | |
| 1626 | D34 | | |
| 1627 | D36 | | |
| 1628 | D45 | | |
| 1629 | D46 | | |
| 1630 | D48 | | |
| 1631 | D63 | | |
| 1632 | D64 | | |
| 1633 | D66 | | |
| 1634 | D73 | | |
| 1635 | D74 | | |
| 1636 | D75 | | |
| 1637 | D76 | | |
| 1638 | D78 | | |
| 1639 | D155 | | |
| 1640 | D182 | | |
| 1641 | D183 | | |
| 1642 | D185 | | |
| 1643 | D246 | | |
| 1644 | D265 | | |
| 1645 | D266 | | |
| 1646 | D267 | | |
| 1647 | D268 | | |
| 1648 | D269 | | |
| 1649 | D296 | | |
| 1650 | D298 | | |
| 1651 | D15 | D267 | |
| 1652 | D16 | | |
| 1653 | D18 | | |
| 1654 | D21 | | |
| 1655 | D33 | | |
| 1656 | D34 | | |
| 1657 | D36 | | |
| 1658 | D45 | | |
| 1659 | D46 | | |
| 1660 | D48 | | |
| 1661 | D63 | | |
| 1662 | D64 | | |
| 1663 | D66 | | |
| 1664 | D73 | | |
| 1665 | D74 | | |
| 1666 | D75 | | |
| 1667 | D76 | | |
| 1668 | D78 | | |
| 1669 | D155 | | |
| 1670 | D182 | | |
| 1671 | D183 | | |
| 1672 | D185 | | |
| 1673 | D246 | | |
| 1674 | D265 | | |
| 1675 | D266 | | |
| 1676 | D267 | | |
| 1677 | D268 | | |
| 1678 | D269 | | |
| 1679 | D296 | | |
| 1680 | D298 | | |

| No. | $R^1$ = H | | |
|---|---|---|---|
| | $R^2$ | $R^3$ | $R^4$ |
| 1681 | D15 | D268 | Ar16 |
| 1682 | D16 | | |
| 1683 | D18 | | |
| 1684 | D21 | | |
| 1685 | D33 | | |
| 1686 | D34 | | |
| 1687 | D36 | | |
| 1688 | D45 | | |
| 1689 | D46 | | |
| 1690 | D48 | | |
| 1691 | D63 | | |
| 1692 | D64 | | |
| 1693 | D66 | | |
| 1694 | D73 | | |
| 1695 | D74 | | |
| 1696 | D75 | | |
| 1697 | D76 | | |
| 1698 | D78 | | |
| 1699 | D155 | | |
| 1700 | D182 | | |
| 1701 | D183 | | |
| 1702 | D185 | | |
| 1703 | D246 | | |
| 1704 | D265 | | |
| 1705 | D266 | | |
| 1706 | D267 | | |
| 1707 | D268 | | |
| 1708 | D269 | | |
| 1709 | D296 | | |
| 1710 | D298 | | |
| 1711 | D15 | D269 | |
| 1712 | D16 | | |
| 1713 | D18 | | |
| 1714 | D21 | | |
| 1715 | D33 | | |
| 1716 | D34 | | |
| 1717 | D36 | | |
| 1718 | D45 | | |
| 1719 | D46 | | |
| 1720 | D48 | | |
| 1721 | D63 | | |
| 1722 | D64 | | |
| 1723 | D66 | | |
| 1724 | D73 | | |
| 1725 | D74 | | |
| 1726 | D75 | | |
| 1727 | D76 | | |
| 1728 | D78 | | |
| 1729 | D155 | | |
| 1730 | D182 | | |
| 1731 | D183 | | |
| 1732 | D185 | | |
| 1733 | D246 | | |
| 1734 | D265 | | |
| 1735 | D266 | | |
| 1736 | D267 | | |
| 1737 | D268 | | |
| 1738 | D269 | | |
| 1739 | D296 | | |
| 1740 | D298 | | |

| No. | $R^1$ = H | | |
|---|---|---|---|
| | $R^2$ | $R^3$ | $R^4$ |
| 1741 | D15 | D296 | Ar16 |
| 1742 | D16 | | |
| 1743 | D18 | | |
| 1744 | D21 | | |
| 1745 | D33 | | |
| 1746 | D34 | | |
| 1747 | D36 | | |
| 1748 | D45 | | |
| 1749 | D46 | | |
| 1750 | D48 | | |
| 1751 | D63 | | |
| 1752 | D64 | | |
| 1753 | D66 | | |
| 1754 | D73 | | |
| 1755 | D74 | | |
| 1756 | D75 | | |
| 1757 | D76 | | |
| 1758 | D78 | | |
| 1759 | D155 | | |
| 1760 | D182 | | |
| 1761 | D183 | | |
| 1762 | D185 | | |
| 1763 | D246 | | |
| 1764 | D265 | | |
| 1765 | D266 | | |
| 1766 | D267 | | |
| 1767 | D268 | | |
| 1768 | D269 | | |
| 1769 | D296 | | |
| 1770 | D298 | | |
| 1771 | D15 | D298 | |
| 1772 | D16 | | |
| 1773 | D18 | | |
| 1774 | D21 | | |
| 1775 | D33 | | |
| 1776 | D34 | | |
| 1777 | D36 | | |
| 1778 | D45 | | |
| 1779 | D46 | | |
| 1780 | D48 | | |
| 1781 | D63 | | |
| 1782 | D64 | | |
| 1783 | D66 | | |
| 1784 | D73 | | |
| 1785 | D74 | | |
| 1786 | D75 | | |
| 1787 | D76 | | |
| 1788 | D78 | | |
| 1789 | D155 | | |
| 1790 | D182 | | |
| 1791 | D183 | | |
| 1792 | D185 | | |
| 1793 | D246 | | |
| 1794 | D265 | | |
| 1795 | D266 | | |
| 1796 | D267 | | |
| 1797 | D268 | | |
| 1798 | D269 | | |
| 1799 | D296 | | |
| 1800 | D298 | | |

| No. | $R^1$ = H | | |
|---|---|---|---|
| | $R^2$ | $R^3$ | $R^4$ |
| 1801 | D15 | D15 | Ar21 |
| 1802 | D16 | | |
| 1803 | D18 | | |
| 1804 | D21 | | |
| 1805 | D33 | | |
| 1806 | D34 | | |
| 1807 | D36 | | |
| 1808 | D45 | | |
| 1809 | D46 | | |
| 1810 | D48 | | |
| 1811 | D63 | | |
| 1812 | D64 | | |
| 1813 | D66 | | |
| 1814 | D73 | | |
| 1815 | D74 | | |
| 1816 | D75 | | |
| 1817 | D76 | | |
| 1818 | D78 | | |
| 1819 | D155 | | |
| 1820 | D182 | | |
| 1821 | D183 | | |
| 1822 | D185 | | |
| 1823 | D246 | | |
| 1824 | D265 | | |
| 1825 | D266 | | |
| 1826 | D267 | | |
| 1827 | D268 | | |
| 1828 | D269 | | |
| 1829 | D296 | | |
| 1830 | D298 | | |
| 1831 | D15 | D16 | |
| 1832 | D16 | | |
| 1833 | D18 | | |
| 1834 | D21 | | |
| 1835 | D33 | | |
| 1836 | D34 | | |
| 1837 | D36 | | |
| 1838 | D45 | | |
| 1839 | D46 | | |
| 1840 | D48 | | |
| 1841 | D63 | | |
| 1842 | D64 | | |
| 1843 | D66 | | |
| 1844 | D73 | | |
| 1845 | D74 | | |
| 1846 | D75 | | |
| 1847 | D76 | | |
| 1848 | D78 | | |
| 1849 | D155 | | |
| 1850 | D182 | | |
| 1851 | D183 | | |
| 1852 | D185 | | |
| 1853 | D246 | | |
| 1854 | D265 | | |
| 1855 | D266 | | |
| 1856 | D267 | | |
| 1857 | D268 | | |
| 1858 | D269 | | |
| 1859 | D296 | | |
| 1860 | D298 | | |

| No. | $R^1$ = H | | |
|---|---|---|---|
| | $R^2$ | $R^3$ | $R^4$ |
| 1861 | D15 | D18 | Ar21 |
| 1862 | D16 | | |
| 1863 | D18 | | |
| 1864 | D21 | | |
| 1865 | D33 | | |
| 1866 | D34 | | |
| 1867 | D36 | | |
| 1868 | D45 | | |
| 1869 | D46 | | |
| 1870 | D48 | | |
| 1871 | D63 | | |
| 1872 | D64 | | |
| 1873 | D66 | | |
| 1874 | D73 | | |
| 1875 | D74 | | |
| 1876 | D75 | | |
| 1877 | D76 | | |
| 1878 | D78 | | |
| 1879 | D155 | | |
| 1880 | D182 | | |
| 1881 | D183 | | |
| 1882 | D185 | | |
| 1883 | D246 | | |
| 1884 | D265 | | |
| 1885 | D266 | | |
| 1886 | D267 | | |
| 1887 | D268 | | |
| 1888 | D269 | | |
| 1889 | D296 | | |
| 1890 | D298 | | |
| 1891 | D15 | D21 | |
| 1892 | D16 | | |
| 1893 | D18 | | |
| 1894 | D21 | | |
| 1895 | D33 | | |
| 1896 | D34 | | |
| 1897 | D36 | | |
| 1898 | D45 | | |
| 1899 | D46 | | |
| 1900 | D48 | | |
| 1901 | D63 | | |
| 1902 | D64 | | |
| 1903 | D66 | | |
| 1904 | D73 | | |
| 1905 | D74 | | |
| 1906 | D75 | | |
| 1907 | D76 | | |
| 1908 | D78 | | |
| 1909 | D155 | | |
| 1910 | D182 | | |
| 1911 | D183 | | |
| 1912 | D185 | | |
| 1913 | D246 | | |
| 1914 | D265 | | |
| 1915 | D266 | | |
| 1916 | D267 | | |
| 1917 | D268 | | |
| 1918 | D269 | | |
| 1919 | D296 | | |
| 1920 | D298 | | |

| No. | $R^1$ = H | | |
|---|---|---|---|
| | $R^2$ | $R^3$ | $R^4$ |
| 1921 | D15 | D33 | Ar21 |
| 1922 | D16 | D33 | Ar21 |
| 1923 | D18 | D33 | Ar21 |
| 1924 | D21 | D33 | Ar21 |
| 1925 | D33 | D33 | Ar21 |
| 1926 | D34 | D33 | Ar21 |
| 1927 | D36 | D33 | Ar21 |
| 1928 | D45 | D33 | Ar21 |
| 1929 | D46 | D33 | Ar21 |
| 1930 | D48 | D33 | Ar21 |
| 1931 | D63 | D33 | Ar21 |
| 1932 | D64 | D33 | Ar21 |
| 1933 | D66 | D33 | Ar21 |
| 1934 | D73 | D33 | Ar21 |
| 1935 | D74 | D33 | Ar21 |
| 1936 | D75 | D33 | Ar21 |
| 1937 | D76 | D33 | Ar21 |
| 1938 | D78 | D33 | Ar21 |
| 1939 | D155 | D33 | Ar21 |
| 1940 | D182 | D33 | Ar21 |
| 1941 | D183 | D33 | Ar21 |
| 1942 | D185 | D33 | Ar21 |
| 1943 | D246 | D33 | Ar21 |
| 1944 | D265 | D33 | Ar21 |
| 1945 | D266 | D33 | Ar21 |
| 1946 | D267 | D33 | Ar21 |
| 1947 | D268 | D33 | Ar21 |
| 1948 | D269 | D33 | Ar21 |
| 1949 | D296 | D33 | Ar21 |
| 1950 | D298 | D33 | Ar21 |
| 1951 | D15 | D34 | Ar21 |
| 1952 | D16 | D34 | Ar21 |
| 1953 | D18 | D34 | Ar21 |
| 1954 | D21 | D34 | Ar21 |
| 1955 | D33 | D34 | Ar21 |
| 1956 | D34 | D34 | Ar21 |
| 1957 | D36 | D34 | Ar21 |
| 1958 | D45 | D34 | Ar21 |
| 1959 | D46 | D34 | Ar21 |
| 1960 | D48 | D34 | Ar21 |
| 1961 | D63 | D34 | Ar21 |
| 1962 | D64 | D34 | Ar21 |
| 1963 | D66 | D34 | Ar21 |
| 1964 | D73 | D34 | Ar21 |
| 1965 | D74 | D34 | Ar21 |
| 1966 | D75 | D34 | Ar21 |
| 1967 | D76 | D34 | Ar21 |
| 1968 | D78 | D34 | Ar21 |
| 1969 | D155 | D34 | Ar21 |
| 1970 | D182 | D34 | Ar21 |
| 1971 | D183 | D34 | Ar21 |
| 1972 | D185 | D34 | Ar21 |
| 1973 | D246 | D34 | Ar21 |
| 1974 | D265 | D34 | Ar21 |
| 1975 | D266 | D34 | Ar21 |
| 1976 | D267 | D34 | Ar21 |
| 1977 | D268 | D34 | Ar21 |
| 1978 | D269 | D34 | Ar21 |
| 1979 | D296 | D34 | Ar21 |
| 1980 | D298 | D34 | Ar21 |

| No. | $R^1$ = H | | |
|---|---|---|---|
| | $R^2$ | $R^3$ | $R^4$ |
| 1981 | D15 | D36 | Ar21 |
| 1982 | D16 | D36 | Ar21 |
| 1983 | D18 | D36 | Ar21 |
| 1984 | D21 | D36 | Ar21 |
| 1985 | D33 | D36 | Ar21 |
| 1986 | D34 | D36 | Ar21 |
| 1987 | D36 | D36 | Ar21 |
| 1988 | D45 | D36 | Ar21 |
| 1989 | D46 | D36 | Ar21 |
| 1990 | D48 | D36 | Ar21 |
| 1991 | D63 | D36 | Ar21 |
| 1992 | D64 | D36 | Ar21 |
| 1993 | D66 | D36 | Ar21 |
| 1994 | D73 | D36 | Ar21 |
| 1995 | D74 | D36 | Ar21 |
| 1996 | D75 | D36 | Ar21 |
| 1997 | D76 | D36 | Ar21 |
| 1998 | D78 | D36 | Ar21 |
| 1999 | D155 | D36 | Ar21 |
| 2000 | D182 | D36 | Ar21 |
| 2001 | D183 | D36 | Ar21 |
| 2002 | D185 | D36 | Ar21 |
| 2003 | D246 | D36 | Ar21 |
| 2004 | D265 | D36 | Ar21 |
| 2005 | D266 | D36 | Ar21 |
| 2006 | D267 | D36 | Ar21 |
| 2007 | D268 | D36 | Ar21 |
| 2008 | D269 | D36 | Ar21 |
| 2009 | D296 | D36 | Ar21 |
| 2010 | D298 | D36 | Ar21 |
| 2011 | D15 | D45 | Ar21 |
| 2012 | D16 | D45 | Ar21 |
| 2013 | D18 | D45 | Ar21 |
| 2014 | D21 | D45 | Ar21 |
| 2015 | D33 | D45 | Ar21 |
| 2016 | D34 | D45 | Ar21 |
| 2017 | D36 | D45 | Ar21 |
| 2018 | D45 | D45 | Ar21 |
| 2019 | D46 | D45 | Ar21 |
| 2020 | D48 | D45 | Ar21 |
| 2021 | D63 | D45 | Ar21 |
| 2022 | D64 | D45 | Ar21 |
| 2023 | D66 | D45 | Ar21 |
| 2024 | D73 | D45 | Ar21 |
| 2025 | D74 | D45 | Ar21 |
| 2026 | D75 | D45 | Ar21 |
| 2027 | D76 | D45 | Ar21 |
| 2028 | D78 | D45 | Ar21 |
| 2029 | D155 | D45 | Ar21 |
| 2030 | D182 | D45 | Ar21 |
| 2031 | D183 | D45 | Ar21 |
| 2032 | D185 | D45 | Ar21 |
| 2033 | D246 | D45 | Ar21 |
| 2034 | D265 | D45 | Ar21 |
| 2035 | D266 | D45 | Ar21 |
| 2036 | D267 | D45 | Ar21 |
| 2037 | D268 | D45 | Ar21 |
| 2038 | D269 | D45 | Ar21 |
| 2039 | D296 | D45 | Ar21 |
| 2040 | D298 | D45 | Ar21 |

| No. | $R^1$ = H | | |
|---|---|---|---|
| | $R^2$ | $R^3$ | $R^4$ |
| 2041 | D15 | D46 | Ar21 |
| 2042 | D16 | D46 | Ar21 |
| 2043 | D18 | D46 | Ar21 |
| 2044 | D21 | D46 | Ar21 |
| 2045 | D33 | D46 | Ar21 |
| 2046 | D34 | D46 | Ar21 |
| 2047 | D36 | D46 | Ar21 |
| 2048 | D45 | D46 | Ar21 |
| 2049 | D46 | D46 | Ar21 |
| 2050 | D48 | D46 | Ar21 |
| 2051 | D63 | D46 | Ar21 |
| 2052 | D64 | D46 | Ar21 |
| 2053 | D66 | D46 | Ar21 |
| 2054 | D73 | D46 | Ar21 |
| 2055 | D74 | D46 | Ar21 |
| 2056 | D75 | D46 | Ar21 |
| 2057 | D76 | D46 | Ar21 |
| 2058 | D78 | D46 | Ar21 |
| 2059 | D155 | D46 | Ar21 |
| 2060 | D182 | D46 | Ar21 |
| 2061 | D183 | D46 | Ar21 |
| 2062 | D185 | D46 | Ar21 |
| 2063 | D246 | D46 | Ar21 |
| 2064 | D265 | D46 | Ar21 |
| 2065 | D266 | D46 | Ar21 |
| 2066 | D267 | D46 | Ar21 |
| 2067 | D268 | D46 | Ar21 |
| 2068 | D269 | D46 | Ar21 |
| 2069 | D296 | D46 | Ar21 |
| 2070 | D298 | D46 | Ar21 |
| 2071 | D15 | D48 | Ar21 |
| 2072 | D16 | D48 | Ar21 |
| 2073 | D18 | D48 | Ar21 |
| 2074 | D21 | D48 | Ar21 |
| 2075 | D33 | D48 | Ar21 |
| 2076 | D34 | D48 | Ar21 |
| 2077 | D36 | D48 | Ar21 |
| 2078 | D45 | D48 | Ar21 |
| 2079 | D46 | D48 | Ar21 |
| 2080 | D48 | D48 | Ar21 |
| 2081 | D63 | D48 | Ar21 |
| 2082 | D64 | D48 | Ar21 |
| 2083 | D66 | D48 | Ar21 |
| 2084 | D73 | D48 | Ar21 |
| 2085 | D74 | D48 | Ar21 |
| 2086 | D75 | D48 | Ar21 |
| 2087 | D76 | D48 | Ar21 |
| 2088 | D78 | D48 | Ar21 |
| 2089 | D155 | D48 | Ar21 |
| 2090 | D182 | D48 | Ar21 |
| 2091 | D183 | D48 | Ar21 |
| 2092 | D185 | D48 | Ar21 |
| 2093 | D246 | D48 | Ar21 |
| 2094 | D265 | D48 | Ar21 |
| 2095 | D266 | D48 | Ar21 |
| 2096 | D267 | D48 | Ar21 |
| 2097 | D268 | D48 | Ar21 |
| 2098 | D269 | D48 | Ar21 |
| 2099 | D296 | D48 | Ar21 |
| 2100 | D298 | D48 | Ar21 |

| No. | $R^1$ = H | | |
|---|---|---|---|
| | $R^2$ | $R^3$ | $R^4$ |
| 2101 | D15 | D63 | Ar21 |
| 2102 | D16 | D63 | Ar21 |
| 2103 | D18 | D63 | Ar21 |
| 2104 | D21 | D63 | Ar21 |
| 2105 | D33 | D63 | Ar21 |
| 2106 | D34 | D63 | Ar21 |
| 2107 | D36 | D63 | Ar21 |
| 2108 | D45 | D63 | Ar21 |
| 2109 | D46 | D63 | Ar21 |
| 2110 | D48 | D63 | Ar21 |
| 2111 | D63 | D63 | Ar21 |
| 2112 | D64 | D63 | Ar21 |
| 2113 | D66 | D63 | Ar21 |
| 2114 | D73 | D63 | Ar21 |
| 2115 | D74 | D63 | Ar21 |
| 2116 | D75 | D63 | Ar21 |
| 2117 | D76 | D63 | Ar21 |
| 2118 | D78 | D63 | Ar21 |
| 2119 | D155 | D63 | Ar21 |
| 2120 | D182 | D63 | Ar21 |
| 2121 | D183 | D63 | Ar21 |
| 2122 | D185 | D63 | Ar21 |
| 2123 | D246 | D63 | Ar21 |
| 2124 | D265 | D63 | Ar21 |
| 2125 | D266 | D63 | Ar21 |
| 2126 | D267 | D63 | Ar21 |
| 2127 | D268 | D63 | Ar21 |
| 2128 | D269 | D63 | Ar21 |
| 2129 | D296 | D63 | Ar21 |
| 2130 | D298 | D63 | Ar21 |
| 2131 | D15 | D64 | Ar21 |
| 2132 | D16 | D64 | Ar21 |
| 2133 | D18 | D64 | Ar21 |
| 2134 | D21 | D64 | Ar21 |
| 2135 | D33 | D64 | Ar21 |
| 2136 | D34 | D64 | Ar21 |
| 2137 | D36 | D64 | Ar21 |
| 2138 | D45 | D64 | Ar21 |
| 2139 | D46 | D64 | Ar21 |
| 2140 | D48 | D64 | Ar21 |
| 2141 | D63 | D64 | Ar21 |
| 2142 | D64 | D64 | Ar21 |
| 2143 | D66 | D64 | Ar21 |
| 2144 | D73 | D64 | Ar21 |
| 2145 | D74 | D64 | Ar21 |
| 2146 | D75 | D64 | Ar21 |
| 2147 | D76 | D64 | Ar21 |
| 2148 | D78 | D64 | Ar21 |
| 2149 | D155 | D64 | Ar21 |
| 2150 | D182 | D64 | Ar21 |
| 2151 | D183 | D64 | Ar21 |
| 2152 | D185 | D64 | Ar21 |
| 2153 | D246 | D64 | Ar21 |
| 2154 | D265 | D64 | Ar21 |
| 2155 | D266 | D64 | Ar21 |
| 2156 | D267 | D64 | Ar21 |
| 2157 | D268 | D64 | Ar21 |
| 2158 | D269 | D64 | Ar21 |
| 2159 | D296 | D64 | Ar21 |
| 2160 | D298 | D64 | Ar21 |

| No. | $R^1$ = H | | |
|---|---|---|---|
| | $R^2$ | $R^3$ | $R^4$ |
| 2161 | D15 | D66 | Ar21 |
| 2162 | D16 | | |
| 2163 | D18 | | |
| 2164 | D21 | | |
| 2165 | D33 | | |
| 2166 | D34 | | |
| 2167 | D36 | | |
| 2168 | D45 | | |
| 2169 | D46 | | |
| 2170 | D48 | | |
| 2171 | D63 | | |
| 2172 | D64 | | |
| 2173 | D66 | | |
| 2174 | D73 | | |
| 2175 | D74 | | |
| 2176 | D75 | | |
| 2177 | D76 | | |
| 2178 | D78 | | |
| 2179 | D155 | | |
| 2180 | D182 | | |
| 2181 | D183 | | |
| 2182 | D185 | | |
| 2183 | D246 | | |
| 2184 | D265 | | |
| 2185 | D266 | | |
| 2186 | D267 | | |
| 2187 | D268 | | |
| 2188 | D269 | | |
| 2189 | D296 | | |
| 2190 | D298 | | |
| 2191 | D15 | D73 | |
| 2192 | D16 | | |
| 2193 | D18 | | |
| 2194 | D21 | | |
| 2195 | D33 | | |
| 2196 | D34 | | |
| 2197 | D36 | | |
| 2198 | D45 | | |
| 2199 | D46 | | |
| 2200 | D48 | | |
| 2201 | D63 | | |
| 2202 | D64 | | |
| 2203 | D66 | | |
| 2204 | D73 | | |
| 2205 | D74 | | |
| 2206 | D75 | | |
| 2207 | D76 | | |
| 2208 | D78 | | |
| 2209 | D155 | | |
| 2210 | D182 | | |
| 2211 | D183 | | |
| 2212 | D185 | | |
| 2213 | D246 | | |
| 2214 | D265 | | |
| 2215 | D266 | | |
| 2216 | D267 | | |
| 2217 | D268 | | |
| 2218 | D269 | | |
| 2219 | D296 | | |
| 2220 | D298 | | |

| No. | $R^1$ = H | | |
|---|---|---|---|
| | $R^2$ | $R^3$ | $R^4$ |
| 2221 | D15 | D74 | Ar21 |
| 2222 | D16 | | |
| 2223 | D18 | | |
| 2224 | D21 | | |
| 2225 | D33 | | |
| 2226 | D34 | | |
| 2227 | D36 | | |
| 2228 | D45 | | |
| 2229 | D46 | | |
| 2230 | D48 | | |
| 2231 | D63 | | |
| 2232 | D64 | | |
| 2233 | D66 | | |
| 2234 | D73 | | |
| 2235 | D74 | | |
| 2236 | D75 | | |
| 2237 | D76 | | |
| 2238 | D78 | | |
| 2239 | D155 | | |
| 2240 | D182 | | |
| 2241 | D183 | | |
| 2242 | D185 | | |
| 2243 | D246 | | |
| 2244 | D265 | | |
| 2245 | D266 | | |
| 2246 | D267 | | |
| 2247 | D268 | | |
| 2248 | D269 | | |
| 2249 | D296 | | |
| 2250 | D298 | | |
| 2251 | D15 | D75 | |
| 2252 | D16 | | |
| 2253 | D18 | | |
| 2254 | D21 | | |
| 2255 | D33 | | |
| 2256 | D34 | | |
| 2257 | D36 | | |
| 2258 | D45 | | |
| 2259 | D46 | | |
| 2260 | D48 | | |
| 2261 | D63 | | |
| 2262 | D64 | | |
| 2263 | D66 | | |
| 2264 | D73 | | |
| 2265 | D74 | | |
| 2266 | D75 | | |
| 2267 | D76 | | |
| 2268 | D78 | | |
| 2269 | D155 | | |
| 2270 | D182 | | |
| 2271 | D183 | | |
| 2272 | D185 | | |
| 2273 | D246 | | |
| 2274 | D265 | | |
| 2275 | D266 | | |
| 2276 | D267 | | |
| 2277 | D268 | | |
| 2278 | D269 | | |
| 2279 | D296 | | |
| 2280 | D298 | | |

| No. | $R^1$ = H | | |
|---|---|---|---|
| | $R^2$ | $R^3$ | $R^4$ |
| 2281 | D15 | D76 | Ar21 |
| 2282 | D16 | | |
| 2283 | D18 | | |
| 2284 | D21 | | |
| 2285 | D33 | | |
| 2286 | D34 | | |
| 2287 | D36 | | |
| 2288 | D45 | | |
| 2289 | D46 | | |
| 2290 | D48 | | |
| 2291 | D63 | | |
| 2292 | D64 | | |
| 2293 | D66 | | |
| 2294 | D73 | | |
| 2295 | D74 | | |
| 2296 | D75 | | |
| 2297 | D76 | | |
| 2298 | D78 | | |
| 2299 | D155 | | |
| 2300 | D182 | | |
| 2301 | D183 | | |
| 2302 | D185 | | |
| 2303 | D246 | | |
| 2304 | D265 | | |
| 2305 | D266 | | |
| 2306 | D267 | | |
| 2307 | D268 | | |
| 2308 | D269 | | |
| 2309 | D296 | | |
| 2310 | D298 | | |
| 2311 | D15 | D78 | |
| 2312 | D16 | | |
| 2313 | D18 | | |
| 2314 | D21 | | |
| 2315 | D33 | | |
| 2316 | D34 | | |
| 2317 | D36 | | |
| 2318 | D45 | | |
| 2319 | D46 | | |
| 2320 | D48 | | |
| 2321 | D63 | | |
| 2322 | D64 | | |
| 2323 | D66 | | |
| 2324 | D73 | | |
| 2325 | D74 | | |
| 2326 | D75 | | |
| 2327 | D76 | | |
| 2328 | D78 | | |
| 2329 | D155 | | |
| 2330 | D182 | | |
| 2331 | D183 | | |
| 2332 | D185 | | |
| 2333 | D246 | | |
| 2334 | D265 | | |
| 2335 | D266 | | |
| 2336 | D267 | | |
| 2337 | D268 | | |
| 2338 | D269 | | |
| 2339 | D296 | | |
| 2340 | D298 | | |

| No. | $R^1$ = H | | |
|---|---|---|---|
| | $R^2$ | $R^3$ | $R^4$ |
| 2341 | D15 | D155 | Ar21 |
| 2342 | D16 | | |
| 2343 | D18 | | |
| 2344 | D21 | | |
| 2345 | D33 | | |
| 2346 | D34 | | |
| 2347 | D36 | | |
| 2348 | D45 | | |
| 2349 | D46 | | |
| 2350 | D48 | | |
| 2351 | D63 | | |
| 2352 | D64 | | |
| 2353 | D66 | | |
| 2354 | D73 | | |
| 2355 | D74 | | |
| 2356 | D75 | | |
| 2357 | D76 | | |
| 2358 | D78 | | |
| 2359 | D155 | | |
| 2360 | D182 | | |
| 2361 | D183 | | |
| 2362 | D185 | | |
| 2363 | D246 | | |
| 2364 | D265 | | |
| 2365 | D266 | | |
| 2366 | D267 | | |
| 2367 | D268 | | |
| 2368 | D269 | | |
| 2369 | D296 | | |
| 2370 | D298 | | |
| 2371 | D15 | D182 | |
| 2372 | D16 | | |
| 2373 | D18 | | |
| 2374 | D21 | | |
| 2375 | D33 | | |
| 2376 | D34 | | |
| 2377 | D36 | | |
| 2378 | D45 | | |
| 2379 | D46 | | |
| 2380 | D48 | | |
| 2381 | D63 | | |
| 2382 | D64 | | |
| 2383 | D66 | | |
| 2384 | D73 | | |
| 2385 | D74 | | |
| 2386 | D75 | | |
| 2387 | D76 | | |
| 2388 | D78 | | |
| 2389 | D155 | | |
| 2390 | D182 | | |
| 2391 | D183 | | |
| 2392 | D185 | | |
| 2393 | D246 | | |
| 2394 | D265 | | |
| 2395 | D266 | | |
| 2396 | D267 | | |
| 2397 | D268 | | |
| 2398 | D269 | | |
| 2399 | D296 | | |
| 2400 | D298 | | |

| No. | $R^1$ = H | | |
|---|---|---|---|
| | $R^2$ | $R^3$ | $R^4$ |
| 2401 | D15 | D183 | Ar21 |
| 2402 | D16 | | |
| 2403 | D18 | | |
| 2404 | D21 | | |
| 2405 | D33 | | |
| 2406 | D34 | | |
| 2407 | D36 | | |
| 2408 | D45 | | |
| 2409 | D46 | | |
| 2410 | D48 | | |
| 2411 | D63 | | |
| 2412 | D64 | | |
| 2413 | D66 | | |
| 2414 | D73 | | |
| 2415 | D74 | | |
| 2416 | D75 | | |
| 2417 | D76 | | |
| 2418 | D78 | | |
| 2419 | D155 | | |
| 2420 | D182 | | |
| 2421 | D183 | | |
| 2422 | D185 | | |
| 2423 | D246 | | |
| 2424 | D265 | | |
| 2425 | D266 | | |
| 2426 | D267 | | |
| 2427 | D268 | | |
| 2428 | D269 | | |
| 2429 | D296 | | |
| 2430 | D298 | | |
| 2431 | D15 | D185 | |
| 2432 | D16 | | |
| 2433 | D18 | | |
| 2434 | D21 | | |
| 2435 | D33 | | |
| 2436 | D34 | | |
| 2437 | D36 | | |
| 2438 | D45 | | |
| 2439 | D46 | | |
| 2440 | D48 | | |
| 2441 | D63 | | |
| 2442 | D64 | | |
| 2443 | D66 | | |
| 2444 | D73 | | |
| 2445 | D74 | | |
| 2446 | D75 | | |
| 2447 | D76 | | |
| 2448 | D78 | | |
| 2449 | D155 | | |
| 2450 | D182 | | |
| 2451 | D183 | | |
| 2452 | D185 | | |
| 2453 | D246 | | |
| 2454 | D265 | | |
| 2455 | D266 | | |
| 2456 | D267 | | |
| 2457 | D268 | | |
| 2458 | D269 | | |
| 2459 | D296 | | |
| 2460 | D298 | | |

| No. | $R^1$ = H | | |
|---|---|---|---|
| | $R^2$ | $R^3$ | $R^4$ |
| 2461 | D15 | D246 | Ar21 |
| 2462 | D16 | | |
| 2463 | D18 | | |
| 2464 | D21 | | |
| 2465 | D33 | | |
| 2466 | D34 | | |
| 2467 | D36 | | |
| 2468 | D45 | | |
| 2469 | D46 | | |
| 2470 | D48 | | |
| 2471 | D63 | | |
| 2472 | D64 | | |
| 2473 | D66 | | |
| 2474 | D73 | | |
| 2475 | D74 | | |
| 2476 | D75 | | |
| 2477 | D76 | | |
| 2478 | D78 | | |
| 2479 | D155 | | |
| 2480 | D182 | | |
| 2481 | D183 | | |
| 2482 | D185 | | |
| 2483 | D246 | | |
| 2484 | D265 | | |
| 2485 | D266 | | |
| 2486 | D267 | | |
| 2487 | D268 | | |
| 2488 | D269 | | |
| 2489 | D296 | | |
| 2490 | D298 | | |
| 2491 | D15 | D265 | |
| 2492 | D16 | | |
| 2493 | D18 | | |
| 2494 | D21 | | |
| 2495 | D33 | | |
| 2496 | D34 | | |
| 2497 | D36 | | |
| 2498 | D45 | | |
| 2499 | D46 | | |
| 2500 | D48 | | |
| 2501 | D63 | | |
| 2502 | D64 | | |
| 2503 | D66 | | |
| 2504 | D73 | | |
| 2505 | D74 | | |
| 2506 | D75 | | |
| 2507 | D76 | | |
| 2508 | D78 | | |
| 2509 | D155 | | |
| 2510 | D182 | | |
| 2511 | D183 | | |
| 2512 | D185 | | |
| 2513 | D246 | | |
| 2514 | D265 | | |
| 2515 | D266 | | |
| 2516 | D267 | | |
| 2517 | D268 | | |
| 2518 | D269 | | |
| 2519 | D296 | | |
| 2520 | D298 | | |

| No. | $R^1$ = H | | |
|---|---|---|---|
| | $R^2$ | $R^3$ | $R^4$ |
| 2521 | D15 | D266 | Ar21 |
| 2522 | D16 | | |
| 2523 | D18 | | |
| 2524 | D21 | | |
| 2525 | D33 | | |
| 2526 | D34 | | |
| 2527 | D36 | | |
| 2528 | D45 | | |
| 2529 | D46 | | |
| 2530 | D48 | | |
| 2531 | D63 | | |
| 2532 | D64 | | |
| 2533 | D66 | | |
| 2534 | D73 | | |
| 2535 | D74 | | |
| 2536 | D75 | | |
| 2537 | D76 | | |
| 2538 | D78 | | |
| 2539 | D155 | | |
| 2540 | D182 | | |
| 2541 | D183 | | |
| 2542 | D185 | | |
| 2543 | D246 | | |
| 2544 | D265 | | |
| 2545 | D266 | | |
| 2546 | D267 | | |
| 2547 | D268 | | |
| 2548 | D269 | | |
| 2549 | D296 | | |
| 2550 | D298 | | |
| 2551 | D15 | D267 | |
| 2552 | D16 | | |
| 2553 | D18 | | |
| 2554 | D21 | | |
| 2555 | D33 | | |
| 2556 | D34 | | |
| 2557 | D36 | | |
| 2558 | D45 | | |
| 2559 | D46 | | |
| 2560 | D48 | | |
| 2561 | D63 | | |
| 2562 | D64 | | |
| 2563 | D66 | | |
| 2564 | D73 | | |
| 2565 | D74 | | |
| 2566 | D75 | | |
| 2567 | D76 | | |
| 2568 | D78 | | |
| 2569 | D155 | | |
| 2570 | D182 | | |
| 2571 | D183 | | |
| 2572 | D185 | | |
| 2573 | D246 | | |
| 2574 | D265 | | |
| 2575 | D266 | | |
| 2576 | D267 | | |
| 2577 | D268 | | |
| 2578 | D269 | | |
| 2579 | D296 | | |
| 2580 | D298 | | |

| No. | $R^1$ = H | | |
|---|---|---|---|
| | $R^2$ | $R^3$ | $R^4$ |
| 2581 | D15 | D268 | Ar21 |
| 2582 | D16 | | |
| 2583 | D18 | | |
| 2584 | D21 | | |
| 2585 | D33 | | |
| 2586 | D34 | | |
| 2587 | D36 | | |
| 2588 | D45 | | |
| 2589 | D46 | | |
| 2590 | D48 | | |
| 2591 | D63 | | |
| 2592 | D64 | | |
| 2593 | D66 | | |
| 2594 | D73 | | |
| 2595 | D74 | | |
| 2596 | D75 | | |
| 2597 | D76 | | |
| 2598 | D78 | | |
| 2599 | D155 | | |
| 2600 | D182 | | |
| 2601 | D183 | | |
| 2602 | D185 | | |
| 2603 | D246 | | |
| 2604 | D265 | | |
| 2605 | D266 | | |
| 2606 | D267 | | |
| 2607 | D268 | | |
| 2608 | D269 | | |
| 2609 | D296 | | |
| 2610 | D298 | | |
| 2611 | D15 | D269 | |
| 2612 | D16 | | |
| 2613 | D18 | | |
| 2614 | D21 | | |
| 2615 | D33 | | |
| 2616 | D34 | | |
| 2617 | D36 | | |
| 2618 | D45 | | |
| 2619 | D46 | | |
| 2620 | D48 | | |
| 2621 | D63 | | |
| 2622 | D64 | | |
| 2623 | D66 | | |
| 2624 | D73 | | |
| 2625 | D74 | | |
| 2626 | D75 | | |
| 2627 | D76 | | |
| 2628 | D78 | | |
| 2629 | D155 | | |
| 2630 | D182 | | |
| 2631 | D183 | | |
| 2632 | D185 | | |
| 2633 | D246 | | |
| 2634 | D265 | | |
| 2635 | D266 | | |
| 2636 | D267 | | |
| 2637 | D268 | | |
| 2638 | D269 | | |
| 2639 | D296 | | |
| 2640 | D298 | | |

| No. | $R^1$ = H | | |
|---|---|---|---|
| | $R^2$ | $R^3$ | $R^4$ |
| 2641 | D15 | D296 | Ar21 |
| 2642 | D16 | | |
| 2643 | D18 | | |
| 2644 | D21 | | |
| 2645 | D33 | | |
| 2646 | D34 | | |
| 2647 | D36 | | |
| 2648 | D45 | | |
| 2649 | D46 | | |
| 2650 | D48 | | |
| 2651 | D63 | | |
| 2652 | D64 | | |
| 2653 | D66 | | |
| 2654 | D73 | | |
| 2655 | D74 | | |
| 2656 | D75 | | |
| 2657 | D76 | | |
| 2658 | D78 | | |
| 2659 | D155 | | |
| 2660 | D182 | | |
| 2661 | D183 | | |
| 2662 | D185 | | |
| 2663 | D246 | | |
| 2664 | D265 | | |
| 2665 | D266 | | |
| 2666 | D267 | | |
| 2667 | D268 | | |
| 2668 | D269 | | |
| 2669 | D296 | | |
| 2670 | D298 | | |
| 2671 | D15 | D298 | |
| 2672 | D16 | | |
| 2673 | D18 | | |
| 2674 | D21 | | |
| 2675 | D33 | | |
| 2676 | D34 | | |
| 2677 | D36 | | |
| 2678 | D45 | | |
| 2679 | D46 | | |
| 2680 | D48 | | |
| 2681 | D63 | | |
| 2682 | D64 | | |
| 2683 | D66 | | |
| 2684 | D73 | | |
| 2685 | D74 | | |
| 2686 | D75 | | |
| 2687 | D76 | | |
| 2688 | D78 | | |
| 2689 | D155 | | |
| 2690 | D182 | | |
| 2691 | D183 | | |
| 2692 | D185 | | |
| 2693 | D246 | | |
| 2694 | D265 | | |
| 2695 | D266 | | |
| 2696 | D267 | | |
| 2697 | D268 | | |
| 2698 | D269 | | |
| 2699 | D296 | | |
| 2700 | D298 | | |

| No. | $R^1$ = H | | |
|---|---|---|---|
| | $R^2$ | $R^3$ | $R^4$ |
| 2701 | D15 | D15 | Ar24 |
| 2702 | D16 | | |
| 2703 | D18 | | |
| 2704 | D21 | | |
| 2705 | D33 | | |
| 2706 | D34 | | |
| 2707 | D36 | | |
| 2708 | D45 | | |
| 2709 | D46 | | |
| 2710 | D48 | | |
| 2711 | D63 | | |
| 2712 | D64 | | |
| 2713 | D66 | | |
| 2714 | D73 | | |
| 2715 | D74 | | |
| 2716 | D75 | | |
| 2717 | D76 | | |
| 2718 | D78 | | |
| 2719 | D155 | | |
| 2720 | D182 | | |
| 2721 | D183 | | |
| 2722 | D185 | | |
| 2723 | D246 | | |
| 2724 | D265 | | |
| 2725 | D266 | | |
| 2726 | D267 | | |
| 2727 | D268 | | |
| 2728 | D269 | | |
| 2729 | D296 | | |
| 2730 | D298 | | |
| 2731 | D15 | D16 | |
| 2732 | D16 | | |
| 2733 | D18 | | |
| 2734 | D21 | | |
| 2735 | D33 | | |
| 2736 | D34 | | |
| 2737 | D36 | | |
| 2738 | D45 | | |
| 2739 | D46 | | |
| 2740 | D48 | | |
| 2741 | D63 | | |
| 2742 | D64 | | |
| 2743 | D66 | | |
| 2744 | D73 | | |
| 2745 | D74 | | |
| 2746 | D75 | | |
| 2747 | D76 | | |
| 2748 | D78 | | |
| 2749 | D155 | | |
| 2750 | D182 | | |
| 2751 | D183 | | |
| 2752 | D185 | | |
| 2753 | D246 | | |
| 2754 | D265 | | |
| 2755 | D266 | | |
| 2756 | D267 | | |
| 2757 | D268 | | |
| 2758 | D269 | | |
| 2759 | D296 | | |
| 2760 | D298 | | |

| No. | $R^1$ = H | | |
|---|---|---|---|
| | $R^2$ | $R^3$ | $R^4$ |
| 2761 | D15 | D18 | Ar24 |
| 2762 | D16 | | |
| 2763 | D18 | | |
| 2764 | D21 | | |
| 2765 | D33 | | |
| 2766 | D34 | | |
| 2767 | D36 | | |
| 2768 | D45 | | |
| 2769 | D46 | | |
| 2770 | D48 | | |
| 2771 | D63 | | |
| 2772 | D64 | | |
| 2773 | D66 | | |
| 2774 | D73 | | |
| 2775 | D74 | | |
| 2776 | D75 | | |
| 2777 | D76 | | |
| 2778 | D78 | | |
| 2779 | D155 | | |
| 2780 | D182 | | |
| 2781 | D183 | | |
| 2782 | D185 | | |
| 2783 | D246 | | |
| 2784 | D265 | | |
| 2785 | D266 | | |
| 2786 | D267 | | |
| 2787 | D268 | | |
| 2788 | D269 | | |
| 2789 | D296 | | |
| 2790 | D298 | | |
| 2791 | D15 | D21 | |
| 2792 | D16 | | |
| 2793 | D18 | | |
| 2794 | D21 | | |
| 2795 | D33 | | |
| 2796 | D34 | | |
| 2797 | D36 | | |
| 2798 | D45 | | |
| 2799 | D46 | | |
| 2800 | D48 | | |
| 2801 | D63 | | |
| 2802 | D64 | | |
| 2803 | D66 | | |
| 2804 | D73 | | |
| 2805 | D74 | | |
| 2806 | D75 | | |
| 2807 | D76 | | |
| 2808 | D78 | | |
| 2809 | D155 | | |
| 2810 | D182 | | |
| 2811 | D183 | | |
| 2812 | D185 | | |
| 2813 | D246 | | |
| 2814 | D265 | | |
| 2815 | D266 | | |
| 2816 | D267 | | |
| 2817 | D268 | | |
| 2818 | D269 | | |
| 2819 | D296 | | |
| 2820 | D298 | | |

| No. | $R^1$ = H | | |
|---|---|---|---|
| | $R^2$ | $R^3$ | $R^4$ |
| 2821 | D15 | D33 | Ar24 |
| 2822 | D16 | | |
| 2823 | D18 | | |
| 2824 | D21 | | |
| 2825 | D33 | | |
| 2826 | D34 | | |
| 2827 | D36 | | |
| 2828 | D45 | | |
| 2829 | D46 | | |
| 2830 | D48 | | |
| 2831 | D63 | | |
| 2832 | D64 | | |
| 2833 | D66 | | |
| 2834 | D73 | | |
| 2835 | D74 | | |
| 2836 | D75 | | |
| 2837 | D76 | | |
| 2838 | D78 | | |
| 2839 | D155 | | |
| 2840 | D182 | | |
| 2841 | D183 | | |
| 2842 | D185 | | |
| 2843 | D246 | | |
| 2844 | D265 | | |
| 2845 | D266 | | |
| 2846 | D267 | | |
| 2847 | D268 | | |
| 2848 | D269 | | |
| 2849 | D296 | | |
| 2850 | D298 | | |
| 2851 | D15 | D34 | |
| 2852 | D16 | | |
| 2853 | D18 | | |
| 2854 | D21 | | |
| 2855 | D33 | | |
| 2856 | D34 | | |
| 2857 | D36 | | |
| 2858 | D45 | | |
| 2859 | D46 | | |
| 2860 | D48 | | |
| 2861 | D63 | | |
| 2862 | D64 | | |
| 2863 | D66 | | |
| 2864 | D73 | | |
| 2865 | D74 | | |
| 2866 | D75 | | |
| 2867 | D76 | | |
| 2868 | D78 | | |
| 2869 | D155 | | |
| 2870 | D182 | | |
| 2871 | D183 | | |
| 2872 | D185 | | |
| 2873 | D246 | | |
| 2874 | D265 | | |
| 2875 | D266 | | |
| 2876 | D267 | | |
| 2877 | D268 | | |
| 2878 | D269 | | |
| 2879 | D296 | | |
| 2880 | D298 | | |

| No. | $R^1$ = H | | |
|---|---|---|---|
| | $R^2$ | $R^3$ | $R^4$ |
| 2881 | D15 | | |
| 2882 | D16 | | |
| 2883 | D18 | | |
| 2884 | D21 | | |
| 2885 | D33 | | |
| 2886 | D34 | | |
| 2887 | D36 | | |
| 2888 | D45 | | |
| 2889 | D46 | | |
| 2890 | D48 | | |
| 2891 | D63 | | |
| 2892 | D64 | | |
| 2893 | D66 | | |
| 2894 | D73 | | |
| 2895 | D74 | D36 | |
| 2896 | D75 | | |
| 2897 | D76 | | |
| 2898 | D78 | | |
| 2899 | D155 | | |
| 2900 | D182 | | |
| 2901 | D183 | | |
| 2902 | D185 | | |
| 2903 | D246 | | |
| 2904 | D265 | | |
| 2905 | D266 | | |
| 2906 | D267 | | |
| 2907 | D268 | | |
| 2908 | D269 | | |
| 2909 | D296 | | |
| 2910 | D298 | | Ar24 |
| 2911 | D15 | | |
| 2912 | D16 | | |
| 2913 | D18 | | |
| 2914 | D21 | | |
| 2915 | D33 | | |
| 2916 | D34 | | |
| 2917 | D36 | | |
| 2918 | D45 | | |
| 2919 | D46 | | |
| 2920 | D48 | | |
| 2921 | D63 | | |
| 2922 | D64 | | |
| 2923 | D66 | | |
| 2924 | D73 | | |
| 2925 | D74 | D45 | |
| 2926 | D75 | | |
| 2927 | D76 | | |
| 2928 | D78 | | |
| 2929 | D155 | | |
| 2930 | D182 | | |
| 2931 | D183 | | |
| 2932 | D185 | | |
| 2933 | D246 | | |
| 2934 | D265 | | |
| 2935 | D266 | | |
| 2936 | D267 | | |
| 2937 | D268 | | |
| 2938 | D269 | | |
| 2939 | D296 | | |
| 2940 | D298 | | |

| No. | $R^1$ = H | | |
|---|---|---|---|
| | $R^2$ | $R^3$ | $R^4$ |
| 2941 | D15 | | |
| 2942 | D16 | | |
| 2943 | D18 | | |
| 2944 | D21 | | |
| 2945 | D33 | | |
| 2946 | D34 | | |
| 2947 | D36 | | |
| 2948 | D45 | | |
| 2949 | D46 | | |
| 2950 | D48 | | |
| 2951 | D63 | | |
| 2952 | D64 | | |
| 2953 | D66 | | |
| 2954 | D73 | | |
| 2955 | D74 | D46 | |
| 2956 | D75 | | |
| 2957 | D76 | | |
| 2958 | D78 | | |
| 2959 | D155 | | |
| 2960 | D182 | | |
| 2961 | D183 | | |
| 2962 | D185 | | |
| 2963 | D246 | | |
| 2964 | D265 | | |
| 2965 | D266 | | |
| 2966 | D267 | | |
| 2967 | D268 | | |
| 2968 | D269 | | |
| 2969 | D296 | | |
| 2970 | D298 | | Ar24 |
| 2971 | D15 | | |
| 2972 | D16 | | |
| 2973 | D18 | | |
| 2974 | D21 | | |
| 2975 | D33 | | |
| 2976 | D34 | | |
| 2977 | D36 | | |
| 2978 | D45 | | |
| 2979 | D46 | | |
| 2980 | D48 | | |
| 2981 | D63 | | |
| 2982 | D64 | | |
| 2983 | D66 | | |
| 2984 | D73 | | |
| 2985 | D74 | D48 | |
| 2986 | D75 | | |
| 2987 | D76 | | |
| 2988 | D78 | | |
| 2989 | D155 | | |
| 2990 | D182 | | |
| 2991 | D183 | | |
| 2992 | D185 | | |
| 2993 | D246 | | |
| 2994 | D265 | | |
| 2995 | D266 | | |
| 2996 | D267 | | |
| 2997 | D268 | | |
| 2998 | D269 | | |
| 2999 | D296 | | |
| 3000 | D298 | | |

| No. | $R^1$ = H | | |
|---|---|---|---|
| | $R^2$ | $R^3$ | $R^4$ |
| 3001 | D15 | | |
| 3002 | D16 | | |
| 3003 | D18 | | |
| 3004 | D21 | | |
| 3005 | D33 | | |
| 3006 | D34 | | |
| 3007 | D36 | | |
| 3008 | D45 | | |
| 3009 | D46 | | |
| 3010 | D48 | | |
| 3011 | D63 | | |
| 3012 | D64 | | |
| 3013 | D66 | | |
| 3014 | D73 | | |
| 3015 | D74 | D63 | |
| 3016 | D75 | | |
| 3017 | D76 | | |
| 3018 | D78 | | |
| 3019 | D155 | | |
| 3020 | D182 | | |
| 3021 | D183 | | |
| 3022 | D185 | | |
| 3023 | D246 | | |
| 3024 | D265 | | |
| 3025 | D266 | | |
| 3026 | D267 | | |
| 3027 | D268 | | |
| 3028 | D269 | | |
| 3029 | D296 | | |
| 3030 | D298 | | Ar24 |
| 3031 | D15 | | |
| 3032 | D16 | | |
| 3033 | D18 | | |
| 3034 | D21 | | |
| 3035 | D33 | | |
| 3036 | D34 | | |
| 3037 | D36 | | |
| 3038 | D45 | | |
| 3039 | D46 | | |
| 3040 | D48 | | |
| 3041 | D63 | | |
| 3042 | D64 | | |
| 3043 | D66 | | |
| 3044 | D73 | | |
| 3045 | D74 | D64 | |
| 3046 | D75 | | |
| 3047 | D76 | | |
| 3048 | D78 | | |
| 3049 | D155 | | |
| 3050 | D182 | | |
| 3051 | D183 | | |
| 3052 | D185 | | |
| 3053 | D246 | | |
| 3054 | D265 | | |
| 3055 | D266 | | |
| 3056 | D267 | | |
| 3057 | D268 | | |
| 3058 | D269 | | |
| 3059 | D296 | | |
| 3060 | D298 | | |

| No. | $R^1$ = H | | |
|---|---|---|---|
| | $R^2$ | $R^3$ | $R^4$ |
| 3061 | D15 | | |
| 3062 | D16 | | |
| 3063 | D18 | | |
| 3064 | D21 | | |
| 3065 | D33 | | |
| 3066 | D34 | | |
| 3067 | D36 | | |
| 3068 | D45 | | |
| 3069 | D46 | | |
| 3070 | D48 | | |
| 3071 | D63 | | |
| 3072 | D64 | | |
| 3073 | D66 | | |
| 3074 | D73 | | |
| 3075 | D74 | D66 | |
| 3076 | D75 | | |
| 3077 | D76 | | |
| 3078 | D78 | | |
| 3079 | D155 | | |
| 3080 | D182 | | |
| 3081 | D183 | | |
| 3082 | D185 | | |
| 3083 | D246 | | |
| 3084 | D265 | | |
| 3085 | D266 | | |
| 3086 | D267 | | |
| 3087 | D268 | | |
| 3088 | D269 | | |
| 3089 | D296 | | |
| 3090 | D298 | | Ar24 |
| 3091 | D15 | | |
| 3092 | D16 | | |
| 3093 | D18 | | |
| 3094 | D21 | | |
| 3095 | D33 | | |
| 3096 | D34 | | |
| 3097 | D36 | | |
| 3098 | D45 | | |
| 3099 | D46 | | |
| 3100 | D48 | | |
| 3101 | D63 | | |
| 3102 | D64 | | |
| 3103 | D66 | | |
| 3104 | D73 | | |
| 3105 | D74 | D73 | |
| 3106 | D75 | | |
| 3107 | D76 | | |
| 3108 | D78 | | |
| 3109 | D155 | | |
| 3110 | D182 | | |
| 3111 | D183 | | |
| 3112 | D185 | | |
| 3113 | D246 | | |
| 3114 | D265 | | |
| 3115 | D266 | | |
| 3116 | D267 | | |
| 3117 | D268 | | |
| 3118 | D269 | | |
| 3119 | D296 | | |
| 3120 | D298 | | |

| No. | $R^1$ = H | | |
|---|---|---|---|
| | $R^2$ | $R^3$ | $R^4$ |
| 3121 | D15 | | |
| 3122 | D16 | | |
| 3123 | D18 | | |
| 3124 | D21 | | |
| 3125 | D33 | | |
| 3126 | D34 | | |
| 3127 | D36 | | |
| 3128 | D45 | | |
| 3129 | D46 | | |
| 3130 | D48 | | |
| 3131 | D63 | | |
| 3132 | D64 | | |
| 3133 | D66 | | |
| 3134 | D73 | | |
| 3135 | D74 | D74 | |
| 3136 | D75 | | |
| 3137 | D76 | | |
| 3138 | D78 | | |
| 3139 | D155 | | |
| 3140 | D182 | | |
| 3141 | D183 | | |
| 3142 | D185 | | |
| 3143 | D246 | | |
| 3144 | D265 | | |
| 3145 | D266 | | |
| 3146 | D267 | | |
| 3147 | D268 | | |
| 3148 | D269 | | |
| 3149 | D296 | | |
| 3150 | D298 | | Ar24 |
| 3151 | D15 | | |
| 3152 | D16 | | |
| 3153 | D18 | | |
| 3154 | D21 | | |
| 3155 | D33 | | |
| 3156 | D34 | | |
| 3157 | D36 | | |
| 3158 | D45 | | |
| 3159 | D46 | | |
| 3160 | D48 | | |
| 3161 | D63 | | |
| 3162 | D64 | | |
| 3163 | D66 | | |
| 3164 | D73 | | |
| 3165 | D74 | D75 | |
| 3166 | D75 | | |
| 3167 | D76 | | |
| 3168 | D78 | | |
| 3169 | D155 | | |
| 3170 | D182 | | |
| 3171 | D183 | | |
| 3172 | D185 | | |
| 3173 | D246 | | |
| 3174 | D265 | | |
| 3175 | D266 | | |
| 3176 | D267 | | |
| 3177 | D268 | | |
| 3178 | D269 | | |
| 3179 | D296 | | |
| 3180 | D298 | | |

| No. | $R^1$ = H | | |
|---|---|---|---|
| | $R^2$ | $R^3$ | $R^4$ |
| 3181 | D15 | | |
| 3182 | D16 | | |
| 3183 | D18 | | |
| 3184 | D21 | | |
| 3185 | D33 | | |
| 3186 | D34 | | |
| 3187 | D36 | | |
| 3188 | D45 | | |
| 3189 | D46 | | |
| 3190 | D48 | | |
| 3191 | D63 | | |
| 3192 | D64 | | |
| 3193 | D66 | | |
| 3194 | D73 | | |
| 3195 | D74 | D76 | |
| 3196 | D75 | | |
| 3197 | D76 | | |
| 3198 | D78 | | |
| 3199 | D155 | | |
| 3200 | D182 | | |
| 3201 | D183 | | |
| 3202 | D185 | | |
| 3203 | D246 | | |
| 3204 | D265 | | |
| 3205 | D266 | | |
| 3206 | D267 | | |
| 3207 | D268 | | |
| 3208 | D269 | | |
| 3209 | D296 | | |
| 3210 | D298 | | Ar24 |
| 3211 | D15 | | |
| 3212 | D16 | | |
| 3213 | D18 | | |
| 3214 | D21 | | |
| 3215 | D33 | | |
| 3216 | D34 | | |
| 3217 | D36 | | |
| 3218 | D45 | | |
| 3219 | D46 | | |
| 3220 | D48 | | |
| 3221 | D63 | | |
| 3222 | D64 | | |
| 3223 | D66 | | |
| 3224 | D73 | | |
| 3225 | D74 | D78 | |
| 3226 | D75 | | |
| 3227 | D76 | | |
| 3228 | D78 | | |
| 3229 | D155 | | |
| 3230 | D182 | | |
| 3231 | D183 | | |
| 3232 | D185 | | |
| 3233 | D246 | | |
| 3234 | D265 | | |
| 3235 | D266 | | |
| 3236 | D267 | | |
| 3237 | D268 | | |
| 3238 | D269 | | |
| 3239 | D296 | | |
| 3240 | D298 | | |

| No. | $R^1$ = H | | |
|---|---|---|---|
| | $R^2$ | $R^3$ | $R^4$ |
| 3241 | D15 | | |
| 3242 | D16 | | |
| 3243 | D18 | | |
| 3244 | D21 | | |
| 3245 | D33 | | |
| 3246 | D34 | | |
| 3247 | D36 | | |
| 3248 | D45 | | |
| 3249 | D46 | | |
| 3250 | D48 | | |
| 3251 | D63 | | |
| 3252 | D64 | | |
| 3253 | D66 | | |
| 3254 | D73 | | |
| 3255 | D74 | D155 | |
| 3256 | D75 | | |
| 3257 | D76 | | |
| 3258 | D78 | | |
| 3259 | D155 | | |
| 3260 | D182 | | |
| 3261 | D183 | | |
| 3262 | D185 | | |
| 3263 | D246 | | |
| 3264 | D265 | | |
| 3265 | D266 | | |
| 3266 | D267 | | |
| 3267 | D268 | | |
| 3268 | D269 | | |
| 3269 | D296 | | |
| 3270 | D298 | | Ar24 |
| 3271 | D15 | | |
| 3272 | D16 | | |
| 3273 | D18 | | |
| 3274 | D21 | | |
| 3275 | D33 | | |
| 3276 | D34 | | |
| 3277 | D36 | | |
| 3278 | D45 | | |
| 3279 | D46 | | |
| 3280 | D48 | | |
| 3281 | D63 | | |
| 3282 | D64 | | |
| 3283 | D66 | | |
| 3284 | D73 | | |
| 3285 | D74 | D182 | |
| 3286 | D75 | | |
| 3287 | D76 | | |
| 3288 | D78 | | |
| 3289 | D155 | | |
| 3290 | D182 | | |
| 3291 | D183 | | |
| 3292 | D185 | | |
| 3293 | D246 | | |
| 3294 | D265 | | |
| 3295 | D266 | | |
| 3296 | D267 | | |
| 3297 | D268 | | |
| 3298 | D269 | | |
| 3299 | D296 | | |
| 3300 | D298 | | |

| No. | $R^1$ = H | | |
|---|---|---|---|
| | $R^2$ | $R^3$ | $R^4$ |
| 3301 | D15 | | |
| 3302 | D16 | | |
| 3303 | D18 | | |
| 3304 | D21 | | |
| 3305 | D33 | | |
| 3306 | D34 | | |
| 3307 | D36 | | |
| 3308 | D45 | | |
| 3309 | D46 | | |
| 3310 | D48 | | |
| 3311 | D63 | | |
| 3312 | D64 | | |
| 3313 | D66 | | |
| 3314 | D73 | | |
| 3315 | D74 | D183 | |
| 3316 | D75 | | |
| 3317 | D76 | | |
| 3318 | D78 | | |
| 3319 | D155 | | |
| 3320 | D182 | | |
| 3321 | D183 | | |
| 3322 | D185 | | |
| 3323 | D246 | | |
| 3324 | D265 | | |
| 3325 | D266 | | |
| 3326 | D267 | | |
| 3327 | D268 | | |
| 3328 | D269 | | |
| 3329 | D296 | | |
| 3330 | D298 | | Ar24 |
| 3331 | D15 | | |
| 3332 | D16 | | |
| 3333 | D18 | | |
| 3334 | D21 | | |
| 3335 | D33 | | |
| 3336 | D34 | | |
| 3337 | D36 | | |
| 3338 | D45 | | |
| 3339 | D46 | | |
| 3340 | D48 | | |
| 3341 | D63 | | |
| 3342 | D64 | | |
| 3343 | D66 | | |
| 3344 | D73 | | |
| 3345 | D74 | D185 | |
| 3346 | D75 | | |
| 3347 | D76 | | |
| 3348 | D78 | | |
| 3349 | D155 | | |
| 3350 | D182 | | |
| 3351 | D183 | | |
| 3352 | D185 | | |
| 3353 | D246 | | |
| 3354 | D265 | | |
| 3355 | D266 | | |
| 3356 | D267 | | |
| 3357 | D268 | | |
| 3358 | D269 | | |
| 3359 | D296 | | |
| 3360 | D298 | | |

| No. | $R^1$ = H | | |
|---|---|---|---|
| | $R^2$ | $R^3$ | $R^4$ |
| 3361 | D15 | D246 | Ar24 |
| 3362 | D16 | | |
| 3363 | D18 | | |
| 3364 | D21 | | |
| 3365 | D33 | | |
| 3366 | D34 | | |
| 3367 | D36 | | |
| 3368 | D45 | | |
| 3369 | D46 | | |
| 3370 | D48 | | |
| 3371 | D63 | | |
| 3372 | D64 | | |
| 3373 | D66 | | |
| 3374 | D73 | | |
| 3375 | D74 | | |
| 3376 | D75 | | |
| 3377 | D76 | | |
| 3378 | D78 | | |
| 3379 | D155 | | |
| 3380 | D182 | | |
| 3381 | D183 | | |
| 3382 | D185 | | |
| 3383 | D246 | | |
| 3384 | D265 | | |
| 3385 | D266 | | |
| 3386 | D267 | | |
| 3387 | D268 | | |
| 3388 | D269 | | |
| 3389 | D296 | | |
| 3390 | D298 | | |
| 3391 | D15 | D265 | |
| 3392 | D16 | | |
| 3393 | D18 | | |
| 3394 | D21 | | |
| 3395 | D33 | | |
| 3396 | D34 | | |
| 3397 | D36 | | |
| 3398 | D45 | | |
| 3399 | D46 | | |
| 3400 | D48 | | |
| 3401 | D63 | | |
| 3402 | D64 | | |
| 3403 | D66 | | |
| 3404 | D73 | | |
| 3405 | D74 | | |
| 3406 | D75 | | |
| 3407 | D76 | | |
| 3408 | D78 | | |
| 3409 | D155 | | |
| 3410 | D182 | | |
| 3411 | D183 | | |
| 3412 | D185 | | |
| 3413 | D246 | | |
| 3414 | D265 | | |
| 3415 | D266 | | |
| 3416 | D267 | | |
| 3417 | D268 | | |
| 3418 | D269 | | |
| 3419 | D296 | | |
| 3420 | D298 | | |

| No. | $R^1$ = H | | |
|---|---|---|---|
| | $R^2$ | $R^3$ | $R^4$ |
| 3421 | D15 | D266 | Ar24 |
| 3422 | D16 | | |
| 3423 | D18 | | |
| 3424 | D21 | | |
| 3425 | D33 | | |
| 3426 | D34 | | |
| 3427 | D36 | | |
| 3428 | D45 | | |
| 3429 | D46 | | |
| 3430 | D48 | | |
| 3431 | D63 | | |
| 3432 | D64 | | |
| 3433 | D66 | | |
| 3434 | D73 | | |
| 3435 | D74 | | |
| 3436 | D75 | | |
| 3437 | D76 | | |
| 3438 | D78 | | |
| 3439 | D155 | | |
| 3440 | D182 | | |
| 3441 | D183 | | |
| 3442 | D185 | | |
| 3443 | D246 | | |
| 3444 | D265 | | |
| 3445 | D266 | | |
| 3446 | D267 | | |
| 3447 | D268 | | |
| 3448 | D269 | | |
| 3449 | D296 | | |
| 3450 | D298 | | |
| 3451 | D15 | D267 | |
| 3452 | D16 | | |
| 3453 | D18 | | |
| 3454 | D21 | | |
| 3455 | D33 | | |
| 3456 | D34 | | |
| 3457 | D36 | | |
| 3458 | D45 | | |
| 3459 | D46 | | |
| 3460 | D48 | | |
| 3461 | D63 | | |
| 3462 | D64 | | |
| 3463 | D66 | | |
| 3464 | D73 | | |
| 3465 | D74 | | |
| 3466 | D75 | | |
| 3467 | D76 | | |
| 3468 | D78 | | |
| 3469 | D155 | | |
| 3470 | D182 | | |
| 3471 | D183 | | |
| 3472 | D185 | | |
| 3473 | D246 | | |
| 3474 | D265 | | |
| 3475 | D266 | | |
| 3476 | D267 | | |
| 3477 | D268 | | |
| 3478 | D269 | | |
| 3479 | D296 | | |
| 3480 | D298 | | |

| No. | $R^1$ = H | | |
|---|---|---|---|
| | $R^2$ | $R^3$ | $R^4$ |
| 3481 | D15 | D268 | Ar24 |
| 3482 | D16 | | |
| 3483 | D18 | | |
| 3484 | D21 | | |
| 3485 | D33 | | |
| 3486 | D34 | | |
| 3487 | D36 | | |
| 3488 | D45 | | |
| 3489 | D46 | | |
| 3490 | D48 | | |
| 3491 | D63 | | |
| 3492 | D64 | | |
| 3493 | D66 | | |
| 3494 | D73 | | |
| 3495 | D74 | | |
| 3496 | D75 | | |
| 3497 | D76 | | |
| 3498 | D78 | | |
| 3499 | D155 | | |
| 3500 | D182 | | |
| 3501 | D183 | | |
| 3502 | D185 | | |
| 3503 | D246 | | |
| 3504 | D265 | | |
| 3505 | D266 | | |
| 3506 | D267 | | |
| 3507 | D268 | | |
| 3508 | D269 | | |
| 3509 | D296 | | |
| 3510 | D298 | | |
| 3511 | D15 | D269 | |
| 3512 | D16 | | |
| 3513 | D18 | | |
| 3514 | D21 | | |
| 3515 | D33 | | |
| 3516 | D34 | | |
| 3517 | D36 | | |
| 3518 | D45 | | |
| 3519 | D46 | | |
| 3520 | D48 | | |
| 3521 | D63 | | |
| 3522 | D64 | | |
| 3523 | D66 | | |
| 3524 | D73 | | |
| 3525 | D74 | | |
| 3526 | D75 | | |
| 3527 | D76 | | |
| 3528 | D78 | | |
| 3529 | D155 | | |
| 3530 | D182 | | |
| 3531 | D183 | | |
| 3532 | D185 | | |
| 3533 | D246 | | |
| 3534 | D265 | | |
| 3535 | D266 | | |
| 3536 | D267 | | |
| 3537 | D268 | | |
| 3538 | D269 | | |
| 3539 | D296 | | |
| 3540 | D298 | | |

| No. | $R^1$ = H | | |
|---|---|---|---|
| | $R^2$ | $R^3$ | $R^4$ |
| 3541 | D15 | D296 | Ar24 |
| 3542 | D16 | | |
| 3543 | D18 | | |
| 3544 | D21 | | |
| 3545 | D33 | | |
| 3546 | D34 | | |
| 3547 | D36 | | |
| 3548 | D45 | | |
| 3549 | D46 | | |
| 3550 | D48 | | |
| 3551 | D63 | | |
| 3552 | D64 | | |
| 3553 | D66 | | |
| 3554 | D73 | | |
| 3555 | D74 | | |
| 3556 | D75 | | |
| 3557 | D76 | | |
| 3558 | D78 | | |
| 3559 | D155 | | |
| 3560 | D182 | | |
| 3561 | D183 | | |
| 3562 | D185 | | |
| 3563 | D246 | | |
| 3564 | D265 | | |
| 3565 | D266 | | |
| 3566 | D267 | | |
| 3567 | D268 | | |
| 3568 | D269 | | |
| 3569 | D296 | | |
| 3570 | D298 | | |
| 3571 | D15 | D298 | |
| 3572 | D16 | | |
| 3573 | D18 | | |
| 3574 | D21 | | |
| 3575 | D33 | | |
| 3576 | D34 | | |
| 3577 | D36 | | |
| 3578 | D45 | | |
| 3579 | D46 | | |
| 3580 | D48 | | |
| 3581 | D63 | | |
| 3582 | D64 | | |
| 3583 | D66 | | |
| 3584 | D73 | | |
| 3585 | D74 | | |
| 3586 | D75 | | |
| 3587 | D76 | | |
| 3588 | D78 | | |
| 3589 | D155 | | |
| 3590 | D182 | | |
| 3591 | D183 | | |
| 3592 | D185 | | |
| 3593 | D246 | | |
| 3594 | D265 | | |
| 3595 | D266 | | |
| 3596 | D267 | | |
| 3597 | D268 | | |
| 3598 | D269 | | |
| 3599 | D296 | | |
| 3600 | D298 | | |

| No. | $R^1$ = H | | |
|---|---|---|---|
| | $R^2$ | $R^3$ | $R^4$ |
| 3601 | D15 | D15 | Ar82 |
| 3602 | D16 | D15 | Ar82 |
| 3603 | D18 | D15 | Ar82 |
| 3604 | D21 | D15 | Ar82 |
| 3605 | D33 | D15 | Ar82 |
| 3606 | D34 | D15 | Ar82 |
| 3607 | D36 | D15 | Ar82 |
| 3608 | D45 | D15 | Ar82 |
| 3609 | D46 | D15 | Ar82 |
| 3610 | D48 | D15 | Ar82 |
| 3611 | D63 | D15 | Ar82 |
| 3612 | D64 | D15 | Ar82 |
| 3613 | D66 | D15 | Ar82 |
| 3614 | D73 | D15 | Ar82 |
| 3615 | D74 | D15 | Ar82 |
| 3616 | D75 | D15 | Ar82 |
| 3617 | D76 | D15 | Ar82 |
| 3618 | D78 | D15 | Ar82 |
| 3619 | D155 | D15 | Ar82 |
| 3620 | D182 | D15 | Ar82 |
| 3621 | D183 | D15 | Ar82 |
| 3622 | D185 | D15 | Ar82 |
| 3623 | D246 | D15 | Ar82 |
| 3624 | D265 | D15 | Ar82 |
| 3625 | D266 | D15 | Ar82 |
| 3626 | D267 | D15 | Ar82 |
| 3627 | D268 | D15 | Ar82 |
| 3628 | D269 | D15 | Ar82 |
| 3629 | D296 | D15 | Ar82 |
| 3630 | D298 | D15 | Ar82 |
| 3631 | D15 | D16 | Ar82 |
| 3632 | D16 | D16 | Ar82 |
| 3633 | D18 | D16 | Ar82 |
| 3634 | D21 | D16 | Ar82 |
| 3635 | D33 | D16 | Ar82 |
| 3636 | D34 | D16 | Ar82 |
| 3637 | D36 | D16 | Ar82 |
| 3638 | D45 | D16 | Ar82 |
| 3639 | D46 | D16 | Ar82 |
| 3640 | D48 | D16 | Ar82 |
| 3641 | D63 | D16 | Ar82 |
| 3642 | D64 | D16 | Ar82 |
| 3643 | D66 | D16 | Ar82 |
| 3644 | D73 | D16 | Ar82 |
| 3645 | D74 | D16 | Ar82 |
| 3646 | D75 | D16 | Ar82 |
| 3647 | D76 | D16 | Ar82 |
| 3648 | D78 | D16 | Ar82 |
| 3649 | D155 | D16 | Ar82 |
| 3650 | D182 | D16 | Ar82 |
| 3651 | D183 | D16 | Ar82 |
| 3652 | D185 | D16 | Ar82 |
| 3653 | D246 | D16 | Ar82 |
| 3654 | D265 | D16 | Ar82 |
| 3655 | D266 | D16 | Ar82 |
| 3656 | D267 | D16 | Ar82 |
| 3657 | D268 | D16 | Ar82 |
| 3658 | D269 | D16 | Ar82 |
| 3659 | D296 | D16 | Ar82 |
| 3660 | D298 | D16 | Ar82 |

| No. | $R^1$ = H | | |
|---|---|---|---|
| | $R^2$ | $R^3$ | $R^4$ |
| 3661 | D15 | D18 | Ar82 |
| 3662 | D16 | D18 | Ar82 |
| 3663 | D18 | D18 | Ar82 |
| 3664 | D21 | D18 | Ar82 |
| 3665 | D33 | D18 | Ar82 |
| 3666 | D34 | D18 | Ar82 |
| 3667 | D36 | D18 | Ar82 |
| 3668 | D45 | D18 | Ar82 |
| 3669 | D46 | D18 | Ar82 |
| 3670 | D48 | D18 | Ar82 |
| 3671 | D63 | D18 | Ar82 |
| 3672 | D64 | D18 | Ar82 |
| 3673 | D66 | D18 | Ar82 |
| 3674 | D73 | D18 | Ar82 |
| 3675 | D74 | D18 | Ar82 |
| 3676 | D75 | D18 | Ar82 |
| 3677 | D76 | D18 | Ar82 |
| 3678 | D78 | D18 | Ar82 |
| 3679 | D155 | D18 | Ar82 |
| 3680 | D182 | D18 | Ar82 |
| 3681 | D183 | D18 | Ar82 |
| 3682 | D185 | D18 | Ar82 |
| 3683 | D246 | D18 | Ar82 |
| 3684 | D265 | D18 | Ar82 |
| 3685 | D266 | D18 | Ar82 |
| 3686 | D267 | D18 | Ar82 |
| 3687 | D268 | D18 | Ar82 |
| 3688 | D269 | D18 | Ar82 |
| 3689 | D296 | D18 | Ar82 |
| 3690 | D298 | D18 | Ar82 |
| 3691 | D15 | D21 | Ar82 |
| 3692 | D16 | D21 | Ar82 |
| 3693 | D18 | D21 | Ar82 |
| 3694 | D21 | D21 | Ar82 |
| 3695 | D33 | D21 | Ar82 |
| 3696 | D34 | D21 | Ar82 |
| 3697 | D36 | D21 | Ar82 |
| 3698 | D45 | D21 | Ar82 |
| 3699 | D46 | D21 | Ar82 |
| 3700 | D48 | D21 | Ar82 |
| 3701 | D63 | D21 | Ar82 |
| 3702 | D64 | D21 | Ar82 |
| 3703 | D66 | D21 | Ar82 |
| 3704 | D73 | D21 | Ar82 |
| 3705 | D74 | D21 | Ar82 |
| 3706 | D75 | D21 | Ar82 |
| 3707 | D76 | D21 | Ar82 |
| 3708 | D78 | D21 | Ar82 |
| 3709 | D155 | D21 | Ar82 |
| 3710 | D182 | D21 | Ar82 |
| 3711 | D183 | D21 | Ar82 |
| 3712 | D185 | D21 | Ar82 |
| 3713 | D246 | D21 | Ar82 |
| 3714 | D265 | D21 | Ar82 |
| 3715 | D266 | D21 | Ar82 |
| 3716 | D267 | D21 | Ar82 |
| 3717 | D268 | D21 | Ar82 |
| 3718 | D269 | D21 | Ar82 |
| 3719 | D296 | D21 | Ar82 |
| 3720 | D298 | D21 | Ar82 |

| No. | $R^1$ = H | | |
|---|---|---|---|
| | $R^2$ | $R^3$ | $R^4$ |
| 3721 | D15 | D33 | Ar82 |
| 3722 | D16 | D33 | Ar82 |
| 3723 | D18 | D33 | Ar82 |
| 3724 | D21 | D33 | Ar82 |
| 3725 | D33 | D33 | Ar82 |
| 3726 | D34 | D33 | Ar82 |
| 3727 | D36 | D33 | Ar82 |
| 3728 | D45 | D33 | Ar82 |
| 3729 | D46 | D33 | Ar82 |
| 3730 | D48 | D33 | Ar82 |
| 3731 | D63 | D33 | Ar82 |
| 3732 | D64 | D33 | Ar82 |
| 3733 | D66 | D33 | Ar82 |
| 3734 | D73 | D33 | Ar82 |
| 3735 | D74 | D33 | Ar82 |
| 3736 | D75 | D33 | Ar82 |
| 3737 | D76 | D33 | Ar82 |
| 3738 | D78 | D33 | Ar82 |
| 3739 | D155 | D33 | Ar82 |
| 3740 | D182 | D33 | Ar82 |
| 3741 | D183 | D33 | Ar82 |
| 3742 | D185 | D33 | Ar82 |
| 3743 | D246 | D33 | Ar82 |
| 3744 | D265 | D33 | Ar82 |
| 3745 | D266 | D33 | Ar82 |
| 3746 | D267 | D33 | Ar82 |
| 3747 | D268 | D33 | Ar82 |
| 3748 | D269 | D33 | Ar82 |
| 3749 | D296 | D33 | Ar82 |
| 3750 | D298 | D33 | Ar82 |
| 3751 | D15 | D34 | Ar82 |
| 3752 | D16 | D34 | Ar82 |
| 3753 | D18 | D34 | Ar82 |
| 3754 | D21 | D34 | Ar82 |
| 3755 | D33 | D34 | Ar82 |
| 3756 | D34 | D34 | Ar82 |
| 3757 | D36 | D34 | Ar82 |
| 3758 | D45 | D34 | Ar82 |
| 3759 | D46 | D34 | Ar82 |
| 3760 | D48 | D34 | Ar82 |
| 3761 | D63 | D34 | Ar82 |
| 3762 | D64 | D34 | Ar82 |
| 3763 | D66 | D34 | Ar82 |
| 3764 | D73 | D34 | Ar82 |
| 3765 | D74 | D34 | Ar82 |
| 3766 | D75 | D34 | Ar82 |
| 3767 | D76 | D34 | Ar82 |
| 3768 | D78 | D34 | Ar82 |
| 3769 | D155 | D34 | Ar82 |
| 3770 | D182 | D34 | Ar82 |
| 3771 | D183 | D34 | Ar82 |
| 3772 | D185 | D34 | Ar82 |
| 3773 | D246 | D34 | Ar82 |
| 3774 | D265 | D34 | Ar82 |
| 3775 | D266 | D34 | Ar82 |
| 3776 | D267 | D34 | Ar82 |
| 3777 | D268 | D34 | Ar82 |
| 3778 | D269 | D34 | Ar82 |
| 3779 | D296 | D34 | Ar82 |
| 3780 | D298 | D34 | Ar82 |

| No. | $R^1$ = H | | |
|---|---|---|---|
| | $R^2$ | $R^3$ | $R^4$ |
| 3781 | D15 | D36 | Ar82 |
| 3782 | D16 | D36 | Ar82 |
| 3783 | D18 | D36 | Ar82 |
| 3784 | D21 | D36 | Ar82 |
| 3785 | D33 | D36 | Ar82 |
| 3786 | D34 | D36 | Ar82 |
| 3787 | D36 | D36 | Ar82 |
| 3788 | D45 | D36 | Ar82 |
| 3789 | D46 | D36 | Ar82 |
| 3790 | D48 | D36 | Ar82 |
| 3791 | D63 | D36 | Ar82 |
| 3792 | D64 | D36 | Ar82 |
| 3793 | D66 | D36 | Ar82 |
| 3794 | D73 | D36 | Ar82 |
| 3795 | D74 | D36 | Ar82 |
| 3796 | D75 | D36 | Ar82 |
| 3797 | D76 | D36 | Ar82 |
| 3798 | D78 | D36 | Ar82 |
| 3799 | D155 | D36 | Ar82 |
| 3800 | D182 | D36 | Ar82 |
| 3801 | D183 | D36 | Ar82 |
| 3802 | D185 | D36 | Ar82 |
| 3803 | D246 | D36 | Ar82 |
| 3804 | D265 | D36 | Ar82 |
| 3805 | D266 | D36 | Ar82 |
| 3806 | D267 | D36 | Ar82 |
| 3807 | D268 | D36 | Ar82 |
| 3808 | D269 | D36 | Ar82 |
| 3809 | D296 | D36 | Ar82 |
| 3810 | D298 | D36 | Ar82 |
| 3811 | D15 | D45 | Ar82 |
| 3812 | D16 | D45 | Ar82 |
| 3813 | D18 | D45 | Ar82 |
| 3814 | D21 | D45 | Ar82 |
| 3815 | D33 | D45 | Ar82 |
| 3816 | D34 | D45 | Ar82 |
| 3817 | D36 | D45 | Ar82 |
| 3818 | D45 | D45 | Ar82 |
| 3819 | D46 | D45 | Ar82 |
| 3820 | D48 | D45 | Ar82 |
| 3821 | D63 | D45 | Ar82 |
| 3822 | D64 | D45 | Ar82 |
| 3823 | D66 | D45 | Ar82 |
| 3824 | D73 | D45 | Ar82 |
| 3825 | D74 | D45 | Ar82 |
| 3826 | D75 | D45 | Ar82 |
| 3827 | D76 | D45 | Ar82 |
| 3828 | D78 | D45 | Ar82 |
| 3829 | D155 | D45 | Ar82 |
| 3830 | D182 | D45 | Ar82 |
| 3831 | D183 | D45 | Ar82 |
| 3832 | D185 | D45 | Ar82 |
| 3833 | D246 | D45 | Ar82 |
| 3834 | D265 | D45 | Ar82 |
| 3835 | D266 | D45 | Ar82 |
| 3836 | D267 | D45 | Ar82 |
| 3837 | D268 | D45 | Ar82 |
| 3838 | D269 | D45 | Ar82 |
| 3839 | D296 | D45 | Ar82 |
| 3840 | D298 | D45 | Ar82 |

| No. | $R^1$ = H | | |
|---|---|---|---|
| | $R^2$ | $R^3$ | $R^4$ |
| 3841 | D15 | D46 | Ar82 |
| 3842 | D16 | D46 | Ar82 |
| 3843 | D18 | D46 | Ar82 |
| 3844 | D21 | D46 | Ar82 |
| 3845 | D33 | D46 | Ar82 |
| 3846 | D34 | D46 | Ar82 |
| 3847 | D36 | D46 | Ar82 |
| 3848 | D45 | D46 | Ar82 |
| 3849 | D46 | D46 | Ar82 |
| 3850 | D48 | D46 | Ar82 |
| 3851 | D63 | D46 | Ar82 |
| 3852 | D64 | D46 | Ar82 |
| 3853 | D66 | D46 | Ar82 |
| 3854 | D73 | D46 | Ar82 |
| 3855 | D74 | D46 | Ar82 |
| 3856 | D75 | D46 | Ar82 |
| 3857 | D76 | D46 | Ar82 |
| 3858 | D78 | D46 | Ar82 |
| 3859 | D155 | D46 | Ar82 |
| 3860 | D182 | D46 | Ar82 |
| 3861 | D183 | D46 | Ar82 |
| 3862 | D185 | D46 | Ar82 |
| 3863 | D246 | D46 | Ar82 |
| 3864 | D265 | D46 | Ar82 |
| 3865 | D266 | D46 | Ar82 |
| 3866 | D267 | D46 | Ar82 |
| 3867 | D268 | D46 | Ar82 |
| 3868 | D269 | D46 | Ar82 |
| 3869 | D296 | D46 | Ar82 |
| 3870 | D298 | D46 | Ar82 |
| 3871 | D15 | D48 | Ar82 |
| 3872 | D16 | D48 | Ar82 |
| 3873 | D18 | D48 | Ar82 |
| 3874 | D21 | D48 | Ar82 |
| 3875 | D33 | D48 | Ar82 |
| 3876 | D34 | D48 | Ar82 |
| 3877 | D36 | D48 | Ar82 |
| 3878 | D45 | D48 | Ar82 |
| 3879 | D46 | D48 | Ar82 |
| 3880 | D48 | D48 | Ar82 |
| 3881 | D63 | D48 | Ar82 |
| 3882 | D64 | D48 | Ar82 |
| 3883 | D66 | D48 | Ar82 |
| 3884 | D73 | D48 | Ar82 |
| 3885 | D74 | D48 | Ar82 |
| 3886 | D75 | D48 | Ar82 |
| 3887 | D76 | D48 | Ar82 |
| 3888 | D78 | D48 | Ar82 |
| 3889 | D155 | D48 | Ar82 |
| 3890 | D182 | D48 | Ar82 |
| 3891 | D183 | D48 | Ar82 |
| 3892 | D185 | D48 | Ar82 |
| 3893 | D246 | D48 | Ar82 |
| 3894 | D265 | D48 | Ar82 |
| 3895 | D266 | D48 | Ar82 |
| 3896 | D267 | D48 | Ar82 |
| 3897 | D268 | D48 | Ar82 |
| 3898 | D269 | D48 | Ar82 |
| 3899 | D296 | D48 | Ar82 |
| 3900 | D298 | D48 | Ar82 |

| No. | $R^1$ = H | | |
|---|---|---|---|
| | $R^2$ | $R^3$ | $R^4$ |
| 3901 | D15 | D63 | Ar82 |
| 3902 | D16 | D63 | Ar82 |
| 3903 | D18 | D63 | Ar82 |
| 3904 | D21 | D63 | Ar82 |
| 3905 | D33 | D63 | Ar82 |
| 3906 | D34 | D63 | Ar82 |
| 3907 | D36 | D63 | Ar82 |
| 3908 | D45 | D63 | Ar82 |
| 3909 | D46 | D63 | Ar82 |
| 3910 | D48 | D63 | Ar82 |
| 3911 | D63 | D63 | Ar82 |
| 3912 | D64 | D63 | Ar82 |
| 3913 | D66 | D63 | Ar82 |
| 3914 | D73 | D63 | Ar82 |
| 3915 | D74 | D63 | Ar82 |
| 3916 | D75 | D63 | Ar82 |
| 3917 | D76 | D63 | Ar82 |
| 3918 | D78 | D63 | Ar82 |
| 3919 | D155 | D63 | Ar82 |
| 3920 | D182 | D63 | Ar82 |
| 3921 | D183 | D63 | Ar82 |
| 3922 | D185 | D63 | Ar82 |
| 3923 | D246 | D63 | Ar82 |
| 3924 | D265 | D63 | Ar82 |
| 3925 | D266 | D63 | Ar82 |
| 3926 | D267 | D63 | Ar82 |
| 3927 | D268 | D63 | Ar82 |
| 3928 | D269 | D63 | Ar82 |
| 3929 | D296 | D63 | Ar82 |
| 3930 | D298 | D63 | Ar82 |
| 3931 | D15 | D64 | Ar82 |
| 3932 | D16 | D64 | Ar82 |
| 3933 | D18 | D64 | Ar82 |
| 3934 | D21 | D64 | Ar82 |
| 3935 | D33 | D64 | Ar82 |
| 3936 | D34 | D64 | Ar82 |
| 3937 | D36 | D64 | Ar82 |
| 3938 | D45 | D64 | Ar82 |
| 3939 | D46 | D64 | Ar82 |
| 3940 | D48 | D64 | Ar82 |
| 3941 | D63 | D64 | Ar82 |
| 3942 | D64 | D64 | Ar82 |
| 3943 | D66 | D64 | Ar82 |
| 3944 | D73 | D64 | Ar82 |
| 3945 | D74 | D64 | Ar82 |
| 3946 | D75 | D64 | Ar82 |
| 3947 | D76 | D64 | Ar82 |
| 3948 | D78 | D64 | Ar82 |
| 3949 | D155 | D64 | Ar82 |
| 3950 | D182 | D64 | Ar82 |
| 3951 | D183 | D64 | Ar82 |
| 3952 | D185 | D64 | Ar82 |
| 3953 | D246 | D64 | Ar82 |
| 3954 | D265 | D64 | Ar82 |
| 3955 | D266 | D64 | Ar82 |
| 3956 | D267 | D64 | Ar82 |
| 3957 | D268 | D64 | Ar82 |
| 3958 | D269 | D64 | Ar82 |
| 3959 | D296 | D64 | Ar82 |
| 3960 | D298 | D64 | Ar82 |

| No. | $R^1$ = H | | |
|---|---|---|---|
| | $R^2$ | $R^3$ | $R^4$ |
| 3961 | D15 | D66 | Ar82 |
| 3962 | D16 | D66 | Ar82 |
| 3963 | D18 | D66 | Ar82 |
| 3964 | D21 | D66 | Ar82 |
| 3965 | D33 | D66 | Ar82 |
| 3966 | D34 | D66 | Ar82 |
| 3967 | D36 | D66 | Ar82 |
| 3968 | D45 | D66 | Ar82 |
| 3969 | D46 | D66 | Ar82 |
| 3970 | D48 | D66 | Ar82 |
| 3971 | D63 | D66 | Ar82 |
| 3972 | D64 | D66 | Ar82 |
| 3973 | D66 | D66 | Ar82 |
| 3974 | D73 | D66 | Ar82 |
| 3975 | D74 | D66 | Ar82 |
| 3976 | D75 | D66 | Ar82 |
| 3977 | D76 | D66 | Ar82 |
| 3978 | D78 | D66 | Ar82 |
| 3979 | D155 | D66 | Ar82 |
| 3980 | D182 | D66 | Ar82 |
| 3981 | D183 | D66 | Ar82 |
| 3982 | D185 | D66 | Ar82 |
| 3983 | D246 | D66 | Ar82 |
| 3984 | D265 | D66 | Ar82 |
| 3985 | D266 | D66 | Ar82 |
| 3986 | D267 | D66 | Ar82 |
| 3987 | D268 | D66 | Ar82 |
| 3988 | D269 | D66 | Ar82 |
| 3989 | D296 | D66 | Ar82 |
| 3990 | D298 | D66 | Ar82 |
| 3991 | D15 | D73 | Ar82 |
| 3992 | D16 | D73 | Ar82 |
| 3993 | D18 | D73 | Ar82 |
| 3994 | D21 | D73 | Ar82 |
| 3995 | D33 | D73 | Ar82 |
| 3996 | D34 | D73 | Ar82 |
| 3997 | D36 | D73 | Ar82 |
| 3998 | D45 | D73 | Ar82 |
| 3999 | D46 | D73 | Ar82 |
| 4000 | D48 | D73 | Ar82 |
| 4001 | D63 | D73 | Ar82 |
| 4002 | D64 | D73 | Ar82 |
| 4003 | D66 | D73 | Ar82 |
| 4004 | D73 | D73 | Ar82 |
| 4005 | D74 | D73 | Ar82 |
| 4006 | D75 | D73 | Ar82 |
| 4007 | D76 | D73 | Ar82 |
| 4008 | D78 | D73 | Ar82 |
| 4009 | D155 | D73 | Ar82 |
| 4010 | D182 | D73 | Ar82 |
| 4011 | D183 | D73 | Ar82 |
| 4012 | D185 | D73 | Ar82 |
| 4013 | D246 | D73 | Ar82 |
| 4014 | D265 | D73 | Ar82 |
| 4015 | D266 | D73 | Ar82 |
| 4016 | D267 | D73 | Ar82 |
| 4017 | D268 | D73 | Ar82 |
| 4018 | D269 | D73 | Ar82 |
| 4019 | D296 | D73 | Ar82 |
| 4020 | D298 | D73 | Ar82 |

| No. | $R^1$ = H | | |
|---|---|---|---|
| | $R^2$ | $R^3$ | $R^4$ |
| 4021 | D15 | D74 | Ar82 |
| 4022 | D16 | D74 | Ar82 |
| 4023 | D18 | D74 | Ar82 |
| 4024 | D21 | D74 | Ar82 |
| 4025 | D33 | D74 | Ar82 |
| 4026 | D34 | D74 | Ar82 |
| 4027 | D36 | D74 | Ar82 |
| 4028 | D45 | D74 | Ar82 |
| 4029 | D46 | D74 | Ar82 |
| 4030 | D48 | D74 | Ar82 |
| 4031 | D63 | D74 | Ar82 |
| 4032 | D64 | D74 | Ar82 |
| 4033 | D66 | D74 | Ar82 |
| 4034 | D73 | D74 | Ar82 |
| 4035 | D74 | D74 | Ar82 |
| 4036 | D75 | D74 | Ar82 |
| 4037 | D76 | D74 | Ar82 |
| 4038 | D78 | D74 | Ar82 |
| 4039 | D155 | D74 | Ar82 |
| 4040 | D182 | D74 | Ar82 |
| 4041 | D183 | D74 | Ar82 |
| 4042 | D185 | D74 | Ar82 |
| 4043 | D246 | D74 | Ar82 |
| 4044 | D265 | D74 | Ar82 |
| 4045 | D266 | D74 | Ar82 |
| 4046 | D267 | D74 | Ar82 |
| 4047 | D268 | D74 | Ar82 |
| 4048 | D269 | D74 | Ar82 |
| 4049 | D296 | D74 | Ar82 |
| 4050 | D298 | D74 | Ar82 |
| 4051 | D15 | D75 | Ar82 |
| 4052 | D16 | D75 | Ar82 |
| 4053 | D18 | D75 | Ar82 |
| 4054 | D21 | D75 | Ar82 |
| 4055 | D33 | D75 | Ar82 |
| 4056 | D34 | D75 | Ar82 |
| 4057 | D36 | D75 | Ar82 |
| 4058 | D45 | D75 | Ar82 |
| 4059 | D46 | D75 | Ar82 |
| 4060 | D48 | D75 | Ar82 |
| 4061 | D63 | D75 | Ar82 |
| 4062 | D64 | D75 | Ar82 |
| 4063 | D66 | D75 | Ar82 |
| 4064 | D73 | D75 | Ar82 |
| 4065 | D74 | D75 | Ar82 |
| 4066 | D75 | D75 | Ar82 |
| 4067 | D76 | D75 | Ar82 |
| 4068 | D78 | D75 | Ar82 |
| 4069 | D155 | D75 | Ar82 |
| 4070 | D182 | D75 | Ar82 |
| 4071 | D183 | D75 | Ar82 |
| 4072 | D185 | D75 | Ar82 |
| 4073 | D246 | D75 | Ar82 |
| 4074 | D265 | D75 | Ar82 |
| 4075 | D266 | D75 | Ar82 |
| 4076 | D267 | D75 | Ar82 |
| 4077 | D268 | D75 | Ar82 |
| 4078 | D269 | D75 | Ar82 |
| 4079 | D296 | D75 | Ar82 |
| 4080 | D298 | D75 | Ar82 |

| No. | $R^1$ = H | | |
|---|---|---|---|
| | $R^2$ | $R^3$ | $R^4$ |
| 4081 | D15 | D76 | Ar82 |
| 4082 | D16 | D76 | Ar82 |
| 4083 | D18 | D76 | Ar82 |
| 4084 | D21 | D76 | Ar82 |
| 4085 | D33 | D76 | Ar82 |
| 4086 | D34 | D76 | Ar82 |
| 4087 | D36 | D76 | Ar82 |
| 4088 | D45 | D76 | Ar82 |
| 4089 | D46 | D76 | Ar82 |
| 4090 | D48 | D76 | Ar82 |
| 4091 | D63 | D76 | Ar82 |
| 4092 | D64 | D76 | Ar82 |
| 4093 | D66 | D76 | Ar82 |
| 4094 | D73 | D76 | Ar82 |
| 4095 | D74 | D76 | Ar82 |
| 4096 | D75 | D76 | Ar82 |
| 4097 | D76 | D76 | Ar82 |
| 4098 | D78 | D76 | Ar82 |
| 4099 | D155 | D76 | Ar82 |
| 4100 | D182 | D76 | Ar82 |
| 4101 | D183 | D76 | Ar82 |
| 4102 | D185 | D76 | Ar82 |
| 4103 | D246 | D76 | Ar82 |
| 4104 | D265 | D76 | Ar82 |
| 4105 | D266 | D76 | Ar82 |
| 4106 | D267 | D76 | Ar82 |
| 4107 | D268 | D76 | Ar82 |
| 4108 | D269 | D76 | Ar82 |
| 4109 | D296 | D76 | Ar82 |
| 4110 | D298 | D76 | Ar82 |
| 4111 | D15 | D78 | Ar82 |
| 4112 | D16 | D78 | Ar82 |
| 4113 | D18 | D78 | Ar82 |
| 4114 | D21 | D78 | Ar82 |
| 4115 | D33 | D78 | Ar82 |
| 4116 | D34 | D78 | Ar82 |
| 4117 | D36 | D78 | Ar82 |
| 4118 | D45 | D78 | Ar82 |
| 4119 | D46 | D78 | Ar82 |
| 4120 | D48 | D78 | Ar82 |
| 4121 | D63 | D78 | Ar82 |
| 4122 | D64 | D78 | Ar82 |
| 4123 | D66 | D78 | Ar82 |
| 4124 | D73 | D78 | Ar82 |
| 4125 | D74 | D78 | Ar82 |
| 4126 | D75 | D78 | Ar82 |
| 4127 | D76 | D78 | Ar82 |
| 4128 | D78 | D78 | Ar82 |
| 4129 | D155 | D78 | Ar82 |
| 4130 | D182 | D78 | Ar82 |
| 4131 | D183 | D78 | Ar82 |
| 4132 | D185 | D78 | Ar82 |
| 4133 | D246 | D78 | Ar82 |
| 4134 | D265 | D78 | Ar82 |
| 4135 | D266 | D78 | Ar82 |
| 4136 | D267 | D78 | Ar82 |
| 4137 | D268 | D78 | Ar82 |
| 4138 | D269 | D78 | Ar82 |
| 4139 | D296 | D78 | Ar82 |
| 4140 | D298 | D78 | Ar82 |

| No. | $R^1$ = H | | |
|---|---|---|---|
| | $R^2$ | $R^3$ | $R^4$ |
| 4141 | D15 | D155 | Ar82 |
| 4142 | D16 | D155 | Ar82 |
| 4143 | D18 | D155 | Ar82 |
| 4144 | D21 | D155 | Ar82 |
| 4145 | D33 | D155 | Ar82 |
| 4146 | D34 | D155 | Ar82 |
| 4147 | D36 | D155 | Ar82 |
| 4148 | D45 | D155 | Ar82 |
| 4149 | D46 | D155 | Ar82 |
| 4150 | D48 | D155 | Ar82 |
| 4151 | D63 | D155 | Ar82 |
| 4152 | D64 | D155 | Ar82 |
| 4153 | D66 | D155 | Ar82 |
| 4154 | D73 | D155 | Ar82 |
| 4155 | D74 | D155 | Ar82 |
| 4156 | D75 | D155 | Ar82 |
| 4157 | D76 | D155 | Ar82 |
| 4158 | D78 | D155 | Ar82 |
| 4159 | D155 | D155 | Ar82 |
| 4160 | D182 | D155 | Ar82 |
| 4161 | D183 | D155 | Ar82 |
| 4162 | D185 | D155 | Ar82 |
| 4163 | D246 | D155 | Ar82 |
| 4164 | D265 | D155 | Ar82 |
| 4165 | D266 | D155 | Ar82 |
| 4166 | D267 | D155 | Ar82 |
| 4167 | D268 | D155 | Ar82 |
| 4168 | D269 | D155 | Ar82 |
| 4169 | D296 | D155 | Ar82 |
| 4170 | D298 | D155 | Ar82 |
| 4171 | D15 | D182 | Ar82 |
| 4172 | D16 | D182 | Ar82 |
| 4173 | D18 | D182 | Ar82 |
| 4174 | D21 | D182 | Ar82 |
| 4175 | D33 | D182 | Ar82 |
| 4176 | D34 | D182 | Ar82 |
| 4177 | D36 | D182 | Ar82 |
| 4178 | D45 | D182 | Ar82 |
| 4179 | D46 | D182 | Ar82 |
| 4180 | D48 | D182 | Ar82 |
| 4181 | D63 | D182 | Ar82 |
| 4182 | D64 | D182 | Ar82 |
| 4183 | D66 | D182 | Ar82 |
| 4184 | D73 | D182 | Ar82 |
| 4185 | D74 | D182 | Ar82 |
| 4186 | D75 | D182 | Ar82 |
| 4187 | D76 | D182 | Ar82 |
| 4188 | D78 | D182 | Ar82 |
| 4189 | D155 | D182 | Ar82 |
| 4190 | D182 | D182 | Ar82 |
| 4191 | D183 | D182 | Ar82 |
| 4192 | D185 | D182 | Ar82 |
| 4193 | D246 | D182 | Ar82 |
| 4194 | D265 | D182 | Ar82 |
| 4195 | D266 | D182 | Ar82 |
| 4196 | D267 | D182 | Ar82 |
| 4197 | D268 | D182 | Ar82 |
| 4198 | D269 | D182 | Ar82 |
| 4199 | D296 | D182 | Ar82 |
| 4200 | D298 | D182 | Ar82 |

| No. | $R^1$ = H | | |
|---|---|---|---|
| | $R^2$ | $R^3$ | $R^4$ |
| 4201 | D15 | D183 | Ar82 |
| 4202 | D16 | D183 | Ar82 |
| 4203 | D18 | D183 | Ar82 |
| 4204 | D21 | D183 | Ar82 |
| 4205 | D33 | D183 | Ar82 |
| 4206 | D34 | D183 | Ar82 |
| 4207 | D36 | D183 | Ar82 |
| 4208 | D45 | D183 | Ar82 |
| 4209 | D46 | D183 | Ar82 |
| 4210 | D48 | D183 | Ar82 |
| 4211 | D63 | D183 | Ar82 |
| 4212 | D64 | D183 | Ar82 |
| 4213 | D66 | D183 | Ar82 |
| 4214 | D73 | D183 | Ar82 |
| 4215 | D74 | D183 | Ar82 |
| 4216 | D75 | D183 | Ar82 |
| 4217 | D76 | D183 | Ar82 |
| 4218 | D78 | D183 | Ar82 |
| 4219 | D155 | D183 | Ar82 |
| 4220 | D182 | D183 | Ar82 |
| 4221 | D183 | D183 | Ar82 |
| 4222 | D185 | D183 | Ar82 |
| 4223 | D246 | D183 | Ar82 |
| 4224 | D265 | D183 | Ar82 |
| 4225 | D266 | D183 | Ar82 |
| 4226 | D267 | D183 | Ar82 |
| 4227 | D268 | D183 | Ar82 |
| 4228 | D269 | D183 | Ar82 |
| 4229 | D296 | D183 | Ar82 |
| 4230 | D298 | D183 | Ar82 |
| 4231 | D15 | D185 | Ar82 |
| 4232 | D16 | D185 | Ar82 |
| 4233 | D18 | D185 | Ar82 |
| 4234 | D21 | D185 | Ar82 |
| 4235 | D33 | D185 | Ar82 |
| 4236 | D34 | D185 | Ar82 |
| 4237 | D36 | D185 | Ar82 |
| 4238 | D45 | D185 | Ar82 |
| 4239 | D46 | D185 | Ar82 |
| 4240 | D48 | D185 | Ar82 |
| 4241 | D63 | D185 | Ar82 |
| 4242 | D64 | D185 | Ar82 |
| 4243 | D66 | D185 | Ar82 |
| 4244 | D73 | D185 | Ar82 |
| 4245 | D74 | D185 | Ar82 |
| 4246 | D75 | D185 | Ar82 |
| 4247 | D76 | D185 | Ar82 |
| 4248 | D78 | D185 | Ar82 |
| 4249 | D155 | D185 | Ar82 |
| 4250 | D182 | D185 | Ar82 |
| 4251 | D183 | D185 | Ar82 |
| 4252 | D185 | D185 | Ar82 |
| 4253 | D246 | D185 | Ar82 |
| 4254 | D265 | D185 | Ar82 |
| 4255 | D266 | D185 | Ar82 |
| 4256 | D267 | D185 | Ar82 |
| 4257 | D268 | D185 | Ar82 |
| 4258 | D269 | D185 | Ar82 |
| 4259 | D296 | D185 | Ar82 |
| 4260 | D298 | D185 | Ar82 |

| No. | $R^1$ = H | | |
|---|---|---|---|
| | $R^2$ | $R^3$ | $R^4$ |
| 4261 | D15 | D246 | Ar82 |
| 4262 | D16 | D246 | Ar82 |
| 4263 | D18 | D246 | Ar82 |
| 4264 | D21 | D246 | Ar82 |
| 4265 | D33 | D246 | Ar82 |
| 4266 | D34 | D246 | Ar82 |
| 4267 | D36 | D246 | Ar82 |
| 4268 | D45 | D246 | Ar82 |
| 4269 | D46 | D246 | Ar82 |
| 4270 | D48 | D246 | Ar82 |
| 4271 | D63 | D246 | Ar82 |
| 4272 | D64 | D246 | Ar82 |
| 4273 | D66 | D246 | Ar82 |
| 4274 | D73 | D246 | Ar82 |
| 4275 | D74 | D246 | Ar82 |
| 4276 | D75 | D246 | Ar82 |
| 4277 | D76 | D246 | Ar82 |
| 4278 | D78 | D246 | Ar82 |
| 4279 | D155 | D246 | Ar82 |
| 4280 | D182 | D246 | Ar82 |
| 4281 | D183 | D246 | Ar82 |
| 4282 | D185 | D246 | Ar82 |
| 4283 | D246 | D246 | Ar82 |
| 4284 | D265 | D246 | Ar82 |
| 4285 | D266 | D246 | Ar82 |
| 4286 | D267 | D246 | Ar82 |
| 4287 | D268 | D246 | Ar82 |
| 4288 | D269 | D246 | Ar82 |
| 4289 | D296 | D246 | Ar82 |
| 4290 | D298 | D246 | Ar82 |
| 4291 | D15 | D265 | Ar82 |
| 4292 | D16 | D265 | Ar82 |
| 4293 | D18 | D265 | Ar82 |
| 4294 | D21 | D265 | Ar82 |
| 4295 | D33 | D265 | Ar82 |
| 4296 | D34 | D265 | Ar82 |
| 4297 | D36 | D265 | Ar82 |
| 4298 | D45 | D265 | Ar82 |
| 4299 | D46 | D265 | Ar82 |
| 4300 | D48 | D265 | Ar82 |
| 4301 | D63 | D265 | Ar82 |
| 4302 | D64 | D265 | Ar82 |
| 4303 | D66 | D265 | Ar82 |
| 4304 | D73 | D265 | Ar82 |
| 4305 | D74 | D265 | Ar82 |
| 4306 | D75 | D265 | Ar82 |
| 4307 | D76 | D265 | Ar82 |
| 4308 | D78 | D265 | Ar82 |
| 4309 | D155 | D265 | Ar82 |
| 4310 | D182 | D265 | Ar82 |
| 4311 | D183 | D265 | Ar82 |
| 4312 | D185 | D265 | Ar82 |
| 4313 | D246 | D265 | Ar82 |
| 4314 | D265 | D265 | Ar82 |
| 4315 | D266 | D265 | Ar82 |
| 4316 | D267 | D265 | Ar82 |
| 4317 | D268 | D265 | Ar82 |
| 4318 | D269 | D265 | Ar82 |
| 4319 | D296 | D265 | Ar82 |
| 4320 | D298 | D265 | Ar82 |

| No. | $R^1$ = H | | |
|---|---|---|---|
| | $R^2$ | $R^3$ | $R^4$ |
| 4321 | D15 | D266 | Ar82 |
| 4322 | D16 | | |
| 4323 | D18 | | |
| 4324 | D21 | | |
| 4325 | D33 | | |
| 4326 | D34 | | |
| 4327 | D36 | | |
| 4328 | D45 | | |
| 4329 | D46 | | |
| 4330 | D48 | | |
| 4331 | D63 | | |
| 4332 | D64 | | |
| 4333 | D66 | | |
| 4334 | D73 | | |
| 4335 | D74 | | |
| 4336 | D75 | | |
| 4337 | D76 | | |
| 4338 | D78 | | |
| 4339 | D155 | | |
| 4340 | D182 | | |
| 4341 | D183 | | |
| 4342 | D185 | | |
| 4343 | D246 | | |
| 4344 | D265 | | |
| 4345 | D266 | | |
| 4346 | D267 | | |
| 4347 | D268 | | |
| 4348 | D269 | | |
| 4349 | D296 | | |
| 4350 | D298 | | |
| 4351 | D15 | D267 | |
| 4352 | D16 | | |
| 4353 | D18 | | |
| 4354 | D21 | | |
| 4355 | D33 | | |
| 4356 | D34 | | |
| 4357 | D36 | | |
| 4358 | D45 | | |
| 4359 | D46 | | |
| 4360 | D48 | | |
| 4361 | D63 | | |
| 4362 | D64 | | |
| 4363 | D66 | | |
| 4364 | D73 | | |
| 4365 | D74 | | |
| 4366 | D75 | | |
| 4367 | D76 | | |
| 4368 | D78 | | |
| 4369 | D155 | | |
| 4370 | D182 | | |
| 4371 | D183 | | |
| 4372 | D185 | | |
| 4373 | D246 | | |
| 4374 | D265 | | |
| 4375 | D266 | | |
| 4376 | D267 | | |
| 4377 | D268 | | |
| 4378 | D269 | | |
| 4379 | D296 | | |
| 4380 | D298 | | |

| No. | $R^1$ = H | | |
|---|---|---|---|
| | $R^2$ | $R^3$ | $R^4$ |
| 4381 | D15 | D268 | Ar82 |
| 4382 | D16 | | |
| 4383 | D18 | | |
| 4384 | D21 | | |
| 4385 | D33 | | |
| 4386 | D34 | | |
| 4387 | D36 | | |
| 4388 | D45 | | |
| 4389 | D46 | | |
| 4390 | D48 | | |
| 4391 | D63 | | |
| 4392 | D64 | | |
| 4393 | D66 | | |
| 4394 | D73 | | |
| 4395 | D74 | | |
| 4396 | D75 | | |
| 4397 | D76 | | |
| 4398 | D78 | | |
| 4399 | D155 | | |
| 4400 | D182 | | |
| 4401 | D183 | | |
| 4402 | D185 | | |
| 4403 | D246 | | |
| 4404 | D265 | | |
| 4405 | D266 | | |
| 4406 | D267 | | |
| 4407 | D268 | | |
| 4408 | D269 | | |
| 4409 | D296 | | |
| 4410 | D298 | | |
| 4411 | D15 | D269 | |
| 4412 | D16 | | |
| 4413 | D18 | | |
| 4414 | D21 | | |
| 4415 | D33 | | |
| 4416 | D34 | | |
| 4417 | D36 | | |
| 4418 | D45 | | |
| 4419 | D46 | | |
| 4420 | D48 | | |
| 4421 | D63 | | |
| 4422 | D64 | | |
| 4423 | D66 | | |
| 4424 | D73 | | |
| 4425 | D74 | | |
| 4426 | D75 | | |
| 4427 | D76 | | |
| 4428 | D78 | | |
| 4429 | D155 | | |
| 4430 | D182 | | |
| 4431 | D183 | | |
| 4432 | D185 | | |
| 4433 | D246 | | |
| 4434 | D265 | | |
| 4435 | D266 | | |
| 4436 | D267 | | |
| 4437 | D268 | | |
| 4438 | D269 | | |
| 4439 | D296 | | |
| 4440 | D298 | | |

| No. | $R^1$ = H | | |
|---|---|---|---|
| | $R^2$ | $R^3$ | $R^4$ |
| 4441 | D15 | D296 | Ar82 |
| 4442 | D16 | | |
| 4443 | D18 | | |
| 4444 | D21 | | |
| 4445 | D33 | | |
| 4446 | D34 | | |
| 4447 | D36 | | |
| 4448 | D45 | | |
| 4449 | D46 | | |
| 4450 | D48 | | |
| 4451 | D63 | | |
| 4452 | D64 | | |
| 4453 | D66 | | |
| 4454 | D73 | | |
| 4455 | D74 | | |
| 4456 | D75 | | |
| 4457 | D76 | | |
| 4458 | D78 | | |
| 4459 | D155 | | |
| 4460 | D182 | | |
| 4461 | D183 | | |
| 4462 | D185 | | |
| 4463 | D246 | | |
| 4464 | D265 | | |
| 4465 | D266 | | |
| 4466 | D267 | | |
| 4467 | D268 | | |
| 4468 | D269 | | |
| 4469 | D296 | | |
| 4470 | D298 | | |
| 4471 | D15 | D298 | |
| 4472 | D16 | | |
| 4473 | D18 | | |
| 4474 | D21 | | |
| 4475 | D33 | | |
| 4476 | D34 | | |
| 4477 | D36 | | |
| 4478 | D45 | | |
| 4479 | D46 | | |
| 4480 | D48 | | |
| 4481 | D63 | | |
| 4482 | D64 | | |
| 4483 | D66 | | |
| 4484 | D73 | | |
| 4485 | D74 | | |
| 4486 | D75 | | |
| 4487 | D76 | | |
| 4488 | D78 | | |
| 4489 | D155 | | |
| 4490 | D182 | | |
| 4491 | D183 | | |
| 4492 | D185 | | |
| 4493 | D246 | | |
| 4494 | D265 | | |
| 4495 | D266 | | |
| 4496 | D267 | | |
| 4497 | D268 | | |
| 4498 | D269 | | |
| 4499 | D296 | | |
| 4500 | D298 | | |

| No. | $R^1$ = H | | |
|---|---|---|---|
| | $R^2$ | $R^3$ | $R^4$ |
| 4501 | D15 | D15 | Ar87 |
| 4502 | D16 | | |
| 4503 | D18 | | |
| 4504 | D21 | | |
| 4505 | D33 | | |
| 4506 | D34 | | |
| 4507 | D36 | | |
| 4508 | D45 | | |
| 4509 | D46 | | |
| 4510 | D48 | | |
| 4511 | D63 | | |
| 4512 | D64 | | |
| 4513 | D66 | | |
| 4514 | D73 | | |
| 4515 | D74 | | |
| 4516 | D75 | | |
| 4517 | D76 | | |
| 4518 | D78 | | |
| 4519 | D155 | | |
| 4520 | D182 | | |
| 4521 | D183 | | |
| 4522 | D185 | | |
| 4523 | D246 | | |
| 4524 | D265 | | |
| 4525 | D266 | | |
| 4526 | D267 | | |
| 4527 | D268 | | |
| 4528 | D269 | | |
| 4529 | D296 | | |
| 4530 | D298 | | |
| 4531 | D15 | D16 | |
| 4532 | D16 | | |
| 4533 | D18 | | |
| 4534 | D21 | | |
| 4535 | D33 | | |
| 4536 | D34 | | |
| 4537 | D36 | | |
| 4538 | D45 | | |
| 4539 | D46 | | |
| 4540 | D48 | | |
| 4541 | D63 | | |
| 4542 | D64 | | |
| 4543 | D66 | | |
| 4544 | D73 | | |
| 4545 | D74 | | |
| 4546 | D75 | | |
| 4547 | D76 | | |
| 4548 | D78 | | |
| 4549 | D155 | | |
| 4550 | D182 | | |
| 4551 | D183 | | |
| 4552 | D185 | | |
| 4553 | D246 | | |
| 4554 | D265 | | |
| 4555 | D266 | | |
| 4556 | D267 | | |
| 4557 | D268 | | |
| 4558 | D269 | | |
| 4559 | D296 | | |
| 4560 | D298 | | |

| No. | $R^1$ = H | | |
|---|---|---|---|
| | $R^2$ | $R^3$ | $R^4$ |
| 4561 | D15 | D18 | Ar87 |
| 4562 | D16 | | |
| 4563 | D18 | | |
| 4564 | D21 | | |
| 4565 | D33 | | |
| 4566 | D34 | | |
| 4567 | D36 | | |
| 4568 | D45 | | |
| 4569 | D46 | | |
| 4570 | D48 | | |
| 4571 | D63 | | |
| 4572 | D64 | | |
| 4573 | D66 | | |
| 4574 | D73 | | |
| 4575 | D74 | | |
| 4576 | D75 | | |
| 4577 | D76 | | |
| 4578 | D78 | | |
| 4579 | D155 | | |
| 4580 | D182 | | |
| 4581 | D183 | | |
| 4582 | D185 | | |
| 4583 | D246 | | |
| 4584 | D265 | | |
| 4585 | D266 | | |
| 4586 | D267 | | |
| 4587 | D268 | | |
| 4588 | D269 | | |
| 4589 | D296 | | |
| 4590 | D298 | | |
| 4591 | D15 | D21 | |
| 4592 | D16 | | |
| 4593 | D18 | | |
| 4594 | D21 | | |
| 4595 | D33 | | |
| 4596 | D34 | | |
| 4597 | D36 | | |
| 4598 | D45 | | |
| 4599 | D46 | | |
| 4600 | D48 | | |
| 4601 | D63 | | |
| 4602 | D64 | | |
| 4603 | D66 | | |
| 4604 | D73 | | |
| 4605 | D74 | | |
| 4606 | D75 | | |
| 4607 | D76 | | |
| 4608 | D78 | | |
| 4609 | D155 | | |
| 4610 | D182 | | |
| 4611 | D183 | | |
| 4612 | D185 | | |
| 4613 | D246 | | |
| 4614 | D265 | | |
| 4615 | D266 | | |
| 4616 | D267 | | |
| 4617 | D268 | | |
| 4618 | D269 | | |
| 4619 | D296 | | |
| 4620 | D298 | | |

| No. | $R^1$ = H | | |
|---|---|---|---|
| | $R^2$ | $R^3$ | $R^4$ |
| 4621 | D15 | D33 | Ar87 |
| 4622 | D16 | | |
| 4623 | D18 | | |
| 4624 | D21 | | |
| 4625 | D33 | | |
| 4626 | D34 | | |
| 4627 | D36 | | |
| 4628 | D45 | | |
| 4629 | D46 | | |
| 4630 | D48 | | |
| 4631 | D63 | | |
| 4632 | D64 | | |
| 4633 | D66 | | |
| 4634 | D73 | | |
| 4635 | D74 | | |
| 4636 | D75 | | |
| 4637 | D76 | | |
| 4638 | D78 | | |
| 4639 | D155 | | |
| 4640 | D182 | | |
| 4641 | D183 | | |
| 4642 | D185 | | |
| 4643 | D246 | | |
| 4644 | D265 | | |
| 4645 | D266 | | |
| 4646 | D267 | | |
| 4647 | D268 | | |
| 4648 | D269 | | |
| 4649 | D296 | | |
| 4650 | D298 | | |
| 4651 | D15 | D34 | |
| 4652 | D16 | | |
| 4653 | D18 | | |
| 4654 | D21 | | |
| 4655 | D33 | | |
| 4656 | D34 | | |
| 4657 | D36 | | |
| 4658 | D45 | | |
| 4659 | D46 | | |
| 4660 | D48 | | |
| 4661 | D63 | | |
| 4662 | D64 | | |
| 4663 | D66 | | |
| 4664 | D73 | | |
| 4665 | D74 | | |
| 4666 | D75 | | |
| 4667 | D76 | | |
| 4668 | D78 | | |
| 4669 | D155 | | |
| 4670 | D182 | | |
| 4671 | D183 | | |
| 4672 | D185 | | |
| 4673 | D246 | | |
| 4674 | D265 | | |
| 4675 | D266 | | |
| 4676 | D267 | | |
| 4677 | D268 | | |
| 4678 | D269 | | |
| 4679 | D296 | | |
| 4680 | D298 | | |

| No. | $R^1$ = H | | |
|---|---|---|---|
| | $R^2$ | $R^3$ | $R^4$ |
| 4681 | D15 | D36 | Ar87 |
| 4682 | D16 | | |
| 4683 | D18 | | |
| 4684 | D21 | | |
| 4685 | D33 | | |
| 4686 | D34 | | |
| 4687 | D36 | | |
| 4688 | D45 | | |
| 4689 | D46 | | |
| 4690 | D48 | | |
| 4691 | D63 | | |
| 4692 | D64 | | |
| 4693 | D66 | | |
| 4694 | D73 | | |
| 4695 | D74 | | |
| 4696 | D75 | | |
| 4697 | D76 | | |
| 4698 | D78 | | |
| 4699 | D155 | | |
| 4700 | D182 | | |
| 4701 | D183 | | |
| 4702 | D185 | | |
| 4703 | D246 | | |
| 4704 | D265 | | |
| 4705 | D266 | | |
| 4706 | D267 | | |
| 4707 | D268 | | |
| 4708 | D269 | | |
| 4709 | D296 | | |
| 4710 | D298 | | |
| 4711 | D15 | D45 | |
| 4712 | D16 | | |
| 4713 | D18 | | |
| 4714 | D21 | | |
| 4715 | D33 | | |
| 4716 | D34 | | |
| 4717 | D36 | | |
| 4718 | D45 | | |
| 4719 | D46 | | |
| 4720 | D48 | | |
| 4721 | D63 | | |
| 4722 | D64 | | |
| 4723 | D66 | | |
| 4724 | D73 | | |
| 4725 | D74 | | |
| 4726 | D75 | | |
| 4727 | D76 | | |
| 4728 | D78 | | |
| 4729 | D155 | | |
| 4730 | D182 | | |
| 4731 | D183 | | |
| 4732 | D185 | | |
| 4733 | D246 | | |
| 4734 | D265 | | |
| 4735 | D266 | | |
| 4736 | D267 | | |
| 4737 | D268 | | |
| 4738 | D269 | | |
| 4739 | D296 | | |
| 4740 | D298 | | |

| No. | $R^1$ = H | | |
|---|---|---|---|
| | $R^2$ | $R^3$ | $R^4$ |
| 4741 | D15 | D46 | Ar87 |
| 4742 | D16 | | |
| 4743 | D18 | | |
| 4744 | D21 | | |
| 4745 | D33 | | |
| 4746 | D34 | | |
| 4747 | D36 | | |
| 4748 | D45 | | |
| 4749 | D46 | | |
| 4750 | D48 | | |
| 4751 | D63 | | |
| 4752 | D64 | | |
| 4753 | D66 | | |
| 4754 | D73 | | |
| 4755 | D74 | | |
| 4756 | D75 | | |
| 4757 | D76 | | |
| 4758 | D78 | | |
| 4759 | D155 | | |
| 4760 | D182 | | |
| 4761 | D183 | | |
| 4762 | D185 | | |
| 4763 | D246 | | |
| 4764 | D265 | | |
| 4765 | D266 | | |
| 4766 | D267 | | |
| 4767 | D268 | | |
| 4768 | D269 | | |
| 4769 | D296 | | |
| 4770 | D298 | | |
| 4771 | D15 | D48 | |
| 4772 | D16 | | |
| 4773 | D18 | | |
| 4774 | D21 | | |
| 4775 | D33 | | |
| 4776 | D34 | | |
| 4777 | D36 | | |
| 4778 | D45 | | |
| 4779 | D46 | | |
| 4780 | D48 | | |
| 4781 | D63 | | |
| 4782 | D64 | | |
| 4783 | D66 | | |
| 4784 | D73 | | |
| 4785 | D74 | | |
| 4786 | D75 | | |
| 4787 | D76 | | |
| 4788 | D78 | | |
| 4789 | D155 | | |
| 4790 | D182 | | |
| 4791 | D183 | | |
| 4792 | D185 | | |
| 4793 | D246 | | |
| 4794 | D265 | | |
| 4795 | D266 | | |
| 4796 | D267 | | |
| 4797 | D268 | | |
| 4798 | D269 | | |
| 4799 | D296 | | |
| 4800 | D298 | | |

| No. | $R^1$ = H | | |
|---|---|---|---|
| | $R^2$ | $R^3$ | $R^4$ |
| 4801 | D15 | D63 | Ar87 |
| 4802 | D16 | | |
| 4803 | D18 | | |
| 4804 | D21 | | |
| 4805 | D33 | | |
| 4806 | D34 | | |
| 4807 | D36 | | |
| 4808 | D45 | | |
| 4809 | D46 | | |
| 4810 | D48 | | |
| 4811 | D63 | | |
| 4812 | D64 | | |
| 4813 | D66 | | |
| 4814 | D73 | | |
| 4815 | D74 | | |
| 4816 | D75 | | |
| 4817 | D76 | | |
| 4818 | D78 | | |
| 4819 | D155 | | |
| 4820 | D182 | | |
| 4821 | D183 | | |
| 4822 | D185 | | |
| 4823 | D246 | | |
| 4824 | D265 | | |
| 4825 | D266 | | |
| 4826 | D267 | | |
| 4827 | D268 | | |
| 4828 | D269 | | |
| 4829 | D296 | | |
| 4830 | D298 | | |
| 4831 | D15 | D64 | |
| 4832 | D16 | | |
| 4833 | D18 | | |
| 4834 | D21 | | |
| 4835 | D33 | | |
| 4836 | D34 | | |
| 4837 | D36 | | |
| 4838 | D45 | | |
| 4839 | D46 | | |
| 4840 | D48 | | |
| 4841 | D63 | | |
| 4842 | D64 | | |
| 4843 | D66 | | |
| 4844 | D73 | | |
| 4845 | D74 | | |
| 4846 | D75 | | |
| 4847 | D76 | | |
| 4848 | D78 | | |
| 4849 | D155 | | |
| 4850 | D182 | | |
| 4851 | D183 | | |
| 4852 | D185 | | |
| 4853 | D246 | | |
| 4854 | D265 | | |
| 4855 | D266 | | |
| 4856 | D267 | | |
| 4857 | D268 | | |
| 4858 | D269 | | |
| 4859 | D296 | | |
| 4860 | D298 | | |

| No. | $R^1$ = H | | |
|---|---|---|---|
| | $R^2$ | $R^3$ | $R^4$ |
| 4861 | D15 | D66 | Ar87 |
| 4862 | D16 | | |
| 4863 | D18 | | |
| 4864 | D21 | | |
| 4865 | D33 | | |
| 4866 | D34 | | |
| 4867 | D36 | | |
| 4868 | D45 | | |
| 4869 | D46 | | |
| 4870 | D48 | | |
| 4871 | D63 | | |
| 4872 | D64 | | |
| 4873 | D66 | | |
| 4874 | D73 | | |
| 4875 | D74 | | |
| 4876 | D75 | | |
| 4877 | D76 | | |
| 4878 | D78 | | |
| 4879 | D155 | | |
| 4880 | D182 | | |
| 4881 | D183 | | |
| 4882 | D185 | | |
| 4883 | D246 | | |
| 4884 | D265 | | |
| 4885 | D266 | | |
| 4886 | D267 | | |
| 4887 | D268 | | |
| 4888 | D269 | | |
| 4889 | D296 | | |
| 4890 | D298 | | |
| 4891 | D15 | D73 | |
| 4892 | D16 | | |
| 4893 | D18 | | |
| 4894 | D21 | | |
| 4895 | D33 | | |
| 4896 | D34 | | |
| 4897 | D36 | | |
| 4898 | D45 | | |
| 4899 | D46 | | |
| 4900 | D48 | | |
| 4901 | D63 | | |
| 4902 | D64 | | |
| 4903 | D66 | | |
| 4904 | D73 | | |
| 4905 | D74 | | |
| 4906 | D75 | | |
| 4907 | D76 | | |
| 4908 | D78 | | |
| 4909 | D155 | | |
| 4910 | D182 | | |
| 4911 | D183 | | |
| 4912 | D185 | | |
| 4913 | D246 | | |
| 4914 | D265 | | |
| 4915 | D266 | | |
| 4916 | D267 | | |
| 4917 | D268 | | |
| 4918 | D269 | | |
| 4919 | D296 | | |
| 4920 | D298 | | |

| No. | $R^1$ = H | | |
|---|---|---|---|
| | $R^2$ | $R^3$ | $R^4$ |
| 4921 | D15 | D74 | Ar87 |
| 4922 | D16 | | |
| 4923 | D18 | | |
| 4924 | D21 | | |
| 4925 | D33 | | |
| 4926 | D34 | | |
| 4927 | D36 | | |
| 4928 | D45 | | |
| 4929 | D46 | | |
| 4930 | D48 | | |
| 4931 | D63 | | |
| 4932 | D64 | | |
| 4933 | D66 | | |
| 4934 | D73 | | |
| 4935 | D74 | | |
| 4936 | D75 | | |
| 4937 | D76 | | |
| 4938 | D78 | | |
| 4939 | D155 | | |
| 4940 | D182 | | |
| 4941 | D183 | | |
| 4942 | D185 | | |
| 4943 | D246 | | |
| 4944 | D265 | | |
| 4945 | D266 | | |
| 4946 | D267 | | |
| 4947 | D268 | | |
| 4948 | D269 | | |
| 4949 | D296 | | |
| 4950 | D298 | | |
| 4951 | D15 | D75 | |
| 4952 | D16 | | |
| 4953 | D18 | | |
| 4954 | D21 | | |
| 4955 | D33 | | |
| 4956 | D34 | | |
| 4957 | D36 | | |
| 4958 | D45 | | |
| 4959 | D46 | | |
| 4960 | D48 | | |
| 4961 | D63 | | |
| 4962 | D64 | | |
| 4963 | D66 | | |
| 4964 | D73 | | |
| 4965 | D74 | | |
| 4966 | D75 | | |
| 4967 | D76 | | |
| 4968 | D78 | | |
| 4969 | D155 | | |
| 4970 | D182 | | |
| 4971 | D183 | | |
| 4972 | D185 | | |
| 4973 | D246 | | |
| 4974 | D265 | | |
| 4975 | D266 | | |
| 4976 | D267 | | |
| 4977 | D268 | | |
| 4978 | D269 | | |
| 4979 | D296 | | |
| 4980 | D298 | | |

| No. | $R^1$ = H | | |
|---|---|---|---|
| | $R^2$ | $R^3$ | $R^4$ |
| 4981 | D15 | D76 | Ar87 |
| 4982 | D16 | | |
| 4983 | D18 | | |
| 4984 | D21 | | |
| 4985 | D33 | | |
| 4986 | D34 | | |
| 4987 | D36 | | |
| 4988 | D45 | | |
| 4989 | D46 | | |
| 4990 | D48 | | |
| 4991 | D63 | | |
| 4992 | D64 | | |
| 4993 | D66 | | |
| 4994 | D73 | | |
| 4995 | D74 | | |
| 4996 | D75 | | |
| 4997 | D76 | | |
| 4998 | D78 | | |
| 4999 | D155 | | |
| 5000 | D182 | | |
| 5001 | D183 | | |
| 5002 | D185 | | |
| 5003 | D246 | | |
| 5004 | D265 | | |
| 5005 | D266 | | |
| 5006 | D267 | | |
| 5007 | D268 | | |
| 5008 | D269 | | |
| 5009 | D296 | | |
| 5010 | D298 | | |
| 5011 | D15 | D78 | |
| 5012 | D16 | | |
| 5013 | D18 | | |
| 5014 | D21 | | |
| 5015 | D33 | | |
| 5016 | D34 | | |
| 5017 | D36 | | |
| 5018 | D45 | | |
| 5019 | D46 | | |
| 5020 | D48 | | |
| 5021 | D63 | | |
| 5022 | D64 | | |
| 5023 | D66 | | |
| 5024 | D73 | | |
| 5025 | D74 | | |
| 5026 | D75 | | |
| 5027 | D76 | | |
| 5028 | D78 | | |
| 5029 | D155 | | |
| 5030 | D182 | | |
| 5031 | D183 | | |
| 5032 | D185 | | |
| 5033 | D246 | | |
| 5034 | D265 | | |
| 5035 | D266 | | |
| 5036 | D267 | | |
| 5037 | D268 | | |
| 5038 | D269 | | |
| 5039 | D296 | | |
| 5040 | D298 | | |

| No. | $R^1$ = H | | |
|---|---|---|---|
| | $R^2$ | $R^3$ | $R^4$ |
| 5041 | D15 | D155 | Ar87 |
| 5042 | D16 | | |
| 5043 | D18 | | |
| 5044 | D21 | | |
| 5045 | D33 | | |
| 5046 | D34 | | |
| 5047 | D36 | | |
| 5048 | D45 | | |
| 5049 | D46 | | |
| 5050 | D48 | | |
| 5051 | D63 | | |
| 5052 | D64 | | |
| 5053 | D66 | | |
| 5054 | D73 | | |
| 5055 | D74 | | |
| 5056 | D75 | | |
| 5057 | D76 | | |
| 5058 | D78 | | |
| 5059 | D155 | | |
| 5060 | D182 | | |
| 5061 | D183 | | |
| 5062 | D185 | | |
| 5063 | D246 | | |
| 5064 | D265 | | |
| 5065 | D266 | | |
| 5066 | D267 | | |
| 5067 | D268 | | |
| 5068 | D269 | | |
| 5069 | D296 | | |
| 5070 | D298 | | |
| 5071 | D15 | D182 | |
| 5072 | D16 | | |
| 5073 | D18 | | |
| 5074 | D21 | | |
| 5075 | D33 | | |
| 5076 | D34 | | |
| 5077 | D36 | | |
| 5078 | D45 | | |
| 5079 | D46 | | |
| 5080 | D48 | | |
| 5081 | D63 | | |
| 5082 | D64 | | |
| 5083 | D66 | | |
| 5084 | D73 | | |
| 5085 | D74 | | |
| 5086 | D75 | | |
| 5087 | D76 | | |
| 5088 | D78 | | |
| 5089 | D155 | | |
| 5090 | D182 | | |
| 5091 | D183 | | |
| 5092 | D185 | | |
| 5093 | D246 | | |
| 5094 | D265 | | |
| 5095 | D266 | | |
| 5096 | D267 | | |
| 5097 | D268 | | |
| 5098 | D269 | | |
| 5099 | D296 | | |
| 5100 | D298 | | |

| No. | $R^1$ = H | | |
|---|---|---|---|
| | $R^2$ | $R^3$ | $R^4$ |
| 5101 | D15 | D183 | Ar87 |
| 5102 | D16 | | |
| 5103 | D18 | | |
| 5104 | D21 | | |
| 5105 | D33 | | |
| 5106 | D34 | | |
| 5107 | D36 | | |
| 5108 | D45 | | |
| 5109 | D46 | | |
| 5110 | D48 | | |
| 5111 | D63 | | |
| 5112 | D64 | | |
| 5113 | D66 | | |
| 5114 | D73 | | |
| 5115 | D74 | | |
| 5116 | D75 | | |
| 5117 | D76 | | |
| 5118 | D78 | | |
| 5119 | D155 | | |
| 5120 | D182 | | |
| 5121 | D183 | | |
| 5122 | D185 | | |
| 5123 | D246 | | |
| 5124 | D265 | | |
| 5125 | D266 | | |
| 5126 | D267 | | |
| 5127 | D268 | | |
| 5128 | D269 | | |
| 5129 | D296 | | |
| 5130 | D298 | | |
| 5131 | D15 | D185 | |
| 5132 | D16 | | |
| 5133 | D18 | | |
| 5134 | D21 | | |
| 5135 | D33 | | |
| 5136 | D34 | | |
| 5137 | D36 | | |
| 5138 | D45 | | |
| 5139 | D46 | | |
| 5140 | D48 | | |
| 5141 | D63 | | |
| 5142 | D64 | | |
| 5143 | D66 | | |
| 5144 | D73 | | |
| 5145 | D74 | | |
| 5146 | D75 | | |
| 5147 | D76 | | |
| 5148 | D78 | | |
| 5149 | D155 | | |
| 5150 | D182 | | |
| 5151 | D183 | | |
| 5152 | D185 | | |
| 5153 | D246 | | |
| 5154 | D265 | | |
| 5155 | D266 | | |
| 5156 | D267 | | |
| 5157 | D268 | | |
| 5158 | D269 | | |
| 5159 | D296 | | |
| 5160 | D298 | | |

| No. | $R^1$ = H | | |
|---|---|---|---|
| | $R^2$ | $R^3$ | $R^4$ |
| 5161 | D15 | D246 | Ar87 |
| 5162 | D16 | | |
| 5163 | D18 | | |
| 5164 | D21 | | |
| 5165 | D33 | | |
| 5166 | D34 | | |
| 5167 | D36 | | |
| 5168 | D45 | | |
| 5169 | D46 | | |
| 5170 | D48 | | |
| 5171 | D63 | | |
| 5172 | D64 | | |
| 5173 | D66 | | |
| 5174 | D73 | | |
| 5175 | D74 | | |
| 5176 | D75 | | |
| 5177 | D76 | | |
| 5178 | D78 | | |
| 5179 | D155 | | |
| 5180 | D182 | | |
| 5181 | D183 | | |
| 5182 | D185 | | |
| 5183 | D246 | | |
| 5184 | D265 | | |
| 5185 | D266 | | |
| 5186 | D267 | | |
| 5187 | D268 | | |
| 5188 | D269 | | |
| 5189 | D296 | | |
| 5190 | D298 | | |
| 5191 | D15 | D265 | |
| 5192 | D16 | | |
| 5193 | D18 | | |
| 5194 | D21 | | |
| 5195 | D33 | | |
| 5196 | D34 | | |
| 5197 | D36 | | |
| 5198 | D45 | | |
| 5199 | D46 | | |
| 5200 | D48 | | |
| 5201 | D63 | | |
| 5202 | D64 | | |
| 5203 | D66 | | |
| 5204 | D73 | | |
| 5205 | D74 | | |
| 5206 | D75 | | |
| 5207 | D76 | | |
| 5208 | D78 | | |
| 5209 | D155 | | |
| 5210 | D182 | | |
| 5211 | D183 | | |
| 5212 | D185 | | |
| 5213 | D246 | | |
| 5214 | D265 | | |
| 5215 | D266 | | |
| 5216 | D267 | | |
| 5217 | D268 | | |
| 5218 | D269 | | |
| 5219 | D296 | | |
| 5220 | D298 | | |

| No. | $R^1$ = H | | |
|---|---|---|---|
| | $R^2$ | $R^3$ | $R^4$ |
| 5221 | D15 | D266 | Ar87 |
| 5222 | D16 | | |
| 5223 | D18 | | |
| 5224 | D21 | | |
| 5225 | D33 | | |
| 5226 | D34 | | |
| 5227 | D36 | | |
| 5228 | D45 | | |
| 5229 | D46 | | |
| 5230 | D48 | | |
| 5231 | D63 | | |
| 5232 | D64 | | |
| 5233 | D66 | | |
| 5234 | D73 | | |
| 5235 | D74 | | |
| 5236 | D75 | | |
| 5237 | D76 | | |
| 5238 | D78 | | |
| 5239 | D155 | | |
| 5240 | D182 | | |
| 5241 | D183 | | |
| 5242 | D185 | | |
| 5243 | D246 | | |
| 5244 | D265 | | |
| 5245 | D266 | | |
| 5246 | D267 | | |
| 5247 | D268 | | |
| 5248 | D269 | | |
| 5249 | D296 | | |
| 5250 | D298 | | |
| 5251 | D15 | D267 | |
| 5252 | D16 | | |
| 5253 | D18 | | |
| 5254 | D21 | | |
| 5255 | D33 | | |
| 5256 | D34 | | |
| 5257 | D36 | | |
| 5258 | D45 | | |
| 5259 | D46 | | |
| 5260 | D48 | | |
| 5261 | D63 | | |
| 5262 | D64 | | |
| 5263 | D66 | | |
| 5264 | D73 | | |
| 5265 | D74 | | |
| 5266 | D75 | | |
| 5267 | D76 | | |
| 5268 | D78 | | |
| 5269 | D155 | | |
| 5270 | D182 | | |
| 5271 | D183 | | |
| 5272 | D185 | | |
| 5273 | D246 | | |
| 5274 | D265 | | |
| 5275 | D266 | | |
| 5276 | D267 | | |
| 5277 | D268 | | |
| 5278 | D269 | | |
| 5279 | D296 | | |
| 5280 | D298 | | |

| No. | $R^1$ = H | | |
|---|---|---|---|
| | $R^2$ | $R^3$ | $R^4$ |
| 5281 | D15 | D268 | Ar87 |
| 5282 | D16 | | |
| 5283 | D18 | | |
| 5284 | D21 | | |
| 5285 | D33 | | |
| 5286 | D34 | | |
| 5287 | D36 | | |
| 5288 | D45 | | |
| 5289 | D46 | | |
| 5290 | D48 | | |
| 5291 | D63 | | |
| 5292 | D64 | | |
| 5293 | D66 | | |
| 5294 | D73 | | |
| 5295 | D74 | | |
| 5296 | D75 | | |
| 5297 | D76 | | |
| 5298 | D78 | | |
| 5299 | D155 | | |
| 5300 | D182 | | |
| 5301 | D183 | | |
| 5302 | D185 | | |
| 5303 | D246 | | |
| 5304 | D265 | | |
| 5305 | D266 | | |
| 5306 | D267 | | |
| 5307 | D268 | | |
| 5308 | D269 | | |
| 5309 | D296 | | |
| 5310 | D298 | | |
| 5311 | D15 | D269 | |
| 5312 | D16 | | |
| 5313 | D18 | | |
| 5314 | D21 | | |
| 5315 | D33 | | |
| 5316 | D34 | | |
| 5317 | D36 | | |
| 5318 | D45 | | |
| 5319 | D46 | | |
| 5320 | D48 | | |
| 5321 | D63 | | |
| 5322 | D64 | | |
| 5323 | D66 | | |
| 5324 | D73 | | |
| 5325 | D74 | | |
| 5326 | D75 | | |
| 5327 | D76 | | |
| 5328 | D78 | | |
| 5329 | D155 | | |
| 5330 | D182 | | |
| 5331 | D183 | | |
| 5332 | D185 | | |
| 5333 | D246 | | |
| 5334 | D265 | | |
| 5335 | D266 | | |
| 5336 | D267 | | |
| 5337 | D268 | | |
| 5338 | D269 | | |
| 5339 | D296 | | |
| 5340 | D298 | | |

| No. | $R^1$ = H | | |
|---|---|---|---|
| | $R^2$ | $R^3$ | $R^4$ |
| 5341 | D15 | D296 | Ar87 |
| 5342 | D16 | | |
| 5343 | D18 | | |
| 5344 | D21 | | |
| 5345 | D33 | | |
| 5346 | D34 | | |
| 5347 | D36 | | |
| 5348 | D45 | | |
| 5349 | D46 | | |
| 5350 | D48 | | |
| 5351 | D63 | | |
| 5352 | D64 | | |
| 5353 | D66 | | |
| 5354 | D73 | | |
| 5355 | D74 | | |
| 5356 | D75 | | |
| 5357 | D76 | | |
| 5358 | D78 | | |
| 5359 | D155 | | |
| 5360 | D182 | | |
| 5361 | D183 | | |
| 5362 | D185 | | |
| 5363 | D246 | | |
| 5364 | D265 | | |
| 5365 | D266 | | |
| 5366 | D267 | | |
| 5367 | D268 | | |
| 5368 | D269 | | |
| 5369 | D296 | | |
| 5370 | D298 | | |
| 5371 | D15 | D298 | |
| 5372 | D16 | | |
| 5373 | D18 | | |
| 5374 | D21 | | |
| 5375 | D33 | | |
| 5376 | D34 | | |
| 5377 | D36 | | |
| 5378 | D45 | | |
| 5379 | D46 | | |
| 5380 | D48 | | |
| 5381 | D63 | | |
| 5382 | D64 | | |
| 5383 | D66 | | |
| 5384 | D73 | | |
| 5385 | D74 | | |
| 5386 | D75 | | |
| 5387 | D76 | | |
| 5388 | D78 | | |
| 5389 | D155 | | |
| 5390 | D182 | | |
| 5391 | D183 | | |
| 5392 | D185 | | |
| 5393 | D246 | | |
| 5394 | D265 | | |
| 5395 | D266 | | |
| 5396 | D267 | | |
| 5397 | D268 | | |
| 5398 | D269 | | |
| 5399 | D296 | | |
| 5400 | D298 | | |

| No. | $R^1$ = H | | |
|---|---|---|---|
| | $R^2$ | $R^3$ | $R^4$ |
| 5401 | D15 | D15 | Ar88 |
| 5402 | D16 | | |
| 5403 | D18 | | |
| 5404 | D21 | | |
| 5405 | D33 | | |
| 5406 | D34 | | |
| 5407 | D36 | | |
| 5408 | D45 | | |
| 5409 | D46 | | |
| 5410 | D48 | | |
| 5411 | D63 | | |
| 5412 | D64 | | |
| 5413 | D66 | | |
| 5414 | D73 | | |
| 5415 | D74 | | |
| 5416 | D75 | | |
| 5417 | D76 | | |
| 5418 | D78 | | |
| 5419 | D155 | | |
| 5420 | D182 | | |
| 5421 | D183 | | |
| 5422 | D185 | | |
| 5423 | D246 | | |
| 5424 | D265 | | |
| 5425 | D266 | | |
| 5426 | D267 | | |
| 5427 | D268 | | |
| 5428 | D269 | | |
| 5429 | D296 | | |
| 5430 | D298 | | |
| 5431 | D15 | D16 | |
| 5432 | D16 | | |
| 5433 | D18 | | |
| 5434 | D21 | | |
| 5435 | D33 | | |
| 5436 | D34 | | |
| 5437 | D36 | | |
| 5438 | D45 | | |
| 5439 | D46 | | |
| 5440 | D48 | | |
| 5441 | D63 | | |
| 5442 | D64 | | |
| 5443 | D66 | | |
| 5444 | D73 | | |
| 5445 | D74 | | |
| 5446 | D75 | | |
| 5447 | D76 | | |
| 5448 | D78 | | |
| 5449 | D155 | | |
| 5450 | D182 | | |
| 5451 | D183 | | |
| 5452 | D185 | | |
| 5453 | D246 | | |
| 5454 | D265 | | |
| 5455 | D266 | | |
| 5456 | D267 | | |
| 5457 | D268 | | |
| 5458 | D269 | | |
| 5459 | D296 | | |
| 5460 | D298 | | |

| No. | $R^1$ = H | | |
|---|---|---|---|
| | $R^2$ | $R^3$ | $R^4$ |
| 5461 | D15 | D18 | Ar88 |
| 5462 | D16 | | |
| 5463 | D18 | | |
| 5464 | D21 | | |
| 5465 | D33 | | |
| 5466 | D34 | | |
| 5467 | D36 | | |
| 5468 | D45 | | |
| 5469 | D46 | | |
| 5470 | D48 | | |
| 5471 | D63 | | |
| 5472 | D64 | | |
| 5473 | D66 | | |
| 5474 | D73 | | |
| 5475 | D74 | | |
| 5476 | D75 | | |
| 5477 | D76 | | |
| 5478 | D78 | | |
| 5479 | D155 | | |
| 5480 | D182 | | |
| 5481 | D183 | | |
| 5482 | D185 | | |
| 5483 | D246 | | |
| 5484 | D265 | | |
| 5485 | D266 | | |
| 5486 | D267 | | |
| 5487 | D268 | | |
| 5488 | D269 | | |
| 5489 | D296 | | |
| 5490 | D298 | | |
| 5491 | D15 | D21 | |
| 5492 | D16 | | |
| 5493 | D18 | | |
| 5494 | D21 | | |
| 5495 | D33 | | |
| 5496 | D34 | | |
| 5497 | D36 | | |
| 5498 | D45 | | |
| 5499 | D46 | | |
| 5500 | D48 | | |
| 5501 | D63 | | |
| 5502 | D64 | | |
| 5503 | D66 | | |
| 5504 | D73 | | |
| 5505 | D74 | | |
| 5506 | D75 | | |
| 5507 | D76 | | |
| 5508 | D78 | | |
| 5509 | D155 | | |
| 5510 | D182 | | |
| 5511 | D183 | | |
| 5512 | D185 | | |
| 5513 | D246 | | |
| 5514 | D265 | | |
| 5515 | D266 | | |
| 5516 | D267 | | |
| 5517 | D268 | | |
| 5518 | D269 | | |
| 5519 | D296 | | |
| 5520 | D298 | | |

| No. | $R^1$ = H | | |
|---|---|---|---|
| | $R^2$ | $R^3$ | $R^4$ |
| 5521 | D15 | D33 | Ar88 |
| 5522 | D16 | | |
| 5523 | D18 | | |
| 5524 | D21 | | |
| 5525 | D33 | | |
| 5526 | D34 | | |
| 5527 | D36 | | |
| 5528 | D45 | | |
| 5529 | D46 | | |
| 5530 | D48 | | |
| 5531 | D63 | | |
| 5532 | D64 | | |
| 5533 | D66 | | |
| 5534 | D73 | | |
| 5535 | D74 | | |
| 5536 | D75 | | |
| 5537 | D76 | | |
| 5538 | D78 | | |
| 5539 | D155 | | |
| 5540 | D182 | | |
| 5541 | D183 | | |
| 5542 | D185 | | |
| 5543 | D246 | | |
| 5544 | D265 | | |
| 5545 | D266 | | |
| 5546 | D267 | | |
| 5547 | D268 | | |
| 5548 | D269 | | |
| 5549 | D296 | | |
| 5550 | D298 | | |
| 5551 | D15 | D34 | |
| 5552 | D16 | | |
| 5553 | D18 | | |
| 5554 | D21 | | |
| 5555 | D33 | | |
| 5556 | D34 | | |
| 5557 | D36 | | |
| 5558 | D45 | | |
| 5559 | D46 | | |
| 5560 | D48 | | |
| 5561 | D63 | | |
| 5562 | D64 | | |
| 5563 | D66 | | |
| 5564 | D73 | | |
| 5565 | D74 | | |
| 5566 | D75 | | |
| 5567 | D76 | | |
| 5568 | D78 | | |
| 5569 | D155 | | |
| 5570 | D182 | | |
| 5571 | D183 | | |
| 5572 | D185 | | |
| 5573 | D246 | | |
| 5574 | D265 | | |
| 5575 | D266 | | |
| 5576 | D267 | | |
| 5577 | D268 | | |
| 5578 | D269 | | |
| 5579 | D296 | | |
| 5580 | D298 | | |

| No. | $R^1$ = H | | |
|---|---|---|---|
| | $R^2$ | $R^3$ | $R^4$ |
| 5581 | D15 | D36 | Ar88 |
| 5582 | D16 | | |
| 5583 | D18 | | |
| 5584 | D21 | | |
| 5585 | D33 | | |
| 5586 | D34 | | |
| 5587 | D36 | | |
| 5588 | D45 | | |
| 5589 | D46 | | |
| 5590 | D48 | | |
| 5591 | D63 | | |
| 5592 | D64 | | |
| 5593 | D66 | | |
| 5594 | D73 | | |
| 5595 | D74 | | |
| 5596 | D75 | | |
| 5597 | D76 | | |
| 5598 | D78 | | |
| 5599 | D155 | | |
| 5600 | D182 | | |
| 5601 | D183 | | |
| 5602 | D185 | | |
| 5603 | D246 | | |
| 5604 | D265 | | |
| 5605 | D266 | | |
| 5606 | D267 | | |
| 5607 | D268 | | |
| 5608 | D269 | | |
| 5609 | D296 | | |
| 5610 | D298 | | |
| 5611 | D15 | D45 | |
| 5612 | D16 | | |
| 5613 | D18 | | |
| 5614 | D21 | | |
| 5615 | D33 | | |
| 5616 | D34 | | |
| 5617 | D36 | | |
| 5618 | D45 | | |
| 5619 | D46 | | |
| 5620 | D48 | | |
| 5621 | D63 | | |
| 5622 | D64 | | |
| 5623 | D66 | | |
| 5624 | D73 | | |
| 5625 | D74 | | |
| 5626 | D75 | | |
| 5627 | D76 | | |
| 5628 | D78 | | |
| 5629 | D155 | | |
| 5630 | D182 | | |
| 5631 | D183 | | |
| 5632 | D185 | | |
| 5633 | D246 | | |
| 5634 | D265 | | |
| 5635 | D266 | | |
| 5636 | D267 | | |
| 5637 | D268 | | |
| 5638 | D269 | | |
| 5639 | D296 | | |
| 5640 | D298 | | |

| No. | $R^1$ = H | | |
|---|---|---|---|
| | $R^2$ | $R^3$ | $R^4$ |
| 5641 | D15 | D46 | Ar88 |
| 5642 | D16 | | |
| 5643 | D18 | | |
| 5644 | D21 | | |
| 5645 | D33 | | |
| 5646 | D34 | | |
| 5647 | D36 | | |
| 5648 | D45 | | |
| 5649 | D46 | | |
| 5650 | D48 | | |
| 5651 | D63 | | |
| 5652 | D64 | | |
| 5653 | D66 | | |
| 5654 | D73 | | |
| 5655 | D74 | | |
| 5656 | D75 | | |
| 5657 | D76 | | |
| 5658 | D78 | | |
| 5659 | D155 | | |
| 5660 | D182 | | |
| 5661 | D183 | | |
| 5662 | D185 | | |
| 5663 | D246 | | |
| 5664 | D265 | | |
| 5665 | D266 | | |
| 5666 | D267 | | |
| 5667 | D268 | | |
| 5668 | D269 | | |
| 5669 | D296 | | |
| 5670 | D298 | | |
| 5671 | D15 | D48 | |
| 5672 | D16 | | |
| 5673 | D18 | | |
| 5674 | D21 | | |
| 5675 | D33 | | |
| 5676 | D34 | | |
| 5677 | D36 | | |
| 5678 | D45 | | |
| 5679 | D46 | | |
| 5680 | D48 | | |
| 5681 | D63 | | |
| 5682 | D64 | | |
| 5683 | D66 | | |
| 5684 | D73 | | |
| 5685 | D74 | | |
| 5686 | D75 | | |
| 5687 | D76 | | |
| 5688 | D78 | | |
| 5689 | D155 | | |
| 5690 | D182 | | |
| 5691 | D183 | | |
| 5692 | D185 | | |
| 5693 | D246 | | |
| 5694 | D265 | | |
| 5695 | D266 | | |
| 5696 | D267 | | |
| 5697 | D268 | | |
| 5698 | D269 | | |
| 5699 | D296 | | |
| 5700 | D298 | | |

| No. | $R^1$ = H | | |
|---|---|---|---|
| | $R^2$ | $R^3$ | $R^4$ |
| 5701 | D15 | D63 | Ar88 |
| 5702 | D16 | | |
| 5703 | D18 | | |
| 5704 | D21 | | |
| 5705 | D33 | | |
| 5706 | D34 | | |
| 5707 | D36 | | |
| 5708 | D45 | | |
| 5709 | D46 | | |
| 5710 | D48 | | |
| 5711 | D63 | | |
| 5712 | D64 | | |
| 5713 | D66 | | |
| 5714 | D73 | | |
| 5715 | D74 | | |
| 5716 | D75 | | |
| 5717 | D76 | | |
| 5718 | D78 | | |
| 5719 | D155 | | |
| 5720 | D182 | | |
| 5721 | D183 | | |
| 5722 | D185 | | |
| 5723 | D246 | | |
| 5724 | D265 | | |
| 5725 | D266 | | |
| 5726 | D267 | | |
| 5727 | D268 | | |
| 5728 | D269 | | |
| 5729 | D296 | | |
| 5730 | D298 | | |
| 5731 | D15 | D64 | |
| 5732 | D16 | | |
| 5733 | D18 | | |
| 5734 | D21 | | |
| 5735 | D33 | | |
| 5736 | D34 | | |
| 5737 | D36 | | |
| 5738 | D45 | | |
| 5739 | D46 | | |
| 5740 | D48 | | |
| 5741 | D63 | | |
| 5742 | D64 | | |
| 5743 | D66 | | |
| 5744 | D73 | | |
| 5745 | D74 | | |
| 5746 | D75 | | |
| 5747 | D76 | | |
| 5748 | D78 | | |
| 5749 | D155 | | |
| 5750 | D182 | | |
| 5751 | D183 | | |
| 5752 | D185 | | |
| 5753 | D246 | | |
| 5754 | D265 | | |
| 5755 | D266 | | |
| 5756 | D267 | | |
| 5757 | D268 | | |
| 5758 | D269 | | |
| 5759 | D296 | | |
| 5760 | D298 | | |

| No. | $R^1$ = H | | |
|---|---|---|---|
| | $R^2$ | $R^3$ | $R^4$ |
| 5761 | D15 | D66 | Ar88 |
| 5762 | D16 | | |
| 5763 | D18 | | |
| 5764 | D21 | | |
| 5765 | D33 | | |
| 5766 | D34 | | |
| 5767 | D36 | | |
| 5768 | D45 | | |
| 5769 | D46 | | |
| 5770 | D48 | | |
| 5771 | D63 | | |
| 5772 | D64 | | |
| 5773 | D66 | | |
| 5774 | D73 | | |
| 5775 | D74 | | |
| 5776 | D75 | | |
| 5777 | D76 | | |
| 5778 | D78 | | |
| 5779 | D155 | | |
| 5780 | D182 | | |
| 5781 | D183 | | |
| 5782 | D185 | | |
| 5783 | D246 | | |
| 5784 | D265 | | |
| 5785 | D266 | | |
| 5786 | D267 | | |
| 5787 | D268 | | |
| 5788 | D269 | | |
| 5789 | D296 | | |
| 5790 | D298 | | |
| 5791 | D15 | D73 | |
| 5792 | D16 | | |
| 5793 | D18 | | |
| 5794 | D21 | | |
| 5795 | D33 | | |
| 5796 | D34 | | |
| 5797 | D36 | | |
| 5798 | D45 | | |
| 5799 | D46 | | |
| 5800 | D48 | | |
| 5801 | D63 | | |
| 5802 | D64 | | |
| 5803 | D66 | | |
| 5804 | D73 | | |
| 5805 | D74 | | |
| 5806 | D75 | | |
| 5807 | D76 | | |
| 5808 | D78 | | |
| 5809 | D155 | | |
| 5810 | D182 | | |
| 5811 | D183 | | |
| 5812 | D185 | | |
| 5813 | D246 | | |
| 5814 | D265 | | |
| 5815 | D266 | | |
| 5816 | D267 | | |
| 5817 | D268 | | |
| 5818 | D269 | | |
| 5819 | D296 | | |
| 5820 | D298 | | |

| No. | $R^1$ = H | | |
|---|---|---|---|
| | $R^2$ | $R^3$ | $R^4$ |
| 5821 | D15 | D74 | Ar88 |
| 5822 | D16 | | |
| 5823 | D18 | | |
| 5824 | D21 | | |
| 5825 | D33 | | |
| 5826 | D34 | | |
| 5827 | D36 | | |
| 5828 | D45 | | |
| 5829 | D46 | | |
| 5830 | D48 | | |
| 5831 | D63 | | |
| 5832 | D64 | | |
| 5833 | D66 | | |
| 5834 | D73 | | |
| 5835 | D74 | | |
| 5836 | D75 | | |
| 5837 | D76 | | |
| 5838 | D78 | | |
| 5839 | D155 | | |
| 5840 | D182 | | |
| 5841 | D183 | | |
| 5842 | D185 | | |
| 5843 | D246 | | |
| 5844 | D265 | | |
| 5845 | D266 | | |
| 5846 | D267 | | |
| 5847 | D268 | | |
| 5848 | D269 | | |
| 5849 | D296 | | |
| 5850 | D298 | | |
| 5851 | D15 | D75 | |
| 5852 | D16 | | |
| 5853 | D18 | | |
| 5854 | D21 | | |
| 5855 | D33 | | |
| 5856 | D34 | | |
| 5857 | D36 | | |
| 5858 | D45 | | |
| 5859 | D46 | | |
| 5860 | D48 | | |
| 5861 | D63 | | |
| 5862 | D64 | | |
| 5863 | D66 | | |
| 5864 | D73 | | |
| 5865 | D74 | | |
| 5866 | D75 | | |
| 5867 | D76 | | |
| 5868 | D78 | | |
| 5869 | D155 | | |
| 5870 | D182 | | |
| 5871 | D183 | | |
| 5872 | D185 | | |
| 5873 | D246 | | |
| 5874 | D265 | | |
| 5875 | D266 | | |
| 5876 | D267 | | |
| 5877 | D268 | | |
| 5878 | D269 | | |
| 5879 | D296 | | |
| 5880 | D298 | | |

| No. | $R^1$ = H | | |
|---|---|---|---|
| | $R^2$ | $R^3$ | $R^4$ |
| 5881 | D15 | D76 | Ar88 |
| 5882 | D16 | | |
| 5883 | D18 | | |
| 5884 | D21 | | |
| 5885 | D33 | | |
| 5886 | D34 | | |
| 5887 | D36 | | |
| 5888 | D45 | | |
| 5889 | D46 | | |
| 5890 | D48 | | |
| 5891 | D63 | | |
| 5892 | D64 | | |
| 5893 | D66 | | |
| 5894 | D73 | | |
| 5895 | D74 | | |
| 5896 | D75 | | |
| 5897 | D76 | | |
| 5898 | D78 | | |
| 5899 | D155 | | |
| 5900 | D182 | | |
| 5901 | D183 | | |
| 5902 | D185 | | |
| 5903 | D246 | | |
| 5904 | D265 | | |
| 5905 | D266 | | |
| 5906 | D267 | | |
| 5907 | D268 | | |
| 5908 | D269 | | |
| 5909 | D296 | | |
| 5910 | D298 | | |
| 5911 | D15 | D78 | |
| 5912 | D16 | | |
| 5913 | D18 | | |
| 5914 | D21 | | |
| 5915 | D33 | | |
| 5916 | D34 | | |
| 5917 | D36 | | |
| 5918 | D45 | | |
| 5919 | D46 | | |
| 5920 | D48 | | |
| 5921 | D63 | | |
| 5922 | D64 | | |
| 5923 | D66 | | |
| 5924 | D73 | | |
| 5925 | D74 | | |
| 5926 | D75 | | |
| 5927 | D76 | | |
| 5928 | D78 | | |
| 5929 | D155 | | |
| 5930 | D182 | | |
| 5931 | D183 | | |
| 5932 | D185 | | |
| 5933 | D246 | | |
| 5934 | D265 | | |
| 5935 | D266 | | |
| 5936 | D267 | | |
| 5937 | D268 | | |
| 5938 | D269 | | |
| 5939 | D296 | | |
| 5940 | D298 | | |

| No. | $R^1$ = H | | |
|---|---|---|---|
| | $R^2$ | $R^3$ | $R^4$ |
| 5941 | D15 | D155 | Ar88 |
| 5942 | D16 | | |
| 5943 | D18 | | |
| 5944 | D21 | | |
| 5945 | D33 | | |
| 5946 | D34 | | |
| 5947 | D36 | | |
| 5948 | D45 | | |
| 5949 | D46 | | |
| 5950 | D48 | | |
| 5951 | D63 | | |
| 5952 | D64 | | |
| 5953 | D66 | | |
| 5954 | D73 | | |
| 5955 | D74 | | |
| 5956 | D75 | | |
| 5957 | D76 | | |
| 5958 | D78 | | |
| 5959 | D155 | | |
| 5960 | D182 | | |
| 5961 | D183 | | |
| 5962 | D185 | | |
| 5963 | D246 | | |
| 5964 | D265 | | |
| 5965 | D266 | | |
| 5966 | D267 | | |
| 5967 | D268 | | |
| 5968 | D269 | | |
| 5969 | D296 | | |
| 5970 | D298 | | |
| 5971 | D15 | D182 | |
| 5972 | D16 | | |
| 5973 | D18 | | |
| 5974 | D21 | | |
| 5975 | D33 | | |
| 5976 | D34 | | |
| 5977 | D36 | | |
| 5978 | D45 | | |
| 5979 | D46 | | |
| 5980 | D48 | | |
| 5981 | D63 | | |
| 5982 | D64 | | |
| 5983 | D66 | | |
| 5984 | D73 | | |
| 5985 | D74 | | |
| 5986 | D75 | | |
| 5987 | D76 | | |
| 5988 | D78 | | |
| 5989 | D155 | | |
| 5990 | D182 | | |
| 5991 | D183 | | |
| 5992 | D185 | | |
| 5993 | D246 | | |
| 5994 | D265 | | |
| 5995 | D266 | | |
| 5996 | D267 | | |
| 5997 | D268 | | |
| 5998 | D269 | | |
| 5999 | D296 | | |
| 6000 | D298 | | |

| No. | R[1] = H | | |
|---|---|---|---|
| | $R^2$ | $R^3$ | $R^4$ |
| 6001 | D15 | D183 | Ar88 |
| 6002 | D16 | | |
| 6003 | D18 | | |
| 6004 | D21 | | |
| 6005 | D33 | | |
| 6006 | D34 | | |
| 6007 | D36 | | |
| 6008 | D45 | | |
| 6009 | D46 | | |
| 6010 | D48 | | |
| 6011 | D63 | | |
| 6012 | D64 | | |
| 6013 | D66 | | |
| 6014 | D73 | | |
| 6015 | D74 | | |
| 6016 | D75 | | |
| 6017 | D76 | | |
| 6018 | D78 | | |
| 6019 | D155 | | |
| 6020 | D182 | | |
| 6021 | D183 | | |
| 6022 | D185 | | |
| 6023 | D246 | | |
| 6024 | D265 | | |
| 6025 | D266 | | |
| 6026 | D267 | | |
| 6027 | D268 | | |
| 6028 | D269 | | |
| 6029 | D296 | | |
| 6030 | D298 | | |
| 6031 | D15 | D185 | |
| 6032 | D16 | | |
| 6033 | D18 | | |
| 6034 | D21 | | |
| 6035 | D33 | | |
| 6036 | D34 | | |
| 6037 | D36 | | |
| 6038 | D45 | | |
| 6039 | D46 | | |
| 6040 | D48 | | |
| 6041 | D63 | | |
| 6042 | D64 | | |
| 6043 | D66 | | |
| 6044 | D73 | | |
| 6045 | D74 | | |
| 6046 | D75 | | |
| 6047 | D76 | | |
| 6048 | D78 | | |
| 6049 | D155 | | |
| 6050 | D182 | | |
| 6051 | D183 | | |
| 6052 | D185 | | |
| 6053 | D246 | | |
| 6054 | D265 | | |
| 6055 | D266 | | |
| 6056 | D267 | | |
| 6057 | D268 | | |
| 6058 | D269 | | |
| 6059 | D296 | | |
| 6060 | D298 | | |

| No. | R[1] = H | | |
|---|---|---|---|
| | $R^2$ | $R^3$ | $R^4$ |
| 6061 | D15 | D246 | Ar88 |
| 6062 | D16 | | |
| 6063 | D18 | | |
| 6064 | D21 | | |
| 6065 | D33 | | |
| 6066 | D34 | | |
| 6067 | D36 | | |
| 6068 | D45 | | |
| 6069 | D46 | | |
| 6070 | D48 | | |
| 6071 | D63 | | |
| 6072 | D64 | | |
| 6073 | D66 | | |
| 6074 | D73 | | |
| 6075 | D74 | | |
| 6076 | D75 | | |
| 6077 | D76 | | |
| 6078 | D78 | | |
| 6079 | D155 | | |
| 6080 | D182 | | |
| 6081 | D183 | | |
| 6082 | D185 | | |
| 6083 | D246 | | |
| 6084 | D265 | | |
| 6085 | D266 | | |
| 6086 | D267 | | |
| 6087 | D268 | | |
| 6088 | D269 | | |
| 6089 | D296 | | |
| 6090 | D298 | | |
| 6091 | D15 | D265 | |
| 6092 | D16 | | |
| 6093 | D18 | | |
| 6094 | D21 | | |
| 6095 | D33 | | |
| 6096 | D34 | | |
| 6097 | D36 | | |
| 6098 | D45 | | |
| 6099 | D46 | | |
| 6100 | D48 | | |
| 6101 | D63 | | |
| 6102 | D64 | | |
| 6103 | D66 | | |
| 6104 | D73 | | |
| 6105 | D74 | | |
| 6106 | D75 | | |
| 6107 | D76 | | |
| 6108 | D78 | | |
| 6109 | D155 | | |
| 6110 | D182 | | |
| 6111 | D183 | | |
| 6112 | D185 | | |
| 6113 | D246 | | |
| 6114 | D265 | | |
| 6115 | D266 | | |
| 6116 | D267 | | |
| 6117 | D268 | | |
| 6118 | D269 | | |
| 6119 | D296 | | |
| 6120 | D298 | | |

| No. | R[1] = H | | |
|---|---|---|---|
| | $R^2$ | $R^3$ | $R^4$ |
| 6121 | D15 | D266 | Ar88 |
| 6122 | D16 | | |
| 6123 | D18 | | |
| 6124 | D21 | | |
| 6125 | D33 | | |
| 6126 | D34 | | |
| 6127 | D36 | | |
| 6128 | D45 | | |
| 6129 | D46 | | |
| 6130 | D48 | | |
| 6131 | D63 | | |
| 6132 | D64 | | |
| 6133 | D66 | | |
| 6134 | D73 | | |
| 6135 | D74 | | |
| 6136 | D75 | | |
| 6137 | D76 | | |
| 6138 | D78 | | |
| 6139 | D155 | | |
| 6140 | D182 | | |
| 6141 | D183 | | |
| 6142 | D185 | | |
| 6143 | D246 | | |
| 6144 | D265 | | |
| 6145 | D266 | | |
| 6146 | D267 | | |
| 6147 | D268 | | |
| 6148 | D269 | | |
| 6149 | D296 | | |
| 6150 | D298 | | |
| 6151 | D15 | D267 | |
| 6152 | D16 | | |
| 6153 | D18 | | |
| 6154 | D21 | | |
| 6155 | D33 | | |
| 6156 | D34 | | |
| 6157 | D36 | | |
| 6158 | D45 | | |
| 6159 | D46 | | |
| 6160 | D48 | | |
| 6161 | D63 | | |
| 6162 | D64 | | |
| 6163 | D66 | | |
| 6164 | D73 | | |
| 6165 | D74 | | |
| 6166 | D75 | | |
| 6167 | D76 | | |
| 6168 | D78 | | |
| 6169 | D155 | | |
| 6170 | D182 | | |
| 6171 | D183 | | |
| 6172 | D185 | | |
| 6173 | D246 | | |
| 6174 | D265 | | |
| 6175 | D266 | | |
| 6176 | D267 | | |
| 6177 | D268 | | |
| 6178 | D269 | | |
| 6179 | D296 | | |
| 6180 | D298 | | |

| No. | R[1] = H | | |
|---|---|---|---|
| | $R^2$ | $R^3$ | $R^4$ |
| 6181 | D15 | D268 | Ar88 |
| 6182 | D16 | | |
| 6183 | D18 | | |
| 6184 | D21 | | |
| 6185 | D33 | | |
| 6186 | D34 | | |
| 6187 | D36 | | |
| 6188 | D45 | | |
| 6189 | D46 | | |
| 6190 | D48 | | |
| 6191 | D63 | | |
| 6192 | D64 | | |
| 6193 | D66 | | |
| 6194 | D73 | | |
| 6195 | D74 | | |
| 6196 | D75 | | |
| 6197 | D76 | | |
| 6198 | D78 | | |
| 6199 | D155 | | |
| 6200 | D182 | | |
| 6201 | D183 | | |
| 6202 | D185 | | |
| 6203 | D246 | | |
| 6204 | D265 | | |
| 6205 | D266 | | |
| 6206 | D267 | | |
| 6207 | D268 | | |
| 6208 | D269 | | |
| 6209 | D296 | | |
| 6210 | D298 | | |
| 6211 | D15 | D269 | |
| 6212 | D16 | | |
| 6213 | D18 | | |
| 6214 | D21 | | |
| 6215 | D33 | | |
| 6216 | D34 | | |
| 6217 | D36 | | |
| 6218 | D45 | | |
| 6219 | D46 | | |
| 6220 | D48 | | |
| 6221 | D63 | | |
| 6222 | D64 | | |
| 6223 | D66 | | |
| 6224 | D73 | | |
| 6225 | D74 | | |
| 6226 | D75 | | |
| 6227 | D76 | | |
| 6228 | D78 | | |
| 6229 | D155 | | |
| 6230 | D182 | | |
| 6231 | D183 | | |
| 6232 | D185 | | |
| 6233 | D246 | | |
| 6234 | D265 | | |
| 6235 | D266 | | |
| 6236 | D267 | | |
| 6237 | D268 | | |
| 6238 | D269 | | |
| 6239 | D296 | | |
| 6240 | D298 | | |

| No. | $R^1$ = H | | |
|---|---|---|---|
| | $R^2$ | $R^3$ | $R^4$ |
| 6241 | D15 | D296 | Ar88 |
| 6242 | D16 | D296 | Ar88 |
| 6243 | D18 | D296 | Ar88 |
| 6244 | D21 | D296 | Ar88 |
| 6245 | D33 | D296 | Ar88 |
| 6246 | D34 | D296 | Ar88 |
| 6247 | D36 | D296 | Ar88 |
| 6248 | D45 | D296 | Ar88 |
| 6249 | D46 | D296 | Ar88 |
| 6250 | D48 | D296 | Ar88 |
| 6251 | D63 | D296 | Ar88 |
| 6252 | D64 | D296 | Ar88 |
| 6253 | D66 | D296 | Ar88 |
| 6254 | D73 | D296 | Ar88 |
| 6255 | D74 | D296 | Ar88 |
| 6256 | D75 | D296 | Ar88 |
| 6257 | D76 | D296 | Ar88 |
| 6258 | D78 | D296 | Ar88 |
| 6259 | D155 | D296 | Ar88 |
| 6260 | D182 | D296 | Ar88 |
| 6261 | D183 | D296 | Ar88 |
| 6262 | D185 | D296 | Ar88 |
| 6263 | D246 | D296 | Ar88 |
| 6264 | D265 | D296 | Ar88 |
| 6265 | D266 | D296 | Ar88 |
| 6266 | D267 | D296 | Ar88 |
| 6267 | D268 | D296 | Ar88 |
| 6268 | D269 | D296 | Ar88 |
| 6269 | D296 | D296 | Ar88 |
| 6270 | D298 | D296 | Ar88 |
| 6271 | D15 | D298 | Ar88 |
| 6272 | D16 | D298 | Ar88 |
| 6273 | D18 | D298 | Ar88 |
| 6274 | D21 | D298 | Ar88 |
| 6275 | D33 | D298 | Ar88 |
| 6276 | D34 | D298 | Ar88 |
| 6277 | D36 | D298 | Ar88 |
| 6278 | D45 | D298 | Ar88 |
| 6279 | D46 | D298 | Ar88 |
| 6280 | D48 | D298 | Ar88 |
| 6281 | D63 | D298 | Ar88 |
| 6282 | D64 | D298 | Ar88 |
| 6283 | D66 | D298 | Ar88 |
| 6284 | D73 | D298 | Ar88 |
| 6285 | D74 | D298 | Ar88 |
| 6286 | D75 | D298 | Ar88 |
| 6287 | D76 | D298 | Ar88 |
| 6288 | D78 | D298 | Ar88 |
| 6289 | D155 | D298 | Ar88 |
| 6290 | D182 | D298 | Ar88 |
| 6291 | D183 | D298 | Ar88 |
| 6292 | D185 | D298 | Ar88 |
| 6293 | D246 | D298 | Ar88 |
| 6294 | D265 | D298 | Ar88 |
| 6295 | D266 | D298 | Ar88 |
| 6296 | D267 | D298 | Ar88 |
| 6297 | D268 | D298 | Ar88 |
| 6298 | D269 | D298 | Ar88 |
| 6299 | D296 | D298 | Ar88 |
| 6300 | D298 | D298 | Ar88 |

Compounds derived from compounds 1 to 6300 by substituting H of $R^1$ with D are disclosed herein as compounds 6301 to 12600, respectively, herein. Compounds derived from 1 to 6300 by exchanging $R^1$ and $R^4$ to $R^4$ and $R^1$, respectively are disclosed herein as compounds 12601 to 18900. Compounds derived from compounds 12601 to 18900 by substituting H of $R^2$ with D are disclosed herein as compounds 18901 to 25200, respectively. Compounds derived from compounds 1 to 6300 by changing $R^1$, $R^2$, $R^3$ and $R^4$ to $R^3$, $R^1$, $R^4$ and $R^2$, respectively, are disclosed herein as compounds 25201 to 31500. Compounds derived from compounds 25201 to 31500 by substituting H of $R^3$ with D are disclosed herein as compounds 31501 to 37800, respectively. Compounds derived from compounds 1 to 6300 by changing $R^1$, $R^2$, $R^3$ and $R^4$ to $R^4$, $R^1$, $R^2$ and $R^3$, respectively, are disclosed herein as compounds 37801 to 44100. Compounds derived from compounds 37801 to 44100 by substituting H of $R^4$ with D are disclosed herein as compounds 44101 to 50400, respectively. Compounds derived from compounds 1 to 6300 by changing $R^1$, $R^3$ and $R^4$ to $R^3$, $R^4$ and $R^1$, respectively, are disclosed herein as compounds 50401 to 56700, respectively. Compounds derived from compounds 50401 to 56700 by substituting H of $R^3$ with D are disclosed herein as compounds 56701 to 63000, respectively. Compounds derived from compounds 1 to 6300 by exchanging $R^3$ and $R^4$ to $R^4$ and $R^3$, respectively, are disclosed herein as compounds 63001 to 69300, respectively. Compounds derived from compounds 63001 to 69300 by substituting H of $R^1$ with D are disclosed herein as compounds 69301 to 75600, respectively. These compounds 1 to 75600 are individually specified in point of the structures thereof, and are described as specific compounds in the present description.

The molecular weight of the compound represented by the general formula (1) is, for example, when an organic layer containing the compound represented by the general formula (1) is intended to be formed by an evaporation method and used, preferably 1500 or less, more preferably 1200 or less, even more preferably 1000 or less, further more preferably 900 or less. The lower limit of the molecular weight is a molecular weight of the smallest compound represented by the general formula (1). Preferably, the lower limit is 624 or more.

The compound represented by the general formula (1) can be formed into a layer by a coating method, irrespective of the molecular weight thereof. A coating method can form the compound having a relatively large molecular weight into a layer. The compound represented by the general formula (1) has an advantage that, among cyanobenzene compounds, the compound is readily soluble in an organic compound. Consequently, a coating method is readily applicable to the compound represented by the general formula (1) and, in addition, the compound can be purified to have an increased purity.

By applying the present invention, it is considered that a compound containing plural number of structures represented by the general formula (1) in the molecule can be used as a light emitting material.

For example, it is considered that a polymerizable group is previously introduced into the structure represented by the general formula (1), and the polymer formed by polymerizing the polymerizable group is used as a light emitting material. Specifically, it is considered that a monomer containing a polymerizable functional group in any of Ar, $D^1$ and $D^2$ in the general formula (1) is prepared, and this is homo-polymerized, or is copolymerized with any other monomer to give a polymer having a repeating unit, and the polymer is used as a light emitting material. Or it is also considered that compounds each having the structure represented by the general formula (1) are coupled to give a dimer or a trimer, and these are used as a light emitting material.

Examples of the polymer having a repeating unit that contains the structure represented by the general formula (1) include polymers having a structure represented by the following general formula (4) or (5).

General Formula (4)

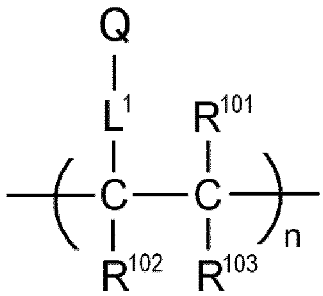

General Formula (5)

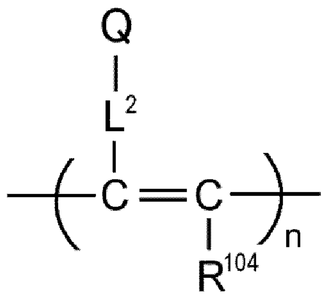

In the general formula (4) or (5), Q represents a group containing the structure represented by the general formula (1), $L^1$ and $L^2$ each represent a linking group. The carbon number of the linking group is preferably 0 to 20, more preferably 1 to 15, even more preferably 2 to 10. The linking group preferably has a structure represented by - $X^{11}$-$L^{11}$-. Here, $X^{11}$ represents an oxygen atom or a sulfur atom, and is preferably an oxygen atom. $L^{11}$ represents a linking group, and is preferably a substituted or unsubstituted alkylene group, or a substituted or unsubstituted arylene group, more preferably a substituted or unsubstituted alkylene group having 1 to 10 carbon atoms, or a substituted or unsubstituted phenylene group.

In the general formula (4) or (5), $R^{101}$, $R^{102}$, $R^{103}$ and $R^{104}$ each independently represent a substituent. Preferably, they each are a substituted or unsubstituted alkyl group having 1 to 6 carbon atoms, a substituted or unsubstituted alkoxy group having 1 to 6 carbon atoms, or a halogen atom, more preferably an unsubstituted alkyl group having 1 to 3 carbon atoms, an unsubstituted alkoxy group having 1 to 3 carbon atoms, a fluorine atom or a chlorine atom, even more preferably an unsubstituted alkyl group having 1 to 3 carbon atoms or an unsubstituted alkoxy group having 1 to 3 carbon atoms.

The linking group represented by $L^1$ and $L^2$ bonds to any of Ar, $D^1$ and $D^2$ in formula (1) that constitutes Q. Two or more linking groups can bond to one Q to form a crosslinked structure or a network structure.

Examples of specific structures of the repeating unit include structures represented by the following formulae (6) to (9).

Formula (6)

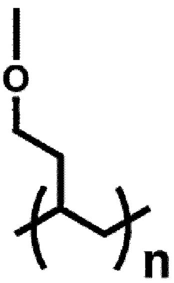

Formula (7)

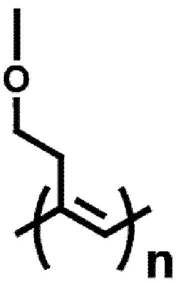

Formula (8)

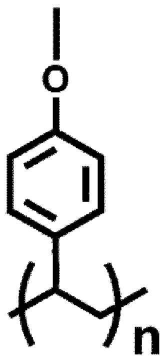

Formula (9)

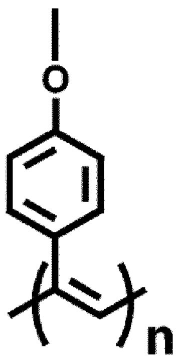

Polymers having a repeating unit that contains any of these formulae (6) to (9) can be synthesized by previously introducing a hydroxy group into any of Ar, $D^1$ and $D^2$ in the general formula (1), then reacting the group serving as a linker with the following compound to thereby introduce a polymerizable group, and polymerizing the polymerizable group.

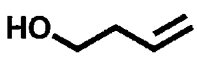

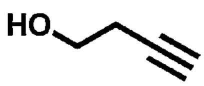

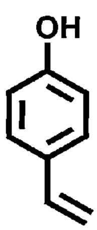

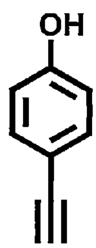

The polymer having a structure represented by the general formula (1) in the molecule can be a polymer having only a repeating unit that has the structure represented by the general formula (1), or can be a polymer containing a

repeating unit that has any other structure. The repeating unit having the structure represented by the general formula (1) to be contained in the polymer may be a single kind or two or more kinds. The repeating unit not having the structure of the general formula (1) includes those derived from monomers used in general copolymerization. For example, it includes repeating units derived from monomers having an ethylenically unsaturated bond, such as ethylene or styrene.

In some embodiments, the compound represented by the general formula (1) is a light emitting material.

In some embodiments, the compound represented by the general formula (1) is a compound that can emit delayed fluorescence.

In some embodiments of the present disclosure, the compound represented by the general formula (1) is, when excited thermally or by an electronic means, able to emit light in a UV region, in a blue, green, yellow, orange or red region in a visible spectrum (e.g., about 420 nm to about 500 nm, about 500 nm to about 600 nm, or about 600 nm to about 700 nm) or in a near IR region.

In some embodiments of the present disclosure, the compound represented by the general formula (1) is, when excited thermally or by an electronic means, able to emit light in a red or orange region in a visible spectrum (e.g., about 620 nm to about 780 nm, about 650 nm).

In some embodiments of the present disclosure, the compound represented by the general formula (1) is, when excited thermally or by an electronic means, able to emit light in an orange or yellow region in a visible spectrum (e.g., about 570 nm to about 620 nm, about 590 nm, about 570 nm).

In some embodiments of the present disclosure, the compound represented by the general formula (1) is, when excited thermally or by an electronic means, able to emit light in a green region in a visible spectrum (e.g., about 490 nm to about 575 nm, about 510 nm).

In some embodiments of the present disclosure, the compound represented by the general formula (1) is, when excited thermally or by an electronic means, able to emit light in a blue region in a visible spectrum (e.g., about 400 nm to about 490 nm, about 475 nm).

In some embodiments of the present disclosure, the compound represented by the general formula (1) is, when excited thermally or by an electronic means, able to emit light in a UV spectral region (e.g., about 280 to 400 nm).

In some embodiments of the present disclosure, the compound represented by the general formula (1) is, when excited thermally or by an electronic means, able to emit light in an IR spectral region (e.g., about 780 nm to 2 $\mu$m).

Electronic characteristics of small-molecule chemical substance libraries can be calculated by known ab initio quantum chemistry calculation. For example, according to time-dependent density functional theory using 6-31G* as a basis, and a functional group known as Becke's three parameters, Lee-Yang-Parr hybrid functionals, the Hartree-Fock equation (TD-DFTB3LYP/6-31G*) is analyzed and molecular fractions (parts) having HOMO not lower than a specific threshold value and LUMO not higher than a specific threshold value can be screened.

With that, for example, in the presence of a HOMO energy (for example, ionizing potential) of -6.5 eV or more, a donor part ("D") can be selected. On the other hand, for example, in the presence of a LUMO energy (for example, electron affinity) of -0.5 eV or less, an acceptor part ("A") can be selected. A bridge part ("B") is a strong conjugated system, for example, capable of strictly limiting the acceptor part and the donor part in a specific three-dimensional configuration, and therefore prevents the donor part and the acceptor part from overlapping in the $\pi$-conjugated system.

In some embodiments, a compound library is screened using at least one of the following characteristics.

1. Light emission around a specific wavelength.
2. A triplet state over a calculated specific energy level.
3. $\Delta E_{ST}$ value lower than a specific value.
4. Quantum yield more than a specific value.
5. HOMO level.
6. LUMO level.

In some embodiments, the difference ($\Delta E_{ST}$) between the lowest singlet excited state and the lowest triplet excited state at 77 K is less than about 0.5 eV, less than about 0.4 eV, less than about 0.3 eV, less than about 0.2 eV, or less than about 0.1 eV In some embodiments, $\Delta E_{ST}$ value is less than about 0.09 eV, less than about 0.08 eV, less than about 0.07 eV, less than about 0.06 eV, less than about 0.05 eV, less than about 0.04 eV, less than about 0.03 eV, less than about 0.02 eV, or less than about 0.01 eV

In some embodiments, the compound represented by the general formula (1) shows a quantum yield of more than 25%, for example, about 30%, about 35%, about 40%, about 45%, about 50%, about 55%, about 60%, about 65%, about 70%, about 75%, about 80%, about 85%, about 90%, about 95% or more.

[Synthesis Method for Compound represented by general formula (1)]

The compound represented by the general formula (1) is a novel compound.

The compound represented by the general formula (1) can be synthesized by combining known reactions. For example, the compound can be synthesized by reacting a difluoroisophthalonitrile, where the positions to which $D^1$ and $D^2$ are introduced are substituted with fluorine atoms, with $D^1$-H and $D^2$-H in the presence of sodium hydride in tetrahydrofuran. In the case where $D^1$-H and $D^2$-H differ, the reaction with $D^1$-H and $D^2$-H can be carried out in two stages. For the specific conditions and the reaction process, reference can be made to Synthesis Examples given hereinafter.

[Structure using compound represented by general formula (1)]

In some embodiments, the compound represented by the general formula (1) is used along with at least one material which is combined with the compound, in which the compound is dispersed, which bonds to the compound in a mode of covalent bonding, which is coated with the compound, which carries the compound or which associates with the compound (for example, small molecules, polymers, metals, metal complexes), and forms a solid film or layer. For example, the compound represented by the general formula (1) can be combined with an electroactive material to form a film. In some cases, the compound represented by the general formula (1) can be combined with a hole transporting polymer. In some cases, the compound represented by the general formula (1) can be combined with an electron transporting polymer. In some cases, the compound represented by the general formula (1) can be combined with a hole transporting polymer and an electron transporting polymer. In some cases, the compound represented by the general formula (1) can be combined with a copolymer having both a hole transporting moiety and an electron transporting moiety. In the above-mentioned embodiments, the electrons and/or holes formed in a solid film or layer can be interacted with the compound represented by the general formula (1).

[Film Formation]

In some embodiments, a film containing the compound represented by the general formula (1) can be formed in a wet process. In the wet process, a solution prepared by dissolving a composition containing the compound of the present invention is applied onto a surface, and then the solvent is removed to form a film. The wet process includes a spin coating method, a slit coating method, an ink jet method (a spraying method), a gravure printing method, an offset printing method and flexographic printing method, which, however are not limitative. In the wet process, an appropriate organic solvent capable of dissolving a composition containing the compound of the present invention is selected and used. In some embodiments, a substituent (for example, an alkyl group) capable of increasing the solubility in an organic solvent can be introduced into the compound contained in composition.

In some embodiments, a film containing the compound of the present invention can be formed in a dry process. In some embodiments, a vacuum evaporation method is employable as a dry process, which, however, is not limitative. In the case where a vacuum evaporation method is employed, compounds to constitute a film can be co-evaporated from individual evaporation sources, or can be co-evaporated from a single evaporation source formed by mixing the compounds. In the case where a single evaporation source is used, a mixed powder prepared by mixing compound powders can be used, or a compression molded body prepared by compression-molding the mixed powder can be used, or a mixture prepared by heating and melting the constituent compounds and cooling the resulting melt can be used. In some embodiments, by coevaporation under the condition where the evaporation rate (weight reduction rate) of the plural compounds contained in a single evaporation source is the same or is nearly the same, a film having a compositional ratio corresponding to the compositional ratio of the plural compounds contained in the evaporation source can be formed. When plural compounds are mixed in the same compositional ratio as the compositional ratio of the film to be formed to prepare an evaporation source, a film having a desired compositional ratio can be formed in a simplified manner. In some embodiments, the temperature at which the compounds to be co-evaporated has the same weight reduction ratio is specifically defined, and the temperature can be employed as the temperature of coevaporation.

[Use Examples of Compound represented by general formula (1)]

Organic Light Emitting Diode:

One embodiment of the present invention relates to use of the compound represented by the general formula (1) of the present invention as a light emitting material for organic light emitting devices. In some embodiments, the compound represented by the general formula (1) of the present invention can be effectively used as a light emitting material in a light emitting layer in an organic light emitting device. In some embodiments, the compound represented by the general formula (1) of the present invention includes delayed fluorescence (delayed fluorescent material) that emits delayed fluorescence. In some embodiments, the present invention provides a delayed fluorescent material having a structure represented by the general formula (1). In some embodiments, the present invention relates to use of the compound represented by the general formula (1) of the present invention as a delayed fluorescent material. In some

embodiments, the compound represented by the general formula (1) of the present invention can be used as a host material, and can be used along with one or more light emitting materials, in which the light emitting material can be a fluorescent material, a phosphorescent material or TADF. In some embodiments, the compound represented by the general formula (1) can also be used as a hole transporting material. In some embodiments, the compound represented by the general formula (1) can be used as an electron transporting material. In some embodiments, the present invention relates to a method of generating delayed fluorescence from the compound represented by the general formula (1). In some embodiments, an organic light emitting device containing a compound as a light emitting material emits delayed fluorescence and exhibits high luminous radiation efficiency.

In some embodiments, the light emitting layer contains the compound represented by the general formula (1), and the compound represented by the general formula (1) is aligned in parallel to the substrate. In some embodiment, the substrate is a film-forming surface. In some embodiment, the alignment of the compound represented by the general formula (1) relative to the film-forming surface can have some influence on the propagation direction of light emitted by the aligned compounds, or can determine the direction. In some embodiments, by aligning the propagation direction of light emitted by the compound represented by the general formula (1), the light extraction efficiency from the light emitting layer can be improved.

One embodiment of the present invention relates to an organic light emitting device. In some embodiments, the organic light emitting device includes a light emitting layer. In some embodiments, the light emitting layer contains, as a light emitting material therein, the compound represented by the general formula (1). In some embodiments, the organic light emitting device is an organic photoluminescent device (organic PL device). In some embodiments, the organic light emitting device is an organic electroluminescent device (organic EL device). In some embodiments, the compound represented by the general formula (1) assists light irradiation from the other light emitting materials contained in the light emitting layer (as a so-called assist dopant). In some embodiments, the compound represented by the general formula (1) contained in the light emitting layer is in a lowest excited singlet energy level, and is contained between the lowest excited singlet energy level of the host material contained in the light emitting layer and the lowest excited singlet energy level of the other light emitting materials contained in the light emitting layer.

In some embodiments, the organic photoluminescent device contains at least one light emitting layer. In some embodiments, the organic electroluminescent device comprises at least an anode, a cathode, and an organic layer between the anode and the cathode. In some embodiments, the organic layer comprises at least a light emitting layer. In some embodiments, the organic layer comprises only a light emitting layer. In some embodiments, the organic layer, comprises one or more organic layers in addition to the light emitting layer. Examples of the organic layer include a hole transporting layer, a hole injection layer, an electron barrier layer, a hole barrier layer, an electron injection layer, an electron transporting layer and an exciton barrier layer. In some embodiments, the hole transporting layer may be a hole injection and transporting layer having a hole injection function, and the electron transporting layer may be an electron injection and transporting layer having an electron injection function. An example of an organic electroluminescent device is shown in Fig. 1.

Light emitting layer:

In some embodiments, the light emitting layer is a layer where holes and electrons injected from the anode and the cathode, respectively, are recombined to form excitons. In some embodiments, the layer emits light.

In some embodiments, only a light emitting material is used as the light emitting layer. In some embodiments, the light emitting layer contains a light emitting material and a host material. In some embodiments, the light emitting material is at least one compound represented by the general formula (1). In some embodiments, for improving luminous radiation efficiency of an organic electroluminescent device and an organic photoluminescence device, the singlet exciton and the triplet exciton generated in a light emitting material is confined inside the light emitting material. In some embodiments, a host material is used in the light emitting layer in addition to a light emitting material therein. In some embodiments, the host material is an organic compound. In some embodiments, the organic compound has an excited singlet energy and an excited triplet energy, and at least one of them is higher than those in the light emitting material of the present invention. In some embodiments, the singlet exciton and the triplet exciton generated in the light emitting material of the present invention are confined in the molecules of the light emitting material of the present invention. In some embodiments, the singlet and triplet excitons are fully confined for improving luminous radiation efficiency. In some embodiments, although high luminous radiation efficiency is still attained, singlet excitons and triplet excitons are not fully confined, that is, a host material capable of attaining high luminous radiation efficiency can be used in the present invention with no specific limitation. In some embodiments, in the light emitting material in the light emitting layer of the device of the present invention, luminous radiation occurs. In some embodiments, radiated light includes both fluorescence and delayed fluorescence. In some embodiments, radiated light includes radiated light from a host material. In some embodiments, radiated light is composed of radiated light from a host material. In some embodiments, radiated light includes radiated light from the compound represented by the general formula (1), and radiated light from a host

material. In some embodiment, a TADF molecule and a host material are used. In some embodiments, TADF is an assist dopant.

When the compound represented by the general formula (1) is used as an assist dopant, various compounds can be employed as a light emitting material (preferably a fluorescent material). Such light emitting materials include an anthracene derivative, a tetracene derivative, a naphthacene derivative, a pyrene derivative, a perylene derivative, a chrysene derivative, a rubrene derivative, a coumarin derivative, a pyran derivative, a stilbene derivative, a fluorene derivative, an anthryl derivative, a pyrromethene derivative, a terphenyl derivative, a terphenylene derivative, a fluoranthene derivative, an amine derivative, a quinacridone derivative, an oxadiazole derivative, a malononitrile derivative, a pyran derivative, a carbazole derivative, a julolidine derivative, a thiazole derivative, and a derivative having a metal (Al or Zn). These exemplified skeletons may have a substituent or may not have a substituent. These exemplified skeletons can be combined with each other.

In the following, examples of light emitting materials that can be combined with the assist dopant represented by the general formula (1) are shown for use herein.

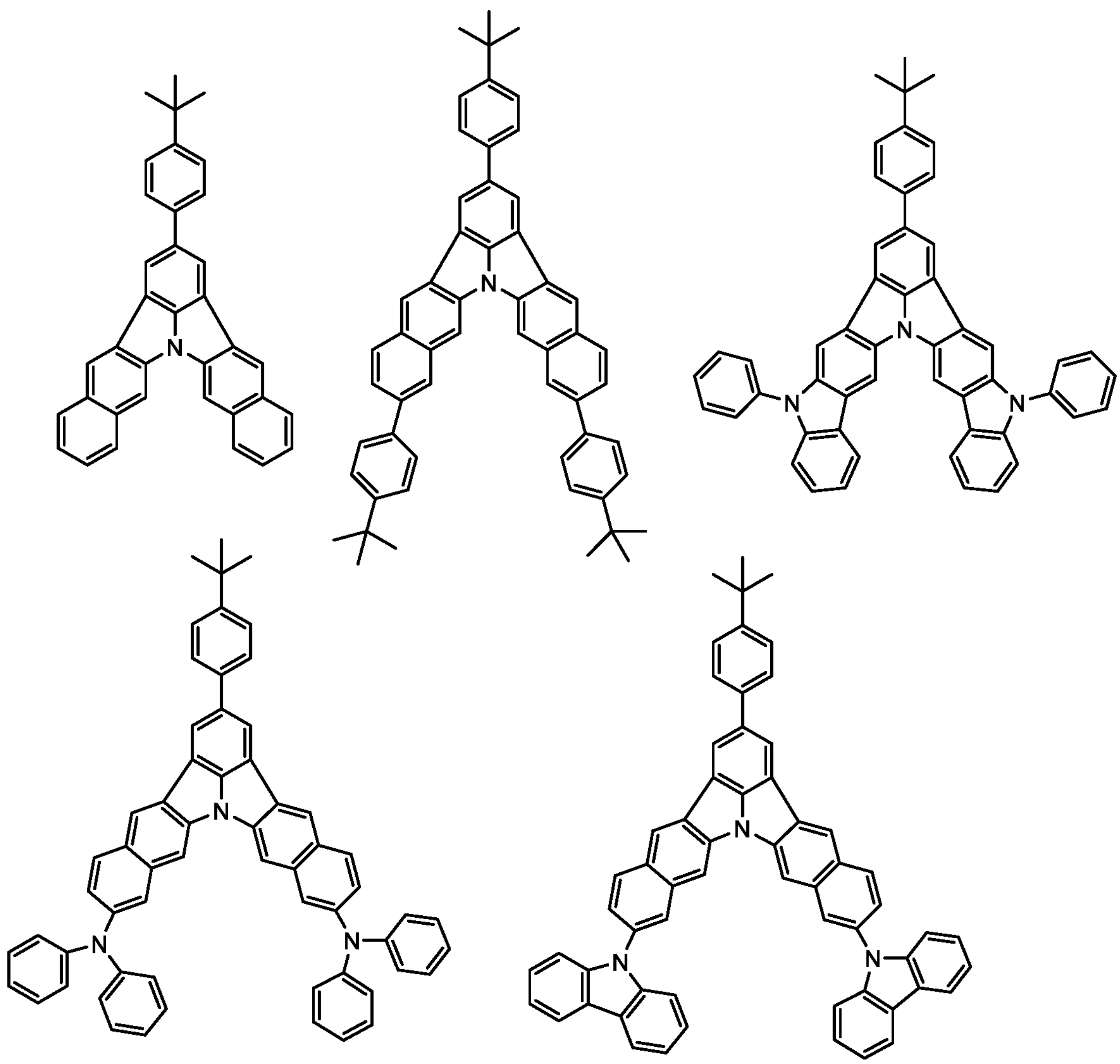

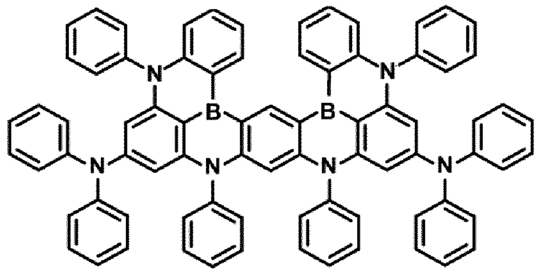

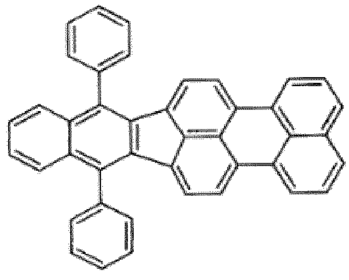

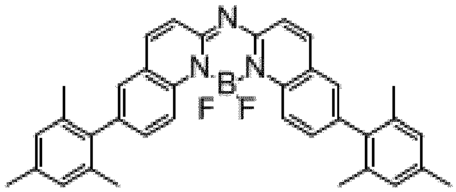

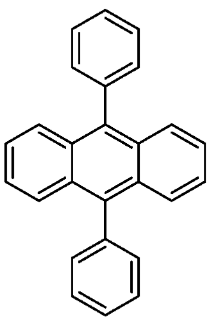

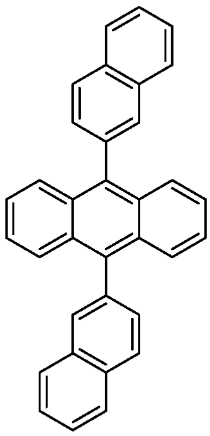

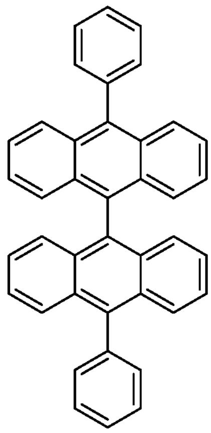

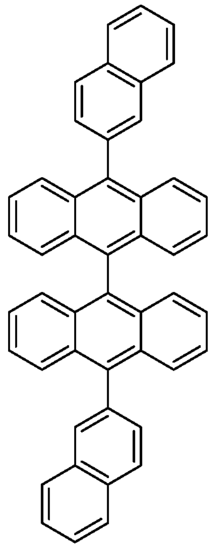

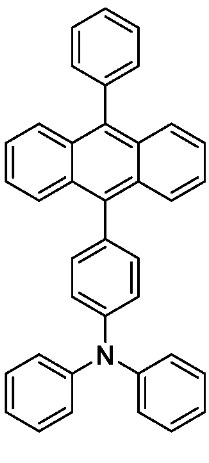

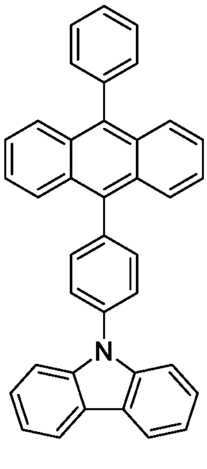

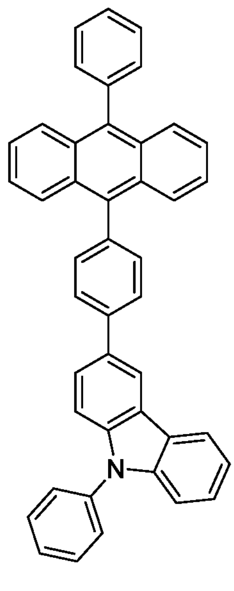

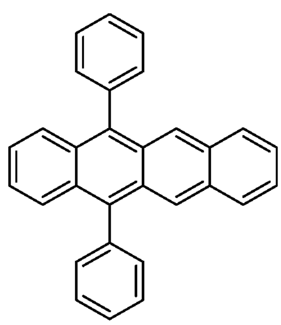

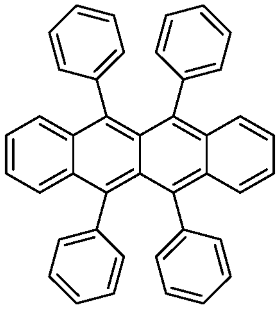
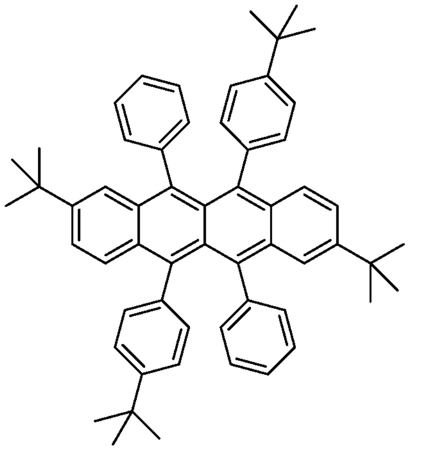
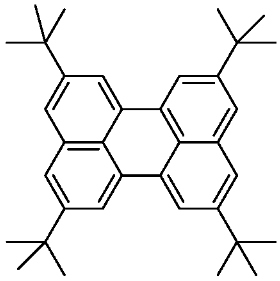

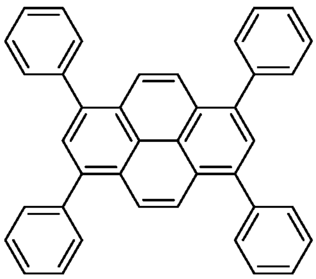
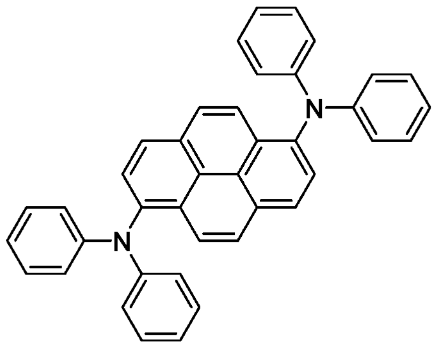
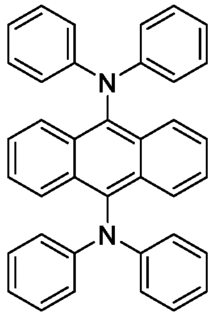

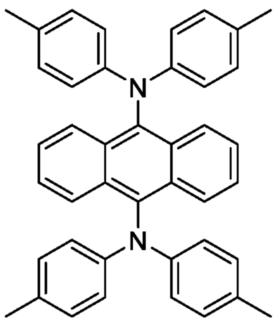
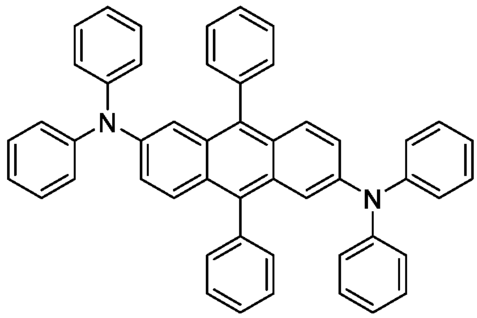
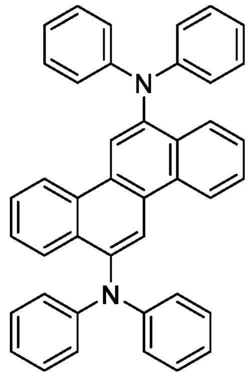

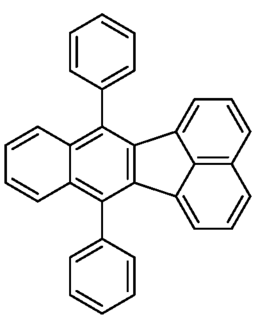
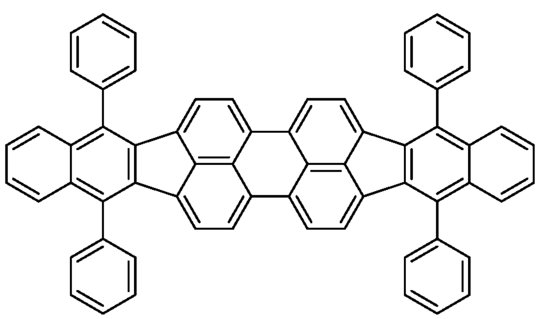
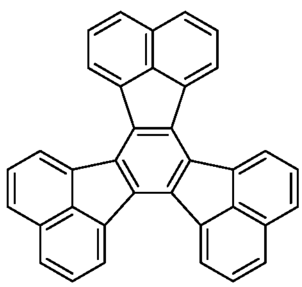

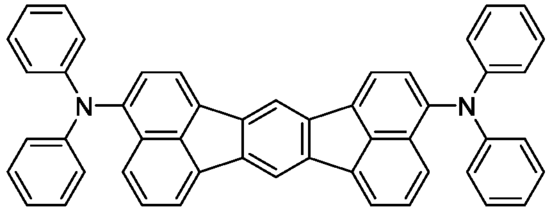
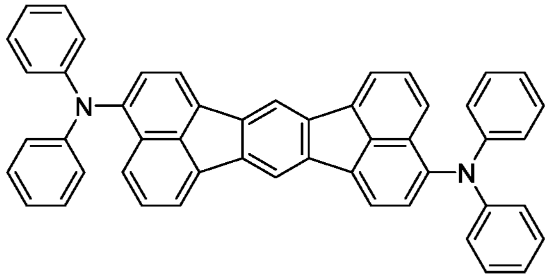

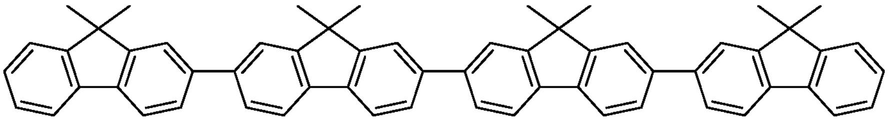

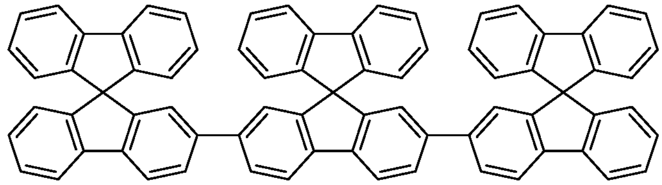

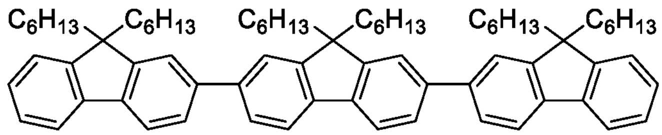

Also the compounds described in WO2015/022974, paragraphs 0220 to 0239 can be especially favorably employed as a light emitting material for use along with the assist dopant represented by the general formula (1).

In some embodiments where a host material is used, the amount of the compound of the present invention contained in the light emitting layer as a light emitting material therein is 0.1% by weight or more. In some embodiments where a host material is used, the amount of the compound of the present invention contained in the light emitting layer as a light emitting material therein is 1% by weight or more. In some embodiments where a host material is used, the amount of the compound of the present invention contained in the light emitting layer as a light emitting material therein is 50% by weight or less. In some embodiments where a host material is used, the amount of the compound of the present invention contained in the light emitting layer as a light emitting material therein is 20% by weight or less. In some embodiments where a host material is used, the amount of the compound of the present invention contained in the light emitting layer as a light emitting material therein is 10% by weight or less.

In some embodiments, the host material in the light emitting layer is an organic compound having a hole transporting function and an electron transporting function. In some embodiments, the host material in the light emitting layer is an organic compound that prevents the wavelength of the emitted light from increasing. In some embodiments, the host material in the light emitting layer is an organic compound having a high glass transition temperature.

In some embodiments, the host material is selected from the group consisting of the following:

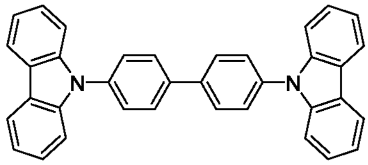
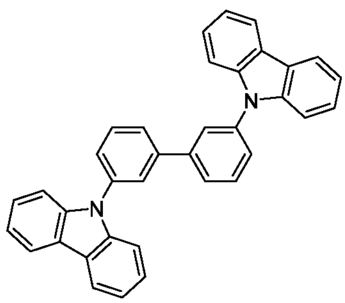
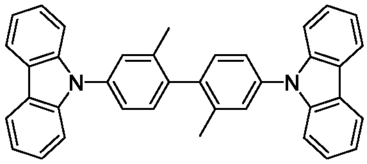

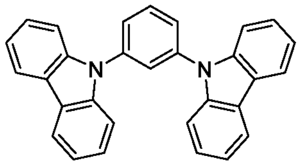
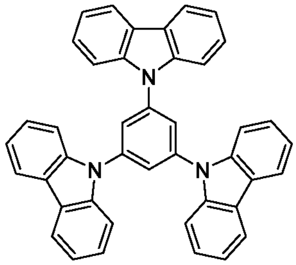
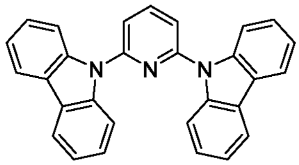

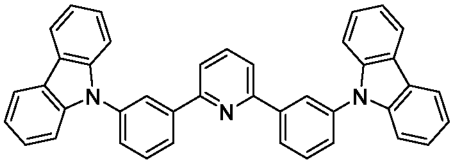
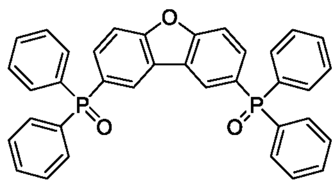

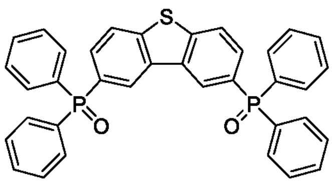
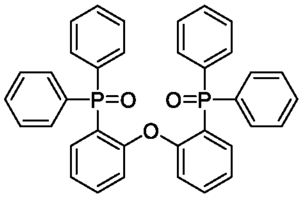
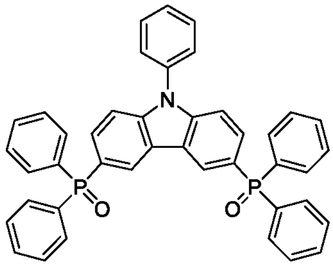

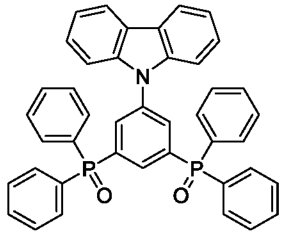
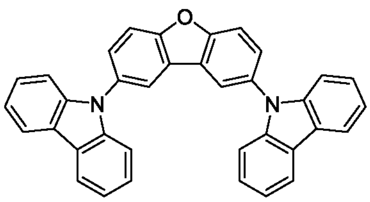
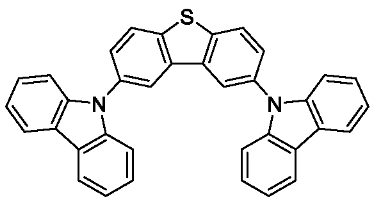

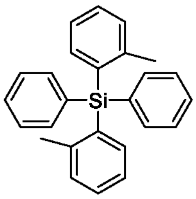
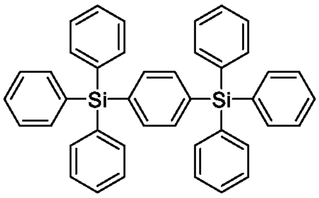
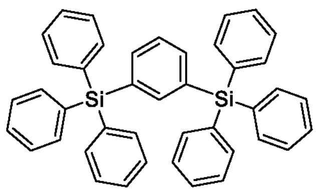
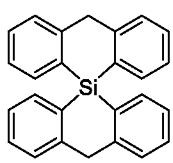

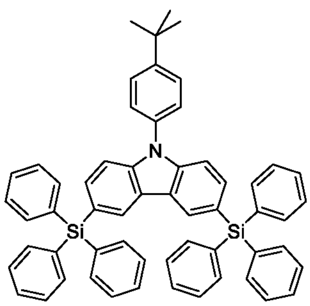
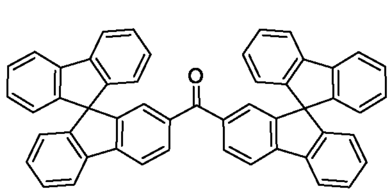
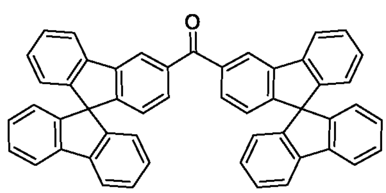

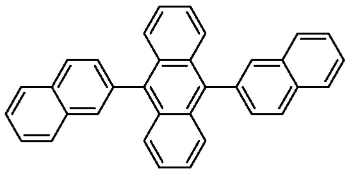
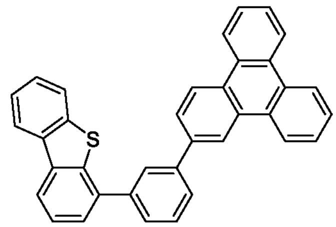

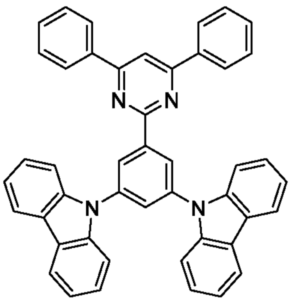
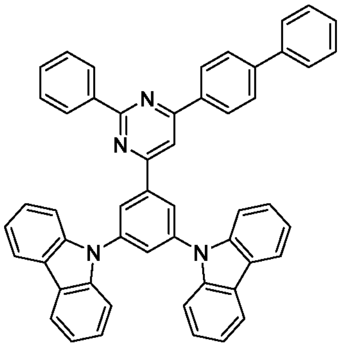
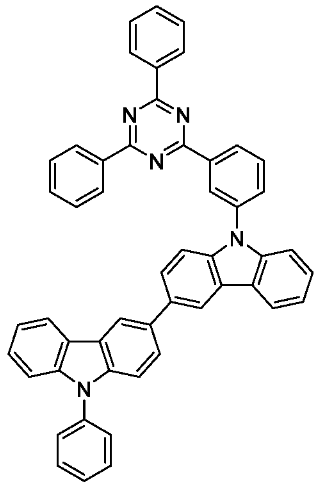

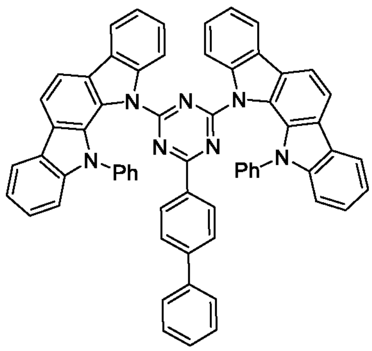
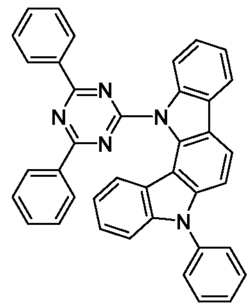

In some embodiments, the light emitting layer contains at least two TADF molecules differing in the structure. For example, the light emitting layer can contain three kinds of materials, a host material, a first TADF molecule and a second TADF molecule whose excited singlet energy level is higher in that order. At that time, the first TADF molecule and the second TADF molecule are preferably such that the difference of $\Delta E_{ST}$ between the lowest excited singlet energy level and the lowest excited triplet energy level at 77 K thereof is 0.3 eV or less, more preferably 0.25 eV or less, even more preferably 0.2 eV or less, further more preferably 0.15 eV or less, further more preferably 0.1 eV or less, further more preferably 0.07 eV or less, further more preferably 0.05 eV or less, further more preferably 0.03 eV or less, especially preferably 0.01 eV or less. The content of the first TADF molecule in the light emitting layer is preferably larger than the content of the second TADF molecule therein. The content of the host material in the light emitting layer is preferably larger than the content of the second TADF molecule therein. The content of the first TADF molecule in the light emitting layer can be larger than the content of the host material therein, or can be smaller than or the same as the latter. In some embodiments, the composition in the light emitting layer can be 10 to 70% by weight of the host material, 10 to 80% by weight of the TADF molecule, and 0.1 to 30% by weight of the second TADF molecule. In some embodiments, the composition in the light emitting layer can be 20 to 45% by weight of the host material, 50 to 75% by weight of the first TADF molecule, and 5 to 20% by weight of the second TADF molecule. In some embodiments, the photoluminescence quantum yield φPL1(A) by photoexcitation of the co-deposited film of the first TADF molecule and the host material (the content of the first TADF molecule in the co-deposited film = A% by weight) and the photoluminescence quantum yield cpPL2(A) by photoexcitation of the co-deposited film of the second TADF molecule and the host material (the content of the second TADF molecule in the co-deposited film = A% by weight) satisfy a relational formula φPL1(A)>φPL2(A).

In some embodiments, the photoluminescence quantum yield φPL2(B) by photoexcitation of the co-deposited film of the second TADF molecule and the host material (the content of the second TADF molecule in the co-deposited film = A% by weight) and the photoluminescence quantum yield φPL2(100) by photoexcitation of the single film of the second TADF molecule satisfy a relational formula φPL2(B)>φPL2(100). In some embodiments, the light emitting layer can contain three TADF molecules differing in the structure. The compound of the present invention can be any of plural TADF compounds contained in the light emitting layer.

In some embodiments, the light emitting layer can be composed of a material selected from the group consisting of a host material, an assist dopant and a light emitting material. In some embodiments, the light emitting layer does not contain a metal element. In some embodiments, the light emitting layer can be composed of a material composed of atoms alone selected from the group consisting of a carbon atom, a hydrogen atom, a deuterium atom, a nitrogen atom, an oxygen atom and a sulfur atom. Or the light emitting layer can also be composed of a material composed of atoms alone selected from the group consisting of a carbon atom, a hydrogen atom, a deuterium atom a nitrogen atom and an oxygen atom. Or the light emitting layer can also be composed of a material composed of atoms alone selected from the group consisting of a carbon atom a hydrogen atom, a nitrogen atom and an oxygen atom.

When the light emitting layer contains a TADF material except the compound of the present invention, the TADF material can be a known delayed fluorescent material. Preferred delayed fluorescent materials are compounds included in the general formulae described in WO2013/154064, paragraphs 0008 to 0048 and 0095 to 0133; WO2013/011954, paragraphs 0007 to 0047 and 0073 to 0085; WO2013/011955, paragraphs 0007 to 0033 and 0059 to 0066; WO2013/081088, paragraphs 0008 to 0071 and 0118 to 0133; JP2013-256490A, paragraphs 0009 to 0046 and 0093 to 0134; JP2013-116975A, paragraphs 0008 to 0020 and 0038 to 0040; WO2013/133359, paragraphs 0007 to 0032 and 0079 to 0084; WO2013/161437, paragraphs 0008 to 0054 and 0101 to 0121; JP2014-9352A, paragraphs 0007 to 0041 and 0060 to 0069; and JP2014-9224A, paragraphs 0008 to 0048 and 0067 to 0076; JP2017-119663A, paragraphs 0013 to 0025; JP2017-119664A, paragraphs 0013 to 0026; JP2017-222623A, paragraphs 0012 to 0025; JP2017-226838A, paragraphs 0010 to 0050; JP2018-100411A, paragraphs 0012 to 0043; WO2018/047853, paragraphs 0016 to 0044; and exemplary compounds therein capable of emitting delayed fluorescence are especially preferred. In addition, light emitting materials capable of emitting delayed fluorescence, as described in JP2013-253121A, WO2013/133359, WO2014/034535, WO2014/115743, WO2014/122895, WO2014/126200, WO2014/136758, WO2014/133121, WO2014/136860, WO2014/196585, WO2014/189122, WO2014/168101, WO2015/008580, WO2014/203840, WO2015/002213, WO2015/016200, WO2015/019725, WO2015/072470, WO2015/108049, WO2015/080182, WO2015/072537, WO2015/080183, JP2015-129240A, WO2015/129714, WO2015/129715, WO2015/133501, WO2015/136880, WO2015/137244, WO2015/137202, WO2015/137136, WO2015/146541 and WO2015/159541, are preferably employed. These patent publications described in this paragraph are hereby incorporated as a part of this description by reference.

In the following, the constituent members and the other layers than the light emitting layer of the organic electroluminescent device are described.

Substrate:

In some embodiments, the organic electroluminescent device of the invention is supported by a substrate, wherein the substrate is not particularly limited and may be any of those that have been commonly used in an organic electroluminescent device, for example those formed of glass, transparent plastics, quartz and silicon.

Anode

In some embodiments, the anode of the organic electroluminescent device is made of a metal, an alloy, an electroconductive compound, or a combination thereof. In some embodiments, the metal, alloy, or electroconductive compound has a large work function (4 eV or more). In some embodiments, the metal is Au. In some embodiments, the electroconductive transparent material is selected from CuI, indium tin oxide (ITO), $SnO_2$, and ZnO. In some embodiments, an amorphous material capable of forming a transparent electroconductive film, such as IDIXO ($In_2O_3$-ZnO), is be used. In some embodiments, the anode is a thin film. In some embodiments the thin film is made by vapor deposition or sputtering. In some embodiments, the film is patterned by a photolithography method. In some embodiments, where the pattern may not require high accuracy (for example, approximately 100 μm or more), the pattern may be formed with a mask having a desired shape on vapor deposition or sputtering of the electrode material. In some embodiments, when a material can be applied as a coating, such as an organic electroconductive compound, a wet film forming method, such as a printing method and a coating method is used. In some embodiments, when the emitted light goes through the anode, the anode has a transmittance of more than 10%, and the anode has a sheet resistance of several hundred Ohm per square or less. In some embodiments, the thickness of the anode is from 10 to 1,000 nm. In some embodiments, the thickness of the anode is from 10 to 200 nm. In some embodiments, the thickness of the anode varies depending

on the material used.

Cathode

In some embodiments, the cathode is made of an electrode material a metal having a small work function (4 eV or less) (referred to as an electron injection metal), an alloy, an electroconductive compound, or a combination thereof. In some embodiments, the electrode material is selected from sodium, a sodium-potassium alloy, magnesium, lithium, a magnesium-cupper mixture, a magnesium-silver mixture, a magnesium-aluminum mixture, a magnesium-indium mixture, an aluminum-aluminum oxide ($Al_2O_3$) mixture, indium, a lithium-aluminum mixture, and a rare earth metal. In some embodiments, a mixture of an electron injection metal and a second metal that is a stable metal having a larger work function than the electron injection metal is used. In some embodiments, the mixture is selected from a magnesium-silver mixture, a magnesium-aluminum mixture, a magnesium-indium mixture, an aluminum-aluminum oxide ($Al_2O_3$) mixture, a lithium-aluminum mixture, and aluminum. In some embodiments, the mixture increases the electron injection property and the durability against oxidation. In some embodiments, the cathode is produced by forming the electrode material into a thin film by vapor deposition or sputtering. In some embodiments, the cathode has a sheet resistance of several hundred Ohm per square or less. In some embodiments, the thickness of the cathode ranges from 10 nm to 5 $\mu$m. In some embodiments, the thickness of the cathode ranges from 50 to 200 nm. In some embodiments, for transmitting the emitted light, any one of the anode and the cathode of the organic electroluminescent device is transparent or translucent. In some embodiments, the transparent or translucent electroluminescent devices enhances the light emission luminance.

In some embodiments, the cathode is formed with an electroconductive transparent material, as described for the anode, to form a transparent or translucent cathode. In some embodiments, a device comprises an anode and a cathode, both being transparent or translucent.

Injection Layer

An injection layer is a layer between the electrode and the organic layer. In some embodiments, the injection layer decreases the driving voltage and enhances the light emission luminance. In some embodiments the injection layer includes a hole injection layer and an electron injection layer. The injection layer can be positioned between the anode and the light emitting layer or the hole transporting layer, and between the cathode and the light emitting layer or the electron transporting layer. In some embodiments, an injection layer is present. In some embodiments, no injection layer is present.

Preferred compound examples for use as a hole injection material are shown below.

$MoO_3$,

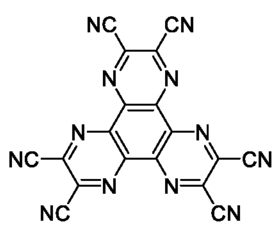
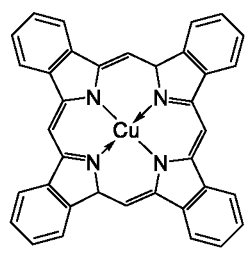
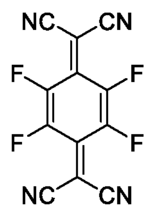
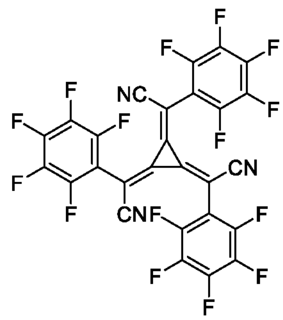

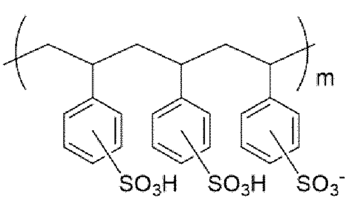
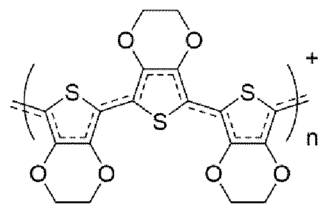

Next, preferred compound examples for use as an electron injection material are shown below.

LiF, CsF,

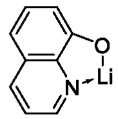

Barrier Layer

A barrier layer is a layer capable of inhibiting charges (electrons or holes) and/or excitons present in the light emitting layer from being diffused outside the light emitting layer. In some embodiments, the electron barrier layer is between the light emitting layer and the hole transporting layer, and inhibits electrons from passing through the light emitting layer toward the hole transporting layer. In some embodiments, the hole barrier layer is between the light emitting layer and the electron transporting layer, and inhibits holes from passing through the light emitting layer toward the electron transporting layer. In some embodiments, the barrier layer inhibits excitons from being diffused outside the light emitting layer. In some embodiments, the electron barrier layer and the hole barrier layer are exciton barrier layers. As used herein, the term "electron barrier layer" or "exciton barrier layer" includes a layer that has the functions of both electron barrier layer and of an exciton barrier layer.

Hole Barrier Layer

A hole barrier layer acts as an electron transporting layer. In some embodiments, the hole barrier layer inhibits holes from reaching the electron transporting layer while transporting electrons. In some embodiments, the hole barrier layer enhances the recombination probability of electrons and holes in the light emitting layer. The material for the hole barrier layer may be the same materials as the ones described for the electron transporting layer.
Preferred compound examples for use for the hole barrier layer are shown below.

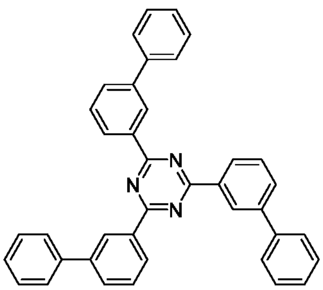
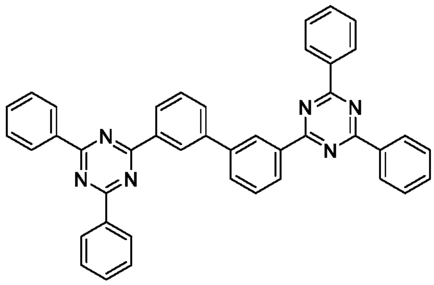
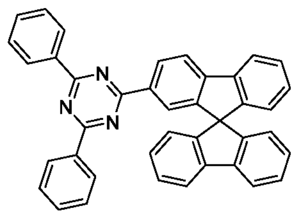

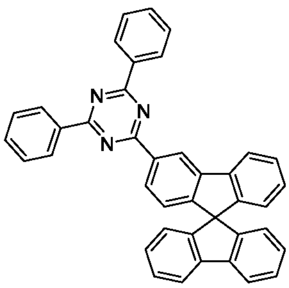
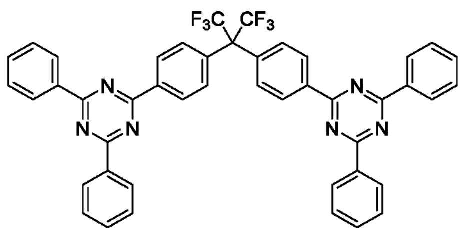
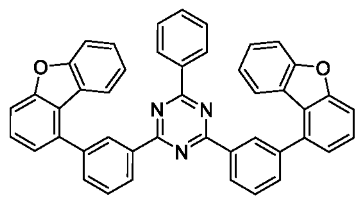

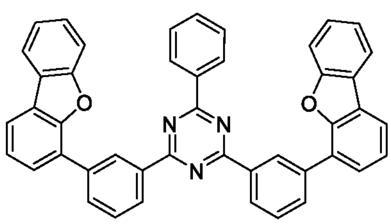
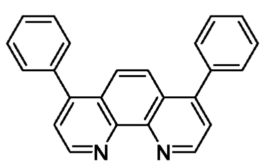
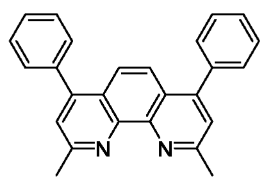

Electron Barrier Layer

As electron barrier layer transports holes. In some embodiments, the electron barrier layer inhibits electrons from reaching the hole transporting layer while transporting holes. In some embodiments, the electron barrier layer enhances the recombination probability of electrons and holes in the light emitting layer.

Preferred compound examples for use as the electron barrier material are shown below.

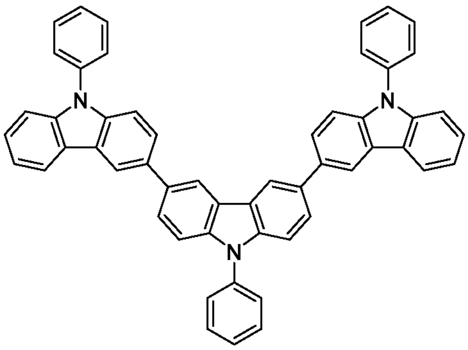

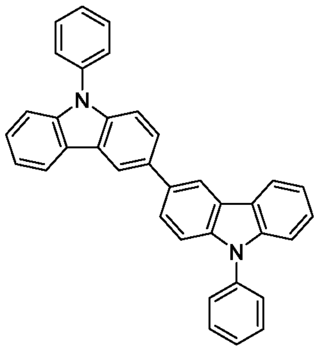

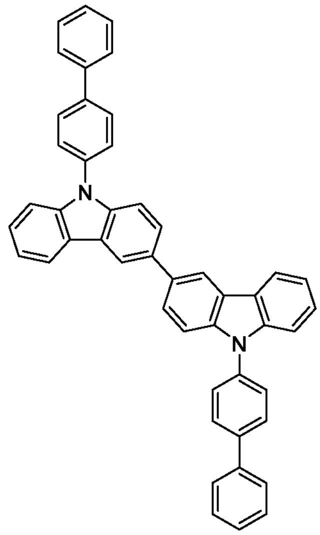

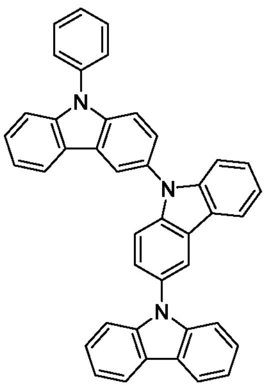

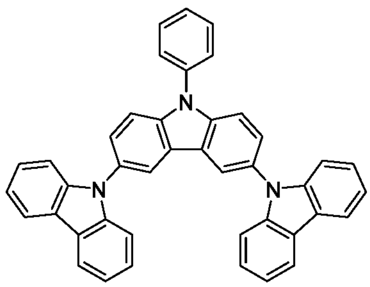

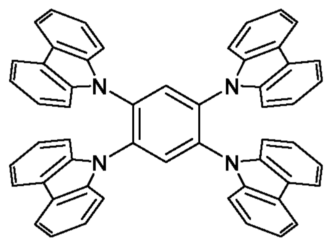

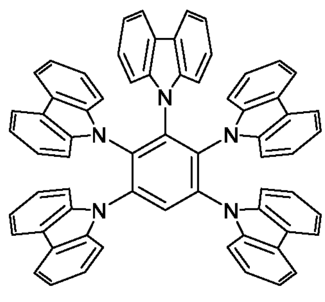

Exciton Barrier Layer

An exciton barrier layer inhibits excitons generated through recombination of holes and electrons in the light emitting layer from being diffused to the charge transporting layer. In some embodiments, the exciton barrier layer enables effective confinement of excitons in the light emitting layer. In some embodiments, the light emission efficiency of the device is enhanced. In some embodiments, the exciton barrier layer is adjacent to the light emitting layer on any of the side of the anode and the side of the cathode, and on both the sides. In some embodiments, where the exciton

barrier layer is on the side of the anode, the layer can be between the hole transporting layer and the light emitting layer and adjacent to the light emitting layer. In some embodiments, where the exciton barrier layer is on the side of the cathode, the layer can be between the light emitting layer and the cathode and adjacent to the light emitting layer. In some embodiments, a hole injection layer, an electron barrier layer, or a similar layer is between the anode and the exciton barrier layer that is adjacent to the light emitting layer on the side of the anode. In some embodiments, a hole injection layer, an electron barrier layer, a hole barrier layer, or a similar layer is between the cathode and the exciton barrier layer that is adjacent to the light emitting layer on the side of the cathode. In some embodiments, the exciton barrier layer comprises excited singlet energy and excited triplet energy, at least one of which is higher than the excited singlet energy and the excited triplet energy of the light emitting material, respectively.

Hole Transporting Layer

The hole transporting layer comprises a hole transporting material. In some embodiments, the hole transporting layer is a single layer. In some embodiments, the hole transporting layer comprises a plurality layers.

In some embodiments, the hole transporting material has one of injection or transporting property of holes and barrier property of electrons. In some embodiments, the hole transporting material is an organic material. In some embodiments, the hole transporting material is an inorganic material. Examples of known hole transporting materials that may be used herein include but are not limited to a triazole derivative, an oxadiazole derivative, an imidazole derivative, a carbazole derivative, an indolocarbazole derivative, a polyarylalkane derivative, a pyrazoline derivative, a pyrazolone derivative, a phenylenediamine derivative, an arylamine derivative, an amino-substituted chalcone derivative, an oxazole derivative, a styrylanthracene derivative, a fluorenone derivative, a hydrazone derivative, a stilbene derivative, a silazane derivative, an aniline copolymer and an electroconductive polymer oligomer, particularly a thiophene oligomer, or a combination thereof. In some embodiments, the hole transporting material is selected from a porphyrin compound, an aromatic tertiary amine compound, and a styrylamine compound. In some embodiments, the hole transporting material is an aromatic tertiary amine compound. Preferred compound examples for use as the hole transporting material are shown below.

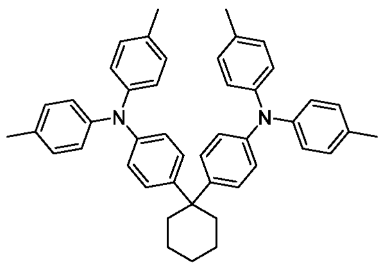
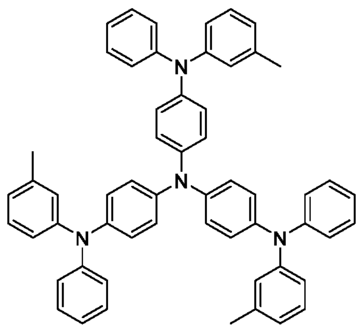
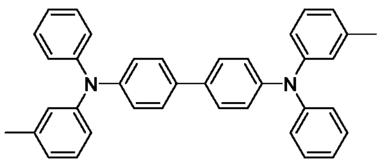

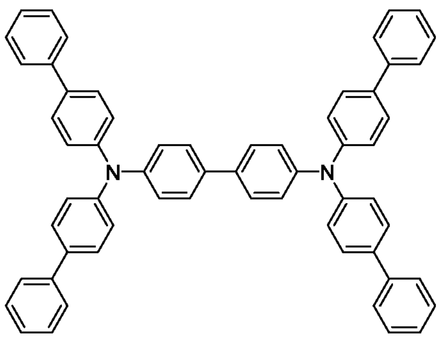
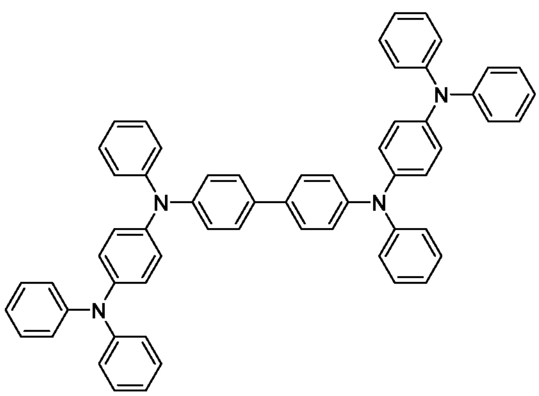

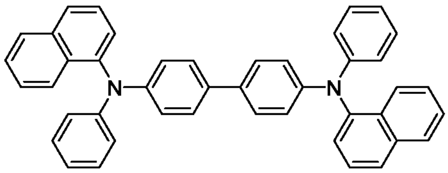
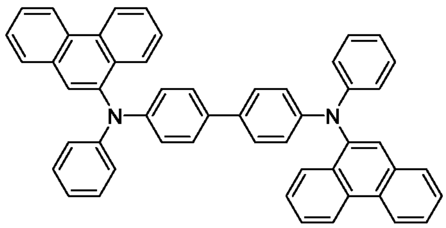

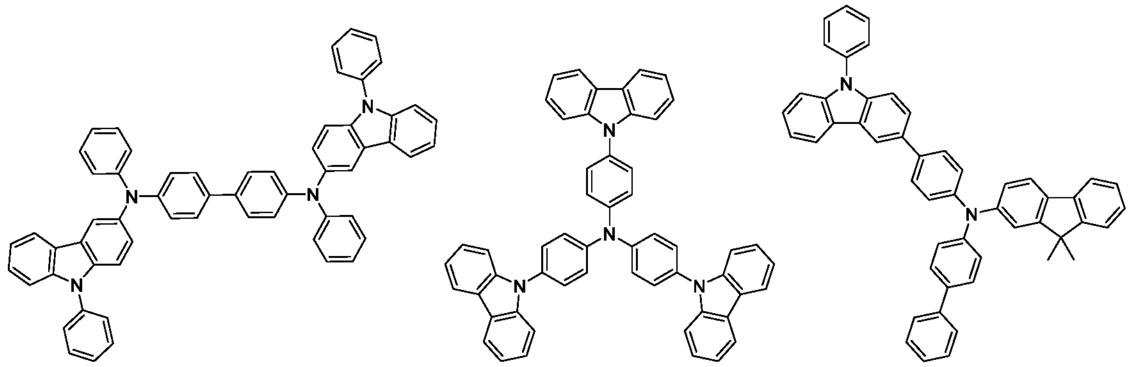

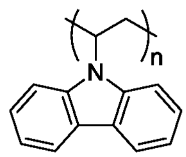
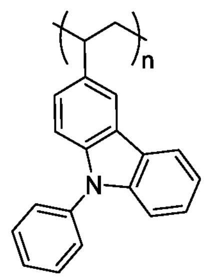
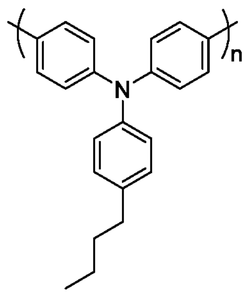

## Electron Transporting Layer

The electron transporting layer comprises an electron transporting material. In some embodiments, the electron transporting layer is a single layer. In some embodiments, the electron transporting layer comprises a plurality of layer.

In some embodiments, the electron transporting material needs only to have a function of transporting electrons, which are injected from the cathode, to the light emitting layer. In some embodiments, the electron transporting material also function as a hole barrier material. Examples of the electron transporting layer that may be used herein include but are not limited to a nitro-substituted fluorene derivative, a diphenylquinone derivative, a thiopyran dioxide derivative, carbodiimide, a fluorenylidene methane derivative, anthraquinodimethane, an anthrone derivatives, an azole derivative, an azine derivative, an oxadiazole derivative, or a combination thereof, or a polymer thereof. In some embodiments, the electron transporting material is a thiadiazole derivative, or a quinoxaline derivative. In some embodiments, the electron transporting material is a polymer material. Preferred compound examples for use as the electron transporting material are shown below.

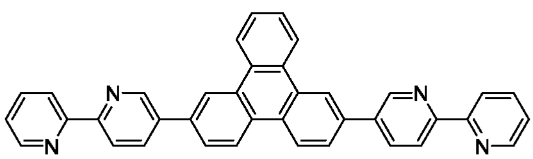
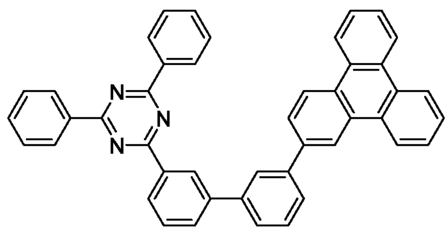

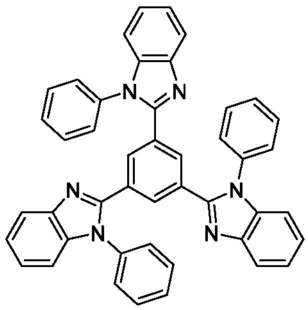
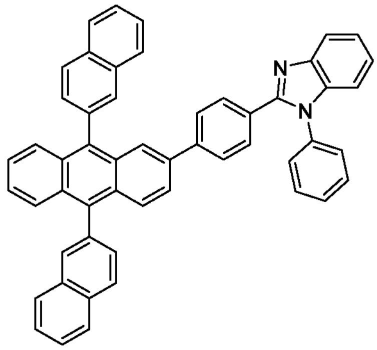

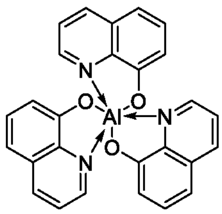
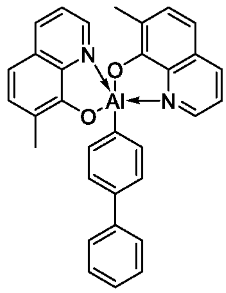

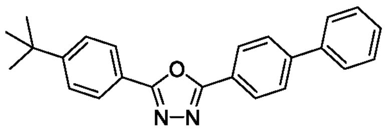
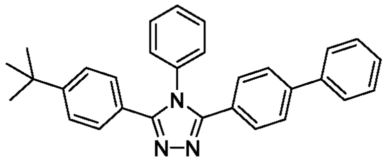

Hereinunder compound examples preferred as a material that can be added to the organic layers are shown. For example, these can be added as a stabilization material.

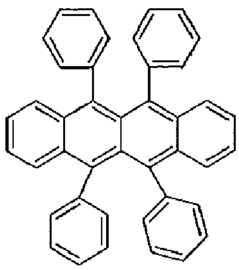
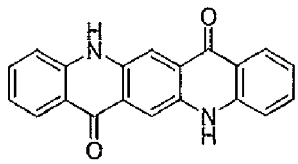
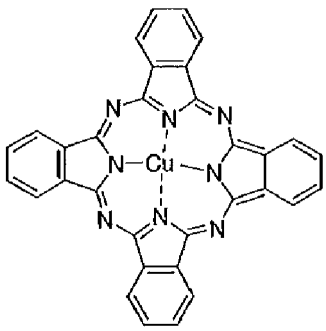

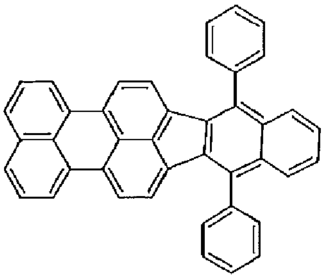
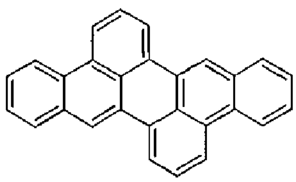

Preferred materials for use in the organic electroluminescent device are specifically shown. However, the materials usable in the invention should not be limitatively interpreted by the following exemplary compounds. Compounds

that are exemplified as materials having a specific function can also be used as materials having any other function.

Devices

In some embodiments, an light emitting layer is incorporated into a device. For example, the device includes, but is not limited to an OLED bulb, an OLED lamp, a television screen, a computer monitor, a mobile phone, and a tablet.

In some embodiments, an electronic device comprises an OLED comprising an anode, a cathode, and at least one organic layer comprising a light emitting layer between the anode and the cathode.

In some embodiments, compositions described herein may be incorporated into various light-sensitive or light-activated devices, such as a OLEDs or photovoltaic devices. In some embodiments, the composition may be useful in facilitating charge transfer or energy transfer within a device and/or as a hole-transport material. The device may be, for example, an organic light emitting diode (OLED), an organic integrated circuit (O-IC), an organic field-effect transistor (O-FET), an organic thin-film transistor (O-TFT), an organic light emitting transistor (O-LET), an organic solar cell (O-SC), an organic optical detector, an organic photoreceptor, an organic field-quench device (O-FQD), a light emitting electrochemical cell (LEC) or an organic laser diode (O-laser).

Bulbs or Lamps

In some embodiments, an electronic device comprises an OLED comprising an anode, a cathode, and at least one organic layer comprising a light emitting layer between the anode and the cathode.

In some embodiments, a device comprises OLEDs that differ in color. In some embodiments, a device comprises an array comprising a combination of OLEDs. In some embodiments, the combination of OLEDs is a combination of three colors (e.g., RGB). In some embodiments, the combination of OLEDs is a combination of colors that are not red, green, or blue (for example, orange and yellow green). In some embodiments, the combination of OLEDs is a combination of two, four, or more colors.

In some embodiments, a device is an OLED light comprising:

a circuit board having a first side with a mounting surface and an opposing second side, and defining at least one aperture;
at least one OLED on the mounting surface, the at least one OLED configured to emanate light, comprising:
an anode, a cathode, and at least one organic layer comprising a light emitting layer between the anode and the cathode;
a housing for the circuit board; and
at least one connector arranged at an end of the housing, the housing and the connector defining a package adapted for installation in a light fixture.

In some embodiments, the OLED light comprises a plurality of OLEDs mounted on a circuit board such that light emanates in a plurality of directions. In some embodiments, a portion of the light emanated in a first direction is deflected to emanate in a second direction. In some embodiments, a reflector is used to deflect the light emanated in a first direction.

Displays or Screens

In some embodiments, the compounds of the invention can be used in a screen or a display. In some embodiments, the compounds of the invention are deposited onto a substrate using a process including, but not limited to, vacuum evaporation, deposition, vapor deposition, or chemical vapor deposition (CVD). In some embodiments, the substrate is a photoplate structure useful in a two-sided etch provides a unique aspect ratio pixel. The screen (which may also be referred to as a mask) is used in a process in the manufacturing of OLED displays. The corresponding artwork pattern design facilitates a very steep and narrow tie-bar between the pixels in the vertical direction and a large, sweeping bevel opening in the horizontal direction. This allows the close patterning of pixels needed for high definition displays while optimizing the chemical deposition onto a TFT backplane.

The internal patterning of the pixel allows the construction of a 3-dimensional pixel opening with varying aspect ratios in the horizontal and vertical directions. Additionally, the use of imaged "stripes" or halftone circles within the pixel area inhibits etching in specific areas until these specific patterns are undercut and fall off the substrate. At that point the entire pixel area is subjected to a similar etch rate but the depths are varying depending on the halftone pattern. Varying the size and spacing of the halftone pattern allows etching to be inhibited at different rates within the pixel allowing for a localized deeper etch needed to create steep vertical bevels.

A preferred material for the deposition mask is invar. Invar is a metal alloy that is cold rolled into long thin sheet

in a steel mill. Invar cannot be electrodeposited onto a rotating mandrel as the nickel mask. A preferred and more cost feasible method for forming the open areas in the mask used for deposition is through a wet chemical etching.

In some embodiments, a screen or display pattern is a pixel matrix on a substrate. In some embodiments, a screen or display pattern is fabricated using lithography (e.g., photolithography and e-beam lithography). In some embodiments, a screen or display pattern is fabricated using a wet chemical etch. In further embodiments, a screen or display pattern is fabricated using plasma etching.

Methods of Manufacturing Devices Using the Disclosed Compounds

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light emitting layer, a counter electrode, and an encapsulation layer, and then is cut from the mother panel.

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light emitting layer, a counter electrode, and an encapsulation layer, and then is cut from the mother panel.

In another aspect, provided herein is a method of manufacturing an organic light emitting diode (OLED) display, the method comprising:

forming a barrier layer on a base substrate of a mother panel;
forming a plurality of display units in units of cell panels on the barrier layer;
forming an encapsulation layer on each of the display units of the cell panels;
applying an organic film to an interface portion between the cell panels.

In some embodiments, the barrier layer is an inorganic film formed of, for example, SiNx, and an edge portion of the barrier layer is covered with an organic film formed of polyimide or acryl. In some embodiments, the organic film helps the mother panel to be softly cut in units of the cell panel.

In some embodiments, the thin film transistor (TFT) layer includes a light emitting layer, a gate electrode, and a source/drain electrode. Each of the plurality of display units may include a thin film transistor (TFT) layer, a planarization film formed on the TFT layer, and a light emitting unit formed on the planarization film, wherein the organic film applied to the interface portion is formed of a same material as a material of the planarization film and is formed at a same time as the planarization film is formed. In some embodiments, a light emitting unit is connected to the TFT layer with a passivation layer and a planarization film therebetween and an encapsulation layer that covers and protects the light emitting unit. In some embodiments of the method of manufacturing, the organic film contacts neither the display units nor the encapsulation layer.

Each of the organic film and the planarization film may include any one of polyimide and acryl. In some embodiments, the barrier layer may be an inorganic film. In some embodiments, the base substrate may be formed of polyimide. The method may further include, before the forming of the barrier layer on one surface of the base substrate formed of polyimide, attaching a carrier substrate formed of a glass material to another surface of the base substrate, and before the cutting along the interface portion, separating the carrier substrate from the base substrate. In some embodiments, the OLED display is a flexible display.

In some embodiments, the passivation layer is an organic film disposed on the TFT layer to cover the TFT layer. In some embodiments, the planarization film is an organic film formed on the passivation layer. In some embodiments, the planarization film is formed of polyimide or acryl, like the organic film formed on the edge portion of the barrier layer. In some embodiments, the planarization film and the organic film are simultaneously formed when the OLED display is manufactured. In some embodiments, the organic film may be formed on the edge portion of the barrier layer such that a portion of the organic film directly contacts the base substrate and a remaining portion of the organic film contacts the barrier layer while surrounding the edge portion of the barrier layer.

In some embodiments, the light emitting layer includes a pixel electrode, a counter electrode, and an organic light emitting layer disposed between the pixel electrode and the counter electrode. In some embodiments, the pixel electrode is connected to the source/drain electrode of the TFT layer.

In some embodiments, when a voltage is applied to the pixel electrode through the TFT layer, an appropriate voltage is formed between the pixel electrode and the counter electrode, and thus the organic light emitting layer emits light, thereby forming an image. Hereinafter, an image forming unit including the TFT layer and the light emitting unit is referred to as a display unit.

In some embodiments, the encapsulation layer that covers the display unit and prevents penetration of external moisture may be formed to have a thin film encapsulation structure in which an organic film and an inorganic film are alternately stacked. In some embodiments, the encapsulation layer has a thin film encapsulation structure in which a plurality of thin films are stacked. In some embodiments, the organic film applied to the interface portion is spaced apart from each of the plurality of display units. In some embodiments, the organic film is formed such that a portion of the organic film directly contacts the base substrate and a remaining portion of the organic film contacts the barrier layer while surrounding an edge portion of the barrier layer.

In one embodiment, the OLED display is flexible and uses the soft base substrate formed of polyimide. In some embodiments, the base substrate is formed on a carrier substrate formed of a glass material, and then the carrier substrate is separated.

In some embodiments, the barrier layer is formed on a surface of the base substrate opposite to the carrier substrate. In one embodiment, the barrier layer is patterned according to a size of each of the cell panels. For example, while the base substrate is formed over the entire surface of a mother panel, the barrier layer is formed according to a size of each of the cell panels, and thus a groove is formed at an interface portion between the barrier layers of the cell panels. Each of the cell panels can be cut along the groove.

In some embodiments, the method of manufacture further comprises cutting along the interface portion, wherein a groove is formed in the barrier layer, wherein at least a portion of the organic film is formed in the groove, and wherein the groove does not penetrate into the base substrate. In some embodiments, the TFT layer of each of the cell panels is formed, and the passivation layer which is an inorganic film and the planarization film which is an organic film are disposed on the TFT layer to cover the TFT layer. At the same time as the planarization film formed of, for example, polyimide or acryl is formed, the groove at the interface portion is covered with the organic film formed of, for example, polyimide or acryl. This is to prevent cracks from occurring by allowing the organic film to absorb an impact generated when each of the cell panels is cut along the groove at the interface portion. That is, if the entire barrier layer is entirely exposed without the organic film, an impact generated when each of the cell panels is cut along the groove at the interface portion is transferred to the barrier layer, thereby increasing the risk of cracks. However, in one embodiment, since the groove at the interface portion between the barrier layers is covered with the organic film and the organic film absorbs an impact that would otherwise be transferred to the barrier layer, each of the cell panels may be softly cut and cracks may be prevented from occurring in the barrier layer. In one embodiment, the organic film covering the groove at the interface portion and the planarization film are spaced apart from each other. For example, if the organic film and the planarization film are connected to each other as one layer, since external moisture may penetrate into the display unit through the planarization film and a portion where the organic film remains, the organic film and the planarization film are spaced apart from each other such that the organic film is spaced apart from the display unit.

In some embodiments, the display unit is formed by forming the light emitting unit, and the encapsulation layer is disposed on the display unit to cover the display unit. As such, once the mother panel is completely manufactured, the carrier substrate that supports the base substrate is separated from the base substrate. In some embodiments, when a laser beam is emitted toward the carrier substrate, the carrier substrate is separated from the base substrate due to a difference in a thermal expansion coefficient between the carrier substrate and the base substrate.

In some embodiments, the mother panel is cut in units of the cell panels. In some embodiments, the mother panel is cut along an interface portion between the cell panels by using a cutter. In some embodiments, since the groove at the interface portion along which the mother panel is cut is covered with the organic film, the organic film absorbs an impact during the cutting. In some embodiments, cracks may be prevented from occurring in the barrier layer during the cutting.

In some embodiments, the methods reduce a defect rate of a product and stabilize its quality.

Another aspect is an OLED display including: a barrier layer that is formed on a base substrate; a display unit that is formed on the barrier layer; an encapsulation layer that is formed on the display unit; and an organic film that is applied to an edge portion of the barrier layer.

Examples

The features of the present invention will be described more specifically with reference to Synthesis Examples and Examples given below. The materials, processes, procedures and the like shown below may be appropriately modified unless they deviate from the substance of the invention. Accordingly, the scope of the invention is not construed as being limited to the specific examples shown below. It should be noted that the light emission characteristics were evaluated using a source meter (available from Keithley Instruments, Inc.: 2400 series), a semiconductor parameter analyzer (available from Agilent Technologies, Inc., E5273A), an optical power meter measurement device (available from Newport Corporation, 1930C), an optical spectroscope (available from Ocean Optics, Inc., USB2000), a spectroradiometer (available from Topcon Corporation, SR-3), and a streak camera (available from Hamamatsu Photonics K.K., Model C4334).

(Synthesis Example 1) Synthesis of Compound C1

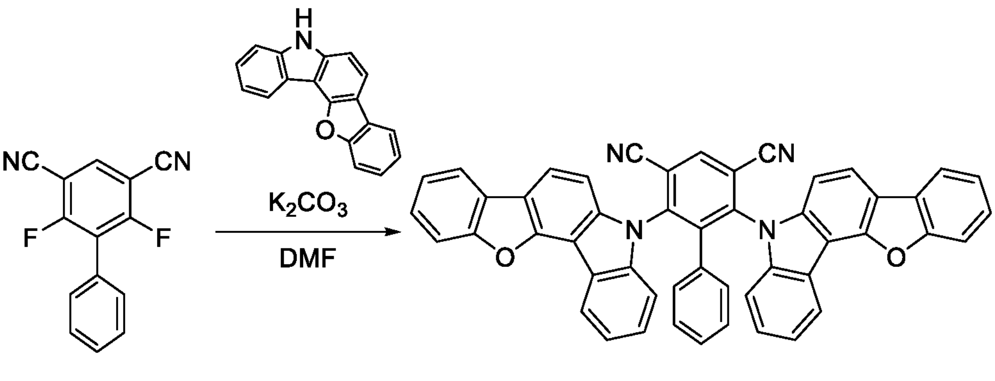


C1

In a nitrogen stream atmosphere, potassium carbonate (1.04 g, 7.5 mmol), benzofuro[2,3-c]carbazole (1.61 g, 6.3 mmol) and 4,6-difluoro-5-phenylisophthalonitrile (0.60 g, 2.5 mmol) were reacted in dimethylformamide (20 mL) at 100°C for 9 hours. Subsequently, this was restored to room temperature, and water and methanol were added to stop the reaction. The precipitated yellow solid was filtered out, and the filtered residue was purified through silica gel column chromatography (toluene) and reprecipitation (toluene/ethanol) to give a yellow solid of a compound C1 (1.33 g, yield 76%).

$^1$H NMR (400 MHz, $CDCl_3$, $\delta$): 8.49 (s, 1H), 8.43 (d, J =7.6 Hz, 2H), 7.96-7.91 (m, 4H), 7.70 (d, J =7.6 Hz, 2H), 7.47-7.36 (m, 8H), 7.14-7.11 (m, 2H), 7.10-7.07 (m, 2H), 6.52-6.46 (m, 3H), 6.37 (t, J =7.6 Hz, 2H).

MS (ASAP): 715.34 (M+H$^+$). Calcd for $C_{50}H_{26}N4O_2$: 714.21.

(Synthesis Example 2) Synthesis of Compound C2

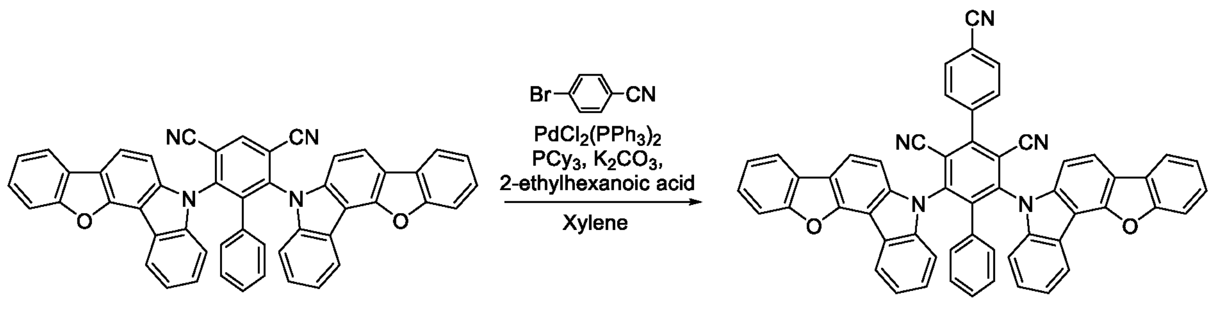


C1 C2

Compound C1 (2.40 g, 3.4 mmol), 4-bromobenzonitrile (1.17 g, 5.0 mmol), potassium carbonate (0.93 g, 6.7 mmol), 2-ethylhexanoic acid (0.10 mg, 0.7 mmol), tricyclohexyl phosphine (0.10 g, 0.5 mmol) and dichlorobis(triphenylphosphine)palladium (0.20 g, 0.3 mmol) were dissolved in xylene (30 mL) in a nitrogen stream atmosphere, and stirred at 120°C for 18 hours. The mixture was restored to room temperature, and a hardly-soluble substance was removed by Celite filtration. The filtrate was concentrated by reduced pressure distillation, and the residue was purified through silica gel column chromatography (chloroform/hexane = 2/1) and reprecipitation (toluene/methanol) to give a white solid of a compound C2 (1.90 g, yield 68%).

$^1$H NMR (400 MHz, $CDCl_3$, $\delta$): 8.43 (d, J =7.6 Hz, 2H), 8.00-7.92 (m, 8H), 7.70 (d, J =8.0 Hz, 2H), 7.48-7.36 (m, 8H), 7.19 (d, J =8.0 Hz, 2H), 7.14 (d, J =8.0 Hz, 2H), 6.54-6.50 (m, 3H), 6.40 (t, J =7.6 Hz, 2H).

MS (ASAP): 816.45 (M+H$^+$). Calcd for $C_{57}H_{29}N_5O_2$: 815.23.

(Synthesis Example 3) Synthesis of Compound C3

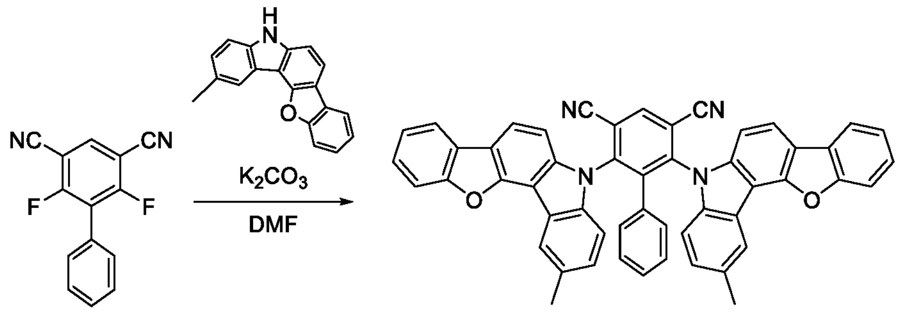


C3

In a nitrogen steam atmosphere, potassium carbonate (1.22 g, 8.8 mmol), benzofuro[2,3-c]carbazole (2.00 g, 7.4 mmol) and 4,6-difluoro-5-phenylisophthalonitrile (0.77 g, 3.0 mmol) were reacted in dimethylformamide (36 mL) at 80°C for 2 hours. Subsequently, this was restored to room temperature and water was added to stop the reaction. The reaction mixture was separated with chloroform added thereto, and the organic layer was dried with magnesium sulfate. The solvent was removed by reduced-pressure distillation, and the residue was purified through silica gel column chromatography (toluene) to give a yellow solid of a compound C3 (2.25 g, yield 90%). $^1H$ NMR (400 MHz, $CDCl_3$, $\delta$): 8.44 (s, 1H), 8.22 (d, J = 5.2 Hz, 2H), 7.92 (t, J = 7.2 Hz, 2H), 7.88-7.81 (m, 2H), 7.69 (d, J = 8.0 Hz, 2H), 7.45-7.40 (m, 2H), 7.38-7.33 (m, 2H), 7.27-7.26 (m, 1H), 7.22 (t, J = 8.4 Hz, 1H), 7.07-6.95 (m, 4H) , 6.49-6.46 (m, 3H), 6.40-6.36 (m, 2H), 2.56 (d, J = 7.2 Hz, 6H).

MS (ASAP): 743.28 [$(M+H)^+$, cal. $C_{52}H_{31}N_4O_2$, 743.24].

(Synthesis Example 4) Synthesis of Compound C4

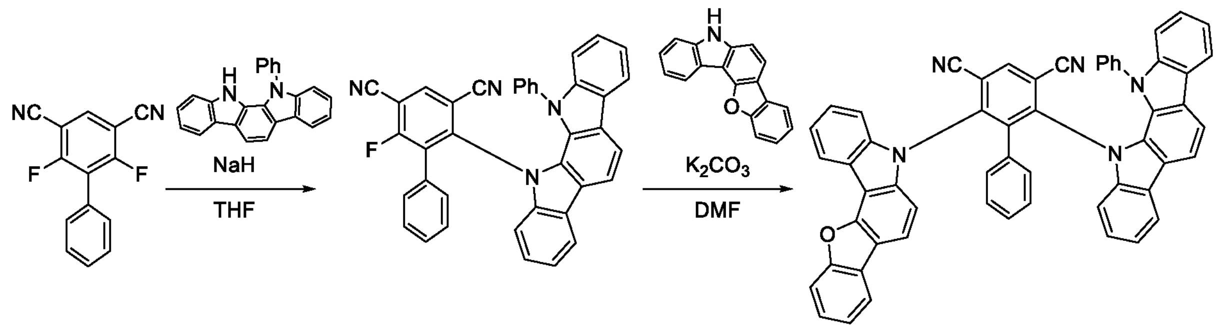


1 C4

In a nitrogen stream atmosphere, sodium hydride (0.30 g, 7.5 mmol) and 11-phenyl-11,12-dihydroindolo[2,3-a]carbazole (2.08 g, 6.3 mmol) were stirred in THF (20 mL) at room temperature for 1 hour, then 4,6-difluoro-5-phenylisophthalonitrile (1.50 g, 6.24 mmol) dissolved in tetrahydrofuran (50 mL) was dropwise added thereto. After this was reacted at room temperature for 5 hours, water was added to stop the reaction. The reaction mixture was separated with chloroform added thereto, and the organic layer was dried with magnesium sulfate. The solvent was removed by reduced-pressure distillation, and the residue was purified through silica gel column chromatography (toluene) to give a yellow solid of an intermediate 1 (1.82 g, yield 53%).

$^1H$ NMR (400 MHz, $CDCl_3$, $\delta$): 8.10 (d, J = 8.4 Hz, 1H), 7.99 (d, J = 7.6 Hz, 1H), 7.78 (s, 1H), 7.61 (d, J = 6.4 Hz, 1H), 7.48-7.40 (m, 4H), 7.36-7.20 (m, 7H), 6.84-6.82 (br, 1H), 6.70 (t, J = 7.6 Hz, 1H), 6.40 (t, J = 7.6 Hz, 2H), 6.06 (br, 1H).

MS (ASAP): 553.70 [$(M+H)^+$, cal. $C_{38}H_{22}FN_4$, 553.18].

In a nitrogen stream atmosphere, potassium carbonate (0.36 g, 2.6 mmol), benzofuro[2,3-c]carbazole (0.54 g, 2.1 mmol) and the intermediate 1 (0.96 g, 1.7 mmol) were reacted in dimethylformamide (17 mL) at room temperature

for 4 hours. Water was added to stop the reaction, and the reaction mixture was separated with chloroform added thereto. The organic layer was dried with magnesium sulfate. The solvent was removed by reduced-pressure distillation, and the residue was purified through silica gel column chromatography (toluene) to give a yellow solid of a compound C4 (1.36 g, yield 99%).

$^1$H NMR (400 MHz, $CDCl_3$, δ): 8.78 (s, 1.00), 8.55 (d, J = 8.8 Hz, 0.54), 8.27-8.19 (m, 2.38), 8.02-7.97 (m, 1.55), 7.89-7.83 (m, 2.85), 7.94-7.75 (m, 1.99), 7.70-7.62 (m, 1.98), 7.59-7.43 (m, 6.17), 7.39-7.34 (m, 0.51), 7.29-7.26 (m, 2.61), 7.21-7.16 (m, 1.02), 7.06-6.99 (m, 1.00), 6.81 (br, 0.90), 6.67-6.61 (m, 1.00), 6.15 (t, J = 7.6 Hz, 0.5), 6.09 (t, J = 7.6 Hz, 0.5), 5.91 (br, 1.87), 5.57 (br, 0.90), 5.29 (br, 0.90);

MS (ASAP): 790.31 [$(M+H)^+$, cal. $C_{56}H_{32}N_4O$, 790.25].

(Synthesis Example 5) Synthesis of Compound C5

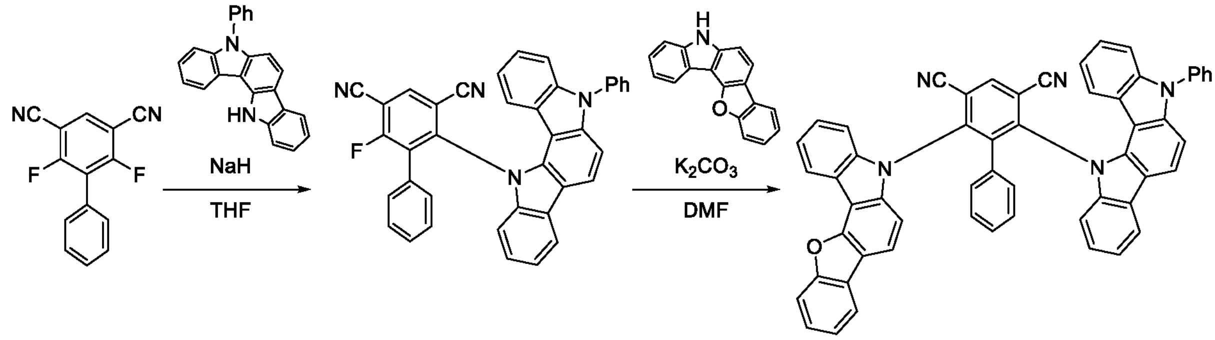


2

C5

In a nitrogen stream atmosphere, sodium hydride (0.30 g, 7.5 mmol) and 5-phenyl-5,12-dihydroindolo[3,2-a]carbazole were stirred in THF (50 mL) at room temperature for 1 hour, and then 4,6-difluoro-5-phenylisophthalonitrile (1.49 g, 6.20 mmol) dissolved in THF (50 mL) was dropwise added thereto. After this was reacted for 15 hours at room temperature, water was added to stop the reaction. The reaction mixture was separated with chloroform added thereto, and the organic layer was dried with magnesium sulfate. The solvent was removed by reduced-pressure distillation, and the residue was purified through silica gel column chromatography (toluene) to give a yellow solid of an intermediate 2 (1.70 g, yield 50%).

$^1$H NMR (400 MHz, $CDCl_3$, δ): 9.05 (d, J = 6.4 Hz, 1H), 8.16 (d, J = 8.8 Hz, 1H), 8.09-8.07 (m, 1H), 7.72-7.68 (m, 2H), 7.61-7.57 (m, 3H), 7.37-7.29 (m, 2H), 7.27-7.19 (m, 4H), 7.14-7.02 (m, 2H), 6.99-6.95 (m, 4H), 6.21 (d, J = 8.0 Hz, 1H).

MS (ASAP): 553.40 [$(M+H)^+$, cal. $C_{38}H_{22}FN_4$, 553.18].

In a nitrogen stream atmosphere, potassium carbonate (0.43 g, 3.1 mmol), benzofuro[2,3-c]carbazole (0.66 g, 2.6 mmol) and the intermediate 2 (1.30 g, 2.4 mmol) were reacted in dimethylformamide (25 mL) at room temperature for 4 hours. Water was added to stop the reaction, and the reaction mixture was separated with chloroform added thereto. The organic layer was dried with magnesium sulfate. The solvent was removed by reduced-pressure distillation, and the residue was purified through silica gel column chromatography (toluene) to give a yellow solid of a compound C5 (1.63 g, yield 88%).

$^1$H NMR (400 MHz, $CDCl_3$, δ): 9.44 (s, 1H), 8.22-8.17 (m, 1H), 8.12-8.07 (m, 1H), 7.76 (d, J = 8.0 Hz, 1H), 7.69-7.26 (m, 17H), 7.08 (d, J = 8.4 Hz, 1H), 6.54-6.49 (m, 1H), 6.40-6.03 (m, 5H).

MS (ASAP): 790.37 [$(M+H)^+$, cal. $C_{56}H_{32}N_4O$, 790.25].

(Synthesis Example 6) Synthesis of Compound C6

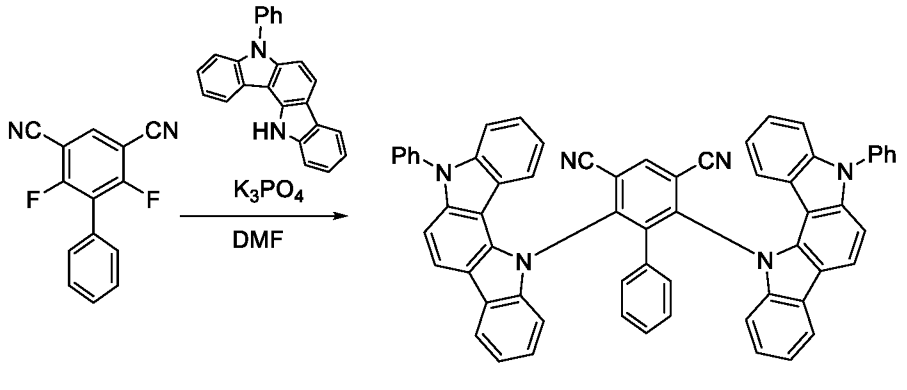


C6

In a nitrogen stream atmosphere, potassium phosphate (1.74 g, 8.2 mmol), 5-phenyl-5,12-dihydroindolo[3,2-a]carbazole (2.28 g, 6.8 mmol) and 4,6-difluoro-5-phenylisophthalonitrile (0.66 g, 2.7 mmol) were reacted in dimethylformamide (30 mL) at 110°C for 6 hours. After the reaction, water was added to stop the reaction, and the reaction mixture was separated with chloroform added thereto. The organic layer was dried with magnesium sulfate. The solvent was removed by reduced-pressure distillation, and the residue was purified through silica gel column chromatography (toluene) to give a yellow solid of a compound C6 (0.97 g, yield 41%).

$^1$H NMR (400 MHz, $CDCl_3$, $\delta$): 9.57 (s, 1H), 8.01 (d, J = 7.6 Hz, 2H), 7.91 (d, J = 8.4 Hz, 2H), 7.64-7.60 (m, 4H), 7.54-7.50 (m, 4H), 7.42-7.35 (m, 10H), 7.26 (t, J = 8.0 Hz, 2H), 7.22-7.19 (m, 2H), 6.91 (d, J = 8.4 Hz, 2H), 6.52-6.49 (m, 2H), 6.21 (t, J = 7.2 Hz, 1H), 5.78 (t, J = 8.0 Hz, 2H), 5.22 (d, J = 7.6 Hz, 2H).

MS (ASAP): 865.47 [$(M+H)^+$, cal. $C_{62}H_{37}N_6$, 865.30].

(Synthesis Example 7) Synthesis of Compound C7

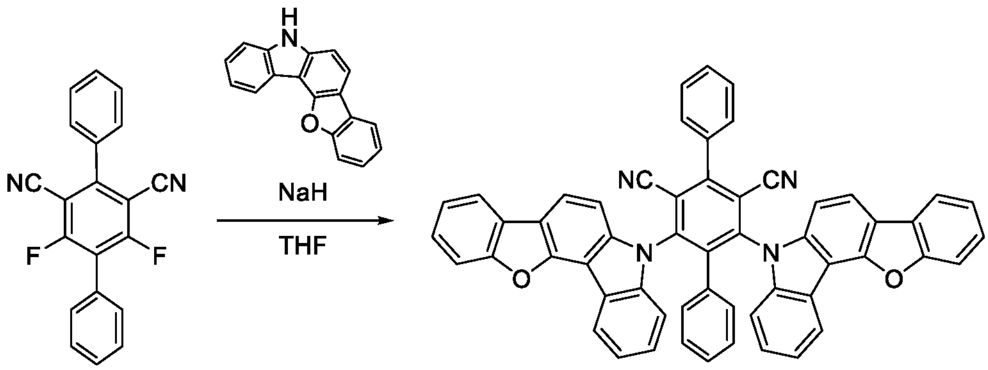


C7

In a nitrogen stream atmosphere, sodium hydride (0.19 g, 4.7 mmol), and benzofuro[3,2-c]carbazole (1.02 g, 4.0 mmol) were stirred in tetrahydrofuran (15 mL) at 0°C for 30 minutes, and then 4,6-difluoro-5-phenylisophthalonitrile was added. The reaction solution was heated up to 40°C and reacted for 6 hours, and then restored to room temperature, and water and methanol were added to stop the reaction. The precipitated yellow solid was filtered out, and the filtered residue was purified through silica gel column chromatography (toluene/hexane = 4/1) and reprecipitation (toluene/methanol) to give a yellow solid of a compound C7 (0.98 g, yield 78%).

$^1$H NMR (400 MHz, $CDCl_3$, $\delta$): 8.44 (d, J =8.0 Hz, 2H), 7.99-7.95 (m, 4H), 7.89-7.86 (m, 2H), 7.73-7.63 (m, 5H), 7.49-7.45 (m, 4H), 7.42-7.37 (m, 4H), 7.25 (d, J =8.4 Hz, 2H), 7.23 (d, J =8.8 Hz, 2H), 6.56-6.50 (m, 3H), 6.40 (t, J =7.6 Hz, 2H).

MS (ASAP): 791.26 ($M+H^+$). Calcd for $C_{56}H_{30}N_4O_2$: 790.24

(Synthesis Example 8) Synthesis of Compound C8

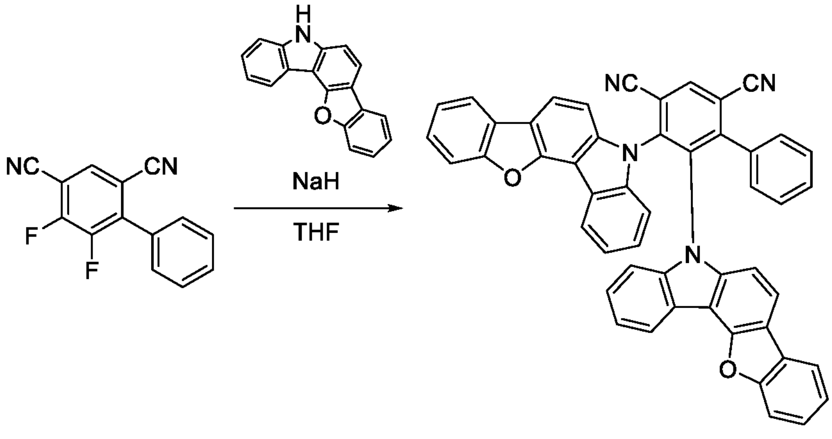


**C8**

In a nitrogen stream atmosphere, sodium hydride (0.49 g, 11.8 mmol), and benzofuro[3,2-c]carbazole (2.54 g, 9.9 mmol) were stirred in tetrahydrofuran (20 mL) at 0°C for 30 minutes, and then 4,5-difluoro-6-phenylisophthalonitrile was added. The reaction solution was restored to room temperature and reacted for 6 hours at room temperature, and then water and methanol were added to stop the reaction. The precipitated yellow solid was filtered out, and the filtered residue was purified through silica gel column chromatography (toluene/chloroform = 10/1) and reprecipitation (toluene/methanol) to give a yellow solid of a compound C8 (0.48 g, yield 17%).

$^1$H NMR (400 MHz, $CDCl_3$, δ): (With the presence of stereoisomers in the sample, the proton number is displayed as a relative ratio.) 8.53 (s, 1H), 8.12 (t, J =7.6 Hz, 1H), 7.97-7.94 (m, 1H), 7.89-7.74 (m, 2H), 7.67 (d, J =8.4 Hz, 0.5H), 7.59-7.50 (m, 3H), 7.45 (d, J =8.4 Hz, 0.5H), 7.41-7.28 (m, 4H), 7.22-7.17 (m, 0.5H), 7.14-6.97 (m, 10H), 6.90-6.84 (m, 2.5H).

MS (ASAP): 715.38 (M+H$^+$). Calcd for $C_{50}H_{26}N_4O_2$: 714.21

(Synthesis Example 9) Synthesis of Compound C9

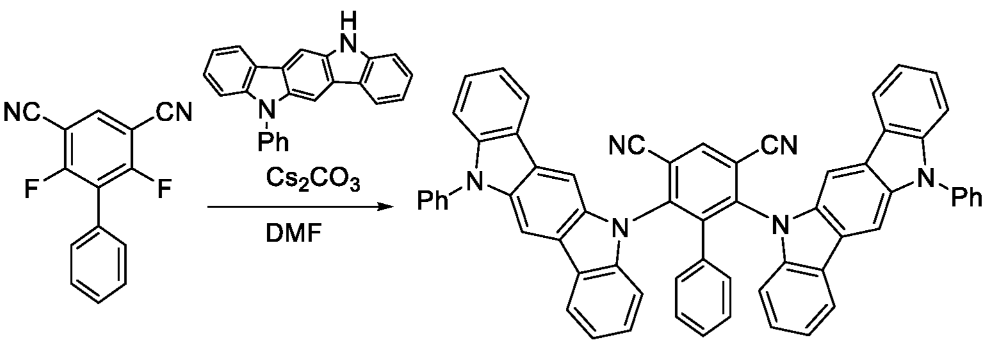


**C9**

In a nitrogen stream atmosphere, cesium carbonate (1.30 g, 4.0 mmol), 5-phenyl-5,11-dihydroindolo[3,2-a]carbazole (0.78 g, 2.3 mmol) and 4,6-difluoro-5-phenylisophthalonitrile (0.24 g, 1.0 mmol) were reacted in dimethylformamide (20 mL) at 80°C for 12 hours. After the reaction, water was added at room temperature to stop the reaction, and the reaction mixture was separated with chloroform added thereto. The organic layer was dried with magnesium sulfate. The solvent was removed by reduced-pressure distillation, and the residue was purified through silica gel column chromatography (toluene) and reprecipitation (toluene/methanol) to give a yellow solid of a compound C9 (0.17 g, yield 22%).

$^1$H NMR (400 MHz, $CDCl_3$, δ): 8.53 (d, J =7.6 Hz, 1H), 8.21 (d, J =8.0 Hz, 1H), 8.15 (d, J =8.0 Hz, 1H), 7.89-7.96

(m, 4H), 7.85 (s, 1H), 7.78 (s, 1H), 7.07-7.66 (m, 22H), 6.95 (d, J =8.0 Hz, 1H), 6.40-6.58 (m, 3H), 6.34 (t, J =8.0 Hz, 2H).

MS (ASAP): 865.58 ($M+H^+$). Calcd for $C_{62}H_{36}N_6$: 864.30

(Synthesis Example 10) Synthesis of Compound C10

A compound C10 was synthesized according to the same method as in Synthesis Example 1 (yield 84%).

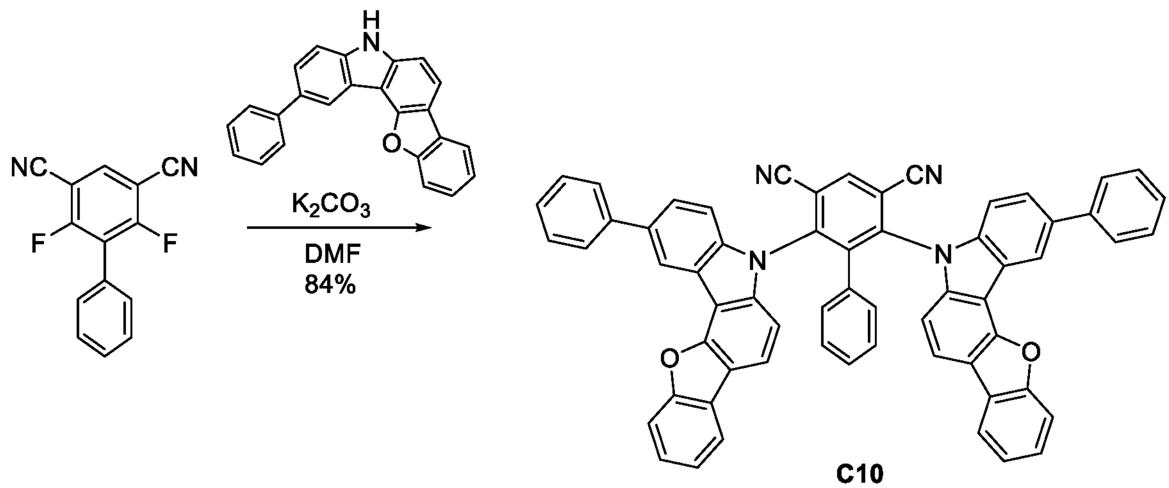


C10

$^1$H NMR (400 MHz, $CDCl_3$, δ): 8.62 (s, 2H), 8.51 (s, 1H), 7.99-7.90 (m, 4H), 7.79-7.71 (m, 6H), 7.69-7.64 (m, 2H), 7.52-7.44 (m, 6H), 7.39-7.35 (m, 4H), 7.21-7.18 (m, 2H), 7.09-7.06 (m, 2H), 6.58-6.50 (m, 3H), 6.41 (t, J = 8.4 Hz, 2H).

MS (ASAP): 867.50 ($M+H^+$). Calcd for $C_{62}H_{34}N_4O_2$: 866.27.

(Synthesis Example 11) Synthesis of Compound C11

A compound C11 was synthesized according to the same method as in Synthesis Example 1 (yield 78%).

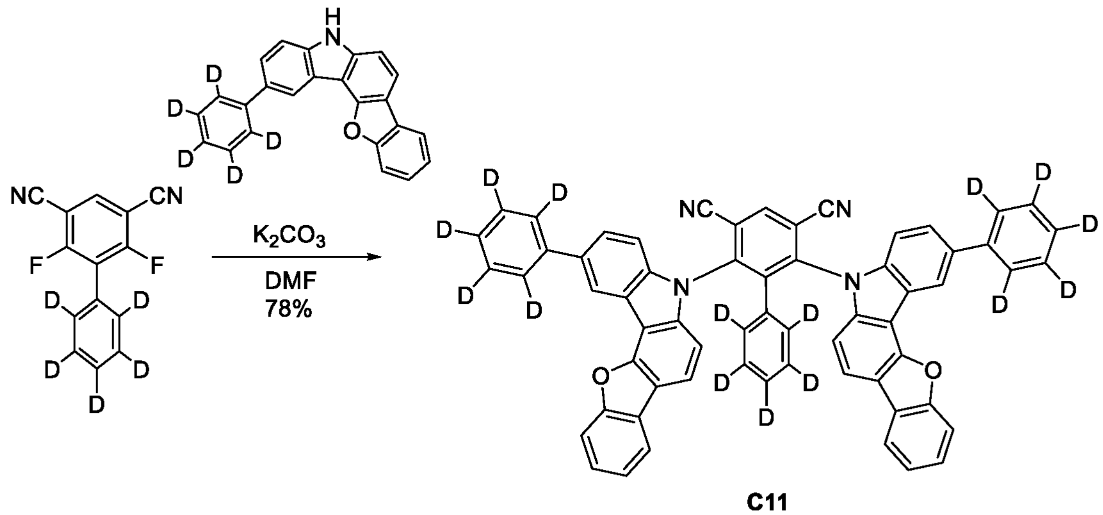


C11

$^1$H NMR (400 MHz, $CDCl_3$, δ): 8.62 (s, 2H), 8.51 (s, 1H), 7.96-7.91 (m, 4H), 7.72 (d, J = 8.2 Hz, 2H), 7.70-7.64 (m, 2H), 7.46-7.37 (m, 2H), 7.21-7.19 (m, 2H), 7.09-7.06 (m, 2H),

MS (ASAP): 882.21 ($M+H^+$). Calcd for $C_{62}H_{19}D_{15}N_4O_2$: 881.36.

(Synthesis Example 12) Synthesis of Compound C12

A compound C12 was synthesized according to the same method as in Synthesis Example 1 (yield 78%).

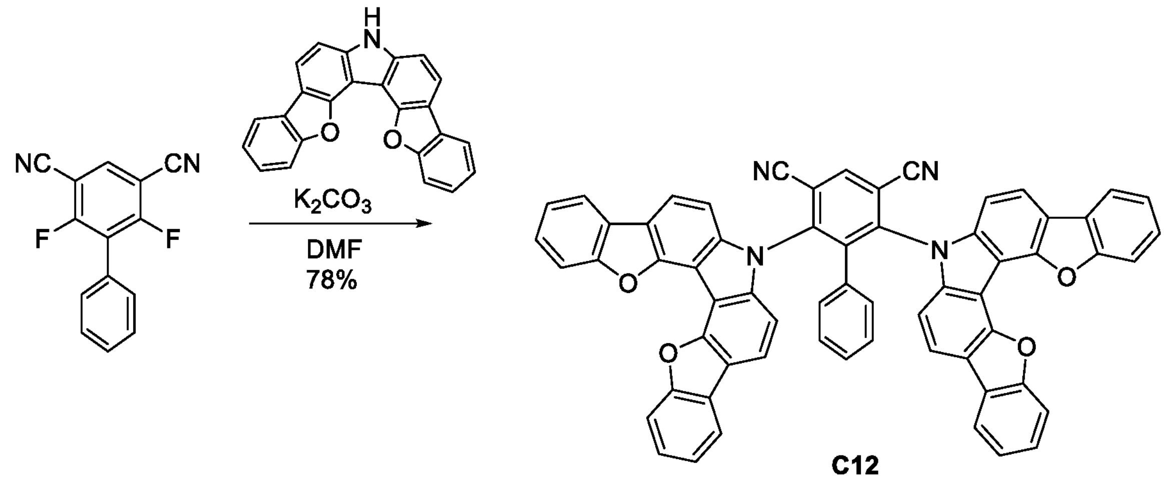


**C12**

$^{1}$H NMR (400 MHz, DMSO, δ): 9.48 (s, 1H), 8.51 (s, 1H), 8.29 (d, J = 8.4 Hz, 4H), 8.19 (d, J = 8.4 Hz, 4H), 7.91 (m J = 8.4 Hz, 4H), 7.84 (t, J = 6.8 Hz, 4H), 7.45 (t, J = 6.8 Hz, 4H), 6.73(t, J = 7.2 Hz ,2H), 6.38 (t, J = 7.2 Hz ,3H),

MS (ASAP): 895.35 (M+H$^+$). Calcd for $C_{62}H_3ON_4O_4$:894.23

(Synthesis Example 13) Synthesis of Compound C13

A compound C13 was synthesized according to the same method as in Synthesis Example 1 (yield 64%).

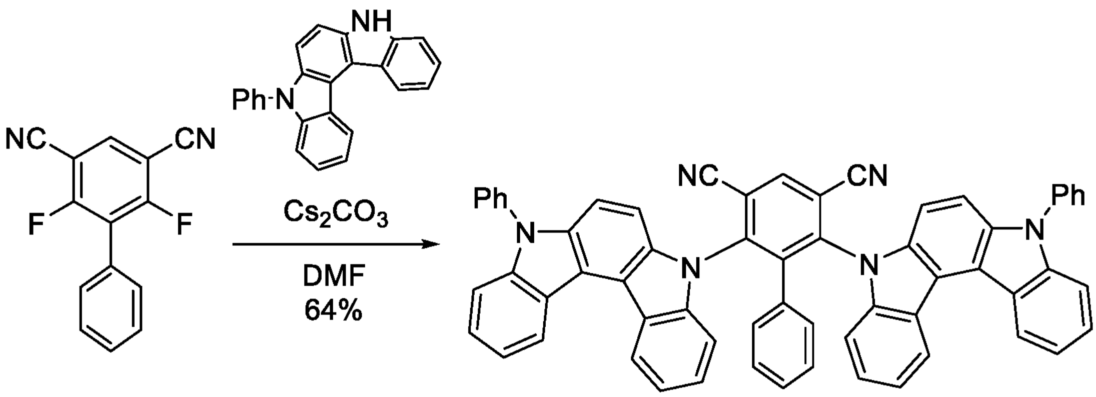


**C13**

$^{1}$H NMR (400 MHz, $CDCl_3$, δ): 8.82-8.76 (m, 4H), 8.45 (s, 1H), 7.64-7.32 (m, 22H), 7.21 (d, J = 9.2 Hz, 2H), 7.08-7.05 (m, 2H), 6.47-6.44 (m, 3H), 6.32 (t, J = 9.2 Hz, 2H). MS (ASAP): 865.27 (M+H$^+$). Calcd for $C_{62}H_{36}N_6$: 864.30

(Synthesis Example 14) Synthesis of Compound C14

A compound C14 was synthesized according to the same method as in Synthesis Example 1 (yield 47%).

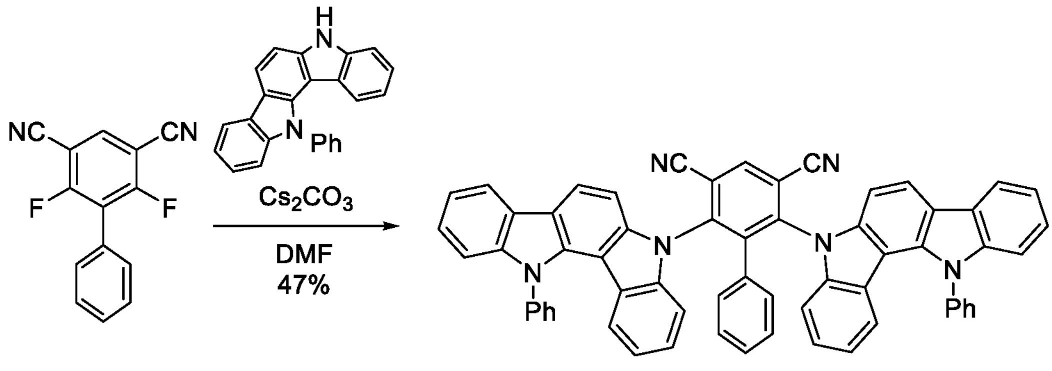


C14

$^1H$ NMR (400 MHz, $CDCl_3$, δ): 8.47 (s, 1H), 8.19-8.10 (m, 4H), 7.68-7.51 (m, 10H), 7.38-7.26 (m, 6H), 7.20-7.14 (m, 2H), 7.06-6.98 (m ,4H), 6.74 (t, J = 7.6 Hz, 2H), 6.47-6.44 (m, 3H), 6.31 (t, J =7.6 Hz, 2H)..

MS (ASAP): 865.37 ($M+H^+$). Calcd for $C_{62}H_{36}N_6$: 864.30

(Examples 1 to 9, Comparative Examples 1 to 4)

Production and Evaluation of Thin Film

On a quartz substrate according to a vacuum evaporation method, the compound C1 and Host 1 were evaporated from different evaporation sources under the condition of a vacuum degree of less than $1\times10^{-3}$ Pa to form a thin film having a thickness of 100 nm in which the concentration of the compound C1 was 20% by mass, and this is a doped thin film of Example 1.

In place of the compound C1, the compounds C2 to C9 were individually used to produce thin films of Examples 2 to 9, respectively. Also using the comparative compound A and PPF and in the same manner, a thin film of Comparative Example 1 was formed. All the compounds used as light emitting materials in Examples and Comparative Examples in the present description were purified by sublimation before use.

The resultant thin films were individually irradiated with 300-nm excitation light, and all the thin films produced photoluminescence. The lifetime ($\tau_d$) of the delayed fluorescence was derived from the transient decay curve of emission, and based on the lifetime of Comparative Example 1, a relative value to Comparative Example 1 was calculated. The results are as shown in the following Table. It is confirmed that the delayed fluorescence lifetime ($\tau_d$) of Examples 1 to 9 is short.

[Table 2]

| | Compound | $\tau_d$ (%) |
|---|---|---|
| Example 1 | Compound C1 | 25.7 |
| Example 2 | Compound C2 | 16.2 |
| Example 3 | Compound C3 | 23.2 |
| Example 4 | Compound C4 | 19.2 |
| Example 5 | Compound C5 | 39.2 |
| Example 6 | Compound C6 | 41.5 |
| Example 7 | Compound C7 | 43.7 |
| Example 8 | Compound C8 | 36.5 |
| Example 9 | Compound C9 | 9.95 |
| Comparative Example 1 | Comparative Compound A | 100 |

(Examples 10 to 14, Comparative Example 1) Production and Evaluation of Organic Electroluminescent Device

On a glass substrate having, as formed thereon, an anode of indium tin oxide (ITO) having a thickness of 100

nm, thin films were laminated at a vacuum degree of $1\times10^{-6}$ Pa in a vacuum evaporation method. First, HATCN was formed on ITO at a thickness of 10 nm, then NPD was formed thereon at a thickness of 30 nm. Next, TrisPCz was formed at a thickness of 10 nm, and Host1 was formed further thereon at a thickness of 5 nm. Next, the compound C1 and Host1 were co-evaporated from different evaporation sources to form a light emitting layer having a thickness of 30 nm. At that time, the concentration of the compound C1 was 35% by weight. On this, SF3TRZ was formed at a thickness of 10 nm, and further on this, SF3TRZ and Liq were co-evaporated from different evaporation sources at a thickness of 30 nm. At that time, SF3TRZ/Liq (by weight) was 7/3. Further, Liq was formed at a thickness of 2 nm, and then aluminum (Al) was evaporated at a thickness of 100 nm to form a cathode. According to the above process, an organic electroluminescent device of Example 10 was produced.

The compound C2, the compound C3, the compound C4 and a comparative compound A were individually used, in place of the compound C1, to produce organic electroluminescent devices of Examples 11 to 14 and Comparative Example 2, respectively.

(Evaluation)

Emission from the organic electroluminescent device of Example 10 was analyzed to measure x and y in the CIE chromaticity coordinate. The results were x = 0.26 and y = 0.57 and confirmed good chromaticity. Of the organic electroluminescent devices of Example 10 and Comparative Example 2, the time in which the emission intensity at 12.6 mA/cm$^2$ lowered to 95% (LT95) was measured. LT95 of Comparative Example 2 was referred to as 1, and based on this, a relative value of Example 10 was calculated. The results are as shown in the following Table. The device lifetime (device durability) of the organic electroluminescent device of Example 10 significantly improved.

[Table 3]

| | Compound | LT95 (relative value) |
|---|---|---|
| Example 10 | Compound C1 | 54.4 |
| Comparative Example 2 | Comparative Compound A | 1 |

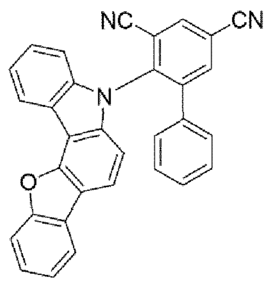

Comparative Compound A

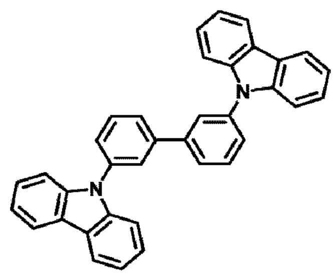

mCBP

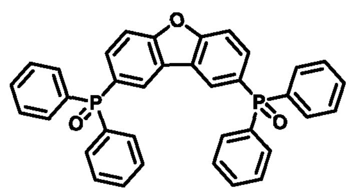

PPF

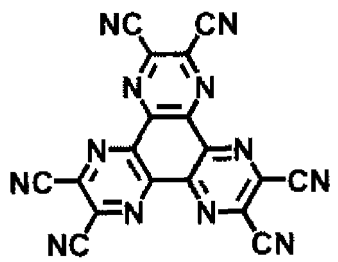

HATCN

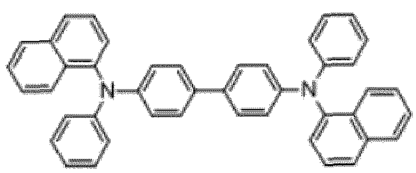

NPD

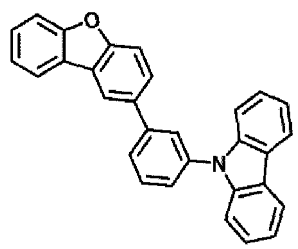

Host1

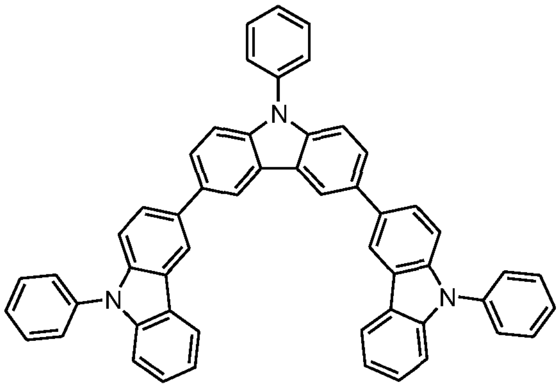

TrisPCz

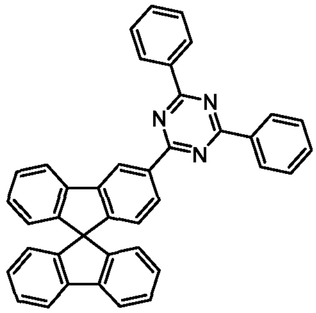

SF3TRZ

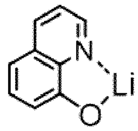

Liq

Reference Signs List

1 Substrate
2 Anode
3 Hole Injection Layer
4 Hole Transporting Layer
5 Light Emitting Layer
6 Electron Transporting Layer
7 Cathode

## Claims

**1.** A compound represented by the following general formula (1):

General Formula (1)

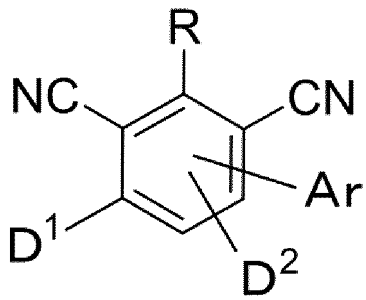

wherein:

R represents a hydrogen atom, a deuterium atom, a substituted or unsubstituted aryl group, or a substituted or unsubstituted heteroaryl group bonding via a carbon atom,
Ar represents a substituted or unsubstituted aryl group, or a substituted or unsubstituted heteroaryl group bonding via a carbon atom, and
$D^1$ and $D^2$ each independently represent a donor group, and at least one of them is a hetero ring-condensed carbazol-9-yl group in which the hetero ring and the carbazol can be substituted.

**2.** The compound according to claim 1, wherein the compound is represented by the following general formula (2):

General Formula (2)

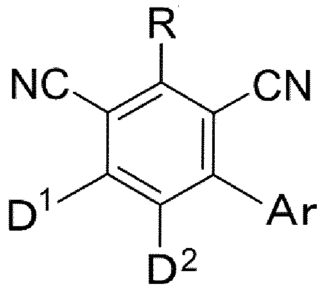

3. The compound according to claim 1, wherein the compound is represented by the following general formula (3):

General Formula (3)

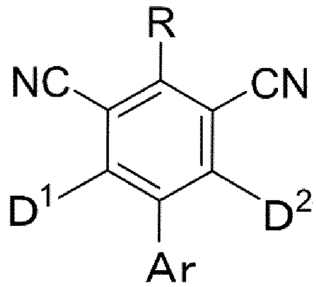

4. The compound according to any one of claims 1 to 3, wherein $D^1$ and $D^2$ are the same.

5. The compound according to any one of claims 1 to 3, wherein $D^1$ and $D^2$ are different.

6. The compound according to any one of claims 1 to 5, wherein the hetero ring condensed with the carbazol-9-yl group of the hetero ring-condensed carbazol-9-yl group is a substituted or unsubstituted furan ring, a substituted or unsubstituted thiophene ring, or a substituted or unsubstituted pyrrole ring, and any other ring can be further condensed with the furan ring, the thiophene ring and the pyrrole ring.

7. The compound according to any one of claims 1 to 6, wherein the hetero ring-condensed carbazol-9-yl group has a structure of any of the following:

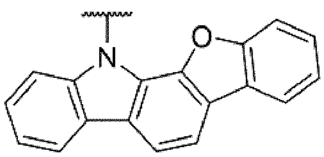

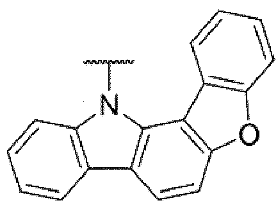

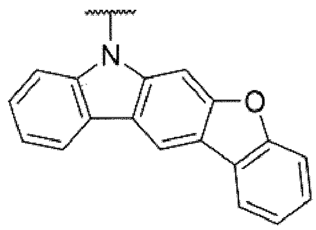

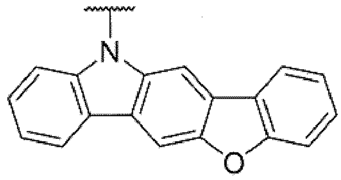
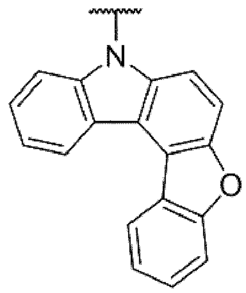
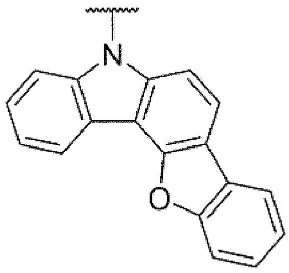

wherein hydrogen atoms can be substituted, with which, however, any further hetero atom is not condensed.

**8.** The compound according to any one of claims 1 to 6, wherein the hetero ring-condensed carbazol-9-yl group has a structure of any of the following:

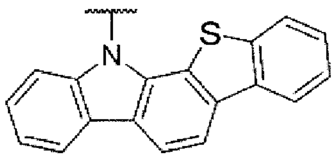
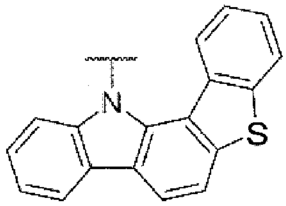
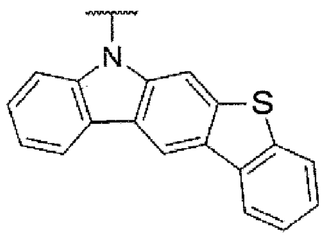

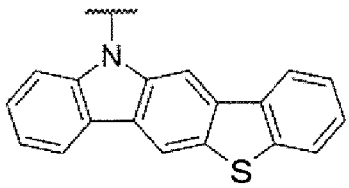
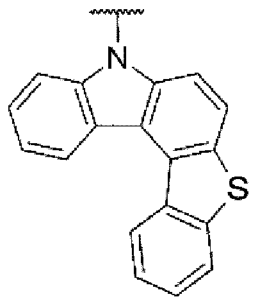
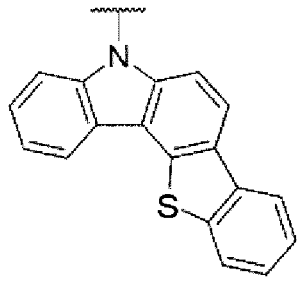

wherein hydrogen atoms can be substituted, with which, however, any further hetero atom is not condensed.

**9.** The compound according to any one of claims 1 to 6, wherein the hetero ring-condensed carbazol-9-yl group has a structure of any of the following:

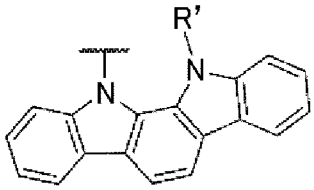
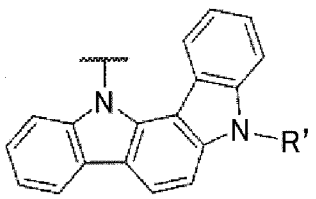
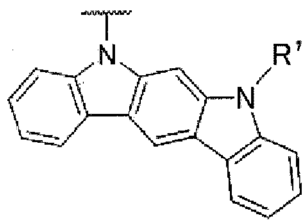

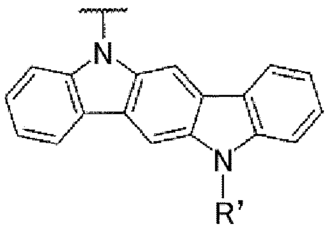 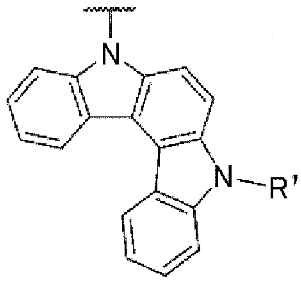 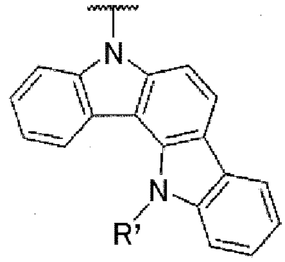

wherein hydrogen atoms can be substituted, with which, however, any further hetero atom is not condensed, and R' represents a hydrogen atom, a deuterium atom or a substituent.

**10.** The compound according to any one of claims 1 to 9, wherein two hetero rings selected from the group consisting of a substituted or unsubstituted furan ring, a substituted or unsubstituted thiophene ring and a substituted or unsubstituted pyrrole ring, in which any other ring can be condensed with the furan ring, the thiophene ring and the pyrrole ring, are condensed with the carbazol-9-yl group of the hetero ring-condensed carbazol-9-yl group.

**11.** The compound according to any one of claims 1 to 10, wherein the hetero ring-condensed carbazol-9-yl group has a structure with a hetero ring condensed at 1,2-positions of the carbazole ring.

**12.** The compound according to any one of claims 1 to 10, wherein the hetero ring-condensed carbazol-9-yl group has a structure with a hetero ring condensed at 2,3-positions of the carbazole ring.

**13.** The compound according to any one of claims 1 to 10, wherein the hetero ring-condensed carbazol-9-yl group has a structure with a hetero ring condensed at 3,4-positions of the carbazole ring.

**14.** The compound according to any one of claims 1 to 13, wherein R and Ar differ.

**15.** The compound according to any one of claims 1 to 13, wherein R is a hydrogen atom or a deuterium atom.

**16.** The compound according to any one of claims 1 to 15, wherein Ar is a substituted or unsubstituted phenyl group, or a substituted or unsubstituted pyridyl group.

**17.** The compound according to any one of claims 1 to 16, which is composed of atoms selected from the group consisting of a carbon atom, a hydrogen atom, a deuterium atom, a nitrogen atom, an oxygen atom and a sulfur atom.

**18.** A light emitting material of the compound of any one of claims 1 to 17.

**19.** A light emitting device containing the compound of any one of claims 1 to 17.

**20.** The light emitting device according to claim 19, wherein the light emitting device has a light emitting layer and the light emitting layer contains the compound and a host material.

**21.** The light emitting device according to claim 20, wherein the light emitting device has a light emitting layer, the light emitting layer contains the compound and a light emitting material, and the light emitting material mainly emits light.

Fig. 1

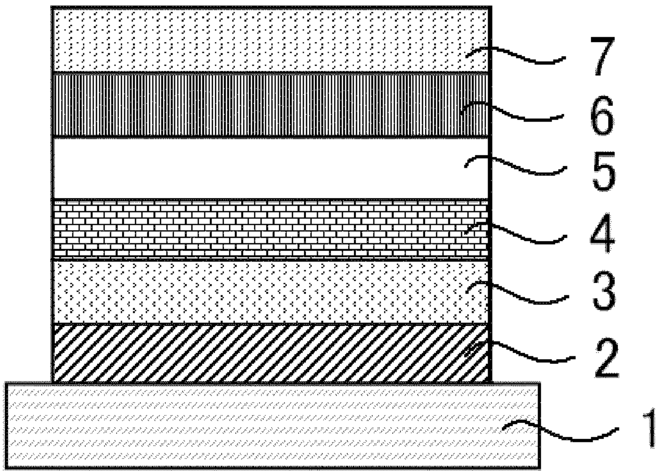

## INTERNATIONAL SEARCH REPORT

International application No.
PCT/JP2021/019431

A. CLASSIFICATION OF SUBJECT MATTER
Int.Cl. C07D491/048(2006.01)i, C09K11/06(2006.01)i, C07D487/04(2006.01)i, C07D519/00(2006.01)i, H01L51/50(2006.01)n
FI: C07D491/048CSP, C09K11/06640, C09K11/06620, C09K11/06690, C07D519/00301, C09K11/06655, C09K11/06650, C07D487/04137, H05B33/14B
According to International Patent Classification (IPC) or to both national classification and IPC

B. FIELDS SEARCHED
Minimum documentation searched (classification system followed by classification symbols)
Int.Cl. C07D491/048, C09K11/06, C07D487/04, C07D519/00, H01L51/50

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2019/195104 A1 (KYULUX, INC.) 10 October 2019 (2019-10-10), particularly, formula (I) | 1-21 |
| X | JP 2016-516085 A (MERCK PATENT GMBH) 02 June 2016 (2016-06-02), particularly, formula (I), compounds 245-247, 252, 253 | 1-21 |
| A | WO 2018/237389 A1 (KYULUX INC.) 27 December 2018 (2018-12-27) | 1-21 |
| A | WO 2014/208698 A1 (IDEMITSU KOSAN CO., LTD.) 31 December 2014 (2014-12-31) | 1-21 |

☒ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 July 2021 | 03 August 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/019431

C (Continuation). DOCUMENTS CONSIDERED TO BE RELEVANT

| Category* | Citation of document, with indication, where appropriate, of the relevant passages | Relevant to claim No. |
|---|---|---|
| A | WO 2020/085765 A1 (LG CHEM, LTD.) 30 April 2020 (2020-04-30) | 1-21 |
| P, X | WO 2021/066059 A1 (IDEMITSU KOSAN CO., LTD.) 08 April 2021 (2021-04-08), particularly, claims, examples | 1-21 |

Form PCT/ISA/210 (second sheet) (January 2015)

**INTERNATIONAL SEARCH REPORT**
Information on patent family members

International application No.
PCT/JP2021/019431

| | | |
|---|---|---|
| WO 2019/195104 A1 | 10 October 2019 | (Family: none) |
| JP 2016-516085 A | 02 June 2016 | US 2016/0072076 A1<br>particularly, formula (I), compounds 245-247, 252, 253<br>WO 2014/146752 A1<br>EP 2976329 A1<br>EP 3375785 A1<br>CN 105051014 A<br>KR 10-2015-0132872 A<br>CN 107739352 A |
| WO 2018/237389 A1 | 27 December 2018 | US 2020/0119287 A1<br>US 2020/0115364 A1<br>US 2020/0115376 A1<br>WO 2018/237385 A1<br>WO 2018/237393 A1<br>EP 3642303 A1<br>KR 10-2020-0019694 A<br>CN 110914378 A<br>KR 10-2020-0023371 A<br>CN 110997866 A |
| WO 2014/208698 A1 | 31 December 2014 | US 2016/0130225 A1<br>US 2020/0148639 A1<br>EP 3015457 A1<br>CN 105209434 A<br>KR 10-2016-0023655 A<br>CN 108822137 A |
| WO 2020/085765 A1 | 30 April 2020 | KR 10-2020-0045433 A |
| WO 2021/066059 A1 | 08 April 2021 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014208698 A1
- WO 2015022974 A
- WO 2013154064 A
- WO 2013011954 A
- WO 2013011955 A
- WO 2013081088 A
- JP 2013256490 A
- JP 2013 A
- JP 116975 A
- WO 2013133359 A
- WO 2013161437 A
- JP 2014009352 A
- JP 2014009224 A
- JP 2017119663 A
- JP 2017119664 A
- JP 2017222623 A
- JP 2017226838 A
- JP 2018100411 A
- WO 2018047853 A
- JP 2013253121 A
- WO 2014034535 A
- WO 2014115743 A
- WO 2014122895 A
- WO 2014126200 A
- WO 2014136758 A
- WO 2014133121 A
- WO 2014136860 A
- WO 2014196585 A
- WO 2014189122 A
- WO 2014168101 A
- WO 2015008580 A
- WO 2014203840 A
- WO 2015002213 A
- WO 2015016200 A
- WO 2015019725 A
- WO 2015072470 A
- WO 2015108049 A
- WO 2015080182 A
- WO 2015072537 A
- WO 2015080183 A
- JP 2015129240 A
- WO 2015129714 A
- WO 2015129715 A
- WO 2015133501 A
- WO 2015136880 A
- WO 2015137244 A
- WO 2015137202 A
- WO 2015137136 A
- WO 2015146541 A
- WO 2015159541 A


### Non-patent literature cited in the description


- **HANSCH.** *Chem. Rev.,* 1991, vol. 91, 165-195